# EUROPEAN PATENT SPECIFICATION

(11) **EP 3 939 979 B1**
(45) Date of publication and mention of the grant of the patent: **02.12.2026**
(21) Application number: 20769246.8
(22) Date of filing: 11.03.2020
(51) Int. Cl.: C07D 471/14, A61K 31/437, A61P 37/00, A61P 17/00, A61P 25/28, A61P 3/10, A61P 35/00

(54) **JAK KINASE INHIBITOR, PREPARATION METHOD FOR SAME, AND APPLICATIONS THEREOF IN FIELD OF MEDICINE**
JAK-KINASE-INHIBITOR, VERFAHREN ZU SEINER HERSTELLUNG UND ANWENDUNGEN IN DER MEDIZIN
INHIBITEUR DE KINASE JAK, SON PROCÉDÉ DE PRÉPARATION ET SES APPLICATIONS DANS LE DOMAINE DE LA MÉDECINE

(30) Priority: 14.03.2019 CN 201910193017
(43) Date of publication of application: 19.01.2022
(73) Proprietor: Shanghai Synergy Pharmaceutical Sciences Co., Ltd, Shanghai 201203 (CN); Zhejiang Huahai Pharmaceutical Co., Ltd., Taizhou, Zhejiang 317024 (CN)
(72) Inventor: XU, Xin, Shanghai 201203 (CN); CHEN, Jia, Shanghai 201203 (CN); ZHANG, Zhen, Shanghai 201203 (CN); WANG, Guan, Shanghai 201203 (CN); ZHANG, Linli, Shanghai 201203 (CN); LI, Qiang, Shanghai 201203 (CN); XI, Chenghao, Shanghai 201203 (CN); QU, Minkai, Shanghai 201203 (CN); ZHANG, Xiaojuan, Shanghai 201203 (CN); CHEN, Chunqiao, Shanghai 201203 (CN); YANG, Fan, Shanghai 201203 (CN); XIA, Xiaoer, Shanghai 201203 (CN); WANG, Yijin, Shanghai 201203 (CN); LI, Yunfei, Shanghai 201203 (CN); GE, Jian, Shanghai 201203 (CN)
(74) Representative: Novagraaf International SA
(86) International application number: PCT/CN2020/078826
(87) International publication number: WO 2020/182159

(56) References cited:
- EP-A1- 2 924 026
- CN-A- 101 578 285
- CN-A- 102 127 078
- CN-A- 102 127 078
- CN-A- 103 748 095
- CN-A- 106 061 973
- CN-A- 106 061 973

## Description

The present application claims the priority of the Chinese Patent Application No. 201910193017.6, with the title of "JAK KINASE INHIBITOR, PREPARATION METHOD FOR SAME, AND APPLICATIONS THEREOF IN FIELD OF MEDICINE", filed on March 14, 2019 before the China National Intellectual Property Administration.

### FIELD OF THE INVENTION

The present application belongs to the field of medicinal chemistry, and relates to a JAK kinase inhibitor, a preparation method for same, and applications thereof in preventing and treating Janus kinase (JAK)-related diseases comprising inflammatory disease, autoimmune disease and the like.

### BACKGROUND OF THE INVENTION

Protein kinases is a kind of enzyme catalyzing a protein phosphorylation reaction, which can transfer a γ-phosphoric acid on adenosine triphosphate (ATP) to an amino acid residue of protein, such as serine, threonine, tyrosine, histidine and other residues, thereby modifying a conformation and activity of a protein. The protein phosphorylation is an important step in a variety of signal transduction pathways, and protein phosphorylation is indispensable for most of important life processes in cells. Protein kinase is mainly responsible for controlling the processes of signal transduction in cells, including regulating a variety of important biological processes, such as cell growth, survival and differentiation, organofaction and morphogenesis, neovascularization, repair and remodeling of tissues. Many diseases are related to abnormal responses in cells caused by abnormal regulations of protein kinase.

Janus kinase (JAK) is a non-receptor tyrosine kinase in cells. Four members of human JAK family are JAK1, JAK2, JAK3 and non-receptor tyrosine kinase 2 (TYK2). JAK1, JAK2 and TYK2 are widely distributed, while JAK3 is only distributed in bone marrow and lymphatic system. The molecular weight of JAK family member is about 120-140 kDa, consisting of more than 1,000 amino acid residues (Leonard, W., O'Shea, J.J., JAKS and STATS: Biological implications. Annu. Rev. Immunol. 1998, 16, 293-322). These members have 7 JAK homology (JH) domains, wherein JH1 domain close to C-terminal is kinase domain, containing certain tyrosine required for JAK activation. After the phosphorylation of these tyrosine, the protein conformation of JAK is modified, thus helpful to combine with downstream substrate; JH2 domain is a "false" kinase domain, regulating the activity of JH1; JH4-JH7 consist of a four-in-one domain, regulating a combination of JAK with cytokine receptors (*Kisseleva, T., Bhattacharya, S., Braunstein, J., Schindler, C.W. Signaling through the JAK*/*STAT pathway, recent advances and future challenges. Gene. 2002, 285 (1-2), 1-24).*

Signal transducer and activator of transcription (STAT) is a set of cytoplasmic protein capable of binding to the DNA of regulatory region of target gene and is a downstream substrate of JAK. STAT family includes 7 members, STAT1, STAT2, STAT3, STAT4, STAT5a, STAT5b and STAT6. Interaction between JAK and STAT plays an important role in signal pathway of cytokine receptor (O'Sullivan, L.A., Liongue, C., Lewis, R. S., Stephenson, S.E.M., Ward, A.C. Cytokine receptor signaling through the Jak Stat pathway in disease. Mol. Immunol, 2007, 44 {10): 2497-2506*).* After the cytokine binds to a specific receptor on its target cell, a subunit of the receptor dimerizes or polymerizes, and JAK binding to each subunit is phosphorylated due to being close to each other. A tyrosine residue of the receptor is phosphorylated under catalysis of activated JAK, forming a "docking site" between corresponding STAT and receptor complex. STAT binds to a phosphotyrosine residue of the receptor molecule by SH2 domain, and then achieves phosphorylation of C-terminal tyrosine residue by JAK. Phosphorylated STAT interacts to form a homo-/heterodimer in nucleus, binding to promoter region of corresponding gene, thereby regulating gene transcription and expression. JAK-STAT pathways may interact with other signal transduction pathways and participate in the development, differentiation, maturation, apoptosis and functional expression of various immune and hematopoietic cells, thus significantly influence the regulation of immune response, immune cell differentiation and development, and inflammatory response. The regulation of many abnormal immune responses, for example, autoimmune diseases such as allergy, asthma, allograft rejection, rheumatoid arthritis, amyotrophic lateral sclerosis and multiple sclerosis, hematological malignant tumor such as myelodysplastic syndrome, leukemia and lymphoma is related to JAK/STAT pathways.

JAK is an important drug target. Some researches have been made regarding JAK inhibitor (e.g., Norman, P. Selective JAK inhibitors in development for rheumatoid arthritis. Expert Opin. investig. Drugs, 2014, 23, 1067-1077*),* in which JAK inhibitor can be used in treatment of deseases such as rheumatoid arthritis, polycythemia vera, psoriasis, primary thrombocythemia and myelofibrosis. For example, Ruxolitinib is a selective inhibitor of JAK1 and JAK2, which was approved by FDA in 2011 and can be used in treating myelofibrosis; Baricitinib is also a selective inhibitor of JAK1 and JAK2 (see CN102026999), which has been approved by EMA in European and Ministry of Health, Labour and Welfare in Japan in treating moderate to severe rheumatoid arthritis, but it is rejected by FDA temporarily; JAK1 selective inhibitor Filgotinib (see CN102482273) and Upadactinib (ABT-494, see CN104370909) specific to multiple indications like rheumatoid arthritis are undergoing clinical Phase III trials; Tofacitinib is the only one selective inhibitor of JAK1 and JAK3 approved by FDA for treating rheumatoid arthritis (Kremer,J.M. etc, "The safety and efficacy of a JAK inhibitor in patients with active rheumatoid arthritis: Results of a double-blind, placebo-controlled phase Ila trial of three dosage levels of CP-690, 550 versus placebo. Arthritis & Rheumatology, 2009, 60(7), 1895-1905*).* However, patients may suffer from some adverse reactions after taking Tofacitinib, for example, possible severe infection and increased risks of cancers and cardiac failure (FDA sets serious infection and malignant tumor as boxed warning).

In addition to the above several JAK inhibitors, currently related patent documents including WO2008109943, WO2011112662, WO2013091539, WO2014128591 and WO2016027195 also disclose JAK inhibitors. Further reference is made to EP2251341.

Though a series of JAK inhibitors have been disclosed currently, these JAK inhibitors already on the market or in the research stage can be still improved in efficacy and safety, and it is necessary to develop novel JAK inhibitors with better effect and safety to provide a better curative effect and to reduce adverse reaction of the patients.

### SUMMARY OF THE INVENTION

The invention is as defined in the accompanying claims. The present application relates to a novel small molecule JAK inhibitor and provides a compound as represented by formula (II), or tautomers, mesomers, racemes, enantiomers, diastereoisomers and mixtures thereof, and pharmaceutically acceptable salts, polymorphs, solvates, prodrugs, metabolites, isotope derivatives and pharmaceutical compositions comprising the compound, which can be used for preventing or treating JAK-related indication, 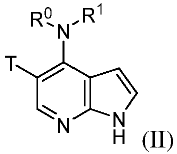
wherein, T is -CN or -CF₃;
R⁰ is hydrogen, halogen, hydroxyl, C₁₋₆ alkyl, C₂₋₆ alkenyl or C₂₋₆ alkynyl; R⁰ is further preferably -H or -C₁₋₃ alkyl;
R¹ is C₃₋₆ cycloalkyl, C₃₋₇ heterocycloalkyl, C₆₋₁₀ aryl, C₁₋₉ heteroaryl, C₆₋₁₂ fused bicyclic group or C₅₋₁₂ fused heterobicyclic group; wherein heteroatoms of the C₃₋₇ heterocycloalkyl, C₁₋₉ heteroaryl or C₅₋₁₂ fused heterobicyclic group can be substituted by R², and hydrogen atoms of C₃₋₆ cycloalkyl, C₃₋₇ heterocycloalkyl, C₆₋₁₀ aryl, C₁₋₉ heteroaryl, C₆₋₁₂ fused bicyclic group or C₅₋₁₂ fused heterobicyclic group can be substituted by R³ or -NHR², and hydrogen atoms of rings having aromaticity can be substituted by R⁷; R¹ is further preferably 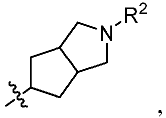 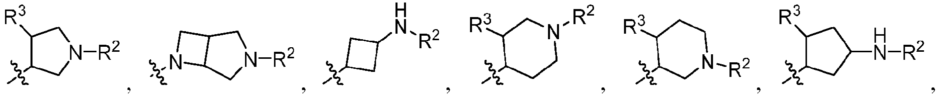 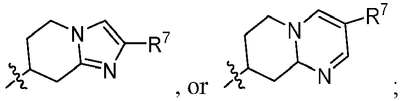
R² is -C(O)R⁴, -C(O)OR⁴, -C(O)NR⁵R⁶, -S(O)R⁴, -S(O)₂R⁴, -S(O)NR⁵R⁶ or -S(O)₂NR⁵R⁶; R² is further preferably -C(O)R⁴, -S(O)₂R⁴ or -C(O)NR⁵R⁶;
R³ is -H, -C₁₋₄ alkyl, C₂₋₆ alkenyl, C₂₋₆ alkynyl, -C₃₋₆ cycloalkyl or C₃₋₇ heterocycloalkyl, wherein the -C₁₋₄ alkyl, C₂₋₆ alkenyl, C₂₋₆ alkynyl, -C₃₋₆ cycloalkyl and C₃₋₇ heterocycloalkyl can be substituted by the following groups: -OH, -C₁₋₃ alkyl, -O-C₁₋₄ alkyl, -NH₂, halogen, phenyl or cyano; R³ is further preferably H or -C₁₋₄ alkyl or -C₃₋₆ cycloalkyl, wherein the -C₁₋₄ alkyl can be subsitituted by the following groups: -OH, -C₁₋₃ alkyl, -O-C₁₋₄ alkyl, -NH₂, halogen, phenyl or cyano; R³ is further preferably H, -C₁₋₄ alkyl or -C₃₋₆ cycloalkyl; R³ is specifically H, -CH₃, -CH₂CH₃, or 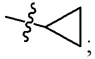
R⁴ is -C₁₋₅ alkyl, -C₂₋₄ alkenyl, -C₂₋₅ alkynyl, -C₃₋₆ cycloalkyl, 4- to 6-membered heterocyclyl, 5- to 10-membered heteroaryl, phenyl, -heterocyclyl-O-R^{e}, -heterocyclyl -NH-R^{e}-heterocyclyl-R^{4a}-O-R^{e} or -heterocyclyl -R^{4a}-NH-R^{e}, wherein the -C₁₋₅ alkyl can be substituted by the following groups: -OH, -C₁₋₃ alkyl, -C₅₋₆ cycloalkyl, 4- to 6-membered heterocyclyl, 5- to 10-membered heteroaryl, -O-C₁₋₄ alkyl, -NH₂, halogen, CF₃, phenyl or cyano; R⁴ is further preferably -C₁₋₅ alkyl, -C₂₋₄ alkenyl, -C₂₋₅ alkynyl, -C₃₋₆ cycloalkyl, 4- to 6-membered heterocyclyl, 5- to 10-membered heteroaryl, phenyl, -heterocyclyl-O-R^{e}, -heterocyclyl -NH-R^{e}-heterocyclyl-R^{4a}-O-R^{e}, -heterocyclyl-R^{4a}-NH-R^{e}, wherein the -C₁₋₅ alkyl can be substituted by the following groups: -OH, -C₁₋₃ alkyl, -O-C₁₋₄ alkyl, -NH₂, halogen, CF₃, phenyl or cyano; R⁴ is specifically methyl, ethyl, propyl, -(CH₂)ₙ-CH₂CN, -(CH₂)ₙ-CH₂CF₃, -CH(OH)-(CH₂)ₙ CH₃, 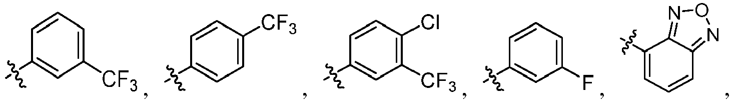 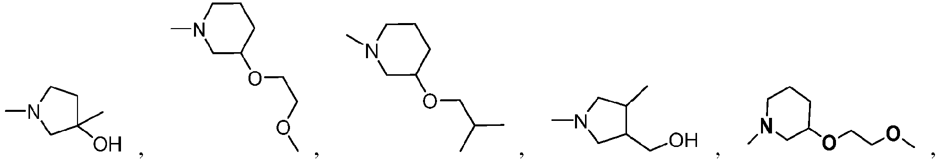 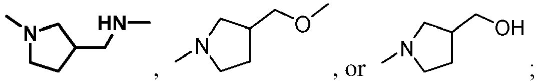
R^{4a} is -C₁₋₃ alkylene; wherein the cycloalkyl, heterocyclyl, heteroaryl or phenyl of R⁴ can be substituted by R^{e};
R⁵ is hydrogen, halogen, hydroxy or C₁₋₆ alkyl; R⁵ is further preferably H or -C₁₋₃ alkyl; R⁵ is specifically H;
R⁶ is -C₁₋₄ alkyl, -C₂₋₄ alkenyl, -C₂₋₄ alkynyl, -C₃₋₆ cycloalkyl, 4- to 6-membered heterocyclyl, phenyl, -CHF₂ or -CH₂CF₂CF₃, wherein the -C₁₋₄ alkyl can be substituted by the following groups: -OH, -C₁₋₃ alkyl, -C₃₋₆ cycloalkyl, 4- to 6-membered heterocyclyl, 5- to 10-membered heteroaryl, -O-C₁₋₄ alkyl, -NH₂, halogen, CF₃, phenyl or cyano group, wherein phenyl can be substituted by R^{e} or R^{g} or R^{e} and R^{g}, hydrogen atoms and heteroatoms of 4- to 6-membered heterocyclyl can be substituted by R^{e} and R^{f}, respectively; R⁶ is further preferably -C₁₋₄ alkyl, -C₃₋₆ cycloalkyl, 4- to 6-membered heterocyclyl, phenyl and 4- to 6-membered heterocyclyl, or 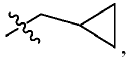 wherein the phenyl can be substituted by R^{e} or R^{g} or R^{e} and R^{g}, hydrogen atoms and heteroatoms of 4- to 6-membered heterocyclyl can be substituted by R^{e} and R^{f}, respectively; -C₁₋₄ alkyl can be substituted by the following groups: -OH, -C₁₋₃ alkyl, -C₃₋₆ cycloalkyl, 4- to 6-membered heterocyclyl, 5- to 10-membered heteroaryl, -O-C₁₋₄ alkyl, -NH₂, halogen, CF₃, phenyl or cyano; R⁶ is further preferably -(CH₂)ₙ-CH₂CF₃, -(CH₂)ₙ-CF₂CF₃, -(CH₂)ₙ-CH₂CN, 4- to 6-membered heterocyclyl, phenyl, or 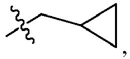 wherein the phenyl can be substituted by R^{e} or R^{g} or R^{e} and R^{g}, hydrogen atoms and heteroatoms of 4- to 6-membered heterocyclyl can be substituted by R^{e} and R^{f}, respectively; R⁶ is further preferably -(CH₂)ₙ-CH₂CF₃, -(CH2)ₙ-CF₂CF₃, -(CH₂)ₙ-CH₂CN, 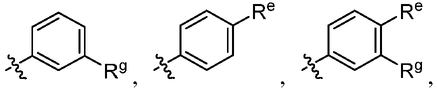 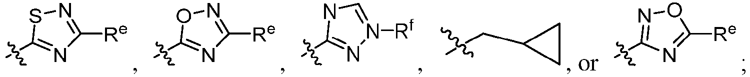
R^{e} and R^{g} are each independently halogen, C₁₋₄ alkyl, alkoxy, 3- to 6-membered cycloalkyl, -NR^{c}R^{d}, -(CH₂)ₙ-NR^{c}R^{d}, -(CH₂)ₙ-OH, -(CH₂)ₙ-CF₃, -(CH₂)ₙ-CN or C(O)OCH₃, wherein the -C₁₋₄ alkyl, alkoxy and cycloalkyl can be monosubstituted or disubstituted by the following substituents: halogen, -OH, -NH₂, -C₁₋₃ alkyl, -O-C₁₋₄ alkyl, CF₃, phenyl or cyano; R^{e} and R^{g} can specifically be -F, -Cl, methyl, ethyl, propyl, -CH(CH₃)-(CH₂)ₙCH₃, -CH₂CH(CH₃)₂, -CH(CH₃)CH(CH₃)₂, -CH₂C(CH₃)₃, -C(CH₃)₂-(CH₂)ₙCH₃, -O-(CH₂)ₙCH₃, -O-(CH₂)ₙCH₂-O-CH₃, -(CH₂)ₙOCH₃, -O-(CH₂)ₙ-CH₂-C(CH₃)₂-OH, -O-(CH₂)ₙ-CH₂-C(CH₃)₂-NH₂, 3- to 6-membered cycloalkyl, -(CH₂)ₙNR^{c}R^{d}, -(CH₂)ₙ-OH, -(CH₂)ₙ-CF₃, -(CH₂)ₙ-CHF₂, -(CH₂)ₙ-CN or -C(O)-O-CH₃;
R^{f} is -C₁₋₄ alkyl or 3- to 6-membered cycloalkyl, wherein the -C₁₋₄ alkyl and cycloalkyl can be substituted by the following substituents: halogen, -OH, -NH₂, -C₁₋₃ alkyl, -O-C₁₋₄ alkyl, CF₃, phenyl or cyano; R^{f} is further preferably -C₁₋₄ alkyl, wherein the -C₁₋₄ alkyl can be substituted by the following substituents: halogen, -OH; R^{f} can specifically be methyl, ethyl or propyl;
R^{c} and R^{d} are each independently hydrogen, -C₁₋₃ alkyl, -C₁₋₄ alkylene-OH, -C₁₋₄ alkylene-CF₃, -C₂₋₄ alkylene-OCH₃, 3- to 6-membered cycloalkyl, or 4- to 6-membered heterocyclyl; R^{c} and R^{d} are further preferably hydrogen, -C₁₋₃ alkyl, -C₁₋₄ alkylene-OH, -C₂₋₄ alkylene-OCF₃; R^{c} and R^{d} can specifically be -H, methyl, ethyl or propyl;
R⁷ is halogen, -C₁₋₄ alkyl, alkoxy, 3- to 6-membered cycloalkyl, -NR^{c}R^{d}, -(CH₂)ₙ-NR^{c}R^{d}, -(CH₂)ₙ-OH, -(CH₂)ₙ-CF₃, -(CH₂)ₙ-CN or C(O)OCH₃, wherein the -C₁₋₄ alkyl, alkoxy and cycloalkyl can be monosubstituted or disubstituted by the following substituents: halogen, -OH, -NH₂, -C₁₋₃ alkyl, -O-C₁₋₄ alkyl, CF₃, phenyl or cyano; R⁷ is further preferably halogen, C₁₋₄ alkyl, alkoxy, -(CH₂)ₙ-OH, -(CH₂)ₙ-CF₃, or -(CH₂)ₙ-CN; R⁷ is further preferably -(CH₂)ₙ-OH, -(CH₂)ₙ-CF₃, or -(CH₂)ₙ-CN; and
n is 0, 1, 2, 3 or 4.

The present application provides a method for preparing a compound represented by formula (II). The compound represented by formula (II) is prepared by the following schemes: 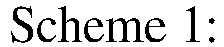 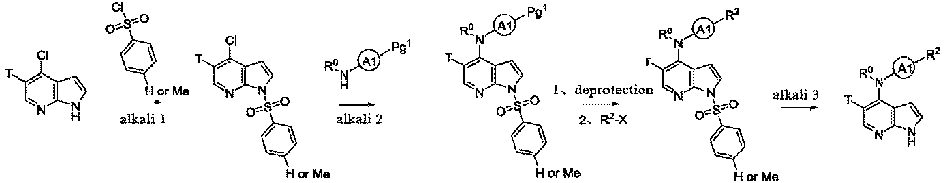 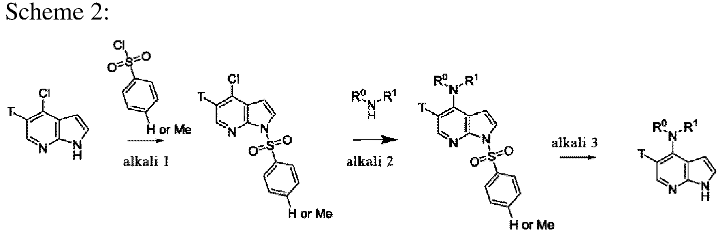 wherein, Pg¹ is an aliphatic amine protecting group, specifically tert-butoxycarbonyl or benzyloxycarbonyl, and A1 is 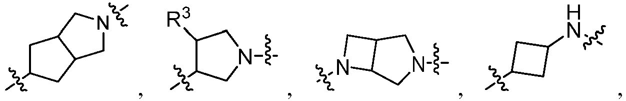 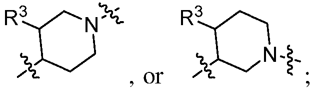 R²-X is an active form of R², and X is preferably selected from the group consisting of chloride, bromine, phenoxyl and p-nitro phenoxy, or R²-X is isocyanate form of R²; alkali 1, alkali 2 and alkali 3 are each independently selected from the group consisting of triethylamine, diisopropyl ethylamine, potassium carbonate, cesium carbonate, sodium carbonate, sodium hydroxide and potassium hydroxide.

According to the compound represented by formula (II) of the present invention and medicinal salt thereof, the compound is specifically:
(cis)-4-({4-ethyl-1-[(2,2,2-trifluoroethyl)carbamoyl)]pyrrolidin-3-yl}amino)-1*H*-pyrrolo[2, 3-b]pyridin-5-carbonitrile;
(cis)-4-({4-ethyl-1-[(2,2,2-trifluoroethyl)carbamoyl)]pyrrolidin-3-yl}-methyl-amino)-1*H*-py rrolo[2,3-b]pyridin-5-carbonitrile;
(cis)-4-{ [4-ethyl-1-(3,3,3-trifluoropropionyl)pyrrolidin-3-yl]-methyl-amino}-1*H*-pyrrolo[2, 3-b]pyridin-5-carbonitrile;
(cis)-4-({4-methyl-1-[(2,2,2-trifluoroethyl)carbamoyl)]pyrrolidin-3-yl}-methyl-amino)-1*H-*pyrrolo[2,3-b]pyridin-5-carbonitrile;
(cis)-4-{[4-methyl-1-(2-cyanoacetyl)pyrrolidin-3-yl]-methyl-amino}-1*H*-pyrrolo[2,3-b]pyri din-5-carbonitrile;
(3aR,5R,6aS)-4-{[2-((2S)-2-hydroxypropionyl)-hexahydrocyclopenta[c]pyrrole-5-yl]-meth yl-amino}-1*H*-pyrrolo[2,3-b]pyridin-5-carbonitrile;
(3aR,5s,6aS)-4-({2-[(3-methoxy-1,2,4-thiadiazole-5-yl)-carbamoyl]-hexahydrocyclopenta[c ]pyrrole-5-yl}-methyl-amino)-1*H*-pyrrolo[2,3-b]pyridin-5-carbonitrile;
(3aR,5s,6aS)-4-({2-[(3-ethyl-1,2,4-thiadiazole-5-yl)-carbamoyl]-hexahydrocyclopenta[c]py rrole-5-yl}-methyl-amino)-1*H*-pyrrolo[2,3-b]pyridin-5-carbonitrile;
4-{[3-(3-trifluoromethyl-benzenesulfonamido)-cyclobutyl-1-yl]-methyl-amino }-1*H*-pyrrolo [2,3-b]pyridin-5-carbonitrile;
4-{[3-(3,3,3-trifluoro-propanesulfonamido)-cyclobutyl-1-yl]-methyl-amino}-1*H*-pyrrolo[2, 3-b]pyridin-5-carbonitrile;
4-{[(3R,4R)-1-(2-cyanoacetyl)-4-methylpiperidin-3-yl]-methyl-amino}-1*H*-pyrrolo[2,3-b]p yridin-5-carbonitrile;
(cis)-4-{[3-(3,3,3-trifluoro-propanesulfonamido)-cyclobutyl-1-yl]-amino}-1*H*-pyrrolo[2,3-b ]pyridin-5-carbonitrile;
(cis)-4-{[3-(3-trifluoromethyl-benzenesulfonamido)-cyclobutyl-1-yl]-amino}-1*H*-pyrrolo[2, 3-b]pyridin-5-carbonitrile;
(cis)-4-{[3-(propanesulfonamido)-cyclobutyl-1-yl]-methyl-amino}-1*H*-pyrrolo[2,3-b]pyridi n-5-carbonitrile;
(cis)-4-{[3-(3,3,3-trifluoro-propanesulfonamido)-cyclobutyl-1-yl]-methyl-amino}-1*H*-pyrro lo[2,3-b]pyridin-5-carbonitrile;
(cis)-4-{[3-(4-trifluoromethyl-benzenesulfonamido)-cyclobutyl-1-yl]-methyl-amino}-1*H*-py rrolo[2,3-b]pyridin-5-carbonitrile;
(cis)-4-{[3-(4-trifluoromethyl-benzenesulfonamido)-cyclobutyl-1-yl]-amino}-1*H*-pyrrolo[2, 3-b]pyridin-5-carbonitrile;
(cis)-4-{[3-(benzo[c][1,2,5]oxadiazole-4-sulfonamido)-cyclobutyl-1-yl]-methyl-amino}-1*H* -pyrrolo[2,3-b]pyridin-5-carbonitrile;
(cis)-4-{[3-(benzo[c][1,2,5]oxadiazole-4-sulfonamido)-cyclobutyl-1-yl]-amino}-1*H*-pyrrolo [2,3-b]pyridin-5-carbonitrile;
(3aR,5s,6aS)-5-((5-cyano-1H-pyrrolo[2,3-b]pyridin-4-yl)amino)-N-(3-methoxy-1,2,4-thiadi azole-5-yl)hexahydrocyclopentadiene[c]pyrrole-2(1H)-carboxamide;
(3aR,5s,6aS)-4-({2-[(3-tert-butyl-1,2,4-thiadiazole-5-yl)-carbamoyl]-hexahydrocyclopenta[ c]pyrrole-5-yl}-methyl-amino)-1*H*-pyrrolo[2,3-b]pyridin-5-carbonitrile;
(3aR,5s,6aS)-4-({2-[(2,2,2-trifluoroethyl)-carbamoyl]-hexahydrocyclopenta[c]pyrrole-5-yl} -methyl-amino)-1*H*-pyrrolo[2,3-b]pyridin-5-carbonitrile;
(3aR,5S,6aS)-4-{ [2-((2S)-2-hydroxypropionyl)-hexahydrocyclopenta[c]pyrrole-5-yl]-methy 1-amino}-1*H*-pyrrolo[2,3-b]pyridin-5-carbonitrile;
4-(methyl((3aR,5s,6aS)-2-((3-(trifluoromethyl)phenyl)sulfonyl)octahydrocyclopentadiene[c ]pyrrole-5-yl)amino-1H-pyrrolo[2,3-b]pyridin-5-carbonitrile;
(3aR,5s,6aS)-N-(4-chloro-3-(trifluoromethyl)phenyl)-5-((5-cyano-1H-pyrrolo[2,3-b]pyridin -4-yl)(methyl)amino)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(3-cyclopropyl-1,2,4-thiadiazole-5-yl)hexahydrocyclopentadiene[c]pyrrole-2(1H)-carboxamide;
4-(methyl((3aR,5s,6aS)-2-((3,3,3-(trifluoropropyl)sulfonyl)octahydrocyclopentadiene[c]pyr role-5-yl)amino-1H-pyrrolo[2,3-b]pyridin-5-carbonitrile;
4-(((3aR,5s,6aS)-2-((3-fluorophenyl)sulfonyl)octahydrocyclopentadiene[c]pyrrole-5-yl)met hyl-amino)- 1H-pyrrolo[2,3-b]pyridin-5-carbonitrile;
(cis)-1-(3-((5-cyano-1H-pyrrolo[2,3-b]pyridin-4-yl)(methyl)amino)cyclobutyl)-3-(3-methox y-1,2,4-thiadiazole-5-yl)urea;
(3aR,5s,6aS)-4-({2-[(3-isopropyl-1,2,4-thiadiazole-5-yl)carbamoyl]-hexahydrocyclopenta[c ]pyrrole-5-yl}-methyl-amino)-1*H*-pyrrolo[2,3-b]pyridin-5-carbonitrile;
(3aR,5s,6aS)-4-({2-[(1-ethyl-1H-1,2,4-triazole-3-yl)carbamoyl]-hexahydrocyclopenta[c]pyr role-5-yl}-methyl-amino)-1*H*-pyrrolo[2,3-b]pyridin-5-carbonitrile;
(3aR,5s,6aS)-4-({2-[(3-cyclopropyl-1,2,4-oxadiazole-5-yl)-carbamoyl]-hexahydrocyclopent a[c]pyrrole-5-yl}-methyl-amino)-1*H*-pyrrolo[2,3-b]pyridin-5-carbonitrile;
(3aR,5s,6aS)-4-({2-[(3-dimethylamino-1,2,4-thiadiazole-5-yl)-carbamoyl]-hexahydrocyclop enta[c]pyrrole-5-yl}-methyl-amino)-1*H*-pyrrolo[2,3-b]pyridin-5-carbonitrile;
4-((1-((3-(trifluoromethyl)phenyl)sulfonyl)piperidin-4-yl)amino)-2,3-dihydro-1H-pyrrolo[2, 3-b]pyridin-5-carbonitrile;
4-((1-((3-(trifluoromethyl)phenyl)sulfonyl)piperidin-4-yl)-methyl-amino)-2,3-dihydro-1H-p yrrolo[2,3-b]pyridin-5-carbonitrile;
3-((5-cyano-1H-pyrrolo[2,3-b]pyridin-4-yl)amino)-N-(2,2,2-trifluoroethyl)pyrrolidin-1-carb oxamide;
3-((5-cyano-1H-pyrrolo[2,3-b]pyridin-4-yl)-methyl-amino)-N-(2,2,2-trifluoroethyl)pyrrolidi n-1-carboxamide;
(3aR,5s,6aS)-5-((5-cyano-1H-pyrrolo[2,3-b]pyridin-4-yl)(methyl)amino)-N-(3-hydroxy-1,2, 4-thiadiazole-5-yl)hexahydrocyclopentadiene[c]pyrrole-2(1H)-carboxamide;
6-(5-cyano-1H-pyrrolo[2,3-b]pyridin-4-yl)-N-(2,2,2-trifluoroethyl)-3,6-diazabicyclo[3.2.0] heptane-3-carboxamide;
(3S,4S)-4-({4-methyl-1-[(2,2,2-trifluoroethyl)carbamoyl)]pyrrolidin-3-yl}amino)-1H-pyrrol o[2,3-b]pyridin-5-carbonitrile;
(3aR,5s,6aS)-5-((5-cyano-1H-pyrrolo[2,3-b]pyridin-4-yl)(methyl)amino)-N-(3-(methyl-ami no)-1,2,4-thiadiazole-5-yl)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(3-hydroxymet hyl-1,2,4-thiadiazole-5-yl)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(3-methoxy-1,2 ,4-oxadiazole-5-yl)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(3-(2-hydroxy-2-methyl-propoxy)-1,2,4-thiadiazole-5-yl)hexahydrocyclopentadiene[c]pyrrole-2(1H)-carboxami de;
(3aR,5s,6aS)-5-((5-cyano-1H-pyrrolo[2,3-b]pyridin-4-yl)(methyl)amino)-N-(3-(2-amino-2-methyl-propoxy)-1,2,4-thiadiazole-5-yl)hexahydrocyclopentadiene[c]pyrrole-2(1H)-carboxamid e;
(cis)-4-{[3-(propanesulfonamido)-cyclobutyl-1-yl]-amino}-1*H*-pyrrolo[2,3-b]pyridin-5-car bonitrile;
(3aR,5s,6aS)-5-((5-cyano-1H-pyrrolo[2,3-b]pyridin-4-yl)(methyl)amino)-N-(3-ethoxy-1,2,4 -oxadiazole-5-yl)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(5-ethoxy-1,2,4 -oxadiazole-3-yl)hexahydrocyclopentadiene[c]pyrrole-2(1H)-carboxamide;
(3aR,5s,6aS)-5-((5-cyano-1H-pyrrolo[2,3-b]pyridin-4-yl)amino)-N-(3-cyclopropyl-1,2,4-thi adiazole-5-yl)hexahydrocyclopentadiene[c]pyrrole-2(1H)-carboxamide;
(3aR,5s,6aS)-5-((5-trifluoromethyl-1H-pyrrolo[2,3-b]pyridin-4-yl)amino)-N-(3-methoxy-1, 2,4-thiadiazole-5-yl)hexahydrocyclopentadiene[c]pyrrole-2(1H)-carboxamide;
(3aR,5s,6aS)-5-((5-cyano-1H-pyrrolo[2,3-b]pyridin-4-yl)(methyl)amino)-N-(3-(2-methoxye thoxy)-1,2,4-thiadiazole-5-yl)hexahydrocyclopenta[c]pyrrole-2(1H)-carboxamide;
(3aR,5s,6aS)-5-((5-cyano-1H-pyrrolo[2,3-b]pyridin-4-yl)amino)-N-(3-(2-methoxyethoxy)-1 ,2,4-thiadiazole-5-yl)hexahydrocyclopenta[c]pyrrole-2(1H)-carboxamide;
methyl 5-((3aR,5s,6aS)-5-((5-cyano-1H-pyrrole[2,3-b]pyridin-4-yl)(methyl)amino) octahydrocyclopenta[c]pyrrole-2-formamido)-1,2,4-thiadiazole-3-carboxylate;
(3aR,5s,6aS)-5-((5-cyano-1H-pyrrole[2,3-b]pyridin-4-yl)(methyl)amino)-N-(3-((dimethyla mino)methyl)-1,2,4-thiadiazole-5-yl)hexahydrocyclopenta[c]pyrrole-2(1H)-carboxamide;
(3aR,5s,6aS)-5-((5-cyano- 1H-pyrrole[2,3-b]pyridin-4-yl)amino)-N-(3-((dimethylamino)met hyl)-1,2,4-thiadiazole-5-yl)hexahydrocyclopenta[c]pyrrole-2(1H)-carboxamide;
(3aR,5s,6aS)-5-((5-cyano-1H-pyrrole[2,3-b]pyridin-4-yl)(methyl)amino)-N-(3-methoxymet hyl)-1,2,4-thiadiazole-5-yl)hexahydrocyclopenta[c]pyrrole-2(1H)-carboxamide;
(3aR,5s,6aS)-5-((5-cyano-1H-pyrrole[2,3-b]pyridin-4-yl)amino)-N-(3-methoxymethyl)-1,2, 4-thiadiazole-5-yl)hexahydrocyclopenta[c]pyridin-2(1H)-carboxamide;
N-(3-((5-cyano-1H-pyrrolo[2,3-b]pyridin-4-yl)amino)-4-ethylcyclopentyl)benzo[c][1,2,5]o xadiazole-4-sulfamide;
N-(3-((5-cyano-1H-pyrrolo[2,3-b]pyridin-4-yl)amino)-4-ethylcyclopentyl)-4,4,4-trifluorobu tanamide;
N-(3-((5-cyano-1H-pyrrolo[2,3-b]pyridin-4-yl)amino)-4-ethylcyclopentyl)-3-(trifluorometh yl)benzsulfamide;
1-((3S,4R)-3-((5-cyano-1H-pyrrolo[2,3-b]pyridin-4-yl)amino)-4-ethylcyclopentyl)-3-(3-met hoxy-1,2,4-thiadiazole-5-yl)urea;
4-((4-((((3-hydroxy-3-methylpyrrolidin-1-yl)sulfonyl)methyl)cyclohexyl)(methyl)amino)-1 H-pyrrolo[2,3-b]pyridin-5-carbonitrile;
trans-4-(methyl((1r,4r)-4-(((4-methylpiperazin-1-yl)sulfonyl)methyl)cyclohexyl)amino)-1H -pyrrolo[2,3-b]pyridin-5-carbonitrile;
4-((trans-4-(((3-isobutoxypiperidin-1-yl)sulfonyl)methyl)cyclohexyl)(methyl)amino)-1H-py rrolo[2,3-b]pyridin-5-acetonitrile;
4-((trans-4-(((trans-3-(hydroxymethyl)-4-methylpyrrolidin-1-yl)sulfonyl)methyl)cyclohexyl )(methyl)amino)-1H-pyrrolo[2,3-b]pyridin-5-carbonitrile;
4-(methyl-(trans-4-(((3-((methylamino)methyl)pyrrolidin-1-yl)sulfonyl)methyl)cyclohexyl) amino)-1H-pyrrolo[2,3-b]pyridin-5-carbonitrile;
4-((trans-4-(((3-(methoxymethyl)pyrrolidin-1-yl)sulfonyl)methyl)cyclohexyl)(methyl)amin o)-1H-pyrrolo[2,3-b]pyridin-5-carbonitrile;
4-((cis-(3-(((3-(2-methoxyethoxy)piperidin-1-yl)sulfonyl)methyl)cyclopentyl)(methyl)amin o)-1H-pyrrolo[2,3-b]pyridin-5-carbonitrile;
4-((trans-3-(((3-((hydroxymethyl)pyrrolidin-1-yl)sulfonyl)methyl)cyclopentyl)(methyl)ami no)-1H-pyrrolo[2,3-b]pyridin-5-carbonitrile;
(R)-7-((5-cyano-1H-pyrrolo[2,3-b]pyridin-4-yl)amino)-N-(2,2,2-trifluoroethyl)-5-azaspiro[ 2.4]heptane-5-carboxamide;
(cis)-3-isopropyl-4-(imidazo[4,5-d]pyrrolo[2,3-b]pyridin-1(6H)-yl)-N-(2,2,2-trifluoroethyl) pyrrolidin-1-carboxamide;
(cis)-3-((5-cyano-1H-pyrrolo[2,3-b]pyridin-4-yl)amino)-4-cyclopropyl-N-isobutylpyrrolidin -1-carboxamide;
(cis)-3-((5-cyano-1H-pyrrolo[2,3-b]pyridin-4-yl)amino)-4-ethyl-N-isobutylpyrrolidin-1-car boxamide;
4-(((cis)-4-ethyl-1-((3,3,3-trifluoropropyl)sulfonyl)pyrrolidin-3-yl)amino)-1H-pyrrolo[2,3-b ]pyridin-5-carbonitrile;
4-(((cis)-4-ethyl-1-(4,4,4-trifluorobutyryl)pyrrolidin-3-yl)amino)-1H-pyrrolo[2,3-b]pyridin-5-carbonitrile;
4-((4-ethyl-1-(2-cyanoacetyl)pyrrolidin-3-yl)amino)-1H-pyrrolo[2,3-b]pyridin-5-carbonitril e;
(cis)-3-((5-cyano-1H-pyrrolo[2,3-b]pyridin-4-yl)amino)-4-ethyl-N-isobutylpyrrolidin-1-car boxamide;
4-(((cis)-4-ethyl-1-(3-methoxy-1,2,4-thiadiazole-5-aminocarbonyl)pyrrolidin-3-yl)amino)-1 H-pyrrolo[2,3-b]pyridin-5-carbonitrile;
4-(((cis)-1-(3-cyanopropionyl)-4-ethylpyrrolidin-3-yl)amino)-1H-pyrrolo[2,3-b]pyridin-5-c arbonitrile;
3-((5-cyano-1H-pyrrolo[2,3-b]pyridin-4-yl)amino)-4-ethyl-N-(cyclopropylmethyl)pyrrolidi n-1-carboxamide;
3-((5-cyano-1-H-pyrrolo[2,3-b]pyridin-4-yl)amino)-4-ethyl-N-(2,2,3,3,3-pentafluoropropyl) -pyrrolidin-1-carboxamide;
(cis)-3-((5-cyano-1H-pyrrolo[2,3-b]pyridin-4-yl)amino)-N-cyclopropyl-4-ethylpyrrolidin-1-carboxamide;
(cis)-3-((5-cyano-1H-pyrrolo[2,3-b]pyridin-4-yl)amino)-4-ethyl-N-(3-methylbutane-2-yl)py rrolidin-1-carboxamide;
(cis)-3-((5-cyano-1H-pyrrolo[2,3-b]pyridin-4-yl)amino)-4-ethyl-N-neopentyl-pyrrolidin-1-c arboxamide;
3-((5-cyano-1H-pyrrolo[2,3-b]pyridin-4-yl)amino)-N-(2,2-difluoroethyl)-4-ethylpyrrolidin-1-carboxamide;
4-(((cis)-1-(2-cyanoethyl)-4-ethylpyrrolidin-3-yl)amino)-1H-pyrrolo[2,3-b]pyridin-5-carbon itrile;
(cis)-3-((5-cyano-1H-pyrrolo[2,3-b]pyridin-4-yl)amino)-N-(2,2-difluoropropyl)-4-ethylpyrr olidin-1-carboxamide;
7-((5-cyano-1H-pyrrolo[2,3-b]pyridin-4-yl)amino)-5,6,7,8-tetrahydroimidazo[1,2-a]pyridin-2-carbonitrile;
(cis)-3-cyclopropyl-N-(2,2,2-trifluoroethyl)-4-((5-(trifluoromethyl)-1H-pyrrolo[2,3-b]pyridi n-4-yl)amino)pyrrolidin-1-carboxamide;
(cis)-3-ethyl-N-(2,2,2-trifluoroethyl)-4-((5-(trifluoromethyl)-1H-pyrrolo[2,3-b]pyridin-4-yl) amino)pyrrolidin-1-carboxamide;
(cis)-3-ethyl-N-(cyanomethyl)-4-((5-(trifluoromethyl)-1H-pyrrolo[2,3-b]pyridin-4-yl)amino ) pyrrolidin-1-carboxamide.

The pharmaceutically acceptable salts of the compounds of the invention include acid addition salts and alkali addition salts thereof.

Suitable acid addition salts are formed by acids that form nontoxic salts. Examples include but not limit to hydrochloride, sulfate/bisulfate, nitrate, phosphate/hydrophosphate/dihydric phosphate, hydrobromide, hydriodate, acetate, lactate, mesylate, citrate, malate, maleate, fumarate, tartrate, salicylate, stearate and analogues thereof.

Suitable alkali addition salts are formed by alkalis that form nontoxic salts, for example alkali metal salts and alkaline earth metal salts, or ammonium salts. Examples include but not limit to sodium salts, potassium salts, calcium salts and magnesium salts, or salts with ammonia or organic amine such as ethylamine, ethanolamine, triethanolamine or amino acid.

A review of the suitable salts is provided in Stahl, P. H. and Wermuth, C. G., "Handbook of Pharmaceutical Salts: Properties, Selection, and Use, 2nd Revised Edition" (Wiley-VCH, 2011).

If the compound of formula (II) contains both acidic group and basic group within its molecule, the invention also includes inner salt or betaine (zwitterion) in addition to the mentioned salt form.

Methods for preparing pharmaceutically acceptable salts of the compounds of the present invention are known to those skilled in the art, for example contacting these compounds with organic or inorganic acids or alkalis in an solvent or dispersant, or anion exchange or cation exchange with other salts.

The compound of formula (II) can be present in a crystal form or an amorphous form. Many conventional analysis techniques including but not limited to single crystal X-Ray Powder diffraction (XRPD) pattern, infrared spectroscopy (IR), Raman spectrum, differential scanning calorimetry (DSC), thermogravimetric analysis (TGA) and solid nuclear magnetic resonance (ssNMR) characterization can be used to distinguish the polymorphism of the compound.

The compounds of the present invention also include a tautomeric form. The tautomeric form is resulted from a conversion of single bond and adjacent double bond with an accompanying migration of proton. The tautomeric form includes prototropic tautomerism, which is an isomeric protonation state having a same empirical formula and total charge. Examples of prototropic tautomerism include keto-enol paris, amide-imidic acid pairs, lactam-lactim pairs, enamine-imine pairs, and cyclic forms in which protons can occupy two or more positions in the heterocyclic system, such as 1H- and 3H-imidazole, 1H-, 2H- and 4H-1,2,4-triazole, 1H- and 2H-isoindole, and 1H- and 2H-pyrazol. Tautomeric forms may be in equilibrium or spatially fixed to one form by appropriate substitution.

The present invention also relates to the compound of formula (II) or its tautomer, mesomer, raceme, enantiomer, diastereoisomer and mixtures thereof, pharmaceutically acceptable salt thereof, or a pharmaceutical composition comprising the same, for use in preparing a medicament for inhibiting JAK kinase; the JAK kinase is preferably JAK1, JAK2 or JAK3.

The present invention also relates to the compound of formula (II) or its tautomers, mesomers, racemes, enantiomers, diastereoisomers and mixtures thereof, pharmaceutically acceptable salts thereof, or a pharmaceutical compositions comprising the same, for use in preparing a medicament for inhibiting JAK kinase, wherein the medicament optionally contains additional one or more agents, anticarcinogen or anti-inflammatory agents that regulate the immune systems of mammals.

The present invention also relates to the compound of formula (II) or its tautomers, mesomers, racemes, enantiomers, diastereoisomers and mixtures thereof, pharmaceutically acceptable salts thereof, or a pharmaceutical compositions comprising same, for use in preparing a medicament for inhibiting JAK kinase, wherein the medicament is used in treating or preventing the following disorders or diseases: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), skin cancer (e.g., cutaneous T-cell lymphoma, cutaneous B-cell lymphoma).

The present invention also relates to the compound of formula (II) or its tautomers, mesomers, racemes, enantiomers, diastereoisomers and mixtures thereof, pharmaceutically acceptable salts thereof, or a pharmaceutical compositions comprising same, for use in preparing a medicament for inhibiting JAK kinase, wherein the medicament optionally includes additional one or more agents, anticarcinogen or anti-inflammatory agents that regulate immune systems of mammals, for treating or preventing the following disorders or diseases: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), and skin cancer (e.g., cutaneous T-cell lymphoma, cutaneous B-cell lymphoma). The mammals are humans.

The present invention further relates to the compound of formula (II) or its tautomers, mesomers, racemes, enantiomers, diastereoisomers and mixtures thereof, pharmaceutically acceptable salts thereof, or a pharmaceutical compositions comprising the same, as a medicament for inhibiting JAK kinase. The JAK kinase is preferably JAK1, JAK2 or JAK3.

The present invention further relates to the compound of formula (II) or its tautomer, mesomer, raceme, enantiomer, diastereoisomer and mixtures thereof, pharmaceutically acceptable salt thereof, or a pharmaceutical composition comprising same, which is further used in combination with additional one or more agents, anticarcinogen or anti-inflammatory agents that regulate immune systems of mammals.

The present invention further relates to the compound of formula (II) or its tautomers, mesomers, racemes, enantiomers, diastereoisomers and mixtures thereof, pharmaceutically acceptable salts thereof, or a pharmaceutical compositions comprising the same, which is used in treating or preventing the following disorders or diseases: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), skin cancer (e.g., cutaneous T-cell lymphoma, cutaneous B-cell lymphoma).

The present invention further relates to the compound of formula (II) or its tautomers, mesomers, racemes, enantiomers, diastereoisomers and mixtures thereof, pharmaceutically acceptable salts thereof, or a pharmaceutical compositions comprising the same, which is further used in combination with additional one or more agents, anticarcinogen or anti-inflammatory agents that regulate immune systems of mammals in treating or preventing following disorders or diseases: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), and skin cancer (e.g., cutaneous T-cell lymphoma, cutaneous B-cell lymphoma)

The invention is set out in the appended set of claims. In addition, any reference to methods of treatment in the subsequent paragraphs of this description is to be interpreted as references to compounds, pharmaceutical compositions and medicaments of the present invention for use in a method of treatment of the human or animal body by therapy.

In other words, the present invention relates to a method for inhibiting JAK kinase, comprising administering an effective amount of the compound of formula (II) or its tautomers, mesomers, racemes, enantiomers, diastereoisomers and mixtures thereof, pharmaceutically acceptable salts thereof, or a pharmaceutical compositions comprising the same to patients in need. Further, the compound of formula (II) or its tautomer, mesomer, raceme, enantiomer, diastereoisomer and mixtures thereof, pharmaceutically acceptable salt thereof, or a pharmaceutical composition comprising the same is used in combination with additional one or more agents, anticarcinogen or anti-inflammatory agents that regulate immune systems of mammals.

The present disclosure further relates to a method for treating or preventing immune system disorders or diseases, comprising administering an effective amount of the compound of formula (II) or its tautomers, mesomers, racemes, enantiomers, diastereoisomers and mixtures thereof, pharmaceutically acceptable salts thereof, or a pharmaceutical compositions comprising the same, to patients in need, wherein the immune system disorders or diseases are selected from the group consisting of: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), skin cancer (e.g., cutaneous T-cell lymphoma, cutaneous B-cell lymphoma).

The present disclosure further relates to a method for treating or preventing immune system disorders or diseases, comprising administering an effective amount of the compound of formula (II) or its tautomers, mesomers, racemes, enantiomers, diastereoisomers and mixtures thereof, pharmaceutically acceptable salts thereof, or a pharmaceutical compositions comprising the same, and additional one or more agents, anticarcinogen or anti-inflammatory agents that regulate immune systems of mammals, to patients in need, wherein the immune system disorders or diseases are selected from the group consisting of: immune system diseases, for example, organ-graft rejection (e.g., allograft rejection and graft-versus-host disease); autoimmune diseases, for example, lupus, multiple sclerosis, rheumatoid arthritis, juvenile arthritis, psoriasis, ulcerative colitis, Crohn's disease, autoimmune thyroid diseases; dermatosis, for example, psora, skin rash, atopic dermatitis; allergic diseases, for example, asthma, rhinitis; viral diseases, for example, hepatitis B, hepatitis C, varicella-zoster virus; type 1 diabetes and diabetic complication; alzheimer disease, xerophthalmia, myelofibrosis, thrombocytosis, polycythemia or leukemia; cancers, for example, solid tumor (e.g., prostate cancer, renal carcinoma, liver cancer, pancreatic cancer, gastric cancer, breast cancer, lung cancer, head-neck carcinoma, thyroid cancer, glioblastoma, melanoma), hematologic malignancy (e.g., lymphoma, leukemia), skin cancer (e.g., cutaneous T-cell lymphoma, cutaneous B-cell lymphoma).

The compounds of the present invention can be formulated in a conventional manner using one or more pharmaceutically acceptable carriers. Therefore, the active compounds of the invention can be formulated into dosage forms for oral, buccal, intranasal, parenteral (e.g., intravenous, intramuscular or subcutaneous) or rectal administration, or into dosage forms which are suitable for inhalation or insufflation administration. The compounds of the invention can be also formulated into a sustained release dosage forms.

Depending on the purpose of treatment, the pharmaceutical composition can be formulated into various types of unit dosage forms, such as a tablet, pill, powder, liquid, suspension, emulsion, granule, capsule, suppository and injection (solution and suspension), or dosage forms which are suitable for inhalation or insufflation administration.

Any excipient known and widely used in the art can be used to formulate a pharmaceutical composition to the tablet form. For example, carriers, such as lactoses, white sugar, sodium chlorides, glucoses, urea, starches, calcium carbonates, kaolins, crystalline celluloses, silicic acids and the like; adhesives, such as water, ethanol, propanol, regular syrup, dextrose solutions, starch solutions, gelatin solutions, carboxymethylcelluloses, shellacs, methyl celluloses, potassium phosphates, polyvinylpyrrolidone and the like; disintegrants, such as dry starches, sodium alginates, agar powder, kelp powder, sodium bicarbonates, calcium carbonates, polyoxyethylene sorbitan fatty acid esters, sodium dodecyl sulfates, monoglyceride stearates, starches, lactoses and the like; disintegration inhibiting agents, such as white sugar, glycerol tristearates, coconut oil and hydrogenated oil; absorptive accelerators, such as quaternary ammonium hydroxide, sodium dodecyl sulfates and the like; humectants, such as glycerol, starches and the like; adsorbents, such as starches, lactoses, kaolins, bentonites, colloid silicic acids and the like; and lubricants, such as pure talcs, stearates, boric acid powder, polyethylene glycol and the like. It can also be made into sugar-coated tablets, gelatin-coated tablets, casing tablets, coated tablets, double-layer film tablets and multi-layer tablets according to needs.

Any excipient known and widely used in the art can be used, such as carriers, e.g. lactose, starch, coconut oil, hydrogenated oil, kaolin, talcum powder and the like; adhesives, e.g. gum acacia powder, gum tragacanth powder, gelatin, ethanol and the like; disintegrants, agar, kelp powder and the like to formulate a pharmaceutical composition to the pill form.

Any excipient known and widely used in the art can be used, such as polyethylene glycol, coconut oil, higher alcohol, ester of higher alcohol, gelatin, semisynthetic glyceride and the like to formulate a pharmaceutical composition to the suppository form.

Solution and suspension can be formulated into an injection after disinfection (preferably adding appropriate amount of sodium chloride, glucose or glycerinum) to prepare a pharmaceutical composition at an isotonic pressure with blood. When preparing an injection, any common carriers in the art can also be used. For example, water, ethanol, propanediol, sulphate-ethoxylated isostearyl alcohol, polyethoxylated isostearyl alcohol, fatty acid ester of polyethylene sorbitan monooleate and the like. Furthermore, a common dissolving agent, buffer agent, analgesic and the like can also be added.

For intranasal administration or inhalation administration, the active compounds of the invention are suitably released in a form of solution or suspension from an absorption or extrusion or sorption pump spray, or in a form of spray form a pressurizing vessel or sprayer, wherein the release utilizes a suitable propellant, such as dichlorodifluoromethane, trichlorofluoromethane, dichlorotetrafluoroethane, carbon dioxide or other suitable gases. In the case of a pressurized aerosol, a dosage unit can be determined by a valve providing metered release. The pressurizing vessel and sprayer can contain a solution or suspension of the active compound. A capsule or cartridge (e.g., made of gelatin) used in an inhaler or insufflator can be formulated into a powder mixture containing the compounds of the invention and suitable powdered substrates such as lactose or starch.

Unless stated to the contrary, terms used in the specification and claims have the following meanings.

The term "stereoisomer" represents an isomer formed by at least an asymmetric center. In the compound having one or more (such as 1, 2, 3 or 4) asymmetric centers, raceme, racemic mixture, mesomer, single enantiomer, mixtures of diastereomers and single diastereoisomer can be formed. Specific individual molecules can also be present as geometrical isomer (cis/trans).

The term "solvate" represents a form of the compound generally physically combined with a solvent by a solvolytic reaction. This physical combination includes hydrogen bonding. Conventional solvents include water, ethanol, methanol, acetic acid and the like. The compound of formula (II) can be prepared in the form of crystal and present in the form of solvate (e.g., the form of hydrate). Suitable solvates include pharmaceutically acceptable solvates (e.g., hydrates) and further include stoichiometric solvates and non-stoichiometric solvates. In some instances, for example, when one or more solvent molecules are incorporated in lattices of crystalline solid, a solvate may dissociate. "Solvate" encompasses solutions and dissociable solvates. Representative solvates include hydrates, ethanolate, methanolate and the like.

The term "prodrug" represents derivatives that are convert to the compound of the present invention by reacting with enzymes, gastric acids, and the like under physiological conditions in vivo, such as by oxidation, reduction, hydrolysis, and the like, each of which is catalyzed by an enzyme.

The term "metabolite" represents all molecules derived from the compounds of the invention in cells or organisms, preferably humans.

The term "isotope derivative" represents the compounds containing isotopes in non-natural proportion at one or more atoms that form the compounds. For example, deuterium (²H or D), tritium (³H or T), carbon-13(¹³C), nitrogen-15 (¹⁵N), oxygen-18 (¹⁸O), etc..

The term "pharmaceutical composition" represents one or more compounds of the invention or physiologically/pharmacologically acceptable salts or mixtures of prodrugs and other chemicals such as physiologically/pharmacologically acceptable carriers and excipients. The aim of the pharmaceutical composition is to facilitate the administration to organisms, which is beneficial to the absorption of the active ingredients to further develop biological activity.

The carriers include all pharmaceutical preparations in the pharmaceutical field that are capable to be formulated for injection and non-injection administration routes, e.g., a diluent, humectant, filler, adhesive, moistening agent, disintegrant, sorbefacient, surfactant, retardant, adsorbent, suspending agent, flocculant, deflocculant, emulgator, common substrate, solubilizer, cosolvent, preservative, corrigent, colorant, antioxygen, buffer, bacteriostat, isoosmotic adjusting agent, pH adjuster, metal ion chelating agent, hardener, thickener and the like.

### DETAILED DESCRIPTION OF THE INVENTION

The following examples are used to further describe the invention, but are not intended to limit the scope of the invention. Experimental methods for which specific conditions are not indicated in the examples of the present invention are usually performed under conventional conditions or as suggested by the manufacturer of the raw material or commodity. Reagents without indicating specific sources are the conventional reagents purchased in the market.

Abbreviations in the present invention have the following meanings:

| Abbreviations | Meanings of abbreviations |
|---|---|
| ACN | acetonitrile |
| Boc- | tert-butoxycarbonyl- |
| Cbz- | carbobenzoxy- |
| CDI | carbonyldiimidazole |
| m-CPBA | meta-chloroperoxybenzoic acid |
| DAST | (diethylamino)sulfur fluoride |
| DBU | 1,8-diazabicyclo[5.4.0]undec-7-ene |
| DCM | dichloromethane |
| DIEA/DIPEA | N,N-diisopropylethylamine |
| DMAP | 4-dimethylaminopyridine |
| DMF | N,N-dimethylformamide |
| DMSO | dimethyl sulfoxide |
| DPPA | diphenylphosphoryl azide |
| EA | ethyl acetate |
| EDC | 1-(3-dimethylaminopropyl)-3-ethylcarbodiimide hydrochloride |
| EtOH | ethanol |
| h | hour |
| HATU | O-(7-azabenzotriazol-1-yl)-N,N,N',N'-tetramethyluronium hexafluorophosphate |
| HCl | hydrochloric acid |
| HOBt | 1-hydroxybenzotriazole |
| HPLC | high performance liquid chromatography |
| LDA | lithium diisopropylamide |
| MeOH | methanol |
| LiHMDS | lithium bis (trimethylsilyl)amide |
| min | minute |
| MS | mass spectrum |
| NCS | N-chlorosuccinimide |
| NMP | N-methylpyrrolidone |
| NMR | nuclear magnetic resonance |
| PE | petroleum ether |
| Pd₂(dba)₃ | tris(dibenzylideneacetone)dipalladium |
| Pd(dppf)Cl₂ | [1,1'-bis(diphenylphosphino)ferrocene]dichloropalladium |
| Pd(PPh₃)₄ | tetrakis(triphenylphosphine)palladium |
| Pd(OAc)₂ | palladium acetate |
| rt | room temperature |
| Rₜ | retention time |
| SEM- | [2-(trimethylsilyl)ethoxy]methyl- |
| TBAF | tetrabutylammonium fluoride |
| TEA | triethylamine |
| TFA | trifluoroacetic acid |
| TFAA | trifluoroacetic anhydride |
| THF | tetrahydrofuran |
| TLC | thin layer chromatography |
| TsCl | para-toluensulfonyl chloride |
| XantPhos | 9,9-dimethyl-4,5-bis(diphenylphosphino)xanthene |

Structures of the compound are determined by nuclear magnetic resonance (NMR) and/or mass spectrum (MS). NMR chemical shift (δ) is expressed in units of 10⁻⁶ (ppm). Test solvents are deuterated dimethyl sulfoxide (DMSO*-*d₆), deuterated chloroform (CDCl₃) and deuterated methanol (CD₃OD), and the internal standard is tetramethylsilane (TMS).

### Example

### Method for preparing intermediates:

### 1. Preparation of 4-chloro-1-benzenesulfonyl-1H-pyrrolo[2,3-b]pyridin-5-carbonitrile I-1

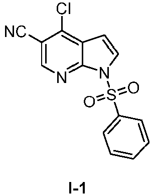

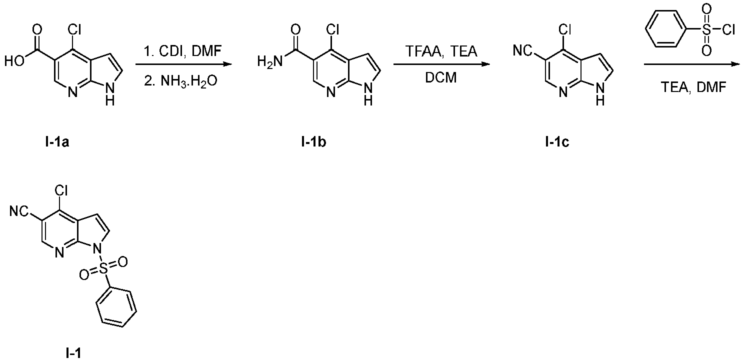

### First step: preparation of 4-chloro-1-benzenesulfonyl-1H-pyrrolo[2,3-b]pyridin-5-carbonitrile I-1b

Compound 4-chloro-1*H*-pyrrolo[2,3-b]pyridin-5-carboxylic acid **I-1a** (1.00 g, 5.10 mmol) and DMF(10 mL) were added into a reaction flask, and then CDI(0.91 g, 5.61 mmol) was added, stirred at room temperature for 1h, and cooled in an ice-water bath. Then, NH₃ • H₂O (1.12 mL) was added at 0 °C. The system was stirred at room temperature for 1h. After the reaction was completed under TLC monitoring, the system was poured into water, extracted with ethyl acetate for three times, and organic phases were combined, washed with water and saturated sodium chloride, dried with anhydrous sodium sulfate, and then concentrated, slurried by dichloromethane/petroleum ether =5ml/1ml, filtered by suction, and dried by a spin drier to obtain the titled compound **I-1b** (0.6 g, 3.08 mmol) with a yield of 60.3%.

LCMS (ESI) m/z: 196 [M+H]⁺. ¹H NMR (400 MHz, DMSO-*d*₆,δppm): 8.29 (s, 1 H), 7.91 (s, 1 H), 7.65-7.59 (m, 2 H), 7.21 (br s, 1 H), 6.57-6.56 (d, J = 3.2 Hz, 1 H).

### Second step: preparation of 4-chloro-1H-pyrrolo[2,3-b]pyridin-5-carbonitrile I-1c

Compound 4-chloro-1*H*-pyrrolo[2,3-b]pyridin-5-carboxamide **I-1b** (300 mg, 1.54 mmol), triethylamine (6 mL) and dichloromethane (6 mL) were added into a reaction flask, and then trifluoroacetic anhydride (3 mL) was added dropwise at room temperature. The system was stirred at room temperature for 6 hours. After the reaction was completed under TLC monitoring, the system was poured into ice water, extracted with dichloromethane for three times, and organic phases were combined, washed with water and saturated sodium chloride, dried with anhydrous sodium sulfate, and concentrated at a low temperature, followed by purification by column chromatography (v/v, dichloromethane/methanol=100:1-50:1) to obtain the titled compound **I-1c** (200mg, 1.13 mmol) with a yield of 73.4%.

LCMS (ESI) m/z: 178 [M+H]⁺. ¹H NMR (400 MHz, DMSO-*d*₆,δppm): 12.69 (br s, 1 H), 8.67 (s, 1 H), 7.834-7.825 (d, J = 3.6 Hz, 1 H), 6.72-6.71 (d, J = 3.6 Hz, 1 H).

### Third step: preparation of 4-chloro-1-benzenesulfonyl-1H-pyrrolo[2,3-b] pyridin-5-carbonitrile 1-1

DMF (5 mL) was added into a reaction flask, and then compound 4-chloro-1*H*-pyrrolo[2,3-b]pyridin-5-carbonitrile **I-1c** (100 mg, 0.57 mmol), triethylamine (114 mg, 1.13 mmol) and benzene sulfonyl chloride (150 mg, 0.85 mmol) were added separately. The system was stirred at room temperature overnight. After the reaction was completed under TLC monitoring, the reaction system was poured into water, extracted with ethyl acetate for three times. Organic phases were combined, washed with water and saturated sodium chloride, dried with anhydrous sodium sulfate, and then concentrated, followed by purification by column chromatography (v/v, petroleum ether/ethyl acetate=20:1-5:1) to obtain the titled compound **I-1** (120 mg, 0.38 mmol) with a yield of 67%.

LCMS (ESI) m/z: 318 [M+H]⁺. ¹H NMR (400 MHz, DMSO-*d*₆,δppm): 8.88 (s,1 H), 8.26-8.25 (d, J = 3.6 Hz, 1 H), 8.17-8.15 (d, J = 7.6 Hz, 2 H), 7.81-7.77 (t, J = 7.6 Hz, 1 H), 7.69-7.65 (t, J = 8.0 Hz, 2 H), 7.07-7.06 (d, J = 4.0 Hz, 1 H).

### Examples of the method for preparing final product:

### Example 1

### Preparation of (cis)-4-({4-ethyl-1-[(2,2,2-trifluoroethyl)carbamoyl)]pyrrolidin-3-yl}amino)-1H-pyrrolo[2,3-b]p yridin-5-carbonitrile H01

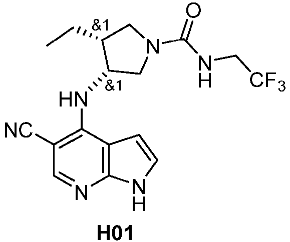

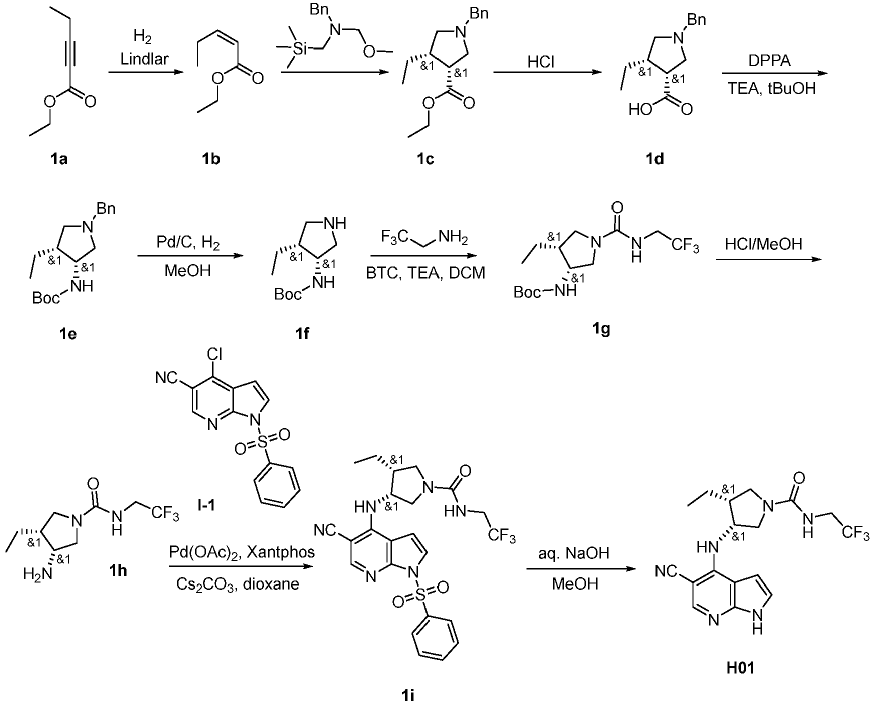

### First step: preparation of (cis)-ethyl 2-pentenoate 1b

Compound ethyl 2-pentynoate **1a** (130 g, 1.03 mol) and anhydrous tetrahydrofuran (1.3 L) were added into a reaction flask, and then quinoline (13 mL) and lindlar catalyst (13.0 g) were added. The system was stirred for 5h under the protection of hydrogen balloon. After the reaction was completed under TLC monitoring, the system was filtered with kieselguhr, dried by a spin drier (10-15 °C) to obtain crude products, followed by purification by column chromatography (petroleum ether) to obtain the titled compound **1b** (70 g, 0.55 mol) with a yield of 53%.

¹H NMR (400 MHz, CDCl₃,δppm): 6.21-6.16 (m, 1 H), 5.75-5.71 (m, 1 H), 4.19-4.14 (m, 2 H), 2.67-2.63 (m, 2 H), 1.30-1.26 (t, J = 7.2 Hz, 3 H), 1.07-1.03 (t, J = 7.6 Hz, 3 H).

### Second step: preparation of (cis)-1-benzyl-4-ethyl-pyrrolidin-3-ethyl formate 1c

Dichloromethane (1.4 L) was added into a reaction flask, then compound (cis)-ethyl 2-pentenoate **1b** (70.0 g, 0.54 mol) and N-(methoxymethyl)-N-(trimethylsilyl)benzylamine (130 g, 0.54 mol) were added separately. The system was cooled to 0 °C, and trifluoroacetic acid (6.20 g, 0.054 mol) was added dropwise, followed by stirred for 2h at an ice-water bath. After the reaction was completed under TLC monitoring, the system was concentrated and dried by a spin drier, and purified by column chromatography (v/v, petroleum ether/ethyl acetate=50:1-5:1) to obtain the titled compound **1c** (70.0 g, 0.27 mol) with a yield of 49%.

LCMS (ESI) m/z: 262 [M+H]⁺. ¹H NMR (400 MHz, CDCl₃,δppm): 7.35-7.28 (m, 5 H), 4.16-4.10 (m, 2 H), 3.65 (s, 2 H), 3.12-3.02 (m, 3 H), 2.70-2.64 (m, , 1 H), 2.40-2.35 (m, 1 H), 2.11-2.06 (t, J = 9.2 Hz, 1 H),1.49-1.43 (m, 1 H), 1.29-1.23 (m, 1 H), 1.28-1.24 (t, J = 6.8 Hz, 3 H), 0.87-0.84 (t, J = 7.6 Hz, 3 H).

### Third step: preparation of (cis)-1-benzyl-4-ethyl-pyrrolidin-3-carboxylic acid 1d

Compound (cis)-1-benzyl-4-ethyl-pyrrolidin-3-ethyl formate **1c** (70.0 g, 0.27 mol) was dissolved in 1,4-dioxane (700 mL), and then concentrated hydrochloric acid (700 mL) was added. The system was stirred for 16h at 90 °C. After the reaction was completed under TLC monitoring, the system was adjusted to pH=9 with 6 mol/L sodium hydroxide solution in an ice-water bath, and then extracted with ethyl acetate (700 mL x 2). The water phase was adjusted to pH=7 with 2 mol/L hydrochloric acid, then dried by a spin drier to obtain crude products. The crude products were slurried by ethanol (200 mL), filtered and dried by a spin drier to obtain the titled compound **1d** (35.0 g, 0.15 mol) with a yield of 56%.

LCMS (ESI) m/z: 234 [M+H]⁺. ¹H NMR (400 MHz, CDCl₃,δppm): 7.60-7.58 (m, 2 H), 7.39-7.37 (m, 3 H), 4.43-4.34 (m, 2 H), 3.66-3.62 (m, 1 H), 3.49-3.42 (m, 2 H), 3.26-3.22 (m, 1 H), 3.02-2.97 (m, 1 H), 2.62-2.55 (m, 1 H), 1.63-1.56 (m, 1 H), 1.41-1.34 (m, 1 H), 0.94-0.90 (t, J = 7.2, 3 H).

### Fourth step: preparation of (cis)-(1-benzyl-4-ethyl-pyrrolidin-3-yl) tert-butyl carbamate 1e

Compound (cis)-1-benzyl-4-ethyl-pyrrolidin-3-carboxylic acid **1d** (2.00 g, 8.58 mmol), triethylamine (1.73 g, 17.2 mmol) and diphenylphosphoryl azide (3.54 g, 12.9 mmol) were added into tertiary butanol (20 mL), stirred for 2h at room temperature and for 16h at 90°C under the protection of nitrogen. After the reaction was completed under TLC monitoring, the system was concentrated, dried by a spin drier, extracted with ethyl acetate, and the organic phases were washed with water and saturated sodium chloride, dried with anhydrous sodium sulfate, followed by purification by column chromatography (v/v, petroleum ether/ethyl acetate=50:1-10:1) to obtain the titled compound **1e** (0.55 g, 1.81 mmol) with a yield of 20%.

¹H NMR (400 MHz, CDCl₃,δppm): 7.33-7.23 (m, 5 H), 4.81-4.78 (m, 1 H), 4.23-4.21 (m, 1 H), 3.66-3.55 (m, 2 H), 2.82-2.78 (m, 1 H), 2.67-2.63 (t, J = 9.2 Hz, 1 H), 2.46-2.43 (m, 1 H), 2.32-2.29 (m, 1 H), 2.19-2.14 (m, 1 H), 1.48-1.43 (m, 1 H), 1.44 (s, 9 H), 1.28-1.18 (m, 1 H), 0.89-0.85 (t, J = 7.2 Hz, 3 H).

### Fifth step: preparation of (cis)-(4-ethyl-pyrrolidin-3-yl) tert-butyl carbamate 1f

Compound (cis)-(1-benzyl-4-ethyl-pyrrolidin-3-yl) tert-butyl carbamate **1e** (10.0 g, 32.9 mmol), methanol (100 mL) and Pd/C (10.0 g) were added into a hydrogenation flask, stirred for 6h under 50psi hydrogen pressure at room temperature. After the reaction was completed under TLC monitoring, the system was filtered with kieselguhr to obtain a filtrate, which was concentrated and dried by a spin drier to obtain the titled compound **1f** (6.00 g, 28.0 mmol) with a yield of 85%.

¹H NMR (400 MHz, CDCl₃,δppm): 4.82 (br s, 1 H), 4.16 (br s, 1 H), 3.21-3.14 (m, 2 H), 2.80-2.77 (m, 1 H), 2.55-2.49 (t, J = 10.0 Hz, 1 H), 2.32-2.26 (m, 1 H), 2.05-2.01 (m, 1 H), 1.51-1.45 (m, 1 H), 1.44 (s, 9 H), 1.31-1.24 (m, 1 H), 0.95-0.92 (t, J = 7.2 Hz, 3 H).

### Sixth step: preparation of (cis)-{4-ethyl-1-[(2,2,2-trifluoroethyl)carbamoyl]pyrrolidin-3-yl} tert-butyl carbamate 1g

Dichloromethane (60 mL), triethylamine (5.67 g, 56.1 mmol) and trifluoroethylamine (2.78 g, 28.0 mmol) were added into a reaction flask, and then triphosgene (3.32 g, 11.2 mmol) was added at 0°C, stirred for 2h at 0°C, and then compound (cis)-(4-ethyl-pyrrolidin-3-yl) tert-butyl carbamate **1f** (6.00 g, 28.0 mmol) was added at 0°C. Then the system was returned to room temperature and stirred for 6h. After reaction was completed under TLC monitoring, the system was poured into water, extracted with dichloromethane, washed with water and saturated sodium chloride, dried with anhydrous sodium sulfate and concentrated, followed by purification by column chromatography (v/v, petroleum ether/ethyl acetate=30:1-2:1) to obtain the titled compound **1g** (2.50 g, 7.37 mmol) with a yield of 26%.

LCMS (ESI) m/z: 362 [M+Na]⁺. ¹H NMR (400 MHz, DMSO-*d*₆,δppm):7.11-7.09 (d, J = 8.8 Hz, 1 H), 6.73-6.70 (t, J = 6.4 Hz, 1 H), 4.04 (br s, 1 H), 3.82-3.73 (m, 2 H), 3.41-3.36 (m, 2 H), 3.20-3.17 (d, J = 10.8 Hz, 1 H), 3.10-3.05 (t, J = 10.0 Hz, 1 H), 2.10 (br s, 1 H), 1.38 (s, 9 H), 1.36-1.34 (m, 1 H), 1.30-1.23 (m, 1 H), 0.87-0.83 (t, J = 7.2 Hz, 3 H).

### Seventh step: preparation of (cis)-3-amino-4-ethyl-N-(2,2,2-trifluoroethyl) pyrrolidin-1-carboxamide 1h

Compound (cis)-{4-ethyl-1-[(2,2,2-trifluoroethyl)carbamoyl]pyrrolidin-3-yl} tert-butyl carbamate **1g** (2.50 g, 7.37 mmol) was dissolved in hydrochloric acid-methanol (25 mL, 4.8 mol/L) at 0°C and stirred for 2h at room temperature. After the reaction was completed under TLC monitoring, the system was concentrated, followed by addition of water, adjusted to pH=9 with sodium hydroxide solution (2 mol/L), and extracted with ethyl acetate. The organic phase was dried with anhydrous sodium sulfate, concentrated and dried by a spin drier to obtain the titled compound **1h** (1.5 g, 6.20 mmol) with a yield of 85%.

LCMS (ESI) m/z: 240 [M+H]⁺. ¹H NMR (400 MHz, DMSO-*d*₆,δppm):4.52 (br s, 1 H), 3.98-3.85 (m, 2 H), 3.55-3.50 (m, 3 H), 3.32-3.30 (m, 1 H), 3.15-3.10 (t, J = 9.6 Hz, 1 H), 2.12-2.07 (m, 1 H), 1.57-1.41 (m, 2 H), 1.25 (br s, 2 H), 1.00-0.96 (t, J = 7.2 Hz, 3 H).

### Eighth step: preparation of (cis)-3-[(5-cyano-1-benzenesulfonyl-1H-pyrrolo[2,3-b]pyridin-4-yl)amino]-4-ethyl-N-(2,2,2-trifluoroethyl)pyrrolidin-1-carboxamide 1i

Dioxane (12 mL) was added into a reaction flask, followed by addition of compound 4-chloro-1-benzenesulfonyl-1*H*-pyrrolo[2,3-b]pyridin-5-carbonitrile **I-1** (240 mg, 0.76 mmol), (cis)-3-amino-4-ethyl-*N*-(2,2,2-trifluoroethyl)pyrrolidin-1-carboxamide **1h** (199 mg, 0.83 mmol), cesium carbonate (740 mg, 2.27 mmol), Xantphos (175 mg, 0.30 mmol) and Pd(OAc)₂ (33.9 mg, 0.15 mmol). The syestem was heated to 100°C and stirred for 6h. After the reaction was completed under TLC monitoring, the system was cooled to room temperature and filtered, poured into water, and extracted with dichloromethane and water for three times. The organic phases were combined, washed with water and saturated sodium chloride, dried with anhydrous sodium sulfate and concentrated to obtain crude products. The crude products were purified with preparative TLC to obtain the titled compound **1i** (100 mg, 0.19 mmol) with a yield of 25.4%. LCMS (ESI) m/z: 521 [M+H]⁺.

### Ninth step: preparation of (cis)-4-({4-ethyl-1-[(2,2,2- trifluoroethyl) carbamoyl)]pyrrolidin-3-yl}amino)1H-pyrrolo[2,3-b]pyridin-5-carbonitrile H01

Methanol (1 mL) was added into a reaction flask, followed by addition of (cis)-3-[(5-cyano-1-benzenesulfonyl-1*H*-pyrrolo[2,3-b]pyridin-4-yl)amino]-4-ethyl-*N*-(2,2,2-trifl uoroethyl)pyrrolidin-1-carboxamide **1i** (40.0 mg, 0.074 mmol) and sodium hydroxide solution (0.15 mL, 0.15 mmol, 1 mol/L). The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were purified with preparative TLC to obtain the titled compound **H01** (26 mg, 0.063 mmol) with a yield of 83.3%. LCMS (ESI) m/z: 381 [M+H]⁺.

¹H NMR (400 MHz, DMSO-d6) δ 11.87 (s, 1H), 8.11 (s, 1H), 7.27 (dd, J = 3.6, 2.5 Hz, 1H), 6.95 - 6.80 (m, 2H), 6.63 (dd, J = 9.1 Hz, 1H), 4.89 - 4.78 (m, 1H), 3.86 - 3.73 (m, 2H), 3.71 - 3.63 (m, 1H), 3.61 - 3.46 (m, 2H), 3.40 - 3.33 (m, 1H), 2.46 - 2.36 (m, 1H), 1.56 - 1.46 (m, 1H), 1.44 - 1.34 (m, 1H), 0.84 (t, J = 7.4 Hz, 3H).

### Example 2

### Preparation of (cis)-4-({4-ethyl-1-[(2,2,2-trifluoroethyl)carbamoyl)]pyrrolidin-3-yl}-methyl-amino)-1H-pyrrolo [2,3-b]pyridin-5-carbonitrile H02

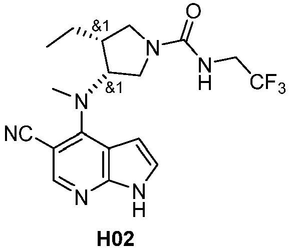

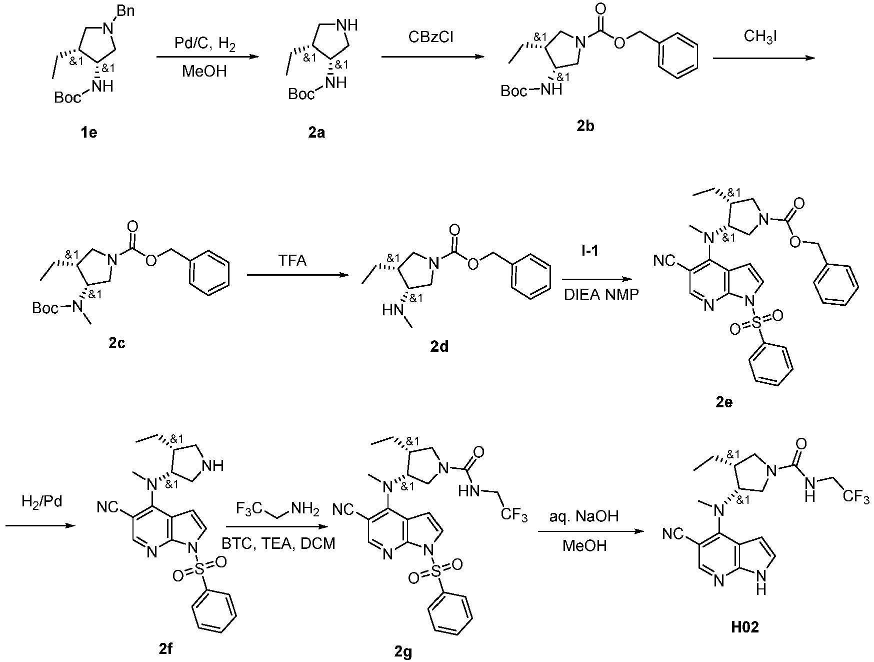

### First step: preparation of (cis)-(4-ethyl-pyrrolidin-3-yl) tert-butyl carbamate 2a

Compound **1e** (3.5 g, 11.5 mmol) and methanol (50 mL) were added into a reaction flask, and then palladium on carbon (300 mg) was added. The system was stirred for 5h under the protection of hydrogen balloon. After the reaction was completed under TLC monitoring, the system was filtered with kieselguhr, dried by a spin drier (10-15 °C) to obtain crude products, followed by purification by column chromatography (petroleum ether) to obtain the titled compound **2a** (2.1 g, 9.8 mmol) with a yield of 85.2 %. LCMS (ESI) m/z: 215 [M+H]⁺.

### Second step: preparation of (cis)-(1-benzyloxycarbonyl-4-ethyl-pyrrolidin-3-yl) tert-butyl carbamate 2b

Compound 2a (2.1 g, 9.8 mmol) and dichloromethane (30 mL) were added into a reaction flask, and then triethylamine (2.0 g, 19.6 mmol) and benzyl chloroformate (2.5g, 14.7mmol) were added, and the system was stirred for 2h at room temperature. After the reaction was completed under TLC monitoring, the system was extracted with dichloromethane and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **2b** (820 mg, 2.48 mmol) with a yield of 25.3%. LCMS (ESI) m/z: 371 [M+Na]⁺.

### Third step: preparation of (cis)-(1-benzyloxycarbonyl-4-ethyl-pyrrolidin-3-yl)-methyl-tert-butyl carbamate 2c

Compound **2b** (0.2 g, 0.57 mmol) and N,N-dimethylformamide (5 mL) were added into a reaction flask, and then sodium hydride (46 mg, 1.15 mmol, 60%) was added. After the system was stirred for 10 min at room temperature, iodomethane (121 mg, 0.86mmol) was added. The system was stirred for 2h at room temperature. After the reaction was completed under TLC monitoring, the system was extracted with ethyl acetate and water. The organic phases were washed with water and saturated sodium chloride, dried with anhydrous sodium sulfate and concentrated to obtain the titled compound 2c (200 mg, 0.55 mmol) with a crude yield of 96.5%. LCMS (ESI) m/z: 363 [M+H]⁺.

### Fourth step: preparation of (cis)-(1-benzyloxycarbonyl-4-ethyl-pyrrolidin-3-yl) -methyl-amine 2d

Compound 2c (0.2 g, 0.55 mmol) and dichloromethane (5 mL) were added into a reaction flask, and then trifluoroacetic acid (0.5 mL) was added, and the system was stirred for 30 min at room temperature. After the reaction was completed under TLC monitoring, the system was concentrated, followed by another addition of dichloromethane, and concentrated again. This step was repeated for three times to obtain trifluoroacetate of the titled compound **2d** (160 mg, 0.45 mmol) with a yield of 81.8%. LCMS (ESI) m/z: 263 [M+H]⁺.

### Fifth step: preparation of (cis)-3-[(5-cyano-1-benzenesulfonyl-1H-pyrrolo[2,3-b] pyridin-4-yl)-methyl-amino]-4-ethyl-pyrrolidin-1-benzyl formate 2e

Compound **2d** (160 mg, 0.45 mmol) and N-methyl pyrrolidone (2 mL) were added into a reaction flask, then intermediate **I-1** (143 mg, 0.45 mmol) and N-isopropyldiethylamine (116 mg, 0.90 mmol) were added. The system was subjected to a microwave reaction for 1h at 170 °C. After the reaction was completed under TLC monitoring, the system was extracted with ethyl acetate and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **2e** (50 mg, 0.092 mmol) with a yield of 20.4%. LCMS (ESI) m/z: 544 [M+H]⁺.

### Sixth step: preparation of (cis)-3-[(5-cyano-1-benzenesulfonyl-1H-pyrrolo[2,3-b] pyridin-4-yl)-methyl-amino]-4-ethyl-pyrrolidine 2f

Compound **2e** (50 mg, 0.092 mmol) and methanol (10 mL) were added into a reaction flask, and then palladium on carbon (30 mg) was added. The system was stirred for 5h under the protection of hydrogen balloon. After the reaction was completed under TLC monitoring, the system was filtered with kieselguhr, dried by a spin drier (10-15 °C) to obtain the titled compound **2f** (36 mg, 0.088 mmol) with a yield of 95.7%. LCMS (ESI) m/z: 410 [M+H]⁺.

### Seventh step: preparation of (cis)-3-[(5-cyano-1-benzenesulfonyl-1H-pyrrolo [2,3-b]pyridin-4-yl)-methyl-amino]-4-ethyl-N-(2,2,2-trifluoroethyl)pyrrolidin-1-carboxamide 2g

Dichloromethane (60 mL), triethylamine (18 mg, 0.176 mmol) and trifluoroethylamine (9 mg, 0.088 mmol) were added into a reaction flask, and then triphosgene (10 mg, 0.035 mmol) was added at 0°C. The system was stirred for 2h at 0°C, and then compound **2f** (36 mg, 0.088 mmol) was added at 0°C. Then the system was returned to room temperature and stirred for 6h. After the reaction was completed under TLC monitoring, the system was poured into water, extracted with dichloromethane, washed with water and saturated sodium chloride, dried with anhydrous sodium sulfate and concentrated, followed by layer chromatography to obtain the titled compound **2g** (10 mg, 0.019 mmol) with a yield of 21.6%. LCMS (ESI) m/z: 535 [M+H]⁺.

### Eighth step: preparation of (cis)-4-({4-ethyl-1-[(2,2,2-trifluoroethyl)carbamoyl)]pyrrolidin -3-yl}-methyl-amino)-1H-pyrrolo[2,3-b]pyridin-5-carbonitrile H02

Methanol (1 mL) was added into a reaction flask, and then compound **2g** (10.0 mg, 0.019 mmol) and sodium hydroxide solution (0.15 mL, 0.15 mmol, 1 mol/L) were added separately. The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were purified by preparative TLC to obtain the titled compound **H02** (6 mg, 0.015 mmol) with a yield of 78.9%. LCMS (ESI) m/z: 395 [M+H]⁺.

¹H NMR (400 MHz, DMSO-*d*₆) δ 12.01 (s, 1H), 8.20 (s, 1H), 7.41 - 7.36 (m, 1H), 6.98 - 6.91 (m, 1H), 6.61 - 6.56 (m, 1H), 4.81 - 4.74 (m, 1H), 3.84-3.57 (m, 5H), 3.33 - 3.30 (m, 1H), 3.13 (s, 3H), 2.42-2.32(m, 1H), 1.55 - 1.34 (m, 2H), 0.71 (t, *J* = 8.0 Hz, 3H).

### Example 3

### Preparation of (cis)-4-{ [4-ethyl-1-(3,3,3- trifluoropropionyl) pyrrolidin-3-yl]-methyl-amino }-1H-pyrrolo[2,3-b]pyridin-5-carbonitrile H03

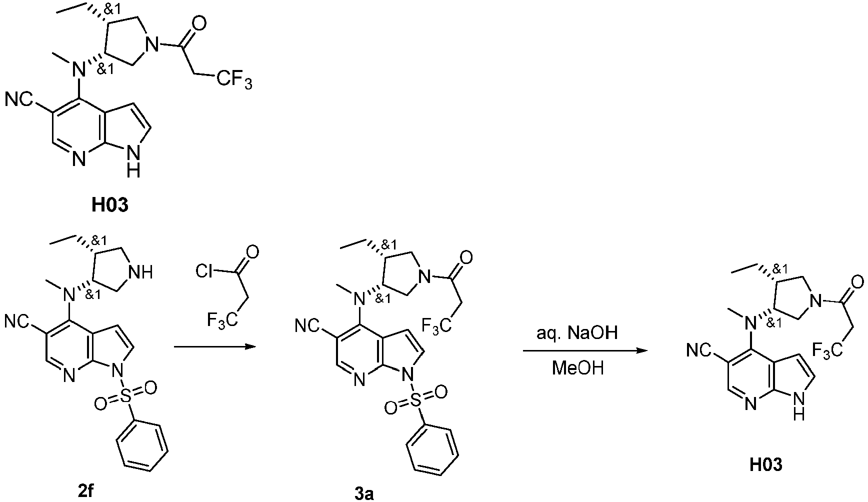

### First step: preparation of (cis)-1-benzenesulfonyl-4-{[4-ethyl-1-(3,3,3-trifluoropropionyl)pyrrolidin-3-yl]-methyl-amino } - 1H-pyrrolo[2,3-b]pyridin-5-carbonitrile 3a

Compound **2f** (100 mg, 0.24 mol) and dichloromethane (30 mL) were added into a reaction flask, and then triethylamine (24 mg, 0.24 mmol) and 3,3,3-trifluoropropanoyl chloride (35 mg, 0.24 mmol) were added. The system was stirred for 2h at room temperature. After the reaction was completed under TLC monitoring, the system was extracted with dichloromethane and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **3a** (40 mg, 0.077 mmol) with a yield of 32.1%. LCMS (ESI) m/z: 520 [M+H]⁺.

### Second step: preparation of (cis)-4-{[4-ethyl-1-(3,3,3-trifluoropropionyl)pyrrolidin-3-yl]-methyl-amino}-1H-pyrrolo[2,3-b]p yridin-5-carbonitrile H03

Methanol (1 mL) was added into a reaction flask, and then compound **3a** (40.0 mg, 0.077 mmol) and sodium hydroxide solution (0.15 mL, 0.15 mmol, 1 mol/L) were added separately. The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were by purified with preparative TLC to obtain the titled compound **H03** (20 mg, 0.053 mmol) with a yield of 68.8%. LCMS (ESI) m/z: 380 [M+H]⁺.

### Example 4

### Preparation of (cis)-4-({4-methyl-1-[(2,2,2-trifluoroethyl)carbamoyl)]pyrrolidin-3-yl}-methyl-amino)-1H-pyrro lo[2,3-b]pyridin-5-carbonitrile H04

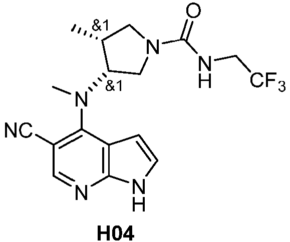

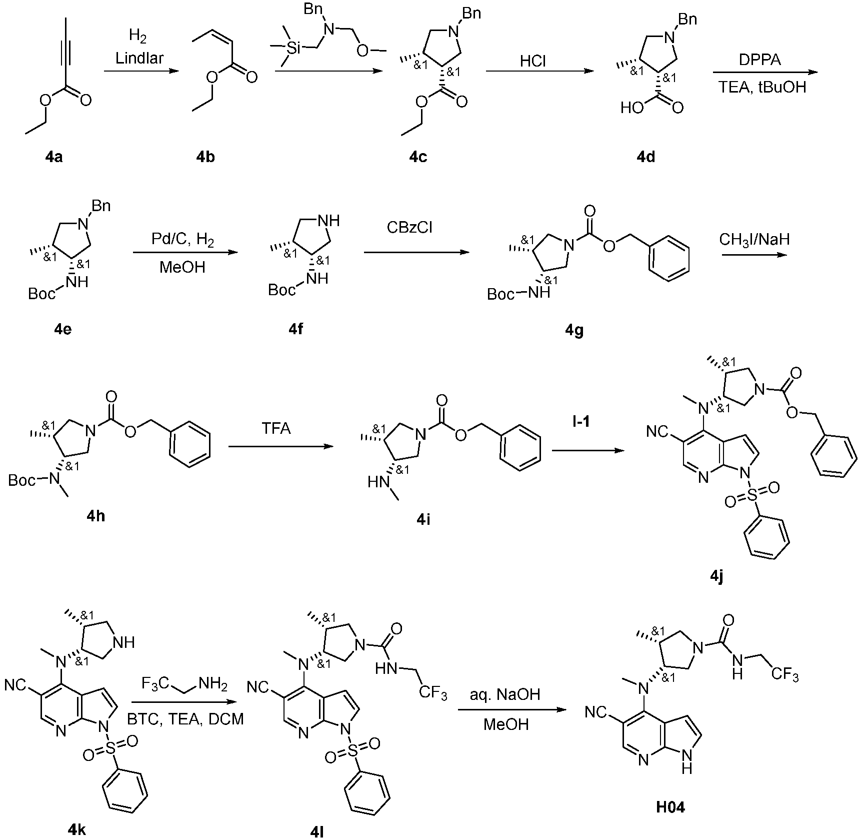

Ethyl 2-butynoate **4a** was used as a raw material to obtain compound **H04** by using the same preparation method as described in Example 2. LCMS (ESI) m/z: 381 [M+H]⁺.

¹H NMR (400 MHz, DMSO-d6) δ 12.04 (s, 1H), 8.22 (d, J = 1.0 Hz, 1H), 7.40 (d, J = 3.5 Hz, 1H), 6.90 (t, J = 6.3 Hz, 1H), 6.62 (d, J = 3.5 Hz, 1H), 4.69 (s, 1H), 3.86 - 3.69 (m, 3H), 3.65 - 3.52 (m, 2H), 3.11 (m, 4H), 2.68 - 2.49 (m, 1H), 0.98 (d, J = 6.9 Hz, 3H).

### Example 5

### Preparation of (cis)-4-{[4-methyl-1-(2-cyanoacetyl)pyrrolidin-3-yl]-methyl-amino}-1H-pyrrolo[2,3-b]pyridin-5 -carbonitrile H05

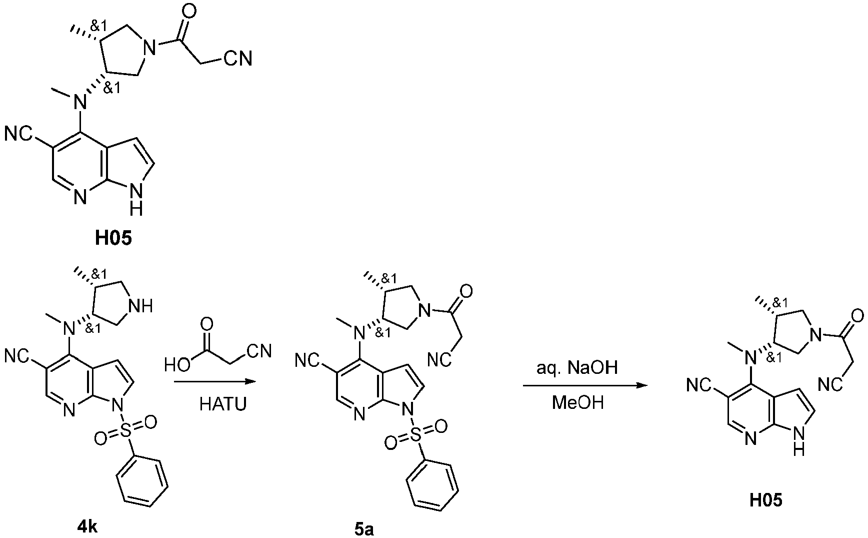

### First step: preparation of (cis)-1-benzenesulfonyl-4-{[4-methyl-1-(2-cyanoacetyl)pyrrolidin-3-yl]-methyl-amino}-1H-pyrr olo[2,3-b]pyridin-5-carbonitrile 5a

Compound **4k** (95 mg, 0.24 mol) and dichloromethane (30 mL) were added into a reaction flask, and then triethylamine (24 mg, 0.24 mmol), cyanoacetic acid (20 mg, 0.24 mmol) and HATU (91 mg, 0.24 mmol) were added. The system was stirred for 2h at room temperature. After the reaction was completed under TLC monitoring, the system was extracted with dichloromethane and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound 5a (50 mg, 0.11 mmol) with a yield of 43.8%. LCMS (ESI) m/z: 463 [M+H]⁺.

### Second step: preparation of (cis)-4-{[4-methyl-1-(2-cyanoacetyl)pyrrolidin-3-yl]-methyl-amino }-1H-pyrrolo[2,3-b]pyridin-5 -carbonitrile H05

Methanol (1 mL) was added into a reaction flask, and then compound **5a** (50.0 mg, 0.11 mmol) and sodium hydroxide solution (0.15 mL, 0.15 mmol, 1 mol/L) were added separately. The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were purified by preparative TLC to obtain the titled compound **H05** (30 mg, 0.093 mmol) with a yield of 84.5%. LCMS (ESI) m/z: 323 [M+H]⁺.

### Example 6

### Preparation of (3aR,5R,6aS)-4-{[2-((2S)-2-hydroxypropionyl)-hexahydrocyclopenta[c]pyrrole-5-yl]-methyl-am ino}-1H-pyrrolo[2,3-b]pyridin-5-carbonitrile H06

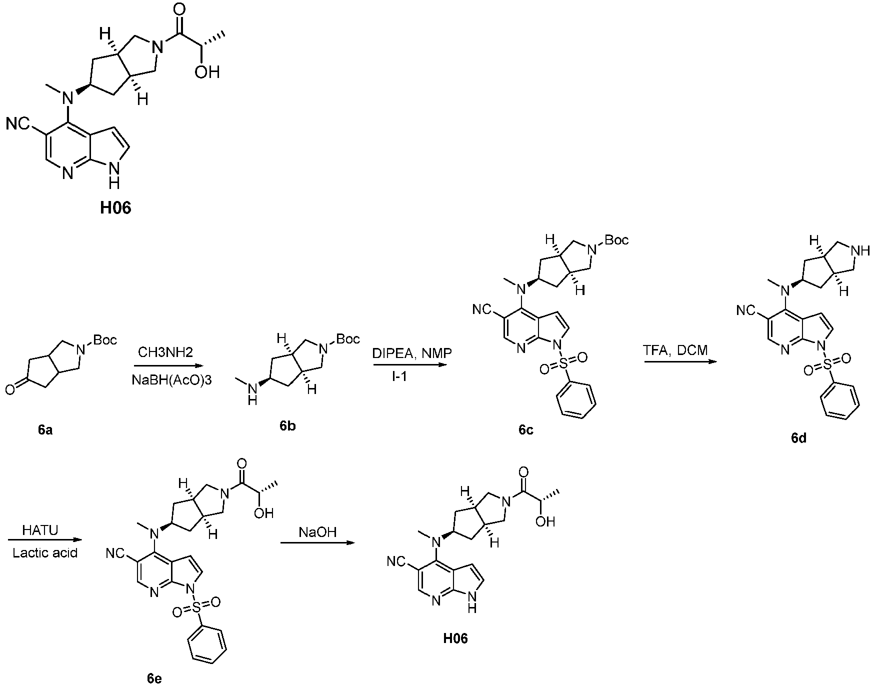

### First step: preparation of N-(2-Boc-hexahydrocyclopenta[c]pyrrole-5-yl)-methylamine 6b

Compound **6a** (200mg, 0.84mmol) was dissolved in 5mL methanol, and then 2mL of 37% methylamine-ethanol solution and sodium triacetoxyborohydride (532mg, 2.5mmol) were added and reacted for 4h. 10 ml saturated ammonium chloride solution was added dropwise to quench the reaction and ethyl acetate was used to extract. The organic phases were combined, dried with anhydrous sodium sulfate, filtered to obtain a filtrate, which was concentrated by reducing pressure, to obtain the titled compound **6b** (108mg, 0.45mmol) with a yield of 53.6%. LCMS (ESI) m/z: 241 [M+H]⁺.

### Second step: preparation of 1-benzenesulfonyl-4-[(2-Boc-hexahydrocyclopenta[c] pyrrole-5-yl]-methyl-amino}-1H-pyrrolo[2,3-b]pyridin-5-carbonitrile 6c

Compound **6b** (108 mg, 0.45 mmol) and N-methyl pyrrolidone (2 mL) were added into a reaction flask, and then intermediates **I-1** (143 mg, 0.45 mmol) and N-isopropyldiethylamine (116 mg, 0.90 mmol) were added. The system was subjected to a microwave reaction for 1h at 170 °C. After the reaction was completed under TLC monitoring, the system was extracted with ethyl acetate and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **6c** (50 mg, 0.096 mmol) with a yield of 21.3%. LCMS (ESI) m/z: 522 [M+H]⁺.

### Third step: preparation of 4-[(hexahydrocyclopenta[c]pyrrole-5-yl)-methyl-amino]-1H-pyrrolo[2,3-b]pyridin-5-carbonitrile 6d

Compound **6c** (50 mg, 0.096 mmol) and dichloromethane (5 mL) were added into a reaction flask, and then trifluoroacetic acid (0.5 mL) was added, stirred for 30 min at room temperature. After the reaction was completed under TLC monitoring, the system was concentrated, followed by another addition of dichloromethane, and concentrated again. This step was repeated for three times to obtain trifluoroacetate of the titled compound 6**d** (40 mg, 0.077 mmol) with a crude yield of 80.2%. LCMS (ESI) m/z: 422 [M+H]⁺.

### Fourth step: preparation of 1-benzenesulfonyl-4-{[2-((2S)-2-hydroxypropionyl)-hexahydrocyclopenta[c]pyrrole-5-yl]-methyl-amino}-1H-pyrrolo[2,3-b]pyridin-5-carbonitrile 6e

Compound **6e** (40 mg, 0.077 mol) and dichloromethane (30 mL) were added into a reaction flask, and then triethylamine (8 mg, 0.077 mmol), sodium L-lactate (9 mg, 0.077 mmol) and HATU (29 mg, 0.077 mmol) were added. The system was stirred for 2h at room temperature. After the reaction was completed under TLC monitoring, the system was extracted with dichloromethane and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **6e** (30 mg, 0.061 mmol) with a yield of 79.2%. LCMS (ESI) m/z: 494 [M+H]⁺.

### Fifth step: preparation of 4-{[2-((2S)-2-hydroxypropionyl)-hexahydrocyclopenta[c] pyrrole-5-yl]-methyl-amino }-1H-pyrrolo[2,3-b]pyridin-5-carbonitrile H06

Methanol (1 mL) was added into a reaction flask, and then compound **6e** (30 mg, 0.061 mmol) and sodium hydroxide solution (0.15 mL, 0.15 mmol, 1 mol/L) were added separately. The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were purified by preparative TLC to obtain the titled compound **H05** (15 mg, 0.042 mmol) with a yield of 68.9%. LCMS (ESI) m/z: 354 [M+H]⁺.

¹H NMR (400 MHz, DMSO-*d*₆) δ 11.95 (s, 1H), 8.17 (s, 1H), 7.36 - 7.31 (m, 1H), 6.67 (dd, *J =* 3.6, 2.0 Hz, 1H), 4.85 (dd, *J =* 6.9, 3.0 Hz, 1H), 4.30 (p, *J =* 6.5 Hz, 1H), 3.61 - 3.57 (m, 1H), 3.56 - 3.51 (m, 2H), 3.47 - 3.44 (m, 1H), 3.19 (s, 3H), 2.72 - 2.55 (m, 2H), 2.17 - 2.06 (m, 2H), 1.77 - 1.62 (m, 2H), 1.23 - 1.15 (m, 3H).

### Example 7

### (3aR,5s,6aS)-4-({2-[(3-methoxy-1,2,4-thiadiazole-5-yl)carbamoyl]-hexahydrocyclopenta[c] pyrrole-5-yl}-methyl-amino)-1H-pyrrolo[2,3-b]pyridin-5-carbonitrile H07

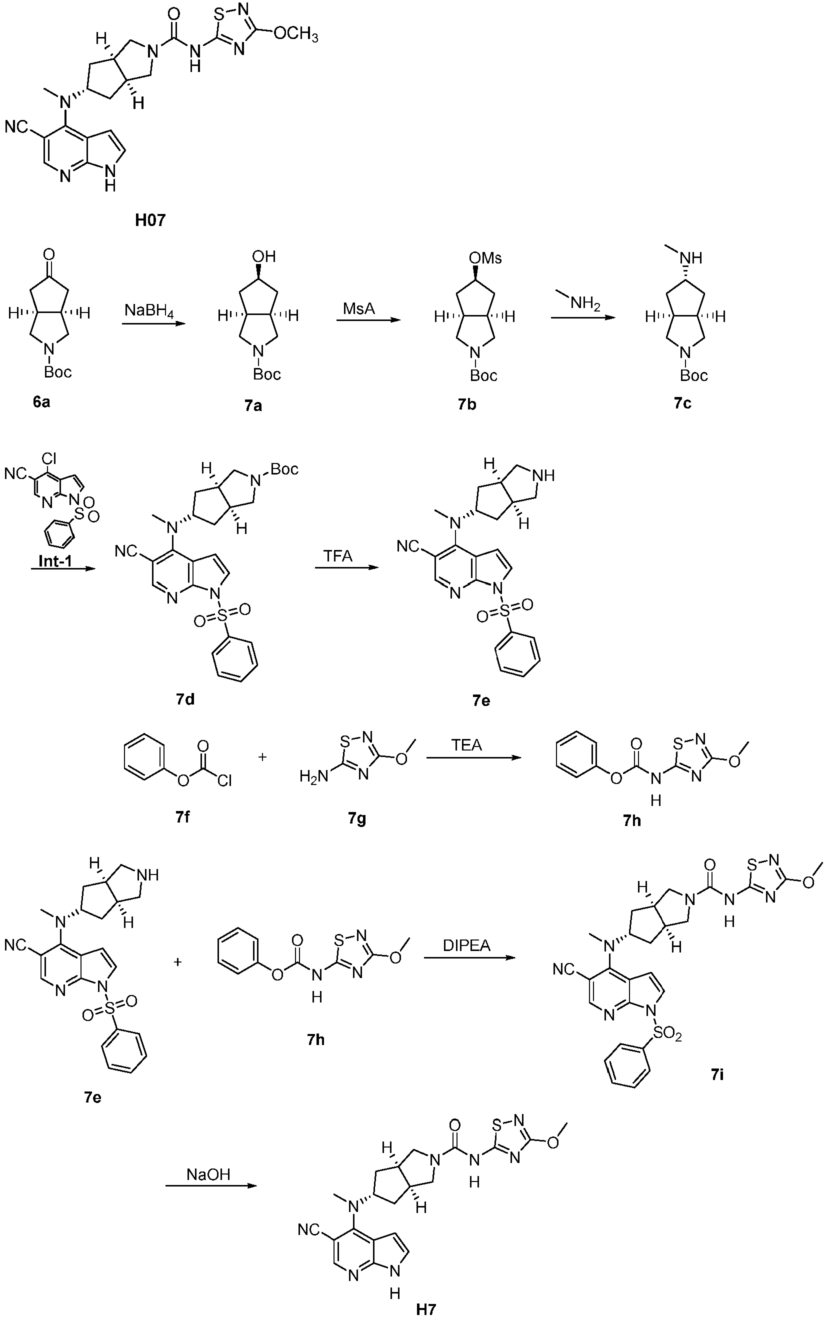

### First step: preparation of tert-butyl (3aR,5r,6aS)-5-hydroxyhexahydrocyclopentadiene[c] pyrrole-2(1H)-carboxylate 7a

Compound **6a** (2.0 g, 8.89 mmol) and methanol (50 mL) were added into a reaction flask, and then NaBH₄ (338mg, 8.89mmol) was added slowly under an ice bath. The system was stirred for 1h at room temperature. After the reaction was completed under TLC monitoring, the system was quenched by adding water, mixed with silica gel, concentrated at low temperature, and purified by column chromatography (v/v, DCM/MeOH=100:1-50:1) to obtain compound **7a** (2.0 g, 8.81 mmol) with a yield of 99.1%.

MS m/z (ESI): 228 [M+H]⁺.

### Second step: preparation of tert-butyl (3aR,5r,6aS)-5-((methylsulfonyl)oxo) hexahydrocyclopentadiene[c]pyrrole-2(1H)-carboxylate 7b

Compound 7a (2.0 g, 8.81 mmol), DIPEA (2.3 g, 17.62mmol) and DCM (20 mL) were added into a reaction flask, and then methanesulfonic anhydride (1.8g, 10.57mmol) was added. After reacting for 4h at room temperature, the reaction was completed. The system was poured into water, extracted with ethyl acetate for three times, and the organic phases were combined, washed with water and saturated sodium chloride, dried with anhydrous sodium sulfate, and concentrated at low temperature, followed by purification by column chromatography (v/v, petroleum ether/ethyl acetate=100:1-50:1) to obtain compound **7b** (2.0g, 6.56 mmol) with a yield of 74.5%.

MS m/z (ESI): 306 [M+H]⁺.

### Third step: preparation of tert-butyl (3aR,5s,6aS)-5-(methylamino) hexahydrocyclopentadiene[c]pyrrole-2(1H)-carboxylate 7c

Compound 7b (1.0 g, 3.28 mmol), methanol amine solution (10 mmol) and methanol (20 mL) were added into a sealed pot. The system was reacted for 8h at 80°C. After the reaction was completed, the system was concentrated at low temperature, and purified by column chromatography (v/v, DCM/MeOH=100:1-50:1) to obtain compound **7c** (700 mg, 2.92 mmol) with a yield of 89.0%.

### Fourth step: preparation of tert-butyl (3aR,5s,6aS)-5-((5-cyano-1-(phenylsulfonyl) -1H-pyrrolo[2,3-b]pyridin-4-yl)(methyl)amino)hexahydrocyclopentadiene[c]pyrrole-2(1H)-carb oxylate 7d

Compound **7c** (380 mg, 1.58 mmol), DIPEA (408 mg, 3.16mmol), **Int-1** (500mg, 1.58mmol) and NMP (5 mL) were added into a reaction flask. The system was subjected to a microwave reaction for 1h at 150 °C. After the reaction was completed, the system was poured into water, extracted with ethyl acetate for three times, and the organic phases were combined, washed with water and saturated sodium chloride, dried with anhydrous sodium sulfate, and concentrated at low temperature, followed by purification by column chromatography (v/v, DCM/MeOH=100:1-50:1) to obtain compound **7d** (300 mg, 0.58 mmol) with a yield of 36.7%.

MS m/z (ESI): 522 [M+H]⁺.

### Fifth step: preparation of 4-(methyl((3aR,5s,6aS)-octahydrocyclopentadiene[c]pyrrole-5-yl)amino)-1-(phenylsulfonyl)-1H -pyrrolo[2,3-b]pyridin-5-carbonitrile 7e

Compound **7d** (300 mg, 0.58 mmol), TFA (2mL) and DCM (5 mL) were added into a reaction flask. The system was reacted for 1h at room temperature. After the reaction was completed, the system was concentrated directly to obtain compound 7e (200 mg, 0.48 mmol) with a yield of 82.3%.

MS m/z (ESI): 422 [M+H]⁺.

### Sixth step: preparation of phenyl (3-methoxy-1,2,4-thiadiazole-5-yl)carbamate 7h

Compound **7f** (1.2 g, 7.63 mmol), compound **7g** (1.0 g, 7.63mmol) and THF (20 mL) were added into a reaction flask, and then triethylamine (1.5g, 15.26mmol) was slowly added dropwise. The system was reacted for 1h at room temperature. After the reaction was completed, the system was concentrated directly and purified by column chromatography (v/v, DCM/MeOH=100:1-50:1) to obtain compound **7h** (90 mg, 0.36 mmol) with a yield of 4.7%.

MS m/z (ESI): 252 [M+H]⁺.

### Seventh step: preparation of (3aR,5s,6aS)-5-((5-cyano-1-(phenylsulfonyl)-1H-pyrrolo [2,3-b]pyridin-4-yl)(methyl)amino)-N-(3-methoxy-1,2,4-thiadiazole-5-yl)hexahydrocyclopentadi ene[c]pyrrole-2(1H)-carboxamide 7i

Compound 7e (50 mg, 0.12 mmol), compound **7h** (30 mg, 0.12 mmol), DIPEA (31mg, 0.24mmol) and THF (10 mL) were added into a reaction flask. The system was reacted for 2h at 60 °C. After the reaction was completed, the system was concentrated directly and purified by column chromatography (v/v, DCM/MeOH=100:1-50:1) to obtain compound 7i (60 mg, 0.10 mmol) with a yield of 83.3%.

MS m/z (ESI): 579 [M+H]⁺.

### Eighth step: preparation of (3aR,5s,6aS)-5-((5-cyano-1H-pyrrolo[2,3-b]pyridin-4-yl)(methyl)amino)-N-(3-methoxy-1,2,4-th iadiazole-5-yl)hexahydrocyclopentadiene[c]pyrrole-2(1H)-carboxamide H7

Compound **7i** (60 mg, 0.10 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H7** (25 mg, 0.057 mmol) with a yield of 57.0%.

MS m/z (ESI): 439 [M+H]⁺.

1H NMR (400 MHz, DMSO-d6) δ 11.93 (s, 1H), 11.57 (s, 1H), 8.15 (s, 1H), 7.31 - 7.24 (m, 1H), 6.68 - 6.61 (m, 1H), 4.79 - 4.68 (m, 1H), 3.89 (s, 3H), 3.73 - 3.60 (m, 2H), 3.19 (s, 3H), 3.17 - 3.15 (m, 2H), 2.96 - 2.79 (m, 2H), 2.21 - 2.08 (m, 2H), 1.93 - 1.80 (m, 2H).

### Example 08

### (3aR,5s,6aS)-4-({2-[(3-ethyl-1,2,4-thiadiazole-5-yl)carbamoyl]-hexahydrocyclopenta[c] pyrrole-5-yl}-methyl-amino)-1H-pyrrolo[2,3-b]pyridin-5-carbonitrile H08

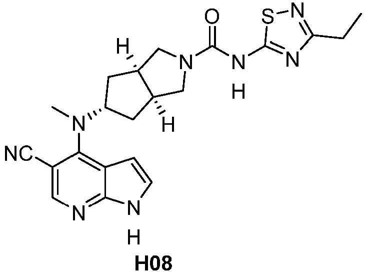

Ethyl 3-ethyl-5-amino-1,2,4-thiadiazole was used as a raw material to obtain compound **H08** by using the same preparation method as described in Example 7. LCMS (ESI) m/z: 437 [M+H]⁺.

¹H NMR (400 MHz, DMSO-*d*₆) δ 11.89 (s, 1H), 11.42 (s, 1H), 8.12 (s, 1H), 7.26 - 7.20 (m, 1H), 6.64 - 6.58 (m, 1H), 4.76 - 4.65 (m, 1H), 3.73 - 3.58 (m, 2H), 3.35 - 3.29 (m, 2H), 3.16 (s, 3H), 2.92 - 2.78 (m, 2H), 2.69 (q, J = 7.5 Hz, 2H), 2.18 - 2.05 (m, 2H), 1.89 - 1.78 (m, 2H), 1.20 (t, J = 7.6 Hz, 3H).

### Example 09

### Preparation of 4-{[3-(3-(trifluoromethyl)benzenesulfonamido)-cyclobutyl-1-yl]-methyl -amino}-1H-pyrrolo[2,3-b]pyridin-5-carbonitrile H09

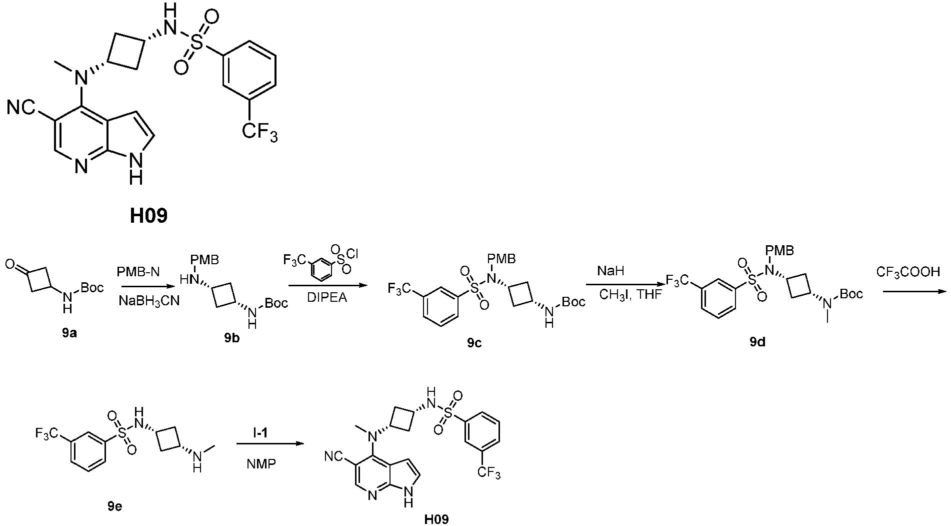

### (1) Preparation of tert-butyl (3-((4-methoxybenzyl)amino)cyclobutyl)carbamate 9b

Compound tert-butyl (3-oxocyclobutyl)carbamate **9a** (1 g, 5.4 mmol), 4-methoxybenzylamine (0.74 g, 5.4 mmol), sodium cyanoborohydride (0.68 g, 10.8 mmol) and 50 mL methanol were added into a reaction flask, stirred for 4h at room temperature and concentrated to obtain crude products. The crude products were purified by column chromatography (DCM: MeOH=10:1) to obtain white solids, compound **9b** tert-butyl (3-((4-methoxybenzyl)amino)cyclobutyl)carbamate (1.3 g) with a yield of 77%. MS m/z (ESI): 307 [M+H]⁺.

### (2) Preparation of tert-butyl (3-((N-(4-methoxybenzyl)-3-(trifluoromethyl)phenyl)sulfonamido)cyclobutyl)carbamate 9c

Tert-butyl (3-((4-methoxybenzyl)amino)cyclobutyl)carbamate **9b** (250 mg, 0.81 mmol) and DIPEA (313 mg, 2.43 mmol) were dissolved in DCM, and then 3-(trifluoromethyl)phenylsulfonyl chloride (200 mg, 0.81 mmol) was added dropwise. After the addition was completed, this mixture was stirred for 4h at room temperature, quenched with water, and extracted with DCM. The organic phases were combined, washed with saturated sodium chloride, dried with anhydrous sodium sulfate, filtered and concentrated to obtain crude products. The crude products were purified by column chromatography (PE: EA=3:1) to obtain 200 mg of compound **9c** tert-butyl (3-((N-(4-methoxybenzyl)-3-(trifluoromethyl)phenyl)sulfonamido)cyclobutyl)carbamate, which was present as a colorless oily substance, with a yield of 48%.

MS m/z (ESI): 515 [M+H]⁺

### (3) Preparation of tert-butyl (3-((N-(4-methoxybenzyl)-3-(trifluoromethyl)phenyl) sulfonamido)cyclobutyl)(methyl)carbamate 9d

Sodium hydride (7.4 mg, 0.42 mmol) was added into tert-butyl (3-((N-(4-methoxybenzyl)-3-(trifluoromethyl)phenyl)sulfonamido)cyclobutyl)carbamate 9c (110 mg, 0.21 mmol) in THF solution, stirred for 1h at room temperature, and then iodomethane (60 mg, 0.42 mmol) was added dropwise. This reaction mixture was stirred for 6h at room temperature, quenched with water and extracted with ethyl acetate. The organic phases were combined, washed with saturated sodium chloride, dried with anhydrous sodium sulfate, filtered and concentrated to obtain crude products tert-butyl (3-((N-(4-methoxybenzyl)-3-(trifluoromethyl)phenyl)sulfonamido)cyclobutyl)(methyl)carbamat e 9d (100 mg), which was present as a colorless oily substance. The crude products were directly used for a reaction of next step.

MS m/z (ESI): 551 [M+Na]

### (4) Preparation of N-(3-(methylamino)cyclobutyl)-3-(trifluoromethyl)benzsulfamide 9e

Tert-butyl (3-((N-(4-methoxybenzyl)-3-(trifluoromethyl)phenyl)sulfonamido)cyclobutyl)(methyl)carbamat e **9d** (100 mg, 0.19 mmol) was dissolved in 2 mL THF, and then trifluoroacetic acid (2 mL) was added. This mixture was stirred for 2h at room temperature, concentrated and dissolved in DCM again, neutralized with saturated sodium bicarbonate solution. The organic phases were washed with saturated sodium chloride, dried with anhydrous sodium sulfate, filtered and concentrated to obtain crude products. The crude products N-(3-(methylamino)cyclobutyl)-3-(trifluoromethyl)benzsulfamide **9e** (50 mg) were directly used for a reaction of next step.

MS m/z (ESI): 309 [M+H]⁺

### (5) N-(3-((5-cyano-1H-pyrrolo[2,3-b]pyridin-4-yl)(methyl)amino)cyclobutyl) -3-(trifluoromethyl)benzsulfamide H09

N-(3-(methylamino)cyclobutyl)-3-(trifluoromethyl)benzsulfamide **9e** (50 mg, 0.16 mmol), 4-chloro-1-(phenylsulfonyl)-1H-pyrrolo[2,3-b]pyridin-5-carbonitrile **I-1** (50 mg, 0.16 mmol) and DIPEA (62 mg, 0.48 mmol) were dissolved in NMP. This mixture was subjected to a microwave reaction for 2h at 170 °C, cooled, quenched with water and extracted with ethyl acetate. The organic phases were combined, washed with saturated sodium chloride, dried with anhydrous sodium sulfate, filtered and concentrated to obtain crude products. The crude products were purified by column chromatography (DCM: MeOH=10:1) to obtain N-(3-((5-cyano-1H-pyrrolo[2,3-b]pyridin-4-yl)(methyl)amino)cyclobutyl)-3-(trifluoromethyl)benzsulfamide **H09** (9.7 mg) with a yield of 13.5%.

MS m/z (ESI): 450 [M+H]⁺

¹H-NMR (400 MHz, Methanol-d4) δ 8.12 - 8.08 (m, 3H), 7.99 - 7.90 (m, 2H), 7.77 (t, J = 7.9 Hz, 1H), 7.22 (d, J = 3.7 Hz, 1H), 6.61 (d, J = 3.7 Hz, 1H), 4.27 (tt, J = 9.1, 7.0 Hz, 1H), 3.64 - 3.53 (m, 1H), 3.24 (s, 3H), 2.53 (dtd, J = 9.8, 7.1, 2.9 Hz, 2H), 2.07 (qd, J = 9.0, 2.8 Hz, 2H).

### Example 10

### Preparation of 4-{[3-(3,3,3-trifluoropropanesulfonamido)-cyclobutyl-1-yl]-methyl-amino} -1H-pyrrolo[2,3-b]pyridin-5-carbonitrile H10

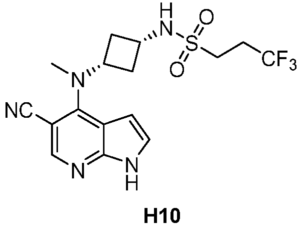

3,3,3-trifluoropropanesulfonyl chloride was used as a raw material to obtain compound **H10** by using the same preparation method as described in Example 9. LCMS (ESI) m/z: 388 [M+H]⁺.

¹H NMR (400 MHz, DMSO-d6) δ 11.93 (s, 1H), 8.15 (s, 1H), 7.76 (d, J = 8.9 Hz, 1H), 7.32 (t, J = 3.1 Hz, 1H), 6.64 (dd, J = 3.7, 2.0 Hz, 1H), 4.24 (p, J = 8.4 Hz, 1H), 3.55 (h, J = 8.6 Hz, 1H), 3.23 (s, 3H), 3.20 (dd, J = 7.2, 3.8 Hz, 2H), 2.64 (dt, J = 10.8, 7.5 Hz, 4H), 2.25 (dt, J = 11.0, 8.6 Hz, 2H).

### Example 11

### Preparation of 4-{[(3R,4R)-1-(2-cyanoacetyl)-4-methylpiperidin-3-yl]-methyl-amino}-1H-pyrrolo[2,3-b]pyridin-5-carbonitrile H11

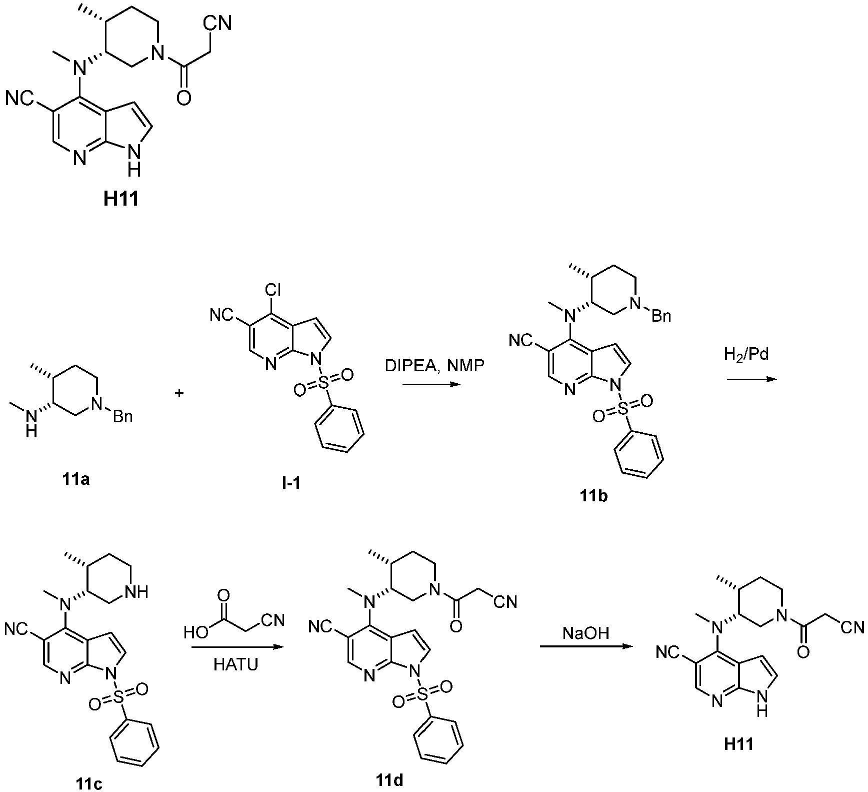

### First step: preparation of 1-benzenesulfonyl-4-[((3R,4R)-1-benzyl-4-methylpiperidin-3-yl) -methyl-amino}-1H-pyrrolo[2,3-b]pyridin-5-carbonitrile 11b

Compound **11a** (293 mg, 1.35 mmol) and N-methyl pyrrolidone (2 mL) were added into a reaction flask, and then intermediates **I-1** (429 mg, 1.35 mmol) and N-isopropyldiethylamine (348 mg, 2.70 mmol) were added. The system was subjected to a microwave reaction for 1h at 170 °C. After the reaction was completed under TLC monitoring, the system was extracted with ethyl acetate and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **11b** (150 mg, 0.30 mmol) with a yield of 22.2%. LCMS (ESI) m/z: 500 [M+H]⁺.

### Second step: preparation of 1-benzenesulfonyl-4-[((3R,4R)-4-methylpiperidin-3-yl) -methyl-amino}-1H-pyrrolo[2,3-b]pyridin-5-carbonitrile 11c

Compound **11b** (150 mg, 0.30 mmol) and methanol (10 mL) were added into a reaction flask, and then palladium on carbon was added (30 mg). The system was stirred for 5h under the protection of hydrogen balloon. After the reaction was completed under TLC monitoring, the system was filtered with kieselguhr, dried by a spin drier (10-15 °C) to obtain the titled compound **11c** (100 mg, 0.24 mmol) with a yield of 80.0%. LCMS (ESI) m/z: 410 [M+H]⁺.

### Third step: preparation of 1-benzenesulfonyl-4-{[(3R,4R)-1-(2-cyanoacetyl) -4-methylpiperidin-3-yl]-methyl-amino}-1H-pyrrolo[2,3-b]pyridin-5-carbonitrile 11d

Compound **11c** (100 mg, 0.24 mol) and dichloromethane (30 mL) were added into a reaction flask, and then triethylamine (24 mg, 0.24 mmol), cyanoacetic acid (20 mg, 0.24 mmol) and HATU (91 mg, 0.24 mmol) were added, the system was stirred for 2h at room temperature. After the reaction was completed under TLC monitoring, the system was extracted with dichloromethane and water. The organic phase was washed with water and saturated sodium chloride, dried with anhydrous sodium sulfate and concentrated, followed by purification by column chromatography (v/v, petroleum ether/ethyl acetate=4:1-2:1) to obtain the titled compound **11d** (50 mg, 0.11 mmol) with a yield of 43.8%. LCMS (ESI) m/z: 477 [M+H]⁺.

### Fourth step: preparation of 4-{[(3R,4R)-1-(2-cyanoacetyl)-4-methylpiperidin-3-yl] -methyl-amino}-1H-pyrrolo[2,3-b]pyridin-5-carbonitrile H11

Methanol (1 mL) was added into a reaction flask, and then compound **11d** (50.0 mg, 0.11 mmol) and sodium hydroxide solution (0.15 mL, 0.15 mmol, 1 mol/L) were added separately. The reaction system was stirred for 12h at room temperature. After the reaction was completed under TLC monitoring, the reaction system was extracted with ethyl acetate and water for three times. The organic phases were combined, washed with water and saturated sodium chloride, dried with anhydrous sodium sulfate and concentrated to obtain crude products. The crude products were purified by preparative TLC to obtain the titled compound **H11** (30 mg, 0.089 mmol) with a yield of 80.9%. LCMS (ESI) m/z: 337 [M+H]⁺.

¹H NMR (400 MHz, DMSO-*d*₆) δ 11.40 (s, 1H), 7.91 (s, 1H), 7.10 (s, 1H), 6.83 (s, 1H), 3.88 - 3.75 (m, 1H), 3.62 - 3.50 (m, 1H), 3.35 - 3.31 (m, 1H), 3.29 (s, 3H), 3.25 - 3.19 (m, 1H), 3.14 - 3.04 (m, 1H), 2.67 (s, 2H), 1.56 - 1.46 (m, 1H), 1.19 (s, 2H), 0.97 (d, *J* = 7.1 Hz, 3H).

### Example 12

### Preparation of (cis)-4-{[3-(3,3,3-trifluoropropanesulfonamido)-cyclobutyl-1-yl]-amino} -1H-pyrrolo[2,3-b]pyridin-5-carbonitrile H12

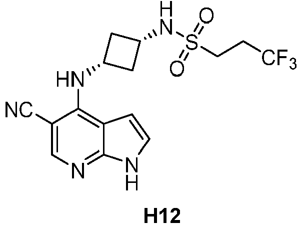

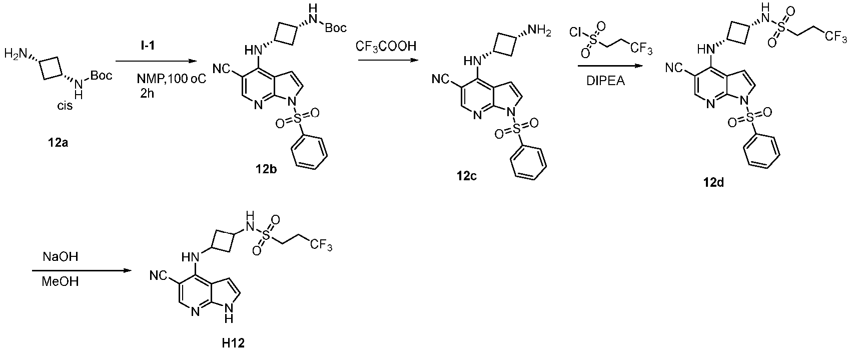

First step: preparation of tert-butyl (3-((5-cyano-1-(benzenesulfonyl)-1H-pyrrole[2,3-b] pyridin-4-yl)amino)cyclobutyl)carbamate**12b**

(Cis)-tert-butyl(3-aminocyclobutyl)carbamate **12a** (200 mg, 1.07 mmol), 4-chloro-1-(phenylsulfonyl)-1H-pyrrolo[2,3-b]pyridin-5-carbonitrile **I-1** (340 mg, 1.07 mmol) and DIPEA (414 mg, 3.21 mmol) were dissolved in NMP. This mixture was reacted at 100 °C for 2h, cooled, quenched with water to precipitate taupe solids, filtered and dried to obtain compound tert-butyl (3-((5-cyano-1-(benzenesulfonyl)-1H-pyrrole[2,3-b]pyridin-4-yl)amino)cyclobutyl) carbamate **12b** (400 mg) with a yield of 80%.

MS m/z (ESI): 468 [M+H]⁺

### Second step: preparation of 4-((3-aminocyclobutyl)amino)-1-(phenylsulfonyl) -1H-pyrrolo[2,3-b]pyridin-5-carbonitrile 12c

Tert-butyl (3-((5-cyano-1-(benzenesulfonyl)-1H-pyrrole[2,3-b]pyridine-4-yl)amino) cyclobutyl)carbamate **12b** (400 mg, 0.85 mmol) was dissolved in 2 mL THF, and then trifluoroacetic acid (2 mL) was added. This mixture was stirred for 2h at room temperature, concentrated and dissolved in DCM again, neutralized with saturated sodium bicarbonate solution. The organic phase was washed with saturated sodium chloride, dried with anhydrous sodium sulfate, filtered and concentrated to obtain crude products. The crude products 4-((3-aminocyclobutyl)amino)-1-(phenylsulfonyl)-1H-pyrrolo[2,3-b]pyridin-5-carbonitrile **12c** (250 mg) were directly used for a reaction of next step. The yield was 80%.

### MS m/z (ESI): 368 [M+H]⁺

### Third step: preparation of N-(3-((5-cyano-1-(phenylsulfonyl)-1H-pyrrolo[2,3-b] pyridin-4-yl)amino)cyclobutyl)-3,3,3-trifluoropropane-1-sulfamide 12d

4-((3-aminocyclobutyl)amino)-1-(phenylsulfonyl)-1H-pyrrolo[2,3-b]pyridin-5-carbonitrile **12c** (100 mg, 0.27 mmol) and DIPEA (104 mg, 0.81 mmol) were dissolved in DCM, and 3,3,3-trifluoropropane-1-sulfonyl chloride (80 mg, 0.4 mmol) was added slowly at room temperature. After the addition was completed, the mixture was stirred for 2h at room temperature, quenched with water, extracted with DCM. The organic phases were combined, washed with saturated sodium chloride, dried with anhydrous sodium sulfate, filtered and concentrated to obtain crude products (**4**) N-(3-((5-cyano-1-(phenylsulfonyl)-1H-pyrrolo[2,3-b] pyridin-4-yl) amino)cyclobutyl)-3,3,3-trifluoropropane-1-sulfamide **12d** (100 mg) for a reaction of next step.

MS m/z (ESI): 528 [M+H]⁺

### Fourth step: preparation of N-(3-((5-cyano-1H-pyrrolo[2,3-b]pyridin-4-yl) amino)cyclobutyl)-3,3,3-trifluoropropane-1-sulfamide H12

N-(3-((5-cyano-1-(phenylsulfonyl)-1H-pyrrolo[2,3-b]pyridin-4-yl)amino)cyclobutyl)-3,3,3-trifluoropropane-1-sulfamide **12d** (100 mg, 0.19 mmol) was dissolved in methanol (4 mL), and then 2 mL of 2 N sodium hydroxide solution was added. This mixture was stirred for 2h at room temperature, extracted with ethyl acetate. The organic phases were combined, washed with saturated sodium chloride, dried with anhydrous sodium sulfate, filtered and concentrated to obtain crude products. The crude products were purified by column chromatography (DCM: MeOH=10:1) to obtain 36 mg N-(3-((5-cyano-1H-pyrrolo[2,3-b]pyridin-4-yl) amino)cyclobutyl)-3,3,3-trifluoropropane-1-sulfamide **H12,** which was present as white solids, with a yield of 48%.

MS m/z (ESI): 388 [M+H]⁺
¹H NMR (400 MHz, DMSO-d6) δ 11.77 (s, 1H), 8.03 (s, 1H), 7.75 (d, J = 7.6 Hz, 1H), 7.21 (t, J = 2.9 Hz, 1H), 7.09 (d, J = 6.9 Hz, 1H), 6.76 (dd, J = 3.7, 1.9 Hz, 1H), 4.19 (p, J = 7.5 Hz, 1H), 3.59 (q, J = 8.0 Hz, 1H), 3.25 - 3.16 (m, 2H), 2.77 (tt, J = 8.8, 4.7 Hz, 2H), 2.69 - 2.58 (m, 2H), 2.14 (dt, J = 11.0, 8.4 Hz, 2H).

### Example 13

### Preparation of (cis)-4-{[3-(3-(trifluoromethyl)benzenesulfonamido)-cyclobutyl-1-yl] -amino}-1H-pyrrolo[2,3-b]pyridin-5-carbonitrile H13

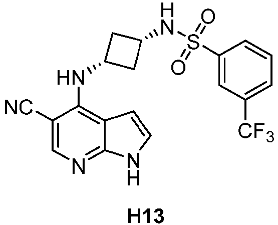

3-trifluoromethylbenzenesulfonyl chloride was used as a raw material to obtain compound **H13** by using the same preparation method as described in Example **12.** LCMS (ESI) m/z: 436 [M+H]⁺.

¹H NMR (400 MHz, Methanol-d4) δ 8.16 - 8.10 (m, 2H), 8.00 (s, 1H), 7.95 - 7.91 (m, 1H), 7.82 - 7.75 (m, 1H), 7.14 (d, J = 3.7 Hz, 1H), 6.66 (d, J = 3.6 Hz, 1H), 4.36 - 4.24 (m, 1H), 3.64 (tt, J = 8.9, 7.3 Hz, 1H), 2.69 (dddd, J = 9.2, 7.2, 4.6, 2.9 Hz, 2H), 1.94 (qd, J = 8.9, 2.9 Hz, 2H).

### Example 14

### Preparation of (cis)-4-{[3-(propanesulfonamido)-cyclobutyl-1-yl]-methyl-amino}-1H-pyrrolo[2,3-b]pyridin-5-carbonitrile H14

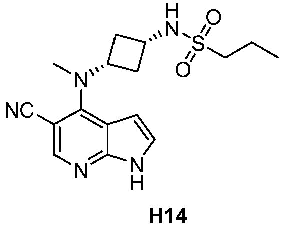

Propanesulfonyl chloride was used as a raw material to obtain compound **H14** by using the same preparation method as described in Example 9. LCMS (ESI) m/z: 348 [M+H]⁺.

¹H NMR (400 MHz, DMSO-d6) δ 11.96 (s, 1H), 8.14 (s, 1H), 7.47 (s, 1H), 7.31 (d, J = 3.6 Hz, 1H), 6.64 (d, J = 3.6 Hz, 1H), 4.28 - 4.18 (m, 1H), 3.48 (s, 1H), 2.93 - 2.84 (m, 2H), 2.68 - 2.58 (m, 2H), 2.24 (qd, J = 9.0, 2.8 Hz, 2H), 1.68 - 1.56 (m, 2H), 0.94 (t, J = 7.4 Hz, 3H).

### Example 15

### Preparation of (cis)-4-{[3-(3,3,3-trifluoropropanesulfonamide)-cyclobutyl-1-yl]-methyl -amino}-1H-pyrrolo[2,3-b]pyridin-5-carbonitrile H15

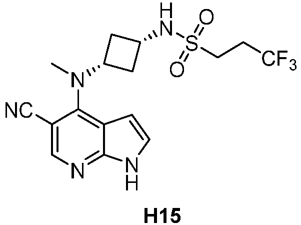

3,3,3-trifluoropropanesulfonyl chloride was used as a raw material to obtain compound **H15** by using the same preparation method as described in Example **9.**

LCMS (ESI) m/z: 402 [M+H]⁺.

¹H NMR (400 MHz, DMSO-d6) δ 11.93 (s, 1H), 8.15 (s, 1H), 7.76 (d, J = 8.9 Hz, 1H), 7.32 (t, J = 3.1 Hz, 1H), 6.64 (dd, J = 3.7, 2.0 Hz, 1H), 4.24 (p, J = 8.4 Hz, 1H), 3.55 (h, J = 8.6 Hz, 1H), 3.23 (s, 3H), 3.20 (dd, J = 7.2, 3.8 Hz, 2H), 2.64 (dt, J = 10.8, 7.5 Hz, 4H), 2.25 (dt, J = 11.0, 8.6 Hz, 2H).

### Example 16

### Preparation of (cis)-4-{ [3-(4-trifluoromethyl benzenesulfonamido)-cyclobutyl-1-yl] -methyl-amino}-1H-pyrrolo[2,3-b]pyridin-5-carbonitrile H16

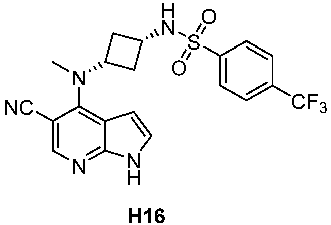

4-trifluoromethylbenzenesulfonyl chloride was used as a raw material to obtain compound **H16** by using the same preparation method as described in Example **9.** LCMS (ESI) m/z: 450 [M+H]⁺.

¹H NMR (400 MHz, DMSO-d6) δ 7.30 (s, 1H), 7.22 (d, J = 8.1 Hz, 2H), 7.06 (d, J = 8.3 Hz, 2H), 6.41 (d, J = 3.7 Hz, 1H), 5.81 (d, J = 3.6 Hz, 1H), 3.50 - 3.40 (m, 1H), 2.77 (p, J = 8.2 Hz, 1H), 2.44 (s, 3H), 1.73 (dtd, J = 9.9, 7.2, 3.0 Hz, 2H), 1.33 - 1.24 (m, 2H).

### Example 17

### Preparation of (cis)-4-{[3-(4-trifluoromethyl benzenesulfonamido)-cyclobutyl-1-yl]-amino} -1H-pyrrolo[2,3-b]pyridin-5-carbonitrile H17

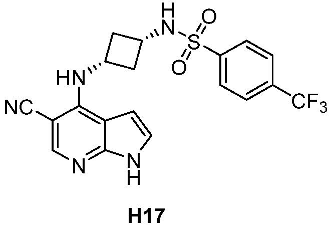

4-trifluoromethylbenzenesulfonyl chloride was used as a raw material to obtain compound **H17** by using the same preparation method as described in Example **12.** LCMS (ESI) m/z: 436 [M+H]⁺.

¹H NMR (400 MHz, Methanol-d4) δ 8.09 - 7.97 (m, 3H), 7.88 (dd, J = 8.7, 2.7 Hz, 2H), 7.13 (t, J = 3.5 Hz, 1H), 6.66 (d, J = 3.6 Hz, 1H), 4.34 - 4.22 (m, 1H), 3.63 (tt, J = 9.0, 7.4 Hz, 1H), 2.75 - 2.65 (m, 2H), 1.96 (qd, J = 8.9, 2.9 Hz, 2H).

### Example 18

### Preparation of (cis)-4-{[3-(benzo[c][1,2,5]oxadiazole-4-sulfonamido)-cyclobutyl-1-yl] -methyl-amino}-1H-pyrrolo[2,3-b]pyridin-5-carbonitrile H18

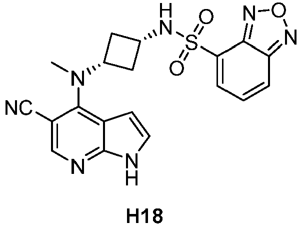

Benzo[c][1,2,5]oxadiazole-4-sulfonyl chloride was used as a raw material to obtain compound **H18** by using the same preparation method as described in Example **9.** LCMS (ESI) m/z: 424 [M+H]⁺.

¹H NMR (400 MHz, DMSO-d6) δ 11.90 (s, 1H), 8.64 (s, 1H), 8.34 (dd, J *=* 9.1, 0.8 Hz, 1H), 8.12 - 8.03 (m, 2H), 7.73 (dd, J = 9.1, 6.8 Hz, 1H), 7.28 (dd, J = 3.7, 2.3 Hz, 1H), 6.55 (dd, J = 3.6, 1.9 Hz, 1H), 4.14 (p, J = 8.5 Hz, 1H), 3.60 (d, J = 8.9 Hz, 1H), 3.13 (s, 3H), 2.37 - 2.29 (m, 2H), 2.17 - 2.01 (m, 2H).

### Example 19

### Preparation of (cis)-4-{[3-(benzo[c][1,2,5]oxadiazole-4-sulfonamido)-cyclobutyl-1-yl] -amino}-1H-pyrrolo[2,3-b]pyridin-5-carbonitrile H19

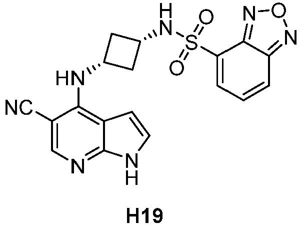

Benzo[c][1,2,5]oxadiazole-4-sulfonyl chloride was used as a raw material to obtain compound **H19** by using the same preparation method as described in Example **12.** LCMS (ESI) m/z: 410 [M+H]⁺.

¹H NMR (400 MHz, Methanol-d4) δ 8.18 (dd, J = 9.1, 0.8 Hz, 1H), 8.10 (dd, J = 6.7, 0.8 Hz, 1H), 7.99 (s, 1H), 7.66 (dd, J = 9.1, 6.7 Hz, 1H), 7.13 (d, J = 3.6 Hz, 1H), 6.64 (d, J = 3.6 Hz, 1H), 4.27 (t, J = 7.7 Hz, 1H), 3.85 - 3.74 (m, 1H), 2.67 (dtd, J = 10.0, 7.3, 3.0 Hz, 2H), 1.97 (td, J = 8.9, 2.9 Hz, 2H).

### Example 20

### Preparation of (3aR,5s,6aS)-5-((5-cyano-1H-pyrrolo[2,3-b]pyridin-4-yl)amino)-N-(3-methoxy-1,2,4- thiadiazole-5-yl)hexahydrocyclopentadiene[c]pyrrole-2(1H)-carboxamide H20

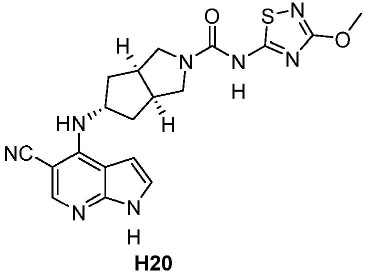

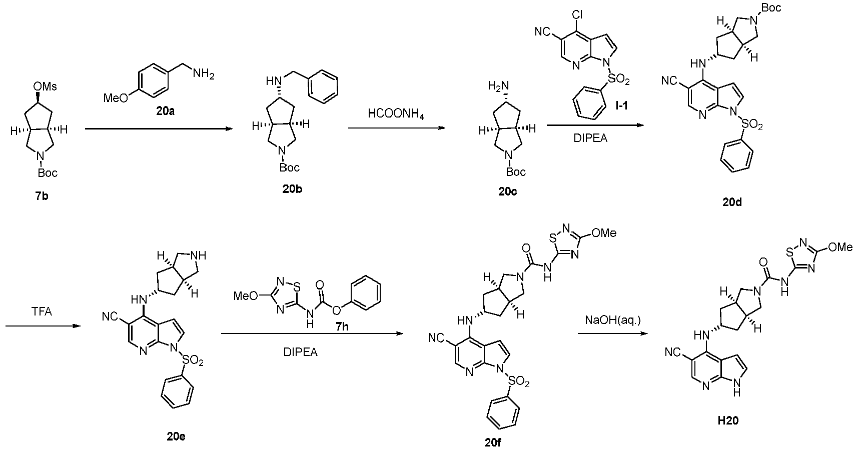

### First step: preparation of tert-butyl (3aR,5s,6aS)-5-(benzylamino) hexahydrocyclopentadiene[c]pyrrole-2(1H)-carboxylate 20b

Compound **7b** (500 mg, 1.6 mmol) and compound **20a** (0.5mL, 4.9 mmol) were added into a reaction flask and heated to 80°C for reacting under a solvent-free condition. After the reaction was completed under TLC monitoring, the system was purified by column chromatography (v/v, DCM/MeOH=100:1-50:1) to obtain compound **20b** (400 mg, 1.3 mmol) with a yield of 77.2%.

MS m/z (ESI): 317 [M+H]⁺.

### Second step: preparation of tert-butyl (3aR,5s,6aS)-5-aminohexahydrocyclopentadiene[c] pyrrole-2(1H)-carboxylate 20c

Compound **20b** (350 mg, 1.1 mmol), ammonium formate (1.4g, 22.1 mmol) and Pd/C (0.7 g) were dissolved in ethanol solvent (15mL) in a reaction flask and reacted at 80°C. After the reaction was completed under TLC monitoring, the system was purified by column chromatography (v/v, DCM/MeOH=100:1-50:1) to obtain compound **20c** (150 mg, 0.66 mmol) with a yield of 59.9%.

MS m/z (ESI): 227 [M+H]⁺.

### Third step: preparation of tert-butyl (3aR,5s,6aS)-5-((5-cyano-1-(phenylsulfonyl)-1H -pyrrolo[2,3-b]pyridin-4-yl)amino)hexahydrocyclopentadiene[c]pyrrole-2(1H)-carboxylate 20d

Compound **20c** (150 mg, 0.66 mmol), DIPEA (1.1 mL, 6.6mmol), **I-1** (421 mg, 1.3mmol) and NMP (5 mL) were added into a reaction flask. The system was subjected to a reflux reaction for 1.5h at 140°C. After the reaction was completed, the system was poured into water, extracted with ethyl acetate for three times. The organic phases were combined, washed with water and saturated sodium chloride, dried with anhydrous sodium sulfate, and concentrated at low temperature, followed by purification by column chromatography (v/v, PE/THF=100:1-50:1) to obtain compound **20d** (80 mg, 0.16 mmol) with a yield of 23.8%.

MS m/z (ESI): 508 [M+H]⁺.

### Fourth step: preparation of 4-(((3aR,5s,6aS)-octahydrocyclopentadiene[c] pyrrole-5-yl)amino)-1-(phenylsulfonyl)-1H-pyrrolo[2,3-b]pyridin-5-carbonitrile 20e

Compound **20d** (80 mg, 0.16 mmol), TFA (0.1 mL) and DCM (5 mL) were added into a reaction flask. The system was reacted for 1h at room temperature. After the reaction was completed, the system was concentrated directly to obtain compound **20e** (110 mg, 0.27 mmol).

MS m/z (ESI): 408 [M+H]⁺.

### Fifth step: preparation of (3aR,5s,6aS)-5-((5-cyano-1-(phenylsulfonyl)-1H-pyrrolo[2,3-b] pyridin-4-yl)amino)-N-(3-methoxy-1,2,4-thiadiazole-5-yl)hexahydrocyclopentadiene[c]pyrrole-2 (1H)-carboxamide 20f

Compound **20e** (110 mg, 0.27 mmol), compound **7h** (68 mg, 0.27 mmol), DIPEA (3.0mL) and THF (5 mL) were added into a reaction flask. The system was reacted for 2h at 60 °C. After the reaction was completed, the system was concentrated directly and purified by column chromatography (v/v, PE/THF=100:1-50:1) to obtain compound **20f** (40 mg, 0.07 mmol) with a yield of 26.2%.

MS m/z (ESI): 566 [M+H]⁺.

### Sixth step: preparation of (3aR,5s,6aS)-5-((5-cyano-1H-pyrrolo[2,3-b]pyridin-4-yl) amino)-N-(3-methoxy-1,2,4-thiadiazole-5-yl)hexahydrocyclopentadiene[c]pyrrole-2(1H)-carbox amide H20

Compound **20f** (40 mg, 0.07 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H20** (12 mg, 0.03 mmol) with a yield of 39.9%.

MS m/z (ESI): 425 [M+H]⁺.

¹H NMR (400 MHz, DMSO-d6) δ 11.78 (s, 1H), 11.58 (s, 1H), 8.06 (s, 1H), 7.26 - 7.17 (m, 1H), 6.88 - 6.70 (m, 2H), 4.75 (q, J = 6.9 Hz, 1H), 3.90 (s, 3H), 3.69 (t, J = 9.4 Hz, 2H), 3.31 - 3.35 (m, 2H), 2.91 (m, 2H), 1.99 (d, J = 7.8 Hz, 4H).

### Example 21

### (3aR,5s,6aS)-4-({2-[(3-tert-butyl-1,2,4-thiadiazole-5-yl)carbamoyl]-hexahydrocyclopenta [c]pyrrole-5-yl}-methyl-amino)-1H-pyrrolo[2,3-b]pyridin-5-carbonitrile H21

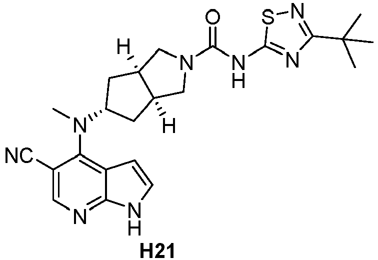

3-tert-butyl-5-amino-1,2,4-thiadiazole was used as a raw material to obtain compound **H21** by using the same preparation method as described in Example **7.**

MS m/z (ESI): 465 [M+H]+.

¹H NMR (400 MHz, DMSO-d₆) δ 11.93 (s, 1H), 11.44 (s, 1H), 8.15 (s, 1H), 7.25 (dd, *J* = 3.6, 2.5 Hz, 1H), 6.64 (dd, *J* = 3.7, 2.0 Hz, 1H), 4.73 (t, *J* = 8.1 Hz, 1H), 3.67 (m, 2H), 3.19 (s, 3H), 2.87 (m, 2H), 2.20 - 2.10 (m, 2H), 1.92 - 1.82 (m, 2H), 1.31 (s, 9H).

### Example 22

### Preparation of (3aR,5s,6aS)-4-({2-[(2,2,2-trifluoroethyl)-carbamoyl]-hexahydrocyclopenta[c]pyrrole-5-yl}-methyl-amino)-1H-pyrrolo[2,3-b]pyridin-5-carbonitrile H22

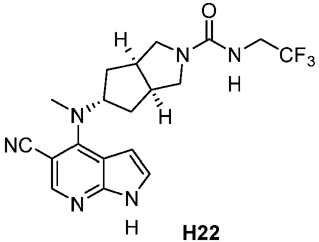

2,2,2-trifluoroethylamine was used as a raw material to obtain compound **H22** by using the same preparation method as described in Example **7.** LCMS (ESI) m/z: 407 [M+H]+.

¹H NMR (400 MHz, DMSO-*d*₆) δ 11.90 (s, 1H), 8.12 (s, 1H), 7.30 - 7.25 (m, 1H), 6.80 - 6.73 (m, 1H), 6.63 - 6.58 (m, 1H), 4.75 - 4.61 (m, 1H), 3.80 - 3.67 (m, 2H), 3.54 - 3.44 (m, 2H), 3.15 (s, 3H), 3.09 - 3.01 (m, 2H), 2.83 - 2.72 (m, 2H), 2.15 - 2.02 (m, 2H), 1.84 - 1.74 (m, 2H).

### Example 23

### Preparation of (3aR,5S,6aS)-4-{[2-((2S)-2-hydroxypropionyl)-hexahydrocyclopenta[c] pyrrole-5-yl]-methyl-amino}-1H-pyrrolo[2,3-b]pyridin-5-carbonitrile H23

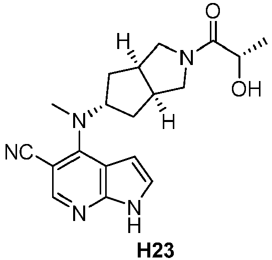

Compound **7e** was used as a raw material to obtain compound **H23** by using the same preparation method as described in Example **6.** LCMS (ESI) m/z: 354 [M+H]+.

¹H NMR (400 MHz, DMSO-*d*₆) δ 11.91 (s, 1H), 8.13 (s, 1H), 7.29 (s, 1H), 6.62 (s, 1H), 4.74 (t, *J* = 7.2 Hz, 1H), 4.70 - 4.60 (m, 1H), 4.26 - 4.17 (m, 1H), 3.80 - 3.48 (m, 2H), 3.28 - 3.19 (m, 1H), 3.16 (s, 3H), 2.91 - 2.67 (m, 2H), 2.16 - 2.02 (m, 2H), 1.88 - 1.73 (m, 2H), 1.17 - 1.03 (m, 3H).

### Example 24

### Preparation of 4-(methyl((3aR,5s,6aS)-2-((3-(trifluoromethyl)phenyl)sulfonyl) octahydrocyclopentadiene[c]pyrrole-5-yl)amino-1H-pyrrolo[2,3-b]pyridin-5-carbonitrile H24

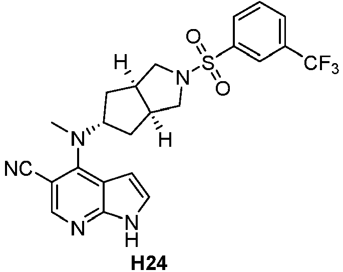

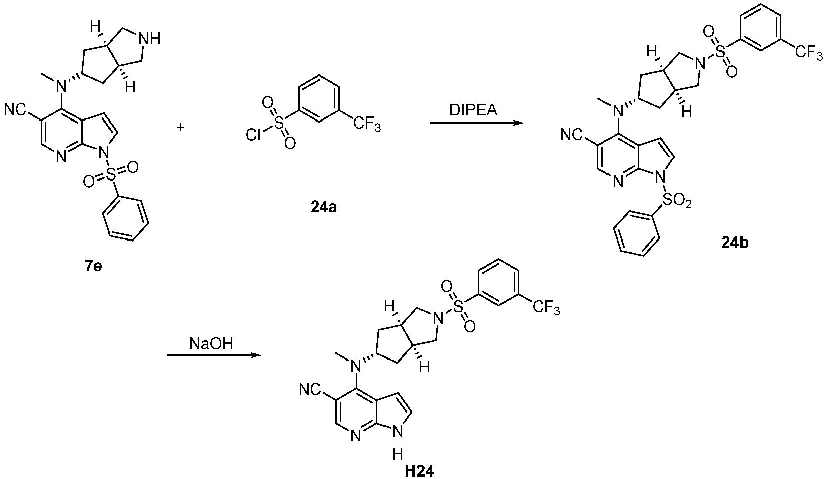

### First step: preparation of 4-(methyl((3aR,5s,6aS)-2-((3-(trifluoromethyl)phenyl)sulfonyl) octahydrocyclopentadiene[c]pyrrole-5-yl)amino-1-(phenylsulfonyl)-1H-pyrrolo[2,3-b]pyridin-5-carbonitrile 24b

Compound **7e** (50 mg, 0.12 mmol), compound **24a** (30 mg, 0.12 mmol), DIPEA (31mg, 0.24mmol) and DCM (10 mL) were added into a reaction flask. The system was reacted for 2h at room temperature. After the reaction was completed, the system was concentrated directly and purified by column chromatography (v/v, DCM/MeOH=100:1-50:1) to obtain compound **24b** (60 mg, 0.095 mmol) with a yield of 79.5%.

MS m/z (ESI): 630 [M+H]+.

### Second step: preparation of 4-(methyl((3aR,5s,6aS)-2-((3-(trifluoromethyl)phenyl)sulfonyl) octahydrocyclopentadiene[c]pyrrole-5-yl)amino-1H-pyrrolo[2,3-b]pyridin-5-carbonitrile H24

Compound **24b** (60 mg, 0.095 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H24** (25 mg, 0.051 mmol) with a yield of 53.8%.

MS m/z (ESI): 490 [M+H]+.

¹H NMR (400 MHz, DMSO-*d*₆) δ 11.92 (s, 1H), 8.16 - 8.03 (m, 3H), 7.94 (s, 1H), 7.90 - 7.82 (m, 1H), 7.30 - 7.24 (m, 1H), 6.55 (dd, *J* = 3.6, 2.0 Hz, 1H), 4.42 - 4.32 (m, 1H), 3.28 - 3.22 (m, 2H), 3.09 (s, 3H), 2.90 - 2.79 (m, 2H), 2.71 - 2.62 (m, 2H), 2.05 - 1.94 (m, 2H), 1.70 - 1.57 (m, 2H).

### Example 25

### Preparation of (3aR,5s,6aS)-N-(4-chloro-3-(trifluoromethyl)phenyl)-5-((5-cyano-1H -pyrrolo[2,3-b]pyridin-4-yl)(methyl)amino)hexahydrocyclopentadiene[c]pyrrole-2(1H)-carboxa mide H25

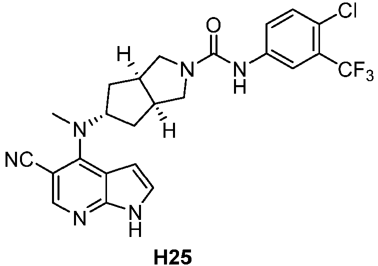

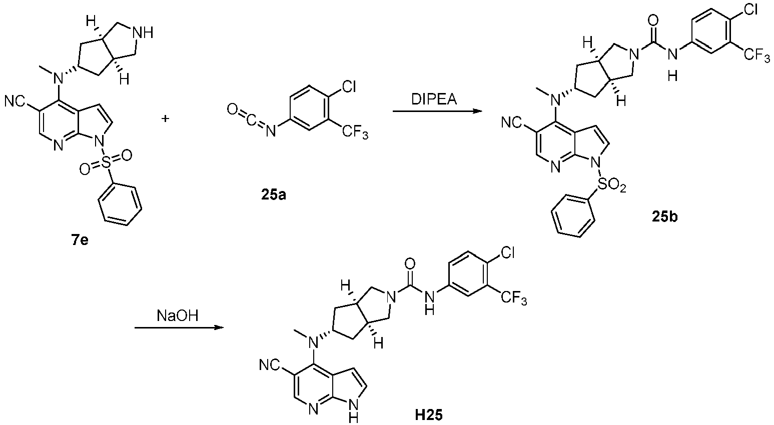

### First step: preparation of (3aR,5s,6aS)-N-(4-chloro-3-(trifluoromethyl) phenyl)-5-((5-cyano-1-(phenylsulfonyl)-1H-pyrrolo[2,3-b]pyridin-4-yl)(methyl)amino)hexahydr ocyclopentadiene[c]pyrrole-2(1H)-carboxamide 25b

Compound **7e** (50 mg, 0.12 mmol), compound **25a** (27 mg, 0.12 mmol), DIPEA (31mg, 0.24mmol) and DCM (10 mL) were added into a reaction flask. The system was reacted for 2h at room temperature. After the reaction was completed, the system was concentrated directly and purified by column chromatography (v/v, DCM/MeOH=100:1-50:1) to obtain compound **24b** (50 mg, 0.078 mmol) with a yield of 64.8%.

MS m/z (ESI): 643 [M+H]+.

### Second step: preparation of (3aR,5s,6aS)-N-(4-chloro-3-(trifluoromethyl)phenyl)-5 -((5-cyano-1H-pyrrolo[2,3-b]pyridin-4-yl)(methyl)amino)hexahydrocyclopentadiene[c]pyrrole-2 (1H)-carboxamide H25

Compound **25b** (50 mg, 0.078 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H25** (20 mg, 0.040 mmol) with a yield of 51.1%.

MS m/z (ESI): 503 [M+H]+.

¹H NMR (400 MHz, DMSO-*d*₆) δ 11.90 (s, 1H), 8.60 (s, 1H), 8.12 (s, 1H), 8.08 (d, *J* = 2.4 Hz, 1H), 7.83 - 7.77 (m, 1H), 7.52 (d, *J* = 8.9 Hz, 1H), 7.28 - 7.22 (m, 1H), 6.68 - 6.60 (m, 1H), 4.77 - 4.68 (m, 1H), 3.68 - 3.57 (m, 2H), 3.26 - 3.21 (m, 2H), 3.17 (s, 3H), 2.89 - 2.77 (m, 2H), 2.17 - 2.08 (m, 2H), 1.89 - 1.80 (m, 2H).

### Example 26

### Preparation of (3aR,5s,6aS)-5-((5-cyano-1H-pyrrolo[2,3-b]pyridin-4-yl)(methyl)amino)-N-(3-cyclopropyl-1,2,4 - thiadiazole-5-yl)hexahydrocyclopentadiene[c]pyrrole-2(1H)-carboxamide H26

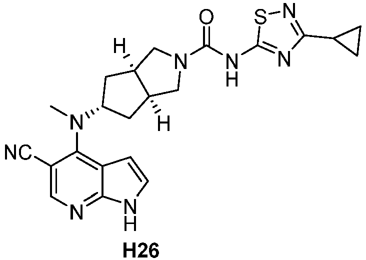

3-cyclopropyl-5-amino-1,2,4-thiadiazole was used as a raw material to obtain compound **H26** by using the same preparation method as described in Example **7.**

MS m/z (ESI): 449 [M+H]+.

¹H NMR (400 MHz, DMSO-*d*₆) δ 11.95 (s, 1H), 11.45 (s, 1H), 8.15 (s, 1H), 7.31 - 7.24 (m, 1H), 6.64 (dd, *J* = 3.5, 1.8 Hz, 1H), 4.78 - 4.68 (m, 1H), 3.74 - 3.61 (m, 2H), 3.34 - 3.28 (m, 2H), 3.19 (s, 3H), 2.95 - 2.78 (m, 2H), 2.20 - 2.05 (m, 3H), 1.91 - 1.81 (m, 2H), 1.00 - 0.85 (m, 4H).

### Example 27

### Preparation of 4-(methyl((3aR,5s,6aS)-2-((3,3,3-trifluoropropyl)sulfonyl) octahydrocyclopentadiene[c]pyrrole-5-yl)amino-1H-pyrrolo[2,3-b]pyridin-5-carbonitrile H27

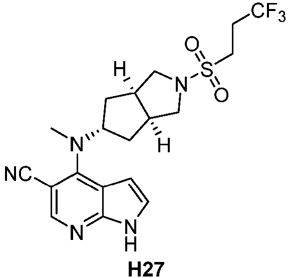

3,3,3-trifluoropropanesulfonyl chloride was used as a raw material to obtain compound **H27** by using the same preparation method as described in Example **24.**

MS m/z (ESI): 442 [M+H]+.

¹H NMR (400 MHz, DMSO-*d*₆) δ 11.92 (s, 1H), 8.13 (s, 1H), 7.33 - 7.26 (m, 1H), 6.65 (dd, *J* = 3.4, 2.0 Hz, 1H), 4.70 - 4.61 (m, 1H), 3.50 - 3.42 (m, 2H), 3.36 - 3.30 (m, 2H), 3.15 (s, 3H), 3.09 - 3.02 (m, 2H), 2.88 - 2.79 (m, 2H), 2.72 - 2.62 (m, 2H), 2.15 - 2.02 (m, 2H), 1.83 - 1.72 (m, 2H).

### Example 28

### Preparation of 4-(((3aR,5s,6aS)-2-((3-fluorophenyl)sulfonyl)octahydrocyclopentadiene[c] pyrrole-5-yl)(methyl)amino-1H-pyrrolo[2,3-b]pyridin-5-carbonitrile H28

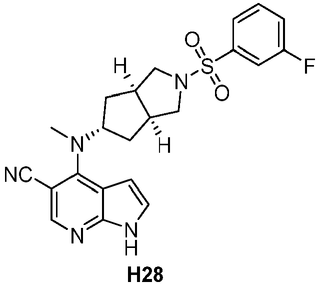

3-trifluorobenzenesulfonyl chloride was used as a raw material to obtain compound **H28** by using the same preparation method as described in Example **24.**

MS m/z (ESI): 440 [M+H]+.

¹H NMR (400 MHz, DMSO-*d*₆) δ 11.92 (s, 1H), 8.10 (s, 1H), 7.71 - 7.62 (m, 1H), 7.62 - 7.50 (m, 3H), 7.32 - 7.25 (m, 1H), 6.54 (dd, *J =* 3.6, 2.0 Hz, 1H), 4.42 - 4.32 (m, 1H), 3.28 - 3.22 (m, 2H), 3.09 (s, 3H), 2.87 - 2.75 (m, 2H), 2.72 - 2.60 (m, 2H), 2.06 - 1.92 (m, 2H), 1.68 - 1.54 (m, 2H).

### Example 29

### Preparation of (cis)-1-(3-((5-cyano-1H-pyrrolo[2,3-b]pyridin-4-yl)(methyl)amino) cyclobutyl)-3-(3-methoxy-1,2,4-thiadiazole-5-yl)urea H29

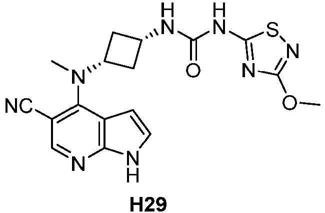

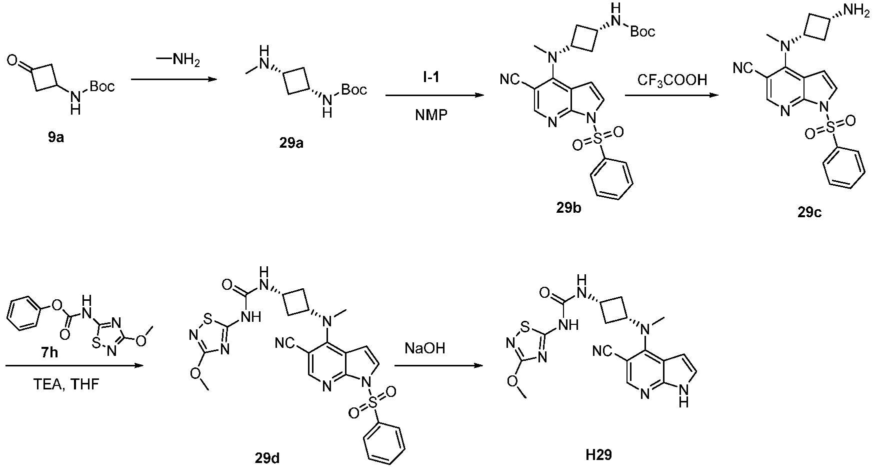

### First step: preparation of tert-butyl(3-(methylamino)cyclobutyl)carbamate 29a

Tert-butyl(3-cyclobutanone)carbamate **9a** (1 g, 5.4 mmol) and methylamine hydrochloride (0.55 g, 8.1 mmol) were dissolved in acetic acid, and then sodium borohydride (0.4 g,10.8 mmol) was added. This mixture was stirred overnight at room temperature, quenched with water, neutralized with saturated sodium bicarbonate aqueous solution, extracted with DCM for 5 times. The organic phases were combined, washed with saturated sodium chloride, dried with anhydrous sodium sulfate, filtered and concentrated to obtain crude products. The crude products were purified by column chromatography (DCM: MeOH=8:1) to obtain tert-butyl(3-(methylamino)cyclobutyl)carbamate 29a (400 mg) with a yield of 37%.

MS m/z (ESI): 201 [M+H]+.

### Second step: preparation of tert-butyl(3-(methylamino)cyclobutyl)carbamate 29b

Tert-butyl(3-(methylamino)cyclobutyl)carbamate **29a** (300 mg, 1.5 mmol), 4-chloro-1-(phenylsulfonyl)-1H-pyrrolo[2,3-b]pyridin-5-carbonitrile **I-1** (475 mg, 1.5 mmol) and DIPEA (580 mg, 4.5 mmol) were dissolved in NMP. This mixture was stirred for 2h at 170°C, cooled, quenched with water and extracted with ethyl acetate. The organic phases were combined, washed with saturated sodium chloride, dried with anhydrous sodium sulfate, filtered and concentrated to obtain crude products. The crude products were purified by column chromatography (PE: EA=1:1) to obtain tert-butyl(3-(methylamino)cyclobutyl)carbamate **29b** (400 mg), which was present as orange solids, with a yield of 55%.

MS m/z (ESI): 482 [M+H]+.

### Third step: preparation of 4-((3-aminocyclobutyl)(methyl)amino) -1-(phenylsulfonyl)-1H-pyrrolo[2,3-b]pyridin-5-carbonitrile 29c

Tert-butyl(3-(methylamino)cyclobutyl) carbamate **29b** (400 mg, 0.83 mmol) was dissolved in 2 mL DCM, and then trifluoroacetic acid (2 mL) was added. This mixture was stirred for 2h at room temperature, concentrated and dissolved in DCM again, neutralized with saturated sodium bicarbonate solution. The organic phases were washed with saturated sodium chloride, dried with anhydrous sodium sulfate, filtered and concentrated to obtain crude products 4-((3-aminocyclobutyl)(methyl)amino)-1-(phenylsulfonyl)-1H-pyrrolo[2,3-b]pyridin-5-carbonitr ile 29c (200 mg) which was directly used for a reaction of next step.

MS m/z (ESI): 382 [M+H]+.

### Fourth step: preparation of 1-(3-((5-cyano-1-(phenylsulfonyl)-1H-pyrrolo[2,3-b] pyridin-4-yl)(methyl)amino)cyclobutyl)-3-(3-methoxy-1,2,4-thiadiazole-5-yl)urea 29d

4-((3-aminocyclobutyl)(methyl)amino)-1-(phenylsulfonyl)-1H-pyrrolo[2,3-b]pyridin-5-carb onitrile **29c** (150 mg, 0.39 mmol), phenyl (3-methoxy-1,2,4-thiadiazole-5-yl) carbamate **7h** (100 mg, 0.39 mol) and DIPEA (150 mg, 1.17 mmol) were dissolved in THF. This mixture was stirred for 1h at 60 °C and concentrated to obtain crude products. The crude products were purified by column chromatography (DCM: MeOH=20:1) to obtain (2) 1-(3-((5-cyano-1-(phenylsulfonyl)-1H-pyrrolo[2,3-b]pyridin-4-yl)(methyl)amino)cyclobutyl)-3-( 3-methoxy-1,2,4-thiadiazole-5-yl)urea **29d** (100 mg) with a yield of 47%.

MS m/z (ESI): 539 [M+H]+.

### Fifth step: preparation of 1-(3-((5-cyano-1H-pyrrolo[2,3-b]pyridin-4-yl)(methyl)amino) cyclobutyl)-3-(3-methoxy-1,2,4-thiadiazole-5-yl)urea H29

1-(3-((5-cyano-1-(phenylsulfonyl)-1H-pyrrolo[2,3-b]pyridin-4-yl)(methyl)amino)cyclobutyl )-3-(3-methoxy-1,2,4-thiadiazole-5-yl)urea **29d** (100 mg, 0.18 mmol) was dissolved in methanol (4 mL), and then 2 mL of 2 N sodium hydroxide solution was added. This mixture was stirred for 2h at room temperature, extracted with ethyl acetate. The organic phases were combined, washed with saturated sodium chloride, dried with anhydrous sodium sulfate, filtered and concentrated to obtain crude products. The crude products were purified by column chromatography (DCM: MeOH=10:1) to obtain 1-(3-((5-cyano-1H-pyrrolo[2,3-b]pyridin-4-yl)(methyl)amino) cyclobutyl)-3-(3-methoxy-1,2,4-thiadiazole-5-yl)urea **H29** (15 mg) with a yield of 21%.

MS m/z (ESI): 399 [M+H]+.

¹H NMR (400 MHz, DMSO-d6) δ 11.92 (s, 1H), 11.39 (s, 1H), 8.15 (s, 1H), 7.41 - 7.24 (m, 2H), 6.65 (dd, J = 3.6, 2.0 Hz, 1H), 4.28 (p, J = 8.2 Hz, 1H), 3.94 - 3.87 (m, 1H), 3.27 (s, 3H), 2.58 (d, J = 8.6 Hz, 2H), 2.32 (dt, J = 11.9, 9.1 Hz, 2H).

### Example 30

### Preparation of (3aR,5s,6aS)-4-({2-[(3-isopropyl-1,2,4-thiadiazole-5-yl)carbamoyl] -hexahydrocyclopenta[c]pyrrole-5-yl}-methyl-amino)-1H-pyrrolo[2,3-b]pyridin-5-carbonitrile H30

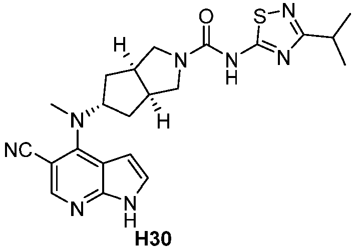

3-isopropyl-5-amino-1,2,4-thiadiazole was used as a raw material to obtain compound **H30** by using the same preparation method as described in Example **7.**

MS m/z (ESI): 451 [M+H]+.

¹H NMR (400 MHz, DMSO-*d*₆) δ 11.90 (s, 1H), 11.44 (s, 1H), 8.11 (s, 1H), 7.28 - 7.15 (m, 1H), 6.61 (dd, *J* = 3.7, 2.0 Hz, 1H), 4.70(m, 1H), 3.63 (m, 2H), 3.31(m, 2H),3.16 (s, 3H), 2.98 (p, *J* = 7.0 Hz, 1H), 2.84 (s, 2H), 2.22 - 2.01 (m, 2H), 1.83 (dd, *J* = 13.2, 7.6 Hz, 2H), 1.21(d, 6H).

### Example 31

### Preparation of (3aR,5s,6aS)-4-({2-[(1-ethyl-1H-1,2,4-triazole-3-yl)-carbamoyl] -hexahydrocyclopenta[c]pyrrole-5-yl}-methyl-amino)-1H-pyrrolo[2,3-b]pyridin-5-carbonitrile H31

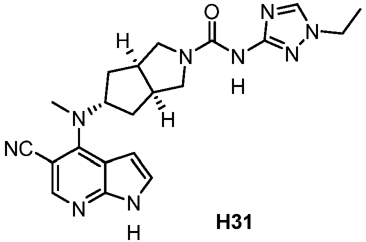

1-ethyl-3-amino-1H-1,2,4-triazole was used as a raw material to obtain compound **H31** by using the same preparation method as described in Example **7.**

MS m/z (ESI): 420 [M+H]+.

¹H NMR (400 MHz, DMSO-*d*₆) δ 11.95 (s, 1H), 8.58 (s, 1H), 8.28 (s, 1H), 8.16 (s, 1H), 7.37 - 7.30 (m, 1H), 6.67 (dd, J = 3.4, 1.9 Hz, 1H), 4.81 - 4.70 (m, 1H), 4.08 (q, *J* = 7.3 Hz, 2H), 3.65 - 3.54 (m, 2H), 3.19 (s, 3H), 3.19 - 3.13 (m, 2H), 2.91 - 2.75 (m, 2H), 2.21 - 2.07 (m, 2H), 1.91 - 1.77 (m, 2H), 1.35 (t, *J* = 7.3 Hz, 3H).

### Example 32

### Preparation of (3aR,5s,6aS)-4-({2-[(3-cyclopropyl-1,2,4-oxadiazole-5-yl)carbamoyl] -hexahydrocyclopenta[c]pyrrole-5-yl}-methyl-amino)-1H-pyrrolo[2,3-b]pyridin-5-carbonitrile H32

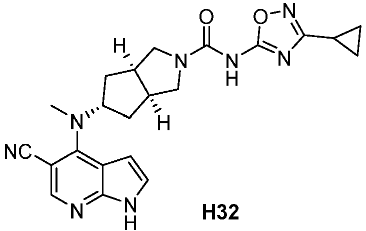

3-cyclopropyl-5-amino-1,2,4-oxadiazole was used as a raw material to obtain compound **H32** by using the same preparation method as described in Example **7.**

MS m/z (ESI): 433 [M+H]+.

¹H NMR (400 MHz, DMSO-*d*₆) δ 11.91 (s, 1H), 10.68 (s, 1H), 8.12 (s, 1H), 7.30 - 7.22 (m, 1H), 6.66 - 6.57 (m, 1H), 4.75 - 4.63 (m, 1H), 3.69 - 3.49 (m, 2H), 3.29 - 3.20 (m, 2H), 3.15 (s, 3H), 2.88 - 2.71 (m, 2H), 2.17 - 2.03 (m, 2H), 2.01 - 1.89 (m, 1H), 1.87 - 1.71 (m, 2H), 1.02 - 0.86 (m, 2H), 0.85 - 0.72 (m, 2H).

### Example 33

### (3aR,5s,6aS)-4-({2-[(3-dimethylamino-1,2,4-thiadiazole-5-yl)carbamoyl]-hexahydrocyclop enta[c]pyrrole-5-yl}-methyl-amino)-1H-pyrrolo[2,3-b]pyridin-5-carbonitrile H33

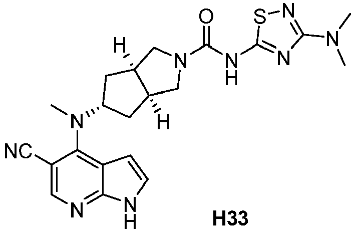

Preparation of 3-(N,N-dimethylamino)-5-amino-1,2,4-thiadiazole was used as a raw material to obtain compound **H33** by using the same preparation method as described in Example **7.**

MS m/z (ESI): 452 [M+H]+.

¹H NMR (400 MHz, DMSO-*d*₆) δ 11.92 (s, 1H), 11.22 (s, 1H), 8.15 (s, 1H), 7.32 - 7.26 (m, 1H), 6.69 - 6.63 (m, 1H), 4.77 - 4.70 (m, 1H), 3.72 - 3.59 (m, 2H), 3.29 (s, 3H), 3.19 (s, 3H), 3.01 (s, 3H), 2.93 - 2.81 (m, 2H), 2.20 - 2.09 (m, 2H), 2.07 - 1.93 (m, 2H), 1.92 - 1.79 (m, 2H).

### Example 34

### Preparation of 4-((1-((3-(trifluoromethyl)phenyl)sulfonyl)piperidin-4-yl)amino) -2,3-dihydro-1H-pyrrolo[2,3-b]pyridin-4-carbonitrile H34

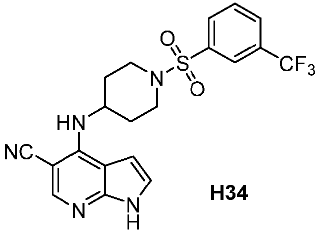

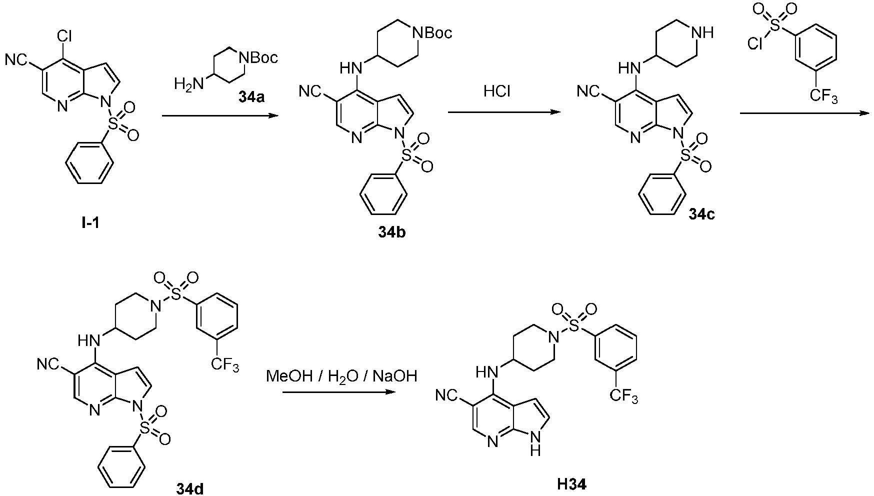

### First step: preparation of tert-butyl 4-((5-cyano-1-(benzenesulfonyl)-2,3-dihydro-1H-pyrrole[2,3-b]pyridin-4-yl)amino)piperidin-1-c arboxylic ester 34b

4-chloro-1-(phenylsulfonyl)-2,3-dihydro-1H-pyrrolo[2,3-b]pyridin-5-carbonitrile **I-1**(500mg, 1.6mmol, 1.0 eq) and tert-butyl 4-aminopiperidin-1-carboxylate **34a**(0.96g, 4.8mmol, 3.0 eq) were added in NMP (5ml), and then DIPEA (0.53ml, 3.2mmol, 2.0 eq) was added. The system was subjected to a microwave reaction for 1h at 100°C, cooled to room temperature, diluted with water (20ml), extracted with ethyl acetate (50ml). The organic layers were washed with brine (10ml), concentrated and purified by falsh column (PE - PE/EA=2: 1) to obtain white solids **34b** (750mg).

MS m/z (ESI): 482 [M+1]⁺

### Second step: preparation of 1-(phenylsulfonyl)-4-(piperidin-4-yl-amino) -2,3-dihydro-1H-pyrrolo[2,3-b]pyridin-5-carbonitrile 34c

Compound **34b** (750mg, 1.6mmol, 1.0 eq) was added into 2N of HCl/EA (30ml), reacted overnight at room temperature, filtered and dried in vacuum to obtain white solids **34c** (600mg).

MS m/z (ESI): 382 [M+1]⁺

### Third step: preparation of 1-(phenylsulfonyl)-4-((1-((3-(trifluoromethyl)phenyl)sulfonyl) piperidin-4-yl)amino)-2,3-dihydro-1H-pyrrolo[2,3-b]pyridin-5-carbonitrile 34d

Compound **34c** (100mg, 0.26mmol, 1.0eq) and TEA (53mg, 0.52mmol, 2.0eq) were dissolved in anhydrous DCM (10ml). 3-trifluoromethylbenzenesulfonyl chloride (64mg, 0.26mmol, 1.0 eq) was added dropwise to DCM solution (5ml). After the addition was completed, the system was reacted for 1h at room temperature. Then, the system was quenched with water (10ml) and layered. The organic layers were dried with anhydrous sodium sulfate, concentrated and purified by flash column to obtain white solids **34d** (160 mg).

MS m/z (ESI): 590 [M+1]⁺

### Fourth step: preparation of 4-((1-((3-(trifluoromethyl)phenyl)sulfonyl)piperidin-4-yl)amino) -2,3-dihydro-1H-pyrrolo[2,3-b]pyridin-5-carbonitrile H34

Compound **34d** (160mg, 0.27mmol, 1.0eq) was dissolved in a mixed solvent of methanol (3ml) and THF (3ml), and then 2N of NaOH (0.7ml, 1.4mmol, 5.0 eq) was added and reacted for 2h at room temperature to obtain a reaction liquid. The reaction liquid was stirred directly and purified by flash column (DCM - DCM/MeOH = 20: 1) to obtain white solid **H34** (56mg).

MS m/z (ESI): 450 [M+1]⁺
¹H NMR (400 MHz, DMSO-*d*₆) δ 11.80 (s, 1H), 8.18 - 8.08 (m, 2H), 8.04 (s, 1H), 8.01 - 7.91 (m, 2H), 7.21 (dd, *J* = 3.5, 2.5 Hz, 1H), 6.75 - 6.65 (m, 2H), 4.07 (s, 1H), 3.76 (d, *J* = 11.8 Hz, 2H), 2.54 (d, *J* = 10.2 Hz, 2H), 2.05 (d, *J* = 11.3 Hz, 2H), 1.75 (qd, *J* = 12.2, 4.0 Hz, 2H).

### Example 35

### Preparation of 4-((1-((3-(trifluoromethyl)phenyl)sulfonyl)piperidin-4-yl)-methyl-amino) -2,3-dihydro-1H-pyrrolo[2,3-b]pyridin-5-carbonitrile H35

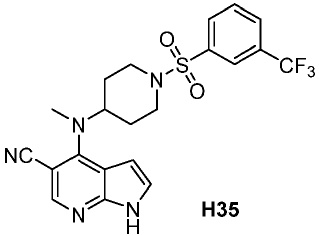

Tert-buty 4-(methylamino)-piperidin-1-carboxylate was used as a raw material to obtain compound **H35** by using the same preparation method as described in Example **34.**

MS m/z (ESI): 464 [M+1]⁺
¹H NMR (400 MHz, DMSO-*d*₆) δ 8.13 (q, *J =* 8.0 Hz, 3H), 8.00 (s, 1H), 7.93 (t, *J =* 7.9 Hz, 1H), 7.32 (t, *J =* 3.0 Hz, 1H), 6.61 - 6.55 (m, 1H), 3.90 (dd, *J* = 44.8, 11.8 Hz, 3H), 3.14 (s, 3H), 2.45 (d, *J* = 11.4 Hz, 2H), 2.03 (q, *J* = 11.1 Hz, 3H), 1.85 (d, *J =* 12.3 Hz, 2H).

### Example 36

### Preparation of 3-((5-cyano-1H-pyrrolo[2,3-b]pyridin-4-yl)amino)-N-(2,2,2-trifluoroethyl) pyrrolidin-1-carboxamide H36

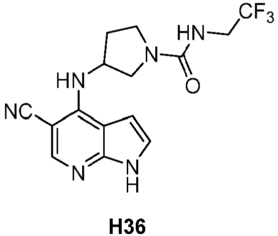

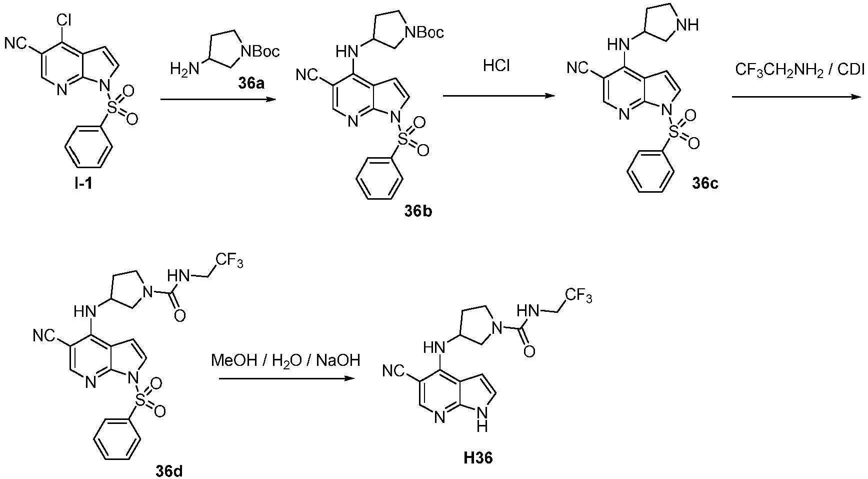

### First step: preparation of tert-butyl 3-((5-cyano-1-(benzenesulfonyl) -1H-pyrrole[2,3-b]pyridin-4-yl)amino)pyrrolidin-1-carboxylic ester 36b

4-chloro-1-(phenylsulfonyl)-2,3-dihydro-1H-pyrrolo[2,3-b]pyridin-5-carbonitrile **I-1** (350mg, 1.1mmol, 1.0 eq) and tert-butyl-3-aminopyrrolidin-1-carboxylate **36a** (0.20g, 1.1mmol, 1.0 eq) were added in NMP (5ml), and then DIPEA (0.28ml, 2.2mmol, 2.0 eq) was added. The system was reacted for 5h at 100 °C, cooled to room temperature, diluted with water (20ml), and extracted with ethyl acetate (50ml). The organic layers were washed with brine (10ml), concentrated and purified by falsh column (PE - PE/EA=1: 1) to obtain white solids **36b** (330mg).

MS m/z (ESI): 468 [M+1]⁺

### Second step: preparation of 1-(phenylsulfonyl)-4-(pyrrolidin-3-yl-amino) -1H-pyrrolo[2,3-b]pyridin-5-carbonitrile 36c

Compound **36b** (150mg, 0.32mmol, 1.0 eq) was added into 2N of HCl/EA (10ml), reacted overnight at room temperature, filtered and dried in vacuum to obtain white solids **36c** (120mg).

MS m/z (ESI): 368 [M+1]⁺

### Third step: preparation of 3-((5-cyano-1-(phenylsulfonyl)-1H-pyrrolo[2,3-b]pyridin-4-yl) amino)-N-(2,2,2-trifluoroethyl)pyrrolidin-1-carboxamide 36d

Trifluoroethylamine (63mg, 0.64mmol, 2.0 eq) was dissolved in anhydrous THF (10ml), and then CDI (0.10g, 0.64mmol, 2.0 eq) was added. The system was reacted for 4h at room temperature to obtain a reaction liquid. Compound **36c** (120mg, 0.32mmol, 1.0 eq) and TEA (97mg, 0.96mmol, 3.0 eq) were dissolved in anhydrous THF (5ml). This solution was pipetted to the above reaction liquid one time and subjected to a reflux reaction for 2h. The solvent was dried under reduced pressure, and ethyl acetate (30ml) was added. The system was washed with water (10ml) and brine (10ml) in sequence, dried with anhydrous sodium sulfate, concentrated and purified by flash column (DCM - DCM/MeOH=20: 1) to obtain white solids **36d** (150mg).

MS m/z (ESI): 493 [M+1]⁺

### Fourth step: preparation of 3-((5-cyano-1H-pyrrolo[2,3-b]pyridin-4-yl)amino)-N-(2,2,2-trifluoroethyl)pyrrolidin-1-carboxamide H36

Compound **36d** (150mg, 0.30mmol, 1.0 eq) was dissolved in a mixed solvent of MeOH/THF (5ml/5ml), and then 2N of NaOH (0.8ml, 1.5mmol, 5.0 eq) was added and reacted for 4h at room temperature to obtain a reaction liquid. The reaction liquid was concentrated and purified by flash column (DCM - DCM/MeOH=10: 1) to obtain white solids **H36** (40mg).

MS m/z (ESI): 353 [M+1]⁺
¹H NMR (400 MHz, DMSO-*d*₆) δ 11.85 (s, 1H), 8.10 (s, 1H), 7.26 (dd, *J* = 3.6, 2.4 Hz, 1H), 7.03 (d, *J =* 7.1 Hz, 1H), 6.89 (t, *J* = 6.2 Hz, 1H), 6.80 (dd, *J* = 3.6, 2.0 Hz, 1H), 4.82 (s, 1H), 3.86 - 3.72 (m, 2H), 3.66 (dd, *J* = 10.7, 6.2 Hz, 1H), 3.56 - 3.36 (m, 3H), 2.30 - 2.05 (m, 2H).

### Example 37

### Preparation of 3-((5-cyano-1H-pyrrolo[2,3-b]pyridin-4-yl)methyl-amino)-N-(2,2,2-trifluoroethyl)pyrrolidin-1-carboxamide H37

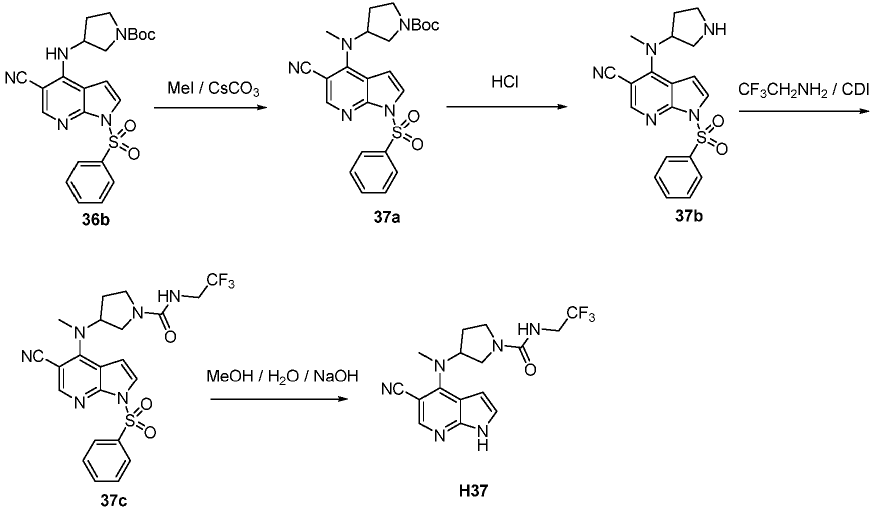

### First step: preparation of tert-butyl-3-((5-cyano-1-(benzenesulfonyl) -1H-pyrrole[2,3-b]pyridin-4-yl)(methyl)amino)pyrrolidin-1-carboxylate 37a

Compound **36b** (150mg, 0.32mmol, 1.0 eq) and cesium carbonate (0.21g, 0.64mmol, 2.0 eq) were added in DMF (10ml) and CH₃I (108mg, 0.76mmol, 2.5 eq) was added dropwise. The system was reacted overnight at room temperature to obtain a reaction liquid. The reaction liquid was diluted with water (50ml) and extracted with ethyl acetate (30ml×2). The organic phases were combined, washed with brine (25ml×3), concentrated and purified by flash column (DCM - DCM/MeOH=50: 1) to obtain white foamed solids **37a** (120mg).

MS m/z (ESI): 482 [M+1]⁺

### Second step: preparation of 4-(methyl(pyrrolidin-3-yl)amino)-1-(phenylsulfonyl) -1H-pyrrolo[2,3-b]pyridin-5-carbonitrile 37b

Compound **37a** (120mg, 0.25mmol, 1.0 eq) was added into 2N of HCl/EA (20ml), reacted overnight at room temperature, filtered and dried in vacuum to obtain white solids **37b** (90mg).

MS m/z (ESI): 382 [M+1]⁺

### Third step: preparation of 3-((5-cyano-1-(phenylsulfonyl)-1H-pyrrolo[2,3-b] pyridin-4-yl)(methyl)amino)-N-(2,2,2-trifluoroethyl)pyrrolidin-1-carboxamide 37c

Trifluoroethylamine (50mg, 0.50mmol, 2.0 eq) was dissolved in anhydrous THF (10ml), and then CDI (81g, 0.50mmol, 2.0 eq) was added. The system was reacted for 4h at room temperature to obtain a reaction liquid. Compound **37b** (90mg, 0.25mmol, 1.0 eq) and TEA (76mg, 0.75mmol, 3.0 eq) were dissolved in anhydrous THF (5ml). This solution was pipetted to the above reaction liquid one time and subjected to a reflux reaction for 2h. The solvent was dried under reduced pressure, and ethyl acetate (30ml) was added. The system was washed with water (10ml) and brine (10ml) in sequence, dried with anhydrous sodium sulfate, concentrated and purified by flash column (DCM - DCM/MeOH=50: 1) to obtain white solids **37c** (110mg).

MS m/z (ESI): 507 [M+1]⁺

### Fourth step: preparation of 3-((5-cyano-1H-pyrrolo[2,3-b]pyridin-4-yl)amino)-N-(2,2,2-trifluoroethyl)pyrrolidin-1-carboxamide H37

Compound **37c** (110mg, 0.22mmol, 1.0 eq) was dissolved in a mixed solvent of MeOH/THF (5ml/5ml), and then 2N of NaOH (0.6ml, 1.1mmol, 5.0 eq) was added and reacted for 4h at room temperature to obtain a reaction liquid. The reaction liquid was concentrated and purified by flash column (DCM - DCM/MeOH=15: 1) to obtain white solids **H37** (47mg).

MS m/z (ESI): 367 [M+1]⁺

¹H NMR (400 MHz, DMSO-*d*₆) δ 12.04 (s, 1H), 8.23 (s, 1H), 7.40 (dd, *J =* 3.7, 2.3 Hz, 1H), 6.92 (t, *J =* 6.3 Hz, 1H), 6.69 (dd, *J =* 3.6, 1.8 Hz, 1H), 4.76 (q, *J =* 7.5 Hz, 1H), 3.90 - 3.72 (m, 2H), 3.67 (dd, *J* = 10.7, 7.7 Hz, 1H), 3.60 - 3.40 (m, 2H), 3.31 - 3.24 (m, 1H), 3.21 (s, 3H), 2.19 (m, 2H).

### Example 38 Preparation of (3aR,5s,6aS)-5-((5-cyano-1H-pyrrolo[2,3-b]pyridin-4-yl) (methyl)amino)-N-(3-hydroxy-1,2,4-thiadiazole-5-yl)hexahydrocyclopentadiene[c]pyrrole-2(1H) -carboxamide H38

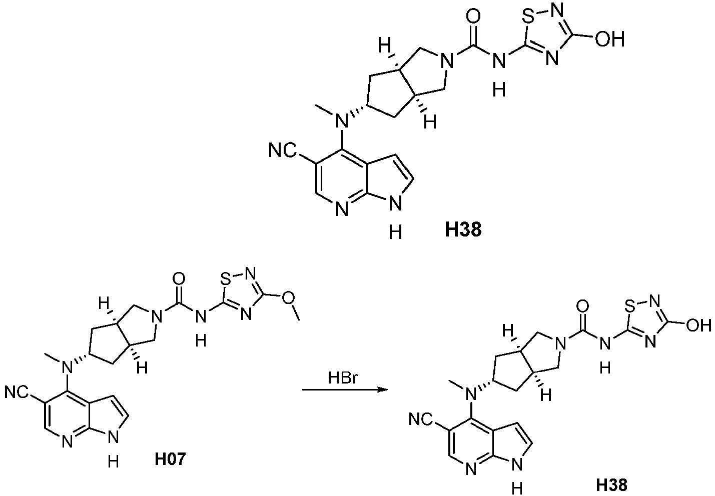

Compound H07 (100 mg, 0.23 mmol) and HBr/AcOH solution (5mL) were added into a reaction flask and stirred for 1h at 80 °C. The system was poured into water, neutralized to be alkalescent, extracted with ethyl acetate for three times. The organic phases were combined, washed with water and saturated sodium chloride, dried with anhydrous sodium sulfate and concentrated at low temperature, followed by purification by column chromatography (v/v, DCM/MeOH=100:1-50:1) to obtain compound **H38** (10 mg, 0.024 mmol) with a yield of 10.4%.

MS m/z (ESI): 425 [M+H]+.

¹H NMR (400 MHz, DMSO-*d*₆) δ 12.12 (brs, 1H), 11.91 (s, 1H), 9.19 (brs, 1H), 8.12 (s, 1H), 7.29 - 7.21 (m, 1H), 6.64 - 6.56 (m, 1H), 4.74 - 4.65 (m, 1H), 3.78 - 3.56 (m, 2H), 3.16 (s, 5H), 2.87 - 2.76 (m, 2H), 2.14 - 2.05 (m, 2H), 1.86 - 1.78 (m, 2H).

### Example 39

### Preparation of 6-(5-cyano-1H-pyrrolo[2,3-b]pyridin-4-yl)-N-(2,2,2-trifluoroethyl)-3,6-diazabicyclo[3.2.0]heptane-3-carboxamide H39

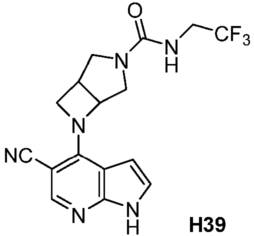

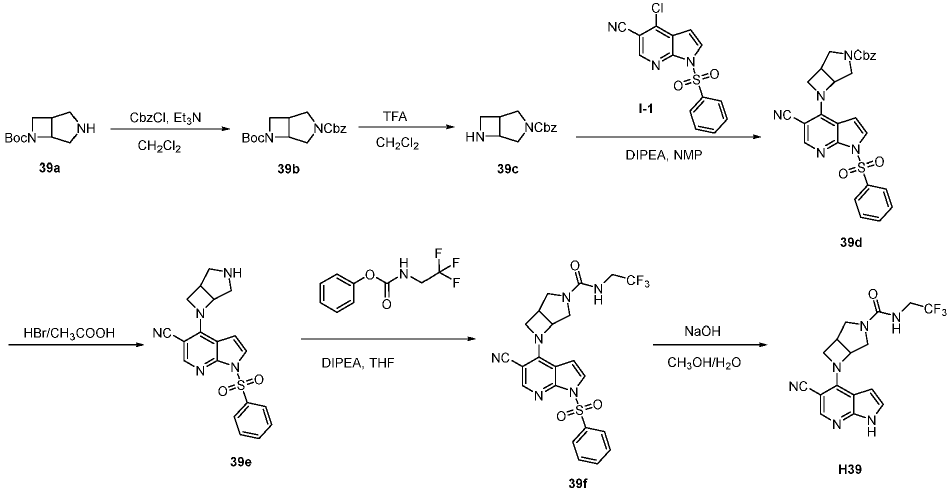

### First step: 3-benzyl 6-(tert-butyl)-3,6-diazabicyclo[3.2.0]heptane-3,6-dicarboxylate 39b

Compound **39a** (300 mg, 1.5 mmol) and dichloromethane (20 mL) were added into a reaction flask, and then triethylamine (303 mg, 3 mmol) and benzyl chloroformate (306 mg, 1.8 mmol) were added in sequence to obtain a reaction liquid. The reaction liquid was stirred for 2h at room temperature and washed with water. The organic phases were dried by a spin drier, mixed with silica gel, concentrated at low temperature, and purified by column chromatography to obtain compound **39b** (470 mg, 1.4mmol) with a yield of 94%.

MS m/z (ESI): 355 [M+23]+

### Second step: benzyl 3,6-diazabicyclo[3.2.0]heptane-3-carboxylate 39c

Compound **39b** (470 mg, 1.4 mmol) was dissolved in dichloromethane (5 mL), and then trifluoroacetic acid (1 mL) was added to obtain a reaction liquid. The reaction liquid was stirred for 2h at room temperature, then concentrated and dried by a spin drier to obtain compound **39c** (328 mg, 1.4 mmol) with a yield of 100%.

MS m/z (ESI): 233 [M+H]+.

### Third step: benzyl 6-(5-cyano-1-(phenylsulfonyl)-1H-pyrrolo[2,3-b]pyridin-4-yl) -3,6-diazabicyclo[3.2.0]heptane-3-carboxylate 39d

Compound **I-1** (200 mg, 0.63 mmol), compound **39c** (146 mg, 0.63 mmol) DIPEA (162 mg, 1.26 mmol) and NMP (5 mL) were added into a reaction flask. The system was subjected to a microwave reaction for 2h at 170 °C. After the reaction was completed, the system was poured into water, extracted with ethyl acetate for three times. The organic phases were combined, washed with water and saturated sodium chloride, dried with anhydrous sodium sulfate, and concentrated at low temperature, followed by purification by column chromatography (v/v, DCM/MeOH=100:1-50:1) to obtain compound **39d** (200 mg, 0.39 mmol) with a yield of 62%.

MS m/z (ESI): 514 [M+H]+.

### Fourth step: 4-(3,6-diazabicyclo[3.2.0]heptane-6-yl)-1-(phenylsulfonyl)-1H-pyrrolo [2,3-b]pyridin-5-carbonitrile 39e

Compound 1e (180 mg, 0.35 mmol) and 40% hydrobromic acid-acetic acid solution (3 mL) were added into a reaction flask to obtain a reaction liquid. The reaction liquid was stirred for 20 minutes at room temperature, then concentrated and dried by a spin drier to obtain compound **39e** (133 mg, 0.35 mmol) with a yield of 100%.

MS m/z (ESI): 380 [M+H]+.

### Fifth step: 6-(5-cyano-1-(phenylsulfonyl)-1H-pyrrolo[2,3-b]pyridin-4-yl)-N-(2,2,2-trifluoroethyl)-3,6-diazabicyclo[3.2.0]heptane-3-carboxamide 39f

Compound **39e** (133 mg, 0.35 mmol), N-(3,3,3-trifluoroethyl)phenyl carbamate (77 mg, 0.35 mmol), DIPEA (90 mg, 0.70 mmol) and THF(10 mL) were added into a reaction flask. The system was reacted for 2h at 60 °C. After the reaction was completed, the system was concentrated directly and purified by column chromatography (v/v, DCM/MeOH=100:1-50:1) to obtain compound **39f** (130 mg, 0.26 mmol) with a yield of 74%.

MS m/z (ESI): 505 [M+H]+.

### Sixth step: 6-(5-cyano-1H-pyrrolo[2,3-b]pyridin-4-yl)-N-(2,2,2-trifluoroethyl)-3,6 -diazabicyclo[3.2.0]heptane-3-carboxamide H39

Compound **39f** (130 mg, 0.26 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H39** (10 mg, 0.027 mmol) with a yield of 11%.

MS m/z (ESI): 365 [M+H]+.

¹H NMR (400 MHz, DMSO-*d*₆) δ 11.85 (s, 1H), 8.01 (s, 1H), 7.26 - 7.11 (m, 2H), 6.45 (s, 1H), 5.37 (dd, *J =* 6.9, 4.5 Hz, 1H), 4.59 (t, *J =* 8.6 Hz, 1H), 4.19 (dd, *J =* 8.9, 4.4 Hz, 1H), 4.04 (d, *J =* 12.4 Hz, 1H), 3.87 - 3.63 (m, 3H), 3.19 (ddd, J = 23.2, 11.9, 5.5 Hz, 2H), 1.96 (q, *J* = 7.1, 6.7 Hz, 1H).

### Example 40

### Preparation of (3S,4S)-4-({4-methyl-1-[(2,2,2-trifluoroethyl)carbamoyl)]pyrrolidin-3-yl} amino)-1H-pyrrolo[2,3-b]pyridin-5-carbonitrile H40

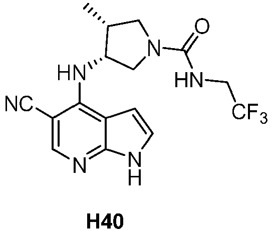

(3S,4S)-(4-methyl-pyrrolidin-3-yl)tert-butyl carbamate was used as a raw material to obtain compound **H40** by using the same preparation method as described in Example **1.**

MS m/z (ESI): 367 [M+H]+.

1H NMR (400 MHz, DMSO-d6) δ 11.86 (s, 1H), 8.11 (s, 1H), 7.28 (t, J = 3.0 Hz, 1H), 6.94 - 6.80 (m, 2H), 6.70 (d, J = 8.8 Hz, 1H), 4.87 (dq, J = 10.9, 5.7 Hz, 1H), 3.81 (ddd, J = 9.4, 6.3, 2.8 Hz, 2H), 3.71 (dd, J = 10.8, 6.5 Hz, 1H), 3.60 - 3.48 (m, 2H), 3.26 (dd, J = 10.2, 7.3 Hz, 1H), 2.61 (p, J = 6.9 Hz, 1H), 0.98 (d, J = 6.9 Hz, 3H).

### Example 41

### Preparation of (3aR,5s,6aS)-5-((5-cyano-1H-pyrrolo[2,3-b]pyridin-4-yl)(methyl)amino)-N -(3-(methylamino)-1,2,4-thiadiazole-5-yl)hexahydrocyclopentadiene[c]pyrrole-2(1H)-carboxami de H41

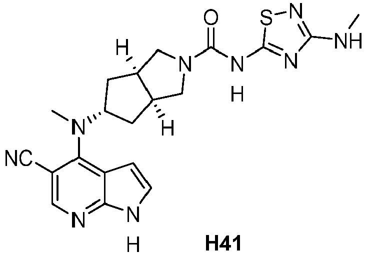

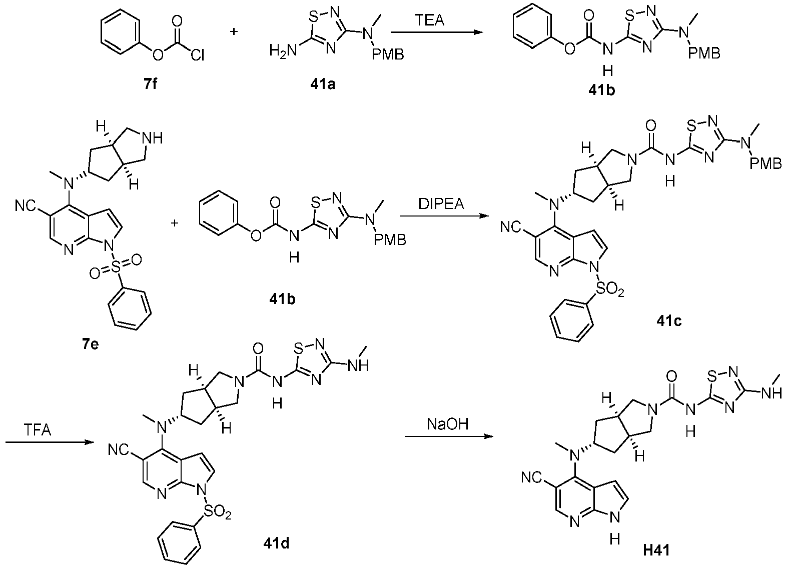

### First step: phenyl(3-((4-methoxyphenyl)(methyl)amino)-1,2,4-thiadiazole-5-yl) carbamate 41b

Compound **41a** (267 mg, 1.71 mmol), compound 92a (388 mg, 1.55 mmol) and DCM (20 mL) were added into a reaction flask, and then triethylamine (313mg, 3.10 mmol) was slowly added dropwise at an ice bath. The system was reacted for 2h at that temperature. After the reaction was completed, water was added. The system was extracted with DCM, dried, concentrated and purified by column chromatography (v/v, DCM/MeOH=100:1-50:1) to obtain compound **41b** (450 mg, 1.22 mmol) with a yield of 78.7%.

MS m/z (ESI): 371 [M+H]+.

### Second step: (3aR,5s,6aS)-5-((5-cyano-1-(phenylsulfonyl)-1H-pyrrolo[2,3-b]pyridin-4-yl) (methyl)amino)-N-(3-((4-methoxyphenyl(methyl)amino)-1,2,4-thiadiazole-5-yl)hexahydrocyclo pentadiene[c]pyrrole-2(1H)-carboxamide 41c

Compound **7e** (50 mg, 0.12 mmol), compound **41b** (44 mg, 0.12 mmol), DIPEA (31mg, 0.24mmol) and THF (10 mL) were added into a reaction flask. The system was reacted for 2h at 60 °C. After the reaction was completed, the system was concentrated directly and purified by column chromatography (v/v, DCM/MeOH=100:1-50:1) to obtain compound **41c** (56 mg, 0.08 mmol) with a yield of 66.7%.

MS m/z (ESI): 698 [M+H]+.

### Third step: (3aR,5s,6aS)-5-((5-cyano-1-(phenylsulfonyl)-1H-pyrrolo[2,3-b]pyridin-4-yl) (methyl)amino)-N-(3-(methylamino)-1,2,4-thiadiazole-5-yl)hexahydrocyclopentadiene[c]pyrrole -2(1H)-carboxamide 41d

Compound **41c** (56 mg, 0.08 mmol), TFA (2 mL) and DCM (10 mL) were added into a reaction flask. The system was reacted for 0.5h at room temperature. After the reaction was completed, the system was concentrated directly and purified by column chromatography (v/v, DCM/MeOH=100:1-50:1) to obtain compound **41d** (40 mg, 0.07 mmol) with a yield of 86.7%.

MS m/z (ESI): 578 [M+H]+.

### Fourth step: (3aR,5s,6aS)-5-((5-cyano-1H-pyrrolo[2,3-b]pyridin-4-yl)(methyl) amino)-N-(3-(methylamino)-1,2,4-thiadiazole-5-yl)hexahydrocyclopentadiene[c]pyrrole-2(1H)-c arboxamide H41

Compound **41d** (40 mg, 0.07 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H41** (10 mg, 0.023 mmol) with a yield of 32.90%.

MS m/z (ESI): 438 [M+H]+.

1H NMR (400 MHz, DMSO-d6) δ 11.90 (s, 1H), 11.13 (s, 1H), 8.12 (s, 1H), 7.28 - 7.19 (m, 1H), 6.65 - 6.59 (m, 1H), 6.56 - 6.44 (m, 1H), 4.73 - 4.64 (m, 1H), 3.67 - 3.56 (m, 2H), 3.29 - 3.23 (m, 2H), 3.16 (s, 3H), 2.88 - 2.77 (m, 2H), 2.71 (s, 3H), 2.16 - 2.05 (m, 2H), 1.88 - 1.78 (m, 2H).

### Example 42

### (3aR,5s,6aS)-5-((5-cyano- 1H-pyrrolo[2,3-b]pyridin-4-yl)(methyl)amino)-N-(3-hydroxymet hyl-1,2,4-thiadiazole-5-yl)hexahydrocyclopentadiene[c]pyrrole-2(1H)-carboxamide H42

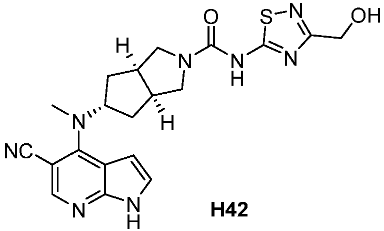

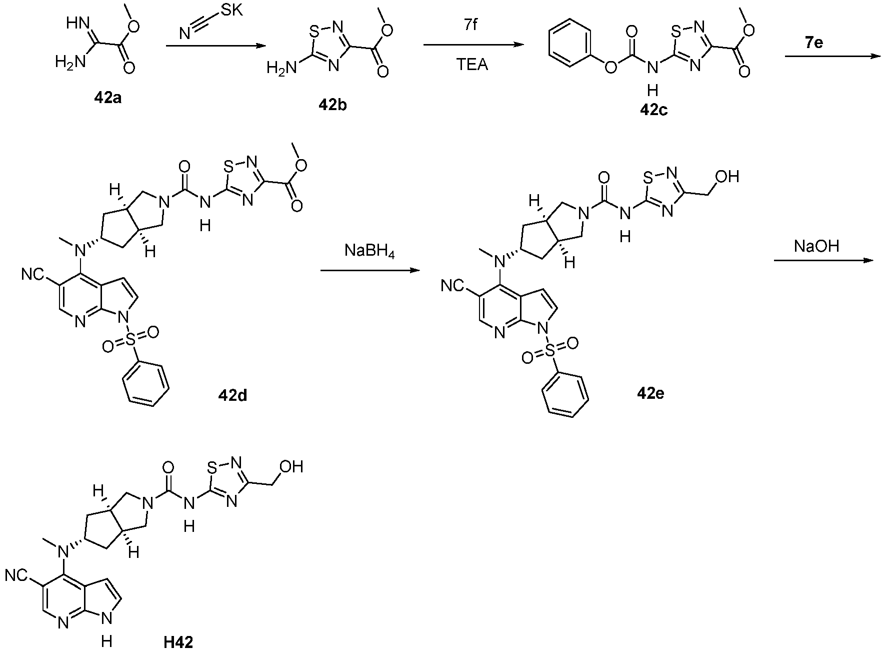

### First step: methyl 5-amino-1,2,4-thiadiazole-3 carboxylate 42b

Compound **42a** (1.0 g, 9.79 mmol), potassium thioisocyanate (1.0 g, 10.3mmol) and methanol (20 mL) were added into a reaction flask, cooled to -5°C and then sodium methoxide (1.0 g, 18.5mmol) was slowly added. The system was reacted for 3h at -5°C, and then heated to room temperature slowly. After the reaction was completed, the system was concentrated directly and purified by column chromatography (v/v, DCM/MeOH=100:1-10:1) to obtain compound **42b** (300 mg, 1.88mmol) with a yield of 19.3%.

MS m/z (ESI): 160.1 [M+H]+.

### Second step: methyl 5-((phenoxyformyl)amino-1,2,4-thiadiazole-3 carboxylate 42c

Compound **42b** (200 mg, 1.26 mmol), compound **7f** (300 mg, 1.92 mmol) and dichloromethane (10 mL) were added into a reaction flask, and then triethylamine (300 mg, 2.96 mmol) was slowly added dropwise. The system was reacted for 1h at room temperature. After the reaction was completed, the system was concentrated directly and purified by column chromatography (v/v, DCM/MeOH=100:1-20:1) to obtain compound **42c** (130 mg, 0.465 mmol) with a yield of 36.9%.

MS m/z (ESI): 280.1 [M+H]+.

### Third step: methyl 5-((3aR,5s,6aS)-5-((5-cyano-1-p-toluenesulfonyl-1H -pyrrolo[2,3-b]pyridin-4-yl)(methyl)amino)hexahydrocyclopentadiene[c]pyrrole-2-formamido)-1 ,2,4-thiadiazole-3-carboxylate 42d

Compound **42c** (100 mg, 0.358 mmol), compound **7e** (80 mg, 0.184 mmol), DIPEA (100mg, 0.774 mmol) and THF (10 mL) were added into a reaction flask. The system was reacted for 2h at 60 °C. After the reaction was completed, the system was concentrated directly and purified by column chromatography (v/v, DCM/MeOH=100:1-10:1) to obtain compound **42d** (100 mg, 0.161 mmol) with a yield of 87.5%.

MS m/z (ESI): 607.4 [M+H]+.

### Fourth step: (3aR,5s,6aS)-5-((5-cyano-1-p-toluenesulfonyl-1H-pyrrolo[2,3-b]pyridin-4-yl) (methyl)amino)-N-(3-hydroxymethyl-1,2,4-thiadiazole-5-yl)hexahydrocyclopentadiene[c]pyrrol e-2(1H)- carboxamide 42e

Compound **42d** (90 mg, 0.145 mmol), sodium borohydride (200 mg, 5.29 mmol) and methanol (10 mL) were added into reaction flask. The system was reacted for 2h at room temperature. After the reaction was completed, the system was concentrated directly and purified by column chromatography (v/v, DCM/MeOH=100:1-5:1) to obtain compound **42e** (60 mg, 0.101 mmol) with a yield of 62.7%.

MS m/z (ESI): 579.3 [M+H]+.

### Fifth step: (3aR,5s,6aS)-5-((5-cyano-1H-pyrrolo[2,3-b]pyridin-4-yl)(methyl)amino)-N-(3-hydroxymethyl-1,2,4-thiadiazole-5-yl)hexahydrocyclopentadiene[c]pyrrole-2(1H)-carboxamide H42

Compound **42e** (60 mg, 0.101 mmol), 2M sodium hydroxide solution (2mL) and methanol (5 mL) were added into a reaction flask. The system was stirred for 1h at room temperature. After the reaction was completed, the system was adjusted to pH=8-10 with diluted hydrochloric acid, concentrated and purified by column chromatography (v/v, DCM/MeOH=100:1-5:1) to obtain compound **H42** (25 mg, 0.057 mmol) with a yield of 56.4%.

MS m/z (ESI): 439.3 [M+H]+.

¹H NMR (400 MHz, DMSO-*d*₆) δ 11.92 (s, 1H), 11.55 (s, 1H), 8.11 (s, 1H), 7.25 - 7.21 (m, 1H), 6.63 - 6.59 (m, 1H), 5.51 - 5.22 (m, 1H), 4.75 - 4.64 (m, 1H), 4.45 (s, 2H), 3.71 - 3.59 (m, 2H), 3.32 - 3.24 (m, 2H), 3.16 (s, 3H), 2.92 - 2.77 (m, 2H), 2.17 - 2.05 (m, 2H), 1.88 - 1.79 (m, 2H).

### Example 43

### (3aR,5s,6aS)-5-((5-cyano-1H-pyrrolo[2,3-b]pyridin-4-yl)(methyl)amino)-N-(3-methoxy-1,2 ,4-oxadiazole-5-yl)hexahydrocyclopentadiene[c]pyrrole-2(1H)-carboxamide H43

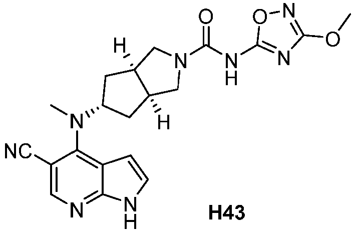

3-methoxy-5-amino-1H-1,2,4-oxadiazole was used as a raw material to obtain compound **H43** by using the same preparation method as described in Example **7.**

MS m/z (ESI): 423 [M+H]+.

### Example 44

### (3aR,5s,6aS)-5-((5-cyano-1H-pyrrolo[2,3-b]pyridin-4-yl)(methyl)amino)-N-(3-(2-amino-2-methyl-propoxy)-1,2,4-thiadiazole-5-yl)hexahydrocyclopentadiene[c]pyrrole-2(1H)-carboxamid eH44

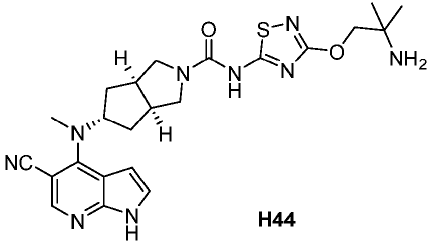

3-(2-amino-2-methyl-propoxy)-5-amino-1H-1,2,4-oxadiazole was used as a raw material to obtain compound **H44** by using the same preparation method as described in Example **7.**

MS m/z (ESI): 496 [M+H]+.

### Example 45

### (3aR,5s,6aS)-5-((5-cyano-1H-pyrrolo[2,3-b]pyridin-4-yl)(methyl)amino)-N-(3-(2-hydroxy-2-methyl-propoxy)-1,2,4-thiadiazole-5-yl)hexahydrocyclopentadiene[c]pyrrole-2(1H)-carboxami de H45

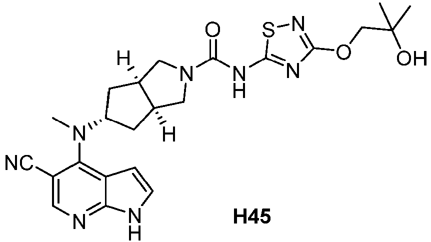

3-(2-hydroxy-2-methyl-propoxy)-5-amino-1H-1,2,4-oxadiazole was used as a raw material to obtain compound **H45** by using the same preparation method as described in Example **7.**

MS m/z (ESI): 497 [M+H]+.

### Example 46

### Preparation of (cis)-4-{[3-(propanesulfonamido)-cyclobutyl-1-yl]-amino}-1H-pyrrolo [2,3-b]pyridin-5-carbonitrile H46

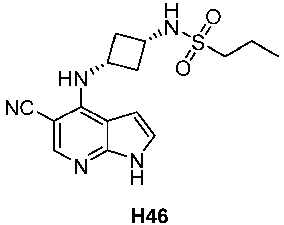

Propanesulfonyl chloride was used as a raw material to obtain compound **H46** by using the same preparation method as described in Example **12.** LCMS (ESI) m/z: 334 [M+H]⁺.

¹H NMR (400 MHz, DMSO-d6) δ 11.76 (s, 1H), 8.03 (s, 1H), 7.42 (d, J = 8.3 Hz, 1H), 7.20 (dd, J = 3.5, 2.4 Hz, 1H), 7.06 (d, J = 6.9 Hz, 1H), 6.77 (dd, J = 3.5, 1.9 Hz, 1H), 4.25 - 4.12 (m, 1H), 3.52 (h, J = 8.4 Hz, 1H), 2.95 - 2.83 (m, 2H), 2.75 (dtd, J = 9.8, 7.2, 2.9 Hz, 2H), 2.12 (qd, J = 8.8, 2.9 Hz, 2H), 1.70 - 1.53 (m, 2H), 0.94 (t, J = 7.4 Hz, 3H).

### Example 47

### (3aR,5s,6aS)-5-((5-cyano-1H-pyrrolo[2,3-b]pyridin-4-yl)(methyl)amino)-N-(3-ethoxy-1,2,4 -oxadiazole-5-yl)hexahydrocyclopentadiene[c]pyrrole-2(1H)-carboxamide H47

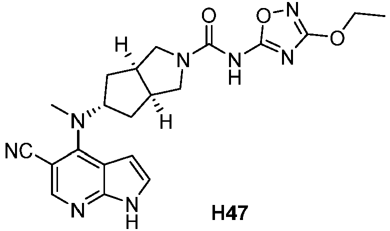

3-ethoxy-5-amino-1H-1,2,4-oxadiazole was used as a raw material to obtain compound **H47** by using the same preparation method as described in Example **7.**

MS m/z (ESI): 437 [M+H]+.

¹H NMR (400 MHz, DMSO-*d*₆) δ 11.95 (s, 1H), 10.78 (s, 1H), 8.16 (s, 1H), 7.31 (t, *J* = 3.0 Hz, 1H), 6.66 (dd, *J* = 3.7, 1.9 Hz, 1H), 4.73 (t, *J* = 8.1 Hz, 1H), 4.24 (q, *J* = 7.0 Hz, 2H), 3.61 (d, *J =* 9.8 Hz, 2H), 3.29 (d, *J =* 12.1 Hz, 2H), 3.19 (s, 3H), 2.91 - 2.79 (m, 2H), 2.21 - 2.09 (m, 2H), 1.85 (dd, *J =* 13.3, 7.6 Hz, 2H), 1.33 (t, *J* = 7.0 Hz, 3H).

### Example 48

### (3aR,5s,6aS)-5-((5-cyano-1H-pyrrolo[2,3-b]pyridin-4-yl)(methyl)amino)-N-(5-ethoxy-1,2,4 -oxadiazole-3-yl)hexahydrocyclopentadiene[c]pyrrole-2(1H)-carboxamide H48

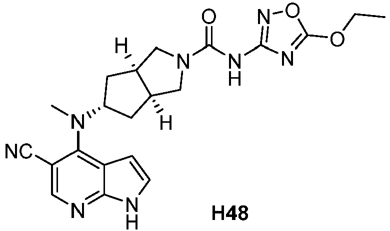

5-ethoxy-3-amino-1H-1,2,4-oxadiazole was used as a raw material to obtain compound **H48** by using the same preparation method as described in Example **7.**

MS m/z (ESI): 437 [M+H]+.
¹H NMR (400 MHz, DMSO-*d*₆)δ 11.93 (s, 1H), 10.32 (s, 1H), 8.16 (s, 1H), 7.31 (dd, J = 3.6, 2.5 Hz, 1H), 6.65 (dd, J = 3.6, 2.0 Hz, 1H), 4.83 - 4.63 (m, 1H), 4.09 (q, J = 7.1 Hz, 2H), 3.70 (dd, J = 10.7, 7.9 Hz, 2H), 3.35 (d, J = 4.3 Hz, 2H), 3.20 (s, 3H), 2.96 (h, J = 3.4 Hz, 2H), 2.17 (dt, J = 12.7, 8.2 Hz, 2H), 1.91 (ddd, J = 13.5, 7.2, 2.0 Hz, 2H), 1.20 (t, J = 7.1 Hz, 3H).

### Example 49

### (3aR,5s,6aS)-5-((5-cyano-1H-pyrrolo[2,3-b]pyridin-4-yl)amino)-N-(3-cyclopropyl-1,2,4-thiadiazole-5-yl)hexahydrocyclopentadiene[c]pyrrole-2(1H)-carboxamide H49

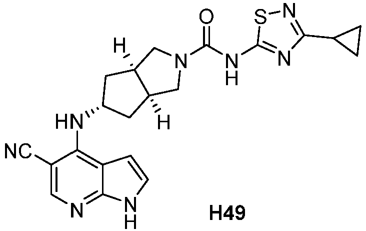

3-cyclopropyl-5-amino-1H-1,2,4-thiadiazole was used as a raw material to obtain compound **H49** by using the same preparation method as described in Example **20.**

MS m/z (ESI): 435 [M+H]+.

¹H NMR (400 MHz, DMSO-*d*₆) δ 11.74 (s, 1H), 11.41 (s, 1H), 8.02 (s, 1H), 7.17 - 7.13 (m, 1H), 6.79 (d, *J =* 8.4 Hz, 1H), 6.71 (dd, *J* = 3.5, 2.0 Hz, 1H), 4.75 - 4.66 (m, 1H), 3.65 - 3.55 (m, 2H), 3.37 - 3.31 (m, 2H), 2.92-2.80 (m, 2H), 2.10-2.03 (m, 1H), 1.99-1.87(m, 4H), 0.95-0.86 (m, 4H).

### Example 50

### Preparation of (3aR,5s,6aS)-5-((5-trifluoromethyl-1H-pyrrolo[2,3-b]pyridin-4-yl) amino)-N-(3-methoxy-1,2,4-thiadiazole-5-yl)hexahydrocyclopentadiene[c]pyrrole-2(1H)-carbox amide H50

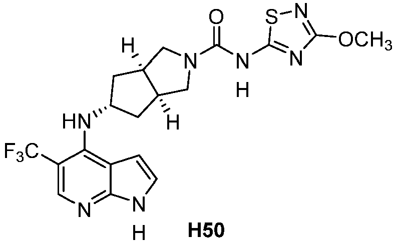

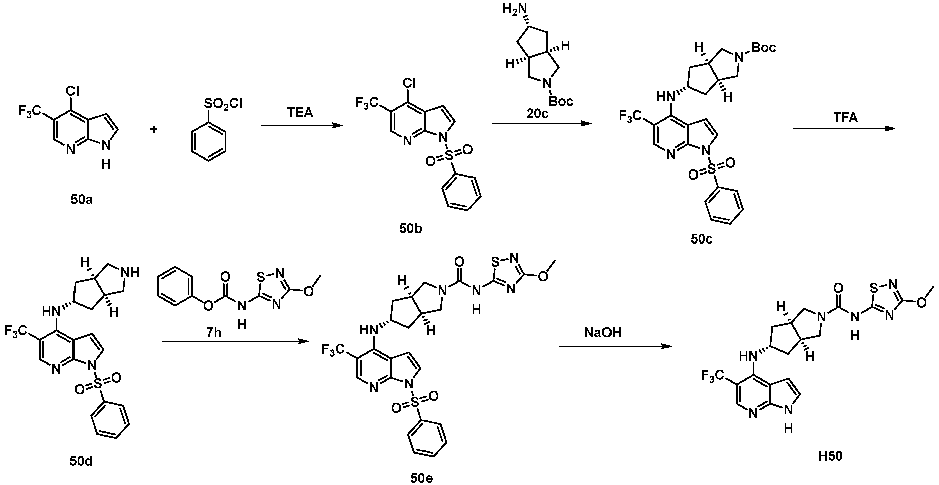

### First step: 4-chloro-1-(phenylsulfonyl)-5-(trifluoromethyl)-1H-pyrrolo[2,3-b]pyridin 50b

Compound **50a** (1.0g, 4.55 mmol), TEA (919 mg, 9.10 mmol) and DMF (10 mL) were added into a reaction flask, and then benzenesulfonyl chloride (800 mg, 4.55 mmol) was slowly added dropwise at an ice bath. The system was reacted for 2h at that temperature. After the reaction was completed, water was added. The system was extracted with EA, dried, concentrated and purified by column chromatography (v/v, DCM/MeOH=100:1-50:1) to obtain compound **50b** (1.4g, 3.89 mmol) with a yield of 85.5%.

MS m/z (ESI): 361 [M+H]+.

### Second step: tert-butyl (3aR,5s,6aS)-5-((1-(phenylsulfonyl)-5-(trifluoromethyl)-1H -pyrrolo[2,3-b]pyridin-4-yl)amino)hexahydrocyclopentadiene[c]pyrrole-2(1H)-tert-butyl carboxylate 50c

Compound **50b** (200mg, 0.56 mmol), compound **20c** (127 mg, 0.56 mmol), DIPEA (144 mg, 1.12mmol) and NMP (10 mL) were added into a reaction flask. The system was subjected to a microwave reaction for 1.5h at 160 °C. After the reaction was completed, the system was concentrated directly and purified by column chromatography (v/v, DCM/MeOH=100:1-50:1) to obtain compound **50c** (80 mg, 0.15 mmol) with a yield of 26.8%.

MS m/z (ESI): 551 [M+H]+.

### Third step: N-((3aR,5S,6AS)-octahydrocyclopenta[c] pyrrole-5-yl)-1-(phenylsulfanyl)-5-(trifluoromethyl)-1H-pyrrolo[2,3-b]pyridin-4-amine 50d

Compound **50c** (80 mg, 0.15 mmol), TFA (2 mL) and DCM (10 mL) were added into a reaction flask. The system was reacted for 0.5h at room temperature. After the reaction was completed, the system was concentrated directly and purified by column chromatography (v/v, DCM/MeOH=100:1-50:1) to obtain compound **50d** (60 mg, 0.13 mmol) with a yield of 86.7%.

MS m/z (ESI): 451 [M+H]+.

### Fourth step: (3aR,5S,6aS)-N-(3-methoxy-1,2,4-thiadiazole-5-yl)-5-((1-(phenylsulfonyl) -5-(trifluoromethyl)-1H-pyrrolo[2,13-b]pyridin-4-yl)amino)hexahydrocyclopenta[c]pyrrole-2(1H )-carboxamide 50e

Compound **50d** (60 mg, 0.13 mmol), compound 7**h** (30 mg, 0.12 mmol), DIPEA (31mg, 0.24mmol) and THF (10 mL) were added into a reaction flask. The system was reacted for 2h at 60 °C. After the reaction was completed, the system was concentrated directly and purified by column chromatography (v/v, DCM/MeOH=100:1-50:1) to obtain compound **50e** (40 mg, 0.07 mmol) with a yield of 53.8%.

MS m/z (ESI): 608 [M+H]+.

### Fifth step: (3aR,5S,6aS)-N-(3-methoxy-1,2,4-thiadiazole-5-yl)-5-((5-(trifluoromethyl)-1H -pyrrolo[2,3-b]piperidinopyridin-4-yl)amino)hexahydrocyclopenta[c]pyrrole-2(1H)-carboxamide H50

Compound **102f** (40 mg, 0.07 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H50** (20 mg, 0.04 mmol) with a yield of 57.10%.

MS m/z (ESI): 468 [M+H]+.

¹H NMR (400 MHz, DMSO-*d*₆) δ 11.66 (s, 1H), 11.56 (s, 1H), 8.04 (s, 1H), 7.22 - 7.16 (m, 1H), 6.60 (dd, *J* = 3.6, 1.8 Hz, 1H), 5.31 (d, *J* = 9.7 Hz, 1H), 4.70 - 4.62 (m, 1H), 3.86 (s, 3H), 3.61 (d, *J* = 7.0 Hz, 2H), 3.41 - 3.34 (m, 2H), 2.88 - 2.75 (m, 2H), 2.03 - 1.83 (m, 4H).

### Example 51

### (3aR,5S,6aS)-5-((5-cyano-1H-pyrrolo[2,3-b]pyridin-4-yl)(methyl)amino)-N-(3-(2-methoxy ethoxy)-1,2,4- thiadiazole-5-yl)hexahydrocyclopenta[c]pyrrole-2(1H)-carboxamide H51

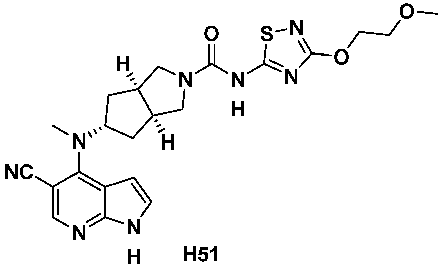

3-(2-methoxyethoxy)-5-amino-1,2,4-thiadiazole was used as a raw material to obtain compound **H51** by using the same preparation method as described in Example **7.**

MS m/z (ESI): 483 [M+H]+.

¹H NMR (400 MHz, DMSO-*d*₆) δ 11.90 (s, 1H), 11.52 (s, 1H), 8.12 (s, 1H), 7.27 - 7.22 (m, 1H), 6.62 (dd, *J* = 3.6, 2.0 Hz, 1H), 4.75 - 4.66 (m, 1H), 4.36 - 4.26 (m, 2H), 3.71 - 3.52 (m, 4H), 3.35-3.27(m, 2H), 3.25 (s, 3H), 3.16 (s, 3H), 2.91 - 2.77 (m, 2H), 2.18 - 2.07 (m, 2H), 1.90 - 1.77 (m, 2H).

### Example 52

### (3aR,5S,6aS)-5-((5-cyano-1H-pyrrolo[2,3-b]pyridin-4-yl)amino)-N-(3-(2-methoxyethoxy)-1,2,4- thiadiazole-5-yl)hexahydrocyclopenta[c]pyrrole-2(1H)-carboxamide H52

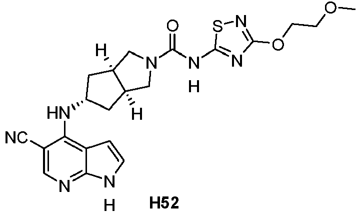

3-(2-methoxyethoxy)-5-amino-1,2,4-thiadiazole was used as a raw material to obtain compound **H52** by using the same preparation method as described in Example **20.**

MS m/z (ESI): 469 [M+H]+.

¹H NMR (400 MHz, DMSO-*d*₆) δ 11.74 (s, 1H), 11.53 (s, 1H), 8.02 (s, 1H), 7.18 - 7.13 (m, 1H), 6.79 (d, *J* = 7.3 Hz, 1H), 6.72 (dd, *J* = 3.5, 2.0 Hz, 1H), 4.76 - 4.67 (m, 1H), 4.36 - 4.29 (m, 2H), 3.71 - 3.56 (m, 4H), 3.36 - 3.29 (m, 2H), 3.26 (s, 3H), 2.93 - 2.81 (m, 2H), 2.01 - 1.90 (m, 4H).

### Example 53

### Methyl 5-((3aR,5s,6as)-5-((5-cyano-1H-pyrrole[2,3-b]pyridin-4-yl)(methyl)amino) octahydrocyclopenta[c]pyrrole-2-formamido)-1,2,4-thiadiazole-3-carboxylate H53

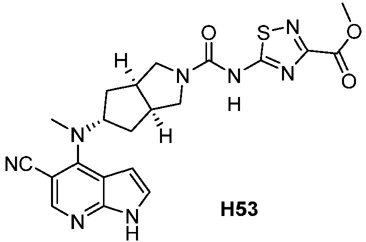

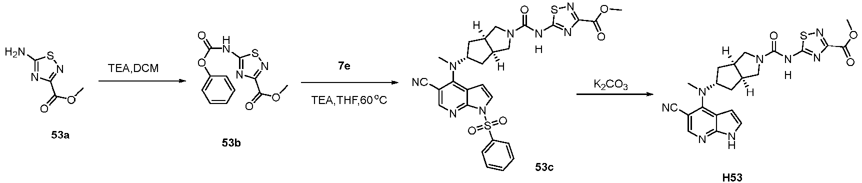

Methyl 5-amino-1,2,4-thiadiazole-3-carboxylate was used as a raw material to obtain compound **H53** by using the same preparation method as described in Example **7.**

MS m/z (ESI): 467 [M+H]⁺
¹H NMR (400 MHz, DMSO-d6) δ12.03 (s, 1H), 11.89 (s, 1H), 8.11 (s, 1H), 7.23 (dd, J = 3.7, 2.5 Hz, 1H), 6.62 (dd, J = 3.7, 2.0 Hz, 1H), 4.71 (q, J = 8.2 Hz, 1H), 3.82 (s, 3H), 3.72-3.60 (m, 2H), 3.40-3.30(m, 2H), 3.16 (s, 3H), 2.90-2.75(m, 2H), 2.12 (dt, J = 12.8, 8.3 Hz, 2H), 1.90 - 1.78 (m, 2H).

### Example 54

### (3aR,5s,6aS)-5-((5-cyano-1H-pyrrole[2,3-b]pyridin-4-yl)(methyl)amino)-N-(3-((dimethyla mino)methyl)-1,2,4-thiadiazole-5-yl)hexahydrocyclopenta[c]pyrrole-2(1H)-carboxamide H54

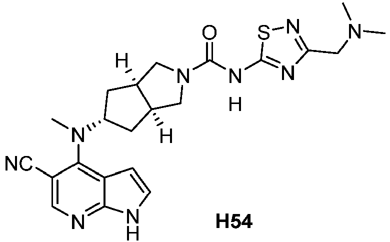

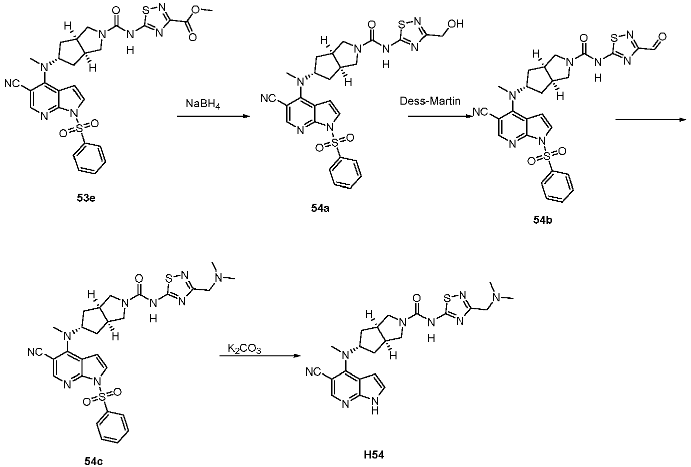

### First step: (3aR,5s,6aS)-5-((5-cyano-1-(benzenesulfonyl)-1H-pyrrolo[2,3-b]pyridin-4-yl) (methyl)amino)-N-(3-(hydroxymethyl)-1,2,4-thiadiazole-5-yl)hexahydrocyclopenta[c]pyrrole-2( 1H)-carboxamide 54a

Compound **53e** (400 mg, 0.66 mmol) was dispersed in THF (20 mL), and sodium borohydride (376 mg, 9.9 mmol) was added in batches. This mixture was stirred for 6h at room temperature and concentrated to obtain crude products. The crude products were purified by column chromatography (DCM: MeOH=15:1) to obtain compound **54a** (250 mg), which was present as gray solids, with a yield of 65%.

### Second step: (3aR,5s,6aS)-5-((5-cyano-1-(benzenesulfonyl)-1H-pyrrolo[2,3-b]pyridin-4-yl) (methyl)amino)-N-(3-formyl-1,2,4-thiadiazole-5-yl)hexahydrocyclopenta[c]pyrrole-2(1H)-carbo xamide 54b

Compound **54a** (200 mg, 0.34 mmol) and Dess-Martin (293 mg, 0.68 mmol) were dispersed in DCM (14 mL). This mixture was stirred for 2h at room temperature to obtain a reaction liquid. The reaction liquid was washed with saturated NaHCO₃ solution and Na₂SO₃ solution. The organic phases were dried and concentrated to obtain crude products **54b,** which were used for a reaction of next step.

### Third step: (3aR,5s,6aS)-5-((5-cyano-1-(benzenesulfonyl)-1H-pyrrolo[2,3-b]pyridin-4-yl) (methyl)amino)-N-(3-((dimethylamino)methyl)-1,2,4-thiadiazole-5-yl)hexahydrocyclopenta[c]p yrrole-2(1H)-carboxamide 54c

Compound **54b** (100 mg, 0.17 mmol) and hydrochloride of dimethylamine (28 mg, 0.34 mmol) were dispersed in THF (10 mL), and sodium acetic acidborohydride (72 mg, 0.34 mmol) was added in batches. This mixture was stirred for 2h at room temperature and concentrated to obtain crude products. The crude products were purified by column chromatography (DCM: MeOH=10:1) to obtain compound **54c** (30 mg), which was present as white solids, with a yield of 29%.

### Fourth step: (3aR,5s,6aS)-5-((5-cyano-1H-pyrrole[2,3-b]pyridin-4-yl)(methyl) amino)-N-(3-((dimethylamino)methyl)-1,2,4-thiadiazole-5-yl)hexahydrocyclopenta[c]pyrrole-2( 1H)-carboxamide H54

Compound **54c** (30 mg, 0.05 mmol) was dissolved in methanol (5 mL), and potassium carbonate (34 mg, 0.25 mmol) was added. This mixture was stirred for 2h at room temperature, and filtered to obtain a filtrate, which was concentrated to obtain crude products. The crude products were purified by column chromatography to obtain compound **H54** (3.5 mg), which was present as white solids, with a yield of 15%.

MS m/z (ESI): 466 [M+H]⁺
¹H NMR (400 MHz, Methanol-d4) δ 8.11 (s, 1H), 7.18 (d, J = 3.7 Hz, 1H), 6.69 (d, J = 3.7 Hz, 1H), 4.90 (s, 2H), 3.73 (dd, J = 11.1, 8.1 Hz, 2H), 3.59 (s, 2H), 3.49 - 3.40 (m, 3H), 3.01 (s, 3H), 2.31 (s, 6H), 2.27 (t, J = 8.9 Hz, 2H), 2.00 (dd, J = 13.6, 7.7 Hz, 3H).

### Example 55

### (3aR,5s,6aS)-5-((5-cyano- 1H-pyrrole[2,3-b]pyridin-4-yl)amino)-N-(3-((dimethylamino)met hyl)-1,2,4-thiadiazole-5-yl)hexahydrocyclopenta[c]pyrrole-2(1H)-carboxamide H55

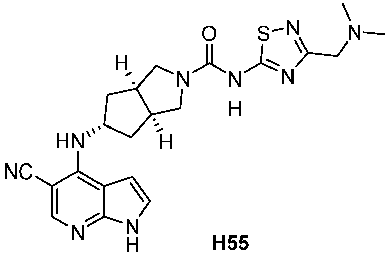

Compound **20e** was used as a raw material to obtain compound **H55** by using the same preparation method as described in Example **54.**

MS m/z (ESI): 452 [M+H]⁺
¹H NMR (400 MHz, Methanol-d4)δ 8.03 (s, 1H), 7.13 (d, J = 3.7 Hz, 1H), 6.70 (d, J = 3.6 Hz, 1H), 4.90-4.75(m, 1H), 3.75 (dd, J = 11.0, 8.1 Hz, 2H), 3.60 (s, 2H), 3.50 - 3.45 (m, 2H), 3.04 (s, 2H), 2.32 (s, 6H), 2.20-2.06 (m, 4H).

### Example 56

### (3aR,5S,6aS)-5-((5-cyano-1H-pyrrole[2,3-b]pyridin-4-yl)(methyl)amino)-N-(3-methoxymet hyl)-1,2,4-thiadiazole-5-yl)hexahydrocyclopenta[c]pyrrole-2(1H)-carboxamide H56

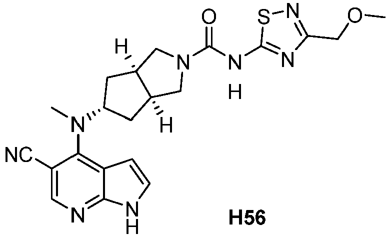

3-(methoxymethyl)-5-amino-1,2,4-thiadiazole was used as a raw material to obtain compound **H56** by using the same preparation method as described in Example **7.**

MS m/z (ESI): 453 [M+H]+.

1H NMR (400 MHz, DMSO-d6) δ 11.90 (s, 1H), 11.61 (s, 1H), 8.12 (s, 1H), 7.23 (dd, J = 3.6, 2.5 Hz, 1H), 6.62 (dd, J = 3.6, 2.0 Hz, 1H), 4.71 (q, J = 8.0 Hz, 1H), 4.41 (s, 2H), 3.65 (m, 2H), 3.37(m, 2H), 3.28(s, 3H), 3.17 (s, 3H), 2.85 (s, 2H), 2.12 (dt, J = 13.4, 8.4 Hz, 2H), 1.84 (dd, J = 13.3, 7.5 Hz, 2H).

### Example 57

### (3aR,5s,6aS)-5-((5-cyano-1H-pyrrole[2,3-b]pyridin-4-yl)amino)-N-(3-methoxymethyl)-1,2, 4- thiadiazole-5-yl)hexahydrocyclopenta[c]pyridin-2(1H)-carboxamide H57

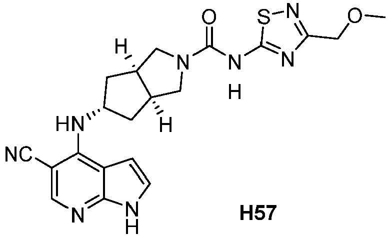

3-(methoxymethyl)-5-amino-1,2,4-thiadiazole was used as a raw material to obtain compound **H57** by using the same preparation method as described in Example **20.**

MS m/z (ESI): 439 [M+H]⁺
¹H NMR (400 MHz, DMSO-d6) δ 11.74 (s, 1H), 11.63 (s, 1H), 8.02 (d, J = 2.1 Hz, 1H), 7.21 - 7.13 (m, 1H), 6.79 (d, J = 7.8 Hz, 1H), 6.72 (dd, J = 3.6, 2.0 Hz, 1H), 4.72 (q, J = 6.8 Hz, 1H), 4.41 (s, 2H), 3.70-3.64 (m, 2H), 3.38-3.30 (m, 2H), 3.28 (s, 3H),2.92-2.85 (m, 2H), 1.99-1.92(m, 4H).

### Example 58

### N-(3-((5-cyano-1H-pyrrolo[2,3-b]pyridin-4-yl)amino)-4-ethylcyclopentyl)benzo[c][1,2,5] oxadiazole-4-sulfamide H58

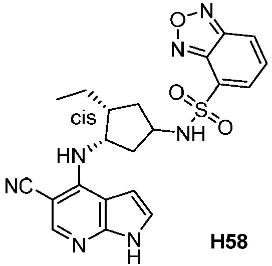

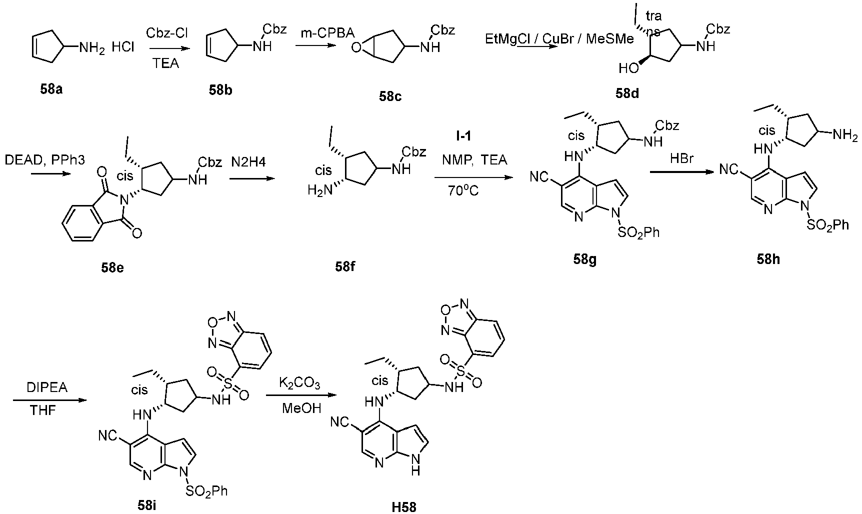

### First step: benzylcyclopentyl-3-ene-1-yl-carbamate 58b

Compound **58a** (5 g, 41.6 mmol) and triethylamine (12.6 g, 124.8 mmol) were dissolved in DCM, and CBZ-Cl (7.8 g, 45.8 mmol) was slowly added dropwise. The mixture was stirred for 2h at room temperature, quenched with water and extracted with DCM. The organic phases were washed with water, dried and concentrated to obtain crude products. The crude products were purified with column chromatography (PE: EA=3:1) to obtain compound **58b** (4 g), which was present as white solids, with a yield of 44%.

MS m/z (ESI): 218 [M+H]⁺

### Second step: benzyl (6-oxabicyclic[3.1.0]hexane-3-yl)carbamate 58c

Compound **58b** (2 g, 9.2 mmol) was dissolved in DCM (60 mL), and m-CPBA (2.3 g, 13.8 mmol) was added in batches at room temperature. The mixture was stirred for 2h at room temperature, and filtered to obtain a filtrate, which was washed with saturated sodium bicarbonate solution and sodium thiosulfate solution. The organic phases were separated, and the aqueous phase was extracted with DCM. The organic phases were combined, dried and concentrated to obtain crude products **58c,** which were used directly for a reaction of next step.

### Third step: benzyl ((3R,4R)-3-ethyl-4-hydroxylcyclopentyl)carbamate 58d

Compound **58c** (2 g, 8.5 mmol) and CuBr-DMS (17 mg, 0.085 mmol) were dissolved in anhydrous THF, and ethylmagnesium bromide (21 ml, 42.5 mmol) was slowly added dropwise under the protection of nitrogen at -60 °C. The reaction temperature was kept below -50°C and this mixture was stirred for 2h. The cold reaction mixture was added into saturated ammonium chloride solution and quenched, extracted with EA, and an organic phase was washed with water, dried and concentrated to obtain crude products. The crude products were purified by column chromatography (PE: EA=2:1) to obtain **58d** (1.9 g), which was present as yellowish solids, with a yield of 84%.

### Fourth step: benzyl ((3S,4R)-3-(1,3-dioxoisoindolinone-2-yl)-4-ethylcyclopentyl)carbamate 58e

Compound **58d** (1.9 g, 7.2 mmol), phthalimide (1.27 g, 8.6 mmol) and triphenyl phosphine (2.25 g, 8.6 mmol) were dissolved in anhydrous THF, and DIAD (1.7 g, 8.6 mmol) was slowly added dropwise at an ice bath, then heated to room temperature and stirred for 4h, quenched with water and extracted with EA. The organic phase was washed with water, dried and concentrated to obtain crude products. The crude products were purified by column chromatography (PE: EA=1:1) to obtain compound **58e** (2.8 g), which was present as white solids, with a yield of 98%.

### Fifth step: benzyl ((3S,4R)-3-amino-4-ethylcyclopentyl)carbamate 58f

Compound **58e** (3 g, 7.6 mmol) was dissolved in ethanol, and hydrazine hydrate (2.4 g, 76 mmol) was added. This reaction mixture was stirred under reflux for 2 hours, cooled, and filtered to obtain a filtrate, which was concentrated to obtain crude products. The crude products were purified by column chromatography (DCM: MeOH=10:1) to obtain compound **58f** (1 g), which was present as a colorless oily substance, with a yield of 50%.

### Sixth step: benzyl ((3S,4R)-3-((5-cyano-1-(benzenesulfonyl)-1H-pyrrole[2,3-b] pyridin-4-yl)amino)-4-ethylcyclopentyl)carbamate 58g

Compounds **58f** (1 g, 2.8 mmol) and **I-1** (0.96 g, 3 mmol) were dissolved in NMP, and DIPEA (1.5 g, 11.4 mmol) was added. This mixture was stirred for 2h at 75°C, cooled, poured into water and extracted with EA. The organic phase was washed with water for three times, dried and concentrated to obtain crude products. The crude products were purified by column chromatography (PE: EA=1:1) to obtain compound **58g** (1.2 g), which was present as yellowish solids, with a yield of 73%.

### Seventh step: 4-(((1S,2R)-4-amino-2-ethylcyclopentyl)amino)-1-(phenylsulfonyl) -1H-pyrrolo[2,3-b]pyridin-5-carbonitrile 58h

Compound **58g** (1.2 g, 2.2 mmol) was dissolved in HBr in acetic acid solution. This mixture was stirred for 2h at room temperature, and concentrated to obtain crude products **58h,** which were used directly fro a reaction of next step.

### Eighth step: N-((3S,4R)-3-((5-cyano-1-(phenylsulfonyl)-1H-pyrrolo[2,3-b]pyridin-4-yl) amino)-4-ethylcyclopentyl)benzo[c][1,2,5]oxadiazole-4-sulfamide 58i

Compound **58h** (100 mg, 0.24 mmol) and triethylamine (72 mg, 0.72 mmol) were dissolved in THF, and benzo[c][1,2,5]oxadiazole-4-sulfonyl chloride (64 mg, 0.29 mmol) was slowly added. This mixture was stirred for 2h at room temperature, quenched with water and extracted with EA. The organic phase was washed with water, dried and concentrated to obtain crude products. The crude products were purified by column chromatography to obtain **58i** (40 mg), which was present as yellowish solids, with a yield of 28%.

### Ninth step: N-((3S,4R)-3-((5-cyano-1H-pyrrolo[2,3-b]pyridin-4-yl)amino)-4-ethylcyclopentyl)benzo[c][1,2,5]oxadiazole-4-sulfamide H58

Compound **58i** (40 mg, 0.06 mmol) and potassium carbonate (41 mg, 0.3 mmol) were dissolved in methanol, stirred for 1h at room temperature and filtered to obtain a filtrate, which was concentrated to obtain crude products. The crude products were purified by column chromatography (DCM: MeOH=10:1) to obtain compound **H58** (18.3 mg), which was present as yellowish solids, with a yield of 67%.

MS m/z (ESI): 452 [M+H]⁺
¹H NMR (400 MHz, DMSO-d6) δ 11.75 (s, 1H), 8.44 (s, 1H), 8.32 (d, J = 9.1 Hz, 1H), 8.06 (dd, J = 6.7, 0.7 Hz, 1H), 8.00 (s, 1H), 7.73 (dd, J = 9.1, 6.8 Hz, 1H), 7.19 (d, J = 3.5 Hz, 1H), 6.69 (d, J = 3.5 Hz, 1H), 6.37 (d, J = 9.1 Hz, 1H), 4.67 (p, J = 7.4 Hz, 1H), 3.94 (s, 1H), 2.10 -1.96 (m, 2H), 1.87 - 1.76 (m, 1H), 1.65 - 1.56 (m, 2H), 1.32 - 1.14 (m, 1H), 1.10-1.02 (m, 1H), 0.63 (t, J = 7.4 Hz, 3H).

### Example 59

### N-(3-((5-cyano-1H-pyrrolo[2,3-b]pyridin-4-yl)amino)-4-ethylcyclopentyl)-4,4,4-trifluorobu tanamide H59

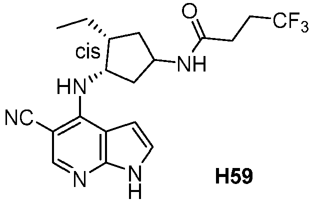

4,4,4-trifluorobutylryl chloride was used as a raw material to obtain compound **H59** by using the same preparation method as described in Example **58.**

MS m/z (ESI): 394 [M+H]⁺
¹H NMR (400 MHz, DMSO-d6) δ 11.77 (s, 1H), 8.11 (d, J = 7.1, Hz, 1H), 8.03 (s, 1H), 7.23 - 7.17 (m, 1H), 6.79 (s, 1H), 6.47 (d, J = 9.3 Hz, 1H), 4.81 - 4.71 (m, 1H), 4.25 - 4.15 (m, 1H), 2.48-2.30 (m, 4H), 2.16 (dt, J = 13.8, 7.3 Hz, 2H), 1.86 (ddt, J = 38.3, 15.4, 7.0 Hz, 2H), 1.67 (dq, J = 12.1, 6.9, 5.5 Hz, 1H), 1.37 (dt, J = 13.2, 6.2 Hz, 1H), 1.20 (t, J = 8.4 Hz, 1H), 0.77 (t, J = 7.4 Hz, 3H).

### Example 60

### N-(3-((5-cyano-1H-pyrrolo[2,3-b]pyridin-4-yl)amino)-4-ethylcyclopentyl)-3-(trifluorometh yl)benzsulfamide H60

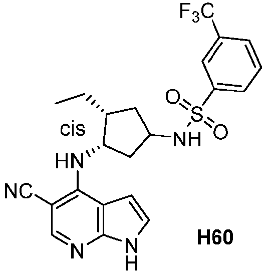

3-trifluoromethylbenzenesulfonyl chloride was used as a raw material to obtain compound **H60** by using the same preparation method as described in Example **58.**

MS m/z (ESI): 478 [M+H]⁺
¹H NMR (400 MHz, DMSO-d6) δ 11.75 (s, 1H), 8.13 - 8.05 (m, 3H), 8.02 (d, J = 8.9 Hz, 2H), 7.84 (t, J = 7.8 Hz, 1H), 7.19 (t, J = 3.0 Hz, 1H), 6.71 (dd, J = 3.6, 1.9 Hz, 1H), 6.43 (d, J = 9.1 Hz, 1H), 4.69 (q, J = 7.6 Hz, 1H), 3.73 (d, J = 6.5 Hz, 1H), 2.12 (q, J = 7.5 Hz, 1H), 1.99 (dt, J = 13.6, 6.9 Hz, 1H), 1.89 - 1.77 (m, 1H), 1.64 - 1.55 (m, 2H), 1.33 - 1.19 (m, 1H), 1.07 (m, 1H), 0.65 (t, J = 7.4 Hz, 3H).

### Example 61

### 1-((3S,4R)-3-((5-cyano-1H-pyrrolo[2,3-b]pyridin-4-yl)amino)-4-ethylcyclopentyl)-3-(3-met hoxy-1,2,4-thiadiazole-5-yl)urea H61

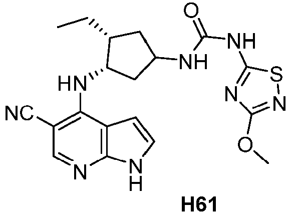

Compound **7h** was used as a raw material to obtain compound **H61** by using the same preparation method as described in Example **58.**

MS m/z (ESI): 427 [M+H]⁺
¹H NMR (400 MHz, DMSO-d6) δ 11.78 (s, 1H), 11.05 (s, 1H), 8.04 (s, 1H), 7.21 (dd, J = 3.6, 2.4 Hz, 1H), 7.06 (s, 1H), 6.79 (dd, J = 3.7, 2.0 Hz, 1H), 6.51 (s, 1H), 4.78 (p, J = 7.2 Hz, 1H), 4.24 - 4.15 (m, 1H), 3.84 (s, 3H), 2.22 (dt, J = 13.9, 7.2 Hz, 2H), 1.99 (ddd, J = 13.0, 7.4, 4.6 Hz, 1H), 1.88 (dt, J = 13.3, 7.7 Hz, 1H), 1.74 (ddd, J = 12.6, 7.4, 4.4 Hz, 1H), 1.44 - 1.29 (m, 1H), 1.25 - 1.12 (m, 1H), 0.78 (t, J = 7.4 Hz, 3H).

### Example 62

### 4-((4-((((3-hydroxy-3-methylpyrrolidin-1-yl)sulfonyl)methyl)cyclohexyl)(methyl)amino)-1 H-pyrrolo[2,3-b]pyridin-5-carbonitrile H62

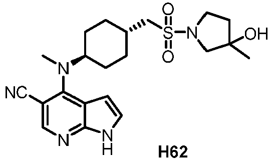

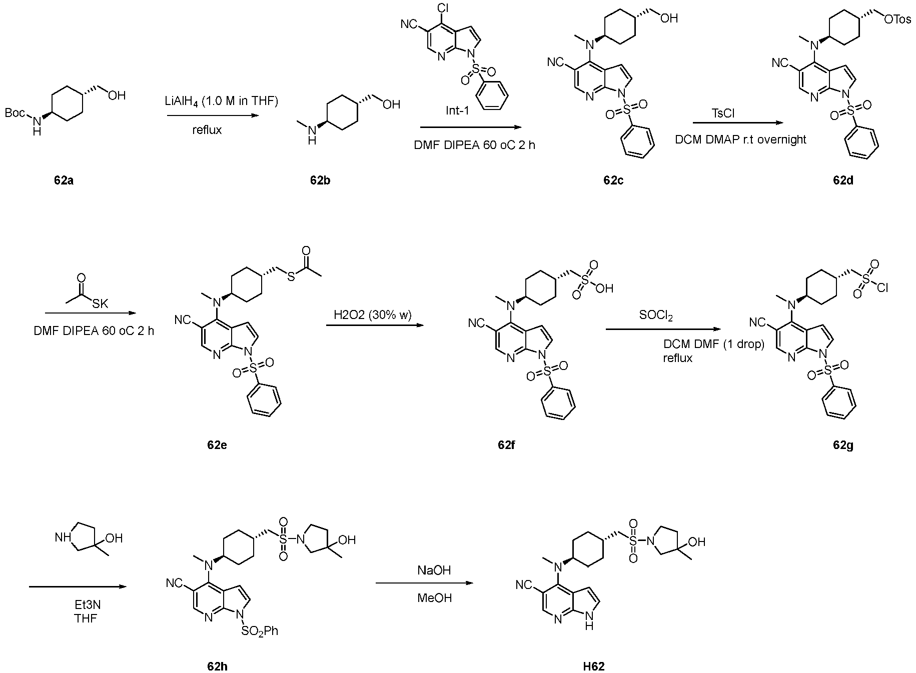

### First step: (trans-4-(methylamino)cyclohexyl)methanol 62b

Compound **62a** (1.0 g, 4.36 mmol) was added in LiAlH₄ (1.0 M in THF, 5 ml) in batches. The reaction liquid was heated to 75 °C and kept stirring for 5h. After the reaction liquid was cooled to room temperature, ethyl acetate (200 ml) was added to the reaction liquid under ice-bath condition to dilute the reaction liquid. NaOH (10 wt %, 5 ml) was added to the above reaction liquid and the reaction liquid was filtered to obtain a filter cake. The filter cake was wash with ethyl acetate twice (100 ml*2). An organic phase was concentrated and dried to obtain white solids compound **62b** (700 mg) with a yield of 70%.

¹H NMR (400 MHz, Chloroform-d) δ 3.42 (d, J = 6.3 Hz, 2H), 2.40 (d, J = 1.6 Hz, 3H), 2.28 (tt, J = 10.8, 3.9 Hz, 1H), 1.97 (dt, J = 10.2, 2.4 Hz, 2H), 1.84 - 1.70 (m, 4H), 1.44 (dddd, J = 11.4, 8.2, 5.8, 3.3 Hz, 1H), 1.08 - 0.94 (m, 4H).

### Second step: trans-4-(((4-hydroxymethyl)cyclohexyl)(methyl)amino)-1-(phenylsulfonyl) -1H-pyrrolo[2,3-b]pyridin-5-carbonitrile 62c

Compound **62b** (310 mg, 2.1 mmol), **Int-1** (450 mg, 1.41 mmol), DIPEA (550 mg, 5.2 mmol) and DMF (5 ml) were added into a 50 ml flask in sequence, gradually heated to 90 °C and reacted for 5h under the protection of nitrogen. TLC (PE/EA=3:1) showed that a small amount of raw material was not completely reacted. After the reaction liquid was cooled to room temperature, ethyl acetate (100 ml) and water (50 ml) were added to the reaction liquid. The organic phase was washed with saturated sodium chloride and dried with anhydrous sodium sulfate to obtain a filtrate. The filtrate was dried by a spin drier to obtain a yellow oily substance. Crude products were separated by silica gel column (PE/EA=10:1-1:1) to obtain 420 mg of yellow solids compound **62c,** with a yield of 70%.

MS (ESI) m/z: 425.2 (M+H⁺)
¹H NMR (400 MHz, Chloroform-d) δ 8.31 (s, 1H), 8.19 - 8.13 (m, 2H), 7.62 - 7.56 (m, 2H), 7.49 (dd, J = 8.5, 7.1 Hz, 2H), 6.63 (d, J = 4.2 Hz, 1H), 3.94 (t, J = 11.7 Hz, 1H), 3.48 (d, J = 6.2 Hz, 2H), 3.20 (s, 3H), 1.95 (d, J = 11.5 Hz, 4H), 1.74 (q, J = 11.4 Hz, 2H), 1.53 - 1.44 (m, 1H), 1.15 (q, J = 11.6 Hz, 2H).

### Third step: trans-(4-((5-cyano-1-(benzenesulfonyl)-1H-pyrrolo[2,3-b]pyridin-4-yl)(methyl) amino)cyclohexyl)4-methyl toluenesulphonate 62d

Compound **62c** (420 mg, 1.0 mmol), triethylamine (150 mmol,1.5 mmol), dichloromethane (10 ml) and TosCl (280 mg, 1.5 mmol) were added into a 25 ml flask in sequence at room temperature, stirred to react overnight to obtain a reaction liquid. TLC (PE/EA=3:1) showed that raw materials were completely reacted. Ethyl acetate (100 ml) and water (50 ml) were added to the reaction liquid. The organic phase was washed with saturated sodium chloride and dried with anhydrous sodium sulfate to obtain a filtrate. The filtrate was dried by spin to obtain crude products. The crude products were separated by preparative plate (PE/EA=2:1) to obtain 500 mg of white solids compound **62d,** with a yield of 86%.

MS (ESI) m/z: 396.2 (M+H⁺)
¹H NMR (400 MHz, Chloroform-d) δ 8.29 (s, 1H), 8.18 - 8.13 (m, 2H), 7.78 - 7.73 (m, 2H), 7.59 (dd, J = 8.4, 5.8 Hz, 2H), 7.49 (t, J = 7.7 Hz, 2H), 7.33 (d, J = 8.1 Hz, 2H), 6.60 (d, J = 4.1 Hz, 1H), 3.88 (s, 1H), 3.81 (d, J = 6.4 Hz, 2H), 3.16 (s, 3H), 2.44 (s, 3H), 1.90 (t, J = 14.5 Hz, 4H), 1.67 (s, 3H), 1.17 - 1.05 (m, 2H).

### Fourth step: trans-((4-((5-cyano-1-(benzenesulfonyl)-1H-pyrrolo[2,3-b]pyridin-4-yl) (methyl)amino)cyclohexyl)methyl)thioacetate 62e

Compound **62d** (500 mg, 0.86 mmol), potassium thioacetate (200 mg, 1.73 mmol) and DMF (5 ml) were added into a 50 ml flask in sequence. The reaction liquid was heated to 60 °C and stirred for 2h. TLC (PE/EA=3:1) showed that raw materials were completely reacted. Ethyl acetate (100 ml) and water (50 ml) were added to the reaction liquid. The organic phase was washed with saturated sodium chloride solution once and dried with anhydrous sodium sulfate to obtain a filtrate. The filtrate was dried by a spin drier to obtain white solids compound **62e** (350 mg), with a yield of 84%.

LCMS (ESI-MS) m/z: 483.3 (M+H⁺).

¹H NMR (400 MHz, Chloroform-d) δ 8.30 (s, 1H), 8.16 (d, J = 7.7 Hz, 2H), 7.59 (dd, J = 9.2, 5.7 Hz, 2H), 7.49 (t, J = 7.7 Hz, 2H), 6.61 (d, J = 4.1 Hz, 1H), 3.92 (s, 1H), 3.16 (s, 3H), 2.79 (d, J = 6.6 Hz, 2H), 2.32 (d, J = 0.8 Hz, 3H), 1.94 (t, J = 16.3 Hz, 4H), 1.68 (d, J = 11.7 Hz, 3H), 1.16 (q, J = 12.1 Hz, 2H).

### Fifth step: trans-(4-((5-cyano-1-(benzenesulfonyl)-1H-pyrrolo[2,3-b]pyridin-4-yl) (methyl)amino)cyclohexyl)methanesulfonic acid 62f

Compound **62e** (250 mg, 0.51 mmol) was dissolved in formic acid (3.0 ml) under an ice-bath condition to obtain a reaction liquid. Hydrogen peroxide (30%aq, 0.5 ml) solution was added in the above reaction liquid dropwise. The reaction liquid was stirred to react overnight at room temperature. LCMS showed that a small amount of raw material remained. Saturated sodium sulfite solution (20 ml) was slowly added in the reaction liquid dropwise under an ice-bath condition to quench the reaction. Starch KI test paper detected that hydrogen peroxide was completely quenched. (Dichloromethane: methanol=10:1, 50ml × 4) was added in the reaction liquid, and the organic phase was concentrated to dryness to obtain crude products. Crude products were separated by silica gel column (dichloromethane: methanol=20:1-8:1) to obtain white solids **62f** (200 mg), with a yield of 80%.

MS (ESI) m/z: 489.2 (M+H⁺)
¹H NMR (400 MHz, DMSO-d6) δ 8.24 (s, 1H), 8.08 - 8.01 (m, 2H), 7.76 - 7.67 (m, 2H), 7.64 - 7.57 (m, 2H), 6.86 (d, J = 4.2 Hz, 1H), 3.89 - 3.79 (m, 1H), 3.13 (s, 3H), 2.28 (d, J = 6.2 Hz, 2H), 2.03 (d, J = 13.0 Hz, 2H), 1.72 (q, J = 6.3 Hz, 5H), 1.07 - 0.94 (m, 2H).

### Sixth step: trans-(4-((5-cyano-1-(benzenesulfonyl)-1H-pyrrolo[2,3-b]pyridin-4-yl) (methyl)amino)cyclohexyl)methylsulfonyl chloride 62g

Compound **62f** (200 mg, 0.4 mmol) was dispersed in dichloromethane (30 ml) to obtain a reaction liquid. Thionyl chloride (1.0 ml, 13.4 mmol) was added in the above reaction liquid, and a drop of DMF was added. The reaction liquid was subjected to a reflux reaction under the protection of nitrogen for 2h. LCMS showed that the reaction was complete substantially. The reaction liquid was directly adjusted to pH=8 with saturated sodium bicarbonate solution without purification under an ice-bath condition to obtain crude products **62g,** which were used for a reaction of next step.

LCMS (ESI-MS) m/z: 507.2 (M+H⁺)

### Seventh step: trans-4-((4-((((3-hydroxy-3-methylpyrrolidin-1-yl)sulfonyl)methyl) cyclohexyl)(methyl)amino)-1-(phenylsulfonyl)-1H-pyrrolo[2,3-b]pyridin-5-carbonitrile 62h

Et₃N (303.3 mg, 3.0mmol) was added in compound Int-2 (202.2mg, 2.0 mmol) in THF solution under an ice-bath condition, and then compound **62g** (0.4 mmol) was added dropwise to obtain a reaction liquid. The reaction liquid was kept reacting at the ice bath for 1h. LCMS showed that raw material was reacted completely. Dichloromethane (50 ml) and water (20 ml) were added in the reaction liquid. The organic phase was dried and concentrated to obtain yellow compound **62h** (100 mg), with a yield of 44%.

LCMS (ESI-MS) m/z: 572.3 (M+H⁺)

### Eighth step: 4-((trans-4-((((3-hydroxy-3-methylpyrrolidin-1-yl)sulfonyl)methyl) cyclohexyl)(methyl)amino)-1H-pyrrolo[2,3-b]pyridin-5-carbonitrile H62

Compound **62h** (100 mg, 0.2 mmol) was dissolved in methanol (3 ml), and NaOH (200uL, 0.6mmol, 4 M in water) was added to obtain a reaction liquid. The reaction liquid was stirred for 2h at room temperature. The reaction liquid was separated by TLC (DCM/MeOH=15:1) silica gel preparative plate to obtain white solids compound **H62** (21 mg), with a yield of 24%.

MS (ESI) *m*/*z*: 432.3 (M+H⁺).

¹H NMR (400 MHz, DMSO-*d*₆) δ 11.88 (s, 1H), 8.10 (s, 1H), 7.28 (dd, *J* = 3.6, 2.5 Hz, 1H), 6.56 (dd, *J* = 3.6, 2.0 Hz, 1H), 4.83 (s, 1H), 3.93 (q, *J* = 6.4, 5.6 Hz, 1H), δ 3.34 (dd, *J* = 10.0, 7.6 Hz, 1H), 3.29 - 3.24 (m, 1H), 3.20 - 3.03 (m, 5H), 3.00 - 2.85 (m, 2H), 2.06 - 1.90 (m, 3H), 1.88 - 1.68 (m, 8H), 1.24 (s, 3H).

### Example 63

### Trans-4-(methyl((1r,4r)-4-(((4-methylpiperazin-1-yl)sulfonyl)methyl)cyclohexyl)amino)-1 H-pyrrolo[2,3-b]pyridin-5-carbonitrile H63

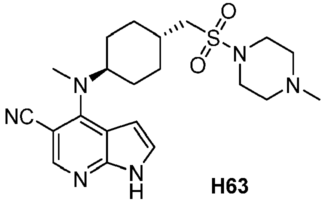

N-methylpiperazine was used as a raw material to obtain compound **H63** by using the same preparation method as described in Example **62.**

MS (ESI) *m*/*z*: 431.3 (M+H⁺).

¹H NMR (400 MHz, DMSO-*d*₆) δ 11.98 (s, 1H), 8.20 (s, 1H), 7.38 (dd, *J* = 3.6, 2.5 Hz, 1H), 6.66 (dd, *J* = 3.6, 2.0 Hz, 1H), 4.06 - 3.96 (m, 1H), 3.26 (s, 3H), 3.20 (t, *J* = 4.9 Hz, 4H), 3.01 (d, *J =* 6.4 Hz, 2H), 2.44 (t, *J* = 4.7 Hz, 4H), 2.27 (s, 3H), 2.10 (d, *J* = 13.0 Hz, 2H), 1.99 - 1.87 (m, 5H), 1.39 - 1.24 (m, 2H).

### Example 64

### trans-4-((4-(((3-(2-methoxyethoxy)piperidin-1-yl)sulfonyl)methyl)cyclopentyl)(methyl)ami no)-1H-pyrrolo[2,3-b]pyridin-5-carbonitrile H64

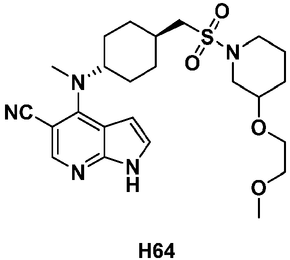

3-(2-methoxyethoxy)piperidine was used as a raw material to obtain compound **H64** by using the same preparation method as described in Example **62.**

MS m/z (ESI): 490 [M+H]+.

¹H NMR (400 MHz, DMSO-*d*₆) δ 11.88 (s, 1H), 8.10 (s, 1H), 7.32 - 7.25 (m, 1H), 6.56 (dd, *J* = 3.5, 2.0 Hz, 1H), 3.95 - 3.87 (m, 1H), 3.57-3.16 (m, 13H), 3.10-2.85 (m, 4H), 2.06 - 1.97 (m, 2H), 1.89 - 1.74 (m, 6H), 1.72 - 1.65 (m, 1H), 1.43 - 1.34 (m, 2H), 1.27 - 1.20 (m, 2H).

### Example 65

### 4-((trans-4-(((3-isobutoxypiperidin-1-yl)sulfonyl)methyl)cyclohexyl)(methyl)amino)-1H-py rrolo[2,3-b]pyridin-5-acetonitrile H65

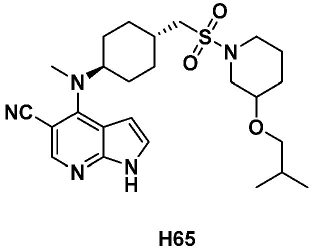

3-(2-methylpropoxy)piperidine was used as a raw material to obtain compound **H65** by using the same preparation method as described in Example **62.**

MS m/z (ESI): 488 [M+H]+.

¹H NMR (400 MHz, DMSO-*d*₆) δ11.88 (s, 1H), 8.11 (s, 1H), 7.31 - 7.25 (m, 1H), 6.59 - 6.52 (m, 1H), 3.95 - 3.87 (m, 1H), 3.45 - 3.38 (m, 1H), 3.34 - 3.29 (m, 1H), 3.27 - 3.18 (m, 2H), 3.18 - 3.11 (m, 4H), 2.99 - 2.80 (m, 4H), 2.04 - 1.96 (m, 2H), 1.91 - 1.74 (m, 6H), 1.75 - 1.66 (m, 2H), 1.44 - 1.33 (m, 2H), 1.27 - 1.20 (m, 2H), 0.82 (s, 6H).

### Example 66

### 4-((trans-4-(((trans-3-(hydroxymethyl)-4-methylpyrrolidin-1-yl)sulfonyl)methyl)cyclohexyl )(methyl)amino)-1H-pyrrolo[2,3-b]pyridin-5-carbonitrile H66

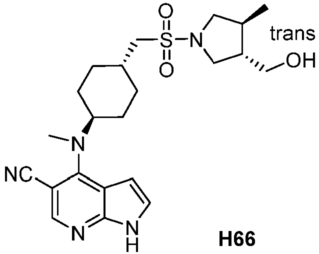

Trans-3-(hydroxymethyl)-4-methylpyrrolidine was used as a raw material to obtain compound **H66** by using the same preparation method as described in Example **62.**

MS (ESI) *m*/*z*: 446 (M+H) ⁺.

¹H NMR (400 MHz, DMSO-d6) δ 11.88 (s, 1H), 8.10 (s, 1H), 7.28 (dd, J = 3.6, 2.5 Hz, 1H), 6.56 (dd, J = 3.6, 2.0 Hz, 1H), 4.64 (t, J = 5.2 Hz, 1H), 3.95 - 3.87 (m, 1H), 3.49 - 3.40 (m, 2H), 3.40 - 3.30 (m, 2H), 3.16 (s, 3H), 3.03 (dd, J = 9.9, 8.1 Hz, 1H), 2.94 (d, J = 6.4 Hz, 2H), 2.78 (dd, J = 9.6, 8.6 Hz, 1H), 2.06 - 1.95 (m, 3H), 1.89 - 1.73 (m, 6H), 1.29 - 1.20 (m, 2H), 0.95 (d, J = 6.5 Hz, 3H).

### Example 67

### 4-(methyl-(trans-4-(((3-((methylamino)methyl)pyrrolidin-1-yl)sulfonyl)methyl)cyclohexyl) amino)-1H-pyrrolo[2,3-b]pyridin-5-carbonitrile H67

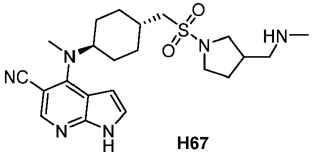

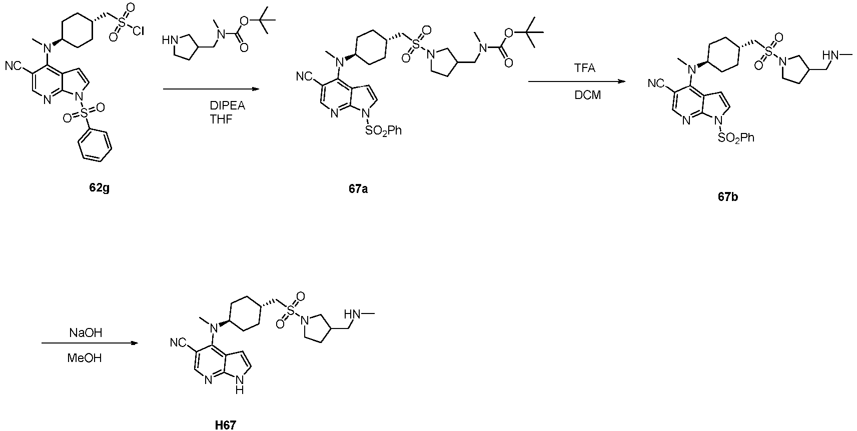

### First step: 4-(methyl(trans-4-(((3-((N-Boc-methylamino)methyl)pyrrolidin-1-yl)sulfonyl) methyl)cyclohexyl)amino)-1-(phenylsulfonyl)-1H-pyrrolo[2,3-b]pyridin-5-carbonitrile 67a

DIEA (387.9 mg, 3.0mmol) was added in compound 3-((N-Boc-methylamino)methyl)pyrrolidine (214.2mg, 1.0 mmol) in THF solution under an ice-bath condition, and then compound **62a** (0.4 mmol) was added dropwise. The system was naturally raised to room temperature to react overnight. LCMS showed that raw material was reacted completely. Dichloromethane (50 ml) and water (20 ml) were added in above reaction liquid. The organic phase was dried and concentrated to obtain yellow compound **67a** (200 mg), with a yield of 73%.

MS (ESI) m/z: 685.4 (M+H⁺)

### Second step: 4-(methyl(trans-4-(((3-((methylamino)methyl)pyrrolidin-1-yl)sulfonyl)methyl) cyclohexyl)amino)-1-(phenylsulfonyl)-1H-pyrrolo[2,3-b]pyridin-5-carbonitrile 67b

Compound **67a** (200 mg, 0.3 mmol) was dissolved in dichloromethane (3 ml), and TFA (100 µL, 1.2mmo) was added to obtain a reaction liquid. The reaction liquid was stirred for 1h at room temperature. The reaction liquid was concentrated directly to obtain crude products compound **67b** (150 mg), with a yield of 86%, which were subjected to a reaction of next step.

### Third step: 4-(methyl-(trans-4-(((3-((methylamino)methyl)methyl)pyrrolidinobutyl-1-yl) sulfonyl)methyl)cyclohexyl)amino)-1H-pyrrolo[2,3-b]pyridin-5-carbonitrile H67

Compound **67b** (150 mg, 0.2 mmol) was dissolved in methanol (3 ml), and NaOH (200uL, 0.6mmol, 4 M in water) was added to obtain a reaction liquid. The reaction liquid was stirred for 2h at room temperature. The reaction liquid was separated by TLC (DCM/MeOH=15:1) silica gel preparative plate to obtain white solids compound **H67** (30 mg), with a yield of 34%.

MS (ESI) *m*/*z*: 445.3 (M+H⁺).

¹H NMR (400 MHz, DMSO-*d*₆) δ 11.89 (s, 1H), 8.10 (s, 1H), 7.28 (d, *J* = 3.6 Hz, 1H), 6.56 (d, *J* = 3.7 Hz, 1H), 4.02 - 3.79 (m, 1H), 3.35 - 3.16 (m, 7H), 2.98 - 2.93 (m, 2H), 2.60 - 2.50 (m, 2H), 2.35 (d, *J* = 7.2 Hz, 1H), 2.30 (s, 3H), 2.06 - 1.98 (m, 2H), 1.97 - 1.90 (m, 1H), 1.87 - 1.73 (m, 5H), 1.56 (dq, *J* = 12.4, 8.1 Hz, 1H), 1.21 (d, *J* = 13.3 Hz, 3H).

### Example 68

### 4-((trans-4-(((3-(methoxymethyl)pyrrolidin-1-yl)sulfonyl)methyl)cyclohexyl)(methyl)amin o)-1H-pyrrolo[2,3-b]pyridin-5-carbonitrile H68

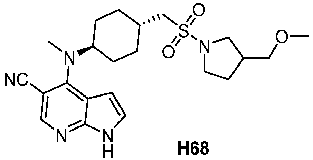

3-(methoxymethyl)piperidine was used as a raw material to obtain compound **H68** by using the same preparation method as described in Example **62.**

MS (ESI-MS) *m*/*z*: 446 (M+H⁺).

¹H NMR (400 MHz, DMSO-*d*₆) δ 11.88 (s, 1H), 8.10 (s, 1H), 7.38 - 7.21 (m, 1H), 6.56 (s, 1H), 3.92 (s, 1H), 3.51-3.10 (m, 12H), 2.93 (dd, *J* = 11.2, 6.8 Hz, 2H), 2.46 (s, 2H), 2.11 - 1.71 (m, 6H), 1.57 (dd, *J* = 12.8, 7.5 Hz, 1H), 1.32-1.20 (m, 3H).

### Example 69

### 4-((trans-4-(((3-(hydroxymethyl)pyrrolidin-1-yl)sulfonyl)methyl)cyclohexyl)(methyl)amino ) -1H-pyrrolo[2,3-b]pyridin-5-carbonitrile H69

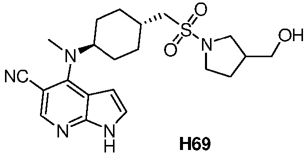

3-(hydroxymethyl)piperidine was used as a raw material to obtain compound **H69** by using the same preparation method as described in Example 62.

MS (ESI) *m*/*z*: 432 (M+H⁺).

¹H NMR (400 MHz, DMSO-*d*₆) δ 11.93 (s, 1H), 8.14 (s, 1H), 7.32 (dd, *J* = 3.6, 2.5 Hz, 1H), 6.60 (dd, *J* = 3.7, 2.0 Hz, 1H), 4.73 (t, *J* = 5.3 Hz, 1H), 3.95 (dt, *J* = 10.1, 4.7 Hz, 1H), 3.44 - 3.21 (m, 8H), 3.08 - 2.91 (m, 3H), 2.32 (p, *J =* 7.0 Hz, 1H), 2.14 - 1.98 (m, 2H), 1.88 (dddd, *J =* 24.3, 10.4, 7.5, 2.8 Hz, 5H), 1.63 (dt, *J* = 12.4, 7.7 Hz, 1H), 1.46 - 1.09 (m, 3H).

### Example 70

### 4-((cis-(3-(((3-((2-methoxyethoxy)piperidin-1-yl)sulfonyl)methyl)cyclopentyl)(methyl)ami no)-1H-pyrrolo[2,3-b]pyridin-5-carbonitrile H70

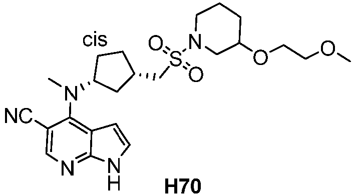

Cis-3-(methylamino)-cyclopentyl-methanol was used as a raw material to obtain compound **H70** by using the same preparation method as described Example **64.**

MS m/z (ESI): 476 [M+H]+.

¹H NMR (400 MHz, DMSO-*d*₆) δ 11.91 (s, 1H), 8.12 (s, 1H), 7.33 - 7.27 (m, 1H), 6.67 - 6.60 (m, 1H), 4.65 - 4.49 (m, 1H), 3.58 -3.15 (m, 15H), 2.99 - 2.82 (m, 2H) , 2.30 - 2.10 (m, 2H), 2.01 - 1.76 (m, 4H), 1.76 - 1.59 (m, 2H), 1.59 - 1.33 (m, 3H).

### Example 71

### 4-((cis-3-(((3-((hydroxymethyl)pyrrolidin-1-yl)sulfonyl)methyl)cyclopentyl)(methyl)amino ) -1H-pyrrolo[2,3-b]pyridin-5-carbonitrile H71

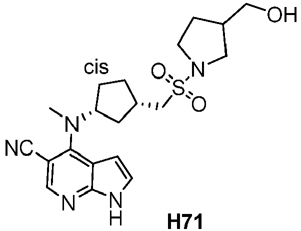

3-(hydroxymethyl)piperidine was used as a raw material to obtain compound **H71** by using the same preparation method as described in Example **70.**

MS (ESI) m/z: 418
¹H NMR (400 MHz, DMSO-d6) δ 11.90 (s, 1H), 8.12 (s, 1H), 7.30 (dd, J = 3.6, 2.4 Hz, 1H), 6.64 (dd, J = 3.6, 1.8 Hz, 1H), 4.69 (t, J = 5.3 Hz, 1H), 4.57 (p, J = 7.6 Hz, 1H), 3.41 - 3.17 (m, 10H), 2.97 (dd, J = 9.8, 6.8 Hz, 1H), 2.29 - 2.17 (m, 3H), 1.98 - 1.78 (m, 3H), 1.74 - 1.44 (m, 3H).

### Example 72

### (R)-7-((5-cyano-1H-pyrrolo[2,3-b]pyridin-4-yl)amino)-N-(2,2,2-trifluoroethyl)-5-azaspiro[ 2.4]heptane-5-carboxamide H72

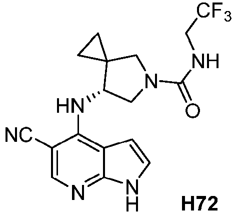

(R)-(5-azaspiro[2.4]heptane-7-yl)tert-butyl carbamate was used as a raw material to obtain compound **H72** by using the same preparation method as described in Example **2.**

MS m/z (ESI): 379 [M+1]⁺
¹H NMR (400 MHz, DMSO-*d*₆) δ 11.85 (s, 1H), 8.09 (s, 1H), 7.27 (dd, *J =* 3.6, 2.5 Hz, 1H), 6.89 (t, *J* = 6.3 Hz, 1H), 6.82 (dd, *J* = 3.5, 2.0 Hz, 1H), 6.71 (d, *J* = 7.6 Hz, 1H), 4.45 (s, 1H), 3.84 - 3.68 (m, 4H), 3.63 (dd, *J* = 10.9, 2.9 Hz, 1H), 3.21 (d, *J* = 10.2 Hz, 1H), 0.87 (dd, *J* = 9.2, 4.5 Hz, 1H), 0.77 (dd, *J* = 9.7, 5.5 Hz, 1H), 0.69 (d, *J* = 6.3 Hz, 2H).

### Example 73

### (cis)-3-isopropyl-4-(imidazo[4,5-d]pyrrolo[2,3-b]pyridin-1(6H)-yl)-N-(2,2,2-trifluoroethyl) pyrrolidin-1-carboxamide H73

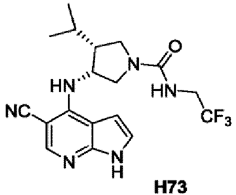

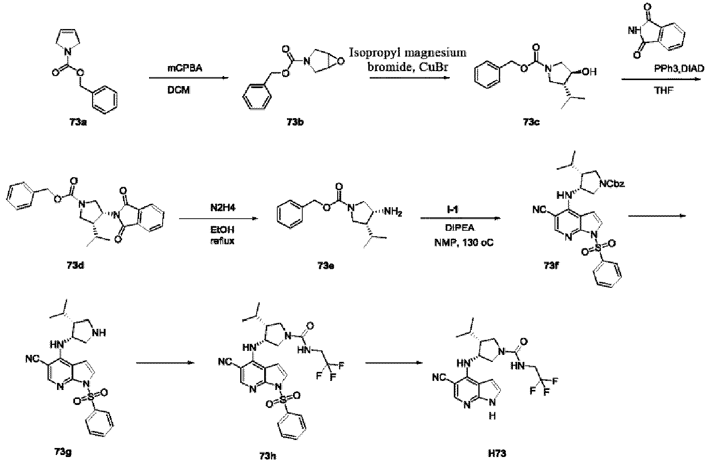

### First step: benzyl 6-oxa-3-azabicyclo[3.1.0]hexyl-3-carboxylate 73b

Compound **73a** (17 g, 83.6 mmol) was dissolved in 120 mL dichloromethane, and m-CPBA (29.4g, 167.2 mmol) was added in batches under an ice bath for reacting overnight at room temperature. After the reaction was completed, the reaction solution was filtered to obtain a filtrate. A sodium hydrogen sulfite solution was added in the filtrate and stirred for 0.5h, extracted with EA and separated by column chromatography (PE : EA = 10:1) to obtain compound **73b** (10g), with a yield of 56%.

MS (ESI) m/z: 242 (M+Na⁺).

### Second step: benzyl (trans)-3-isopropyl-4-hydroxypyrrolidin-1-carboxylate 73c

Compound **73b** (2.19g, 10.0 mmol) was dissolved in 20 mL anhydrous THF, and CuBr (150 mg, 1.0 mmol) was added, substituted by hydrogen for three times, and then cyclopropyl magnesium bromide (30 mL, 90 mmol, 3 M in THF) was added dropwise under a dry ice bath of ethyl acetate, and naturally heated to room temperature to react overnight. After the reaction was completed, the system was quenched with ammonium chloride solution, extracted with EA and separated by column chromatography (PE: EA = 3: 1) to obtain compound **73c** (1.6g), with a yield of 60%.

MS (ESI) m/z: 264 (M+H⁺)

### Third step: benzyl (cis)-3-isopropyl-4-(1,3-dioxoisoindol-2-yl)pyrrolidin-1-carboxylate 73d

Compound **73c** (1.6g, 6.08mmol) was dissolved in 10 mL anhydrous tetrahydrofuran, and PPh₃ (1.91g, 7.3 mmol) and phthalimide (1.07g, 7.3 mmol) were added. The system was substituted by nitrogen for three times. DIAD (1.91g, 7.3mmol) was added under an ice bath and naturally heated to room temperature to react overnight. After the reaction was completed, water was added. The system was extracted with EA and the organic phases were collected. Then the system was separated by column chromatography (PE: EA = 3:1) to obtain crude products **73d** (3.0 g).

MS (ESI-MS) m/z: 393 (M+H⁺).

### Fourth step: benzyl (cis)-3-amino-4-isopropylpyrrolidin-1-carboxylate 73e

Compound **73d** (3.0g, crude products) was dissolved in 10 mL ethanol, and hydrazine hydrate (979.2 mg, 30.6 mmol) was added, heated to 80 °C and reacted for 2h. After the reaction was completed, saturated NaCl solution was added. The system was extracted with DCM, and organic phases were collected. Then the system was separated by column chromatography (DCM: MeOH = 95:5) to obtain compound **73e** (1.2g).

MS (ESI) m/z: 263 (M+H⁺)

### Fifth step: (cis)-3-isopropyl-4-((5-cyano-1-(benzenesulfonyl)-1H-pyrrole[2,3-b] pyridin-4-yl)amino)pyrrolidin-1-benzyl formate 73f

Compound **73e** (496 g, 1.89mmol) was dissolved in 5 mL NMP, and then Int-1 (300mg, 0.94mmol) and DIEA (364 mg, 2.82 mmol) were added, heated to 130 °C and reacted for 3h. After the reaction was completed, water was added to wash NMP, and the system was extracted with DCM, then separated by column chromatography (DCM: MeOH = 98: 2) to obtain compound **73f** (100 mg).

MS (ESI) *m*/*z*: 544 (M+H⁺).

### Sixth step: 4-(((cis)-4-isopropylpyrrolidin-3-yl)amino)-1-(phenylsulfonyl)-1H -pyrrolo[2,3-b]pyridin-5-carbonitrile 73g

Compound **73f** (100mg, crude products) was dissolved in 5 mL dichloromethane, and hydrogen bromide in glacial acetic acid solution (1 ml) was added, stirred and reacted for 2h at room temperature. After the reaction was completed, the system was mixed with silica gel, and subjected to column chromatography (DCM: MeOH = 10:1) to obtain compound **73g** (50 mg), which was present as an oily substance.

MS (ESI) *m*/*z*: 410 (M+H⁺).

### Seventh step: (cis)-3-((5-cyano-1-(phenylsulfonyl)-1H- pyrrolo[2,3-b]pyridin-4-yl)amino) -4-isopropyl-N-(2,2,2-trifluoroethyl)pyrrolidin-1-carboxamide 73h

Compound **73g** (50 mg, 0.12mmol) was dissolved in THF, and phenyl (2,2,2-trifluoroethyl)carbamate (50mg, 0.122mmol) and potassium carbonate (25 mg, 0.18mmol) were added to perform a reflux reaction for 4h to obtain a reaction liquid. After the reaction was completed, the reaction liquid was used directly for next step.

MS (ESI) *m*/*z*: 535 (M+H⁺).

### Eighth step: (cis)-3-((5-cyano-1H-pyrrolo[2,3-b]pyridin-4-yl)amino)-4-isopropyl -N-(2,2,2-trifluoroethyl)pyrrolidin-1-carboxamide H73

Compound **73h** (50 mg, 0.09mmol) was dissolved in 2 mL methanol, and 2N sodium hydroxide (2 mL) was added, stirred for 1h at room temperature. After the reaction was completed, the system was extracted with EA, and the organic phase was dried by a spin drier, concentrated, followed by column chromatography (DCM: MeOH=15:1) to obtain compound **H73** (20 mg).

MS (ESI) *m*/*z*: 395 (M+H⁺).

¹H NMR (400 MHz, Methanol-*d*₄) δ 8.10 (s, 1H), 7.23 (d, *J* = 3.7 Hz, 1H), 6.73 (d, *J* = 3.7 Hz, 1H), 3.87 - 3.66 (m, 5H), 3.42 (t, *J* = 10.9 Hz, 1H), 3.33 (s, 1H), 2.30 (d, *J* = 23.2 Hz, 1H), 1.94 - 1.81 (m, 1H), 1.03 (d, *J* = 6.5 Hz, 3H), 0.93 (d, *J* = 6.5 Hz, 3H).

### Example 74

### (cis)-3-((5-cyano-1H-pyrrolo[2,3-b]pyridin-4-yl)amino)-4-cyclopropyl-N-isobutylpyrrolidin -1-carboxamide H74

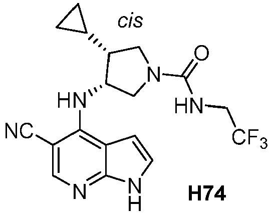

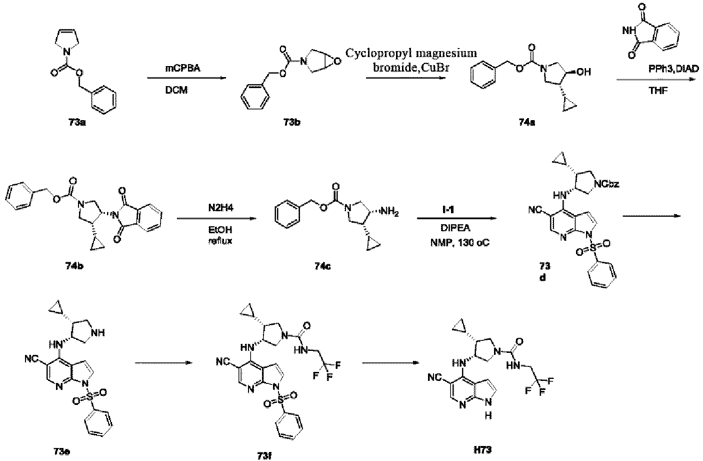

Cyclopropyl magnesium bromide was used as a raw material to obtain compound **H74** by using the same preparation method as described in Example **73.**

MS (ESI) *m*/*z*: 393 (M+H⁺).

¹H NMR (400 MHz, DMSO-*d*₆) δ 11.83 (s, 1H), 8.09 (s, 1H), 7.25 (dd, *J* = 3.6, 2.4 Hz, 1H), 6.89 - 6.79 (m, 2H), 6.62 (d, *J* = 8.7 Hz, 1H), 4.87 (q, *J* = 6.6, 6.1 Hz, 1H), 3.82 - 3.65 (m, 3H), 3.54 - 3.42 (m, 3H), 1.80 (p, *J* = 6.8 Hz, 1H), 0.81 (tq, *J* = 9.5, 5.1, 4.6 Hz, 1H), 0.38 (ddt, *J =* 22.2, 8.3, 4.2 Hz, 2H), 0.18 - 0.04 (m, 2H).

### Example 75

### (cis)-3-((5-cyano-1H-pyrrolo[2,3-b]pyridine-4-yl)amino)-4-ethyl-N-isobutylpyrrolidin-1-ca rboxamide H75

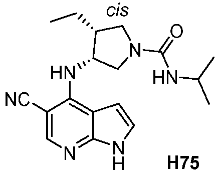

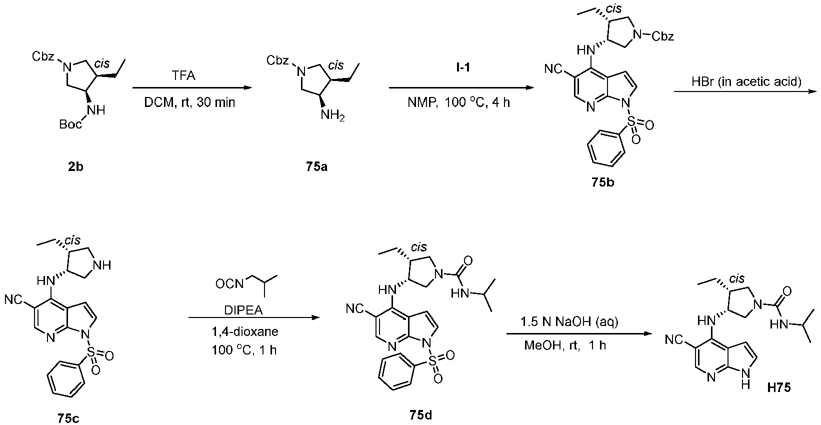

### First step: (cis)-N-carbobenzoxy-4-ethylpyrrolidin-3-amino 75a

Compound **2b** (184 mg, 0.53 mmol) was dissolved in 5 mL dichloromethane and 2 mL TFA was added. After the addition was completed, the mixture was stirred for 0.5h at room temperature to react. After the reaction was completed, the system was dried by a spin drier to remove solvent, thereby obtaining crude products **75a,** which was used directly for next step.

MS (ESI) m/z: 249 (M+H⁺)

### Second step: (cis)-3-((5-cyano-1-(benzenesulfonyl)-1H-pyrrole[2,3-b]pyridin -4-yl)amino)-4-ethylpyrrolidin-1-benzyl formate 75b

Compound **75a** (0.53 mmol) was dissolved in 5 mL NMP, and compound **I-1**(168 mg, 0.53 mmol) was added. After the addition was completed, the mixture was stirred for 2h at 100 °C to react under LCMS monitoring. After the reaction was completed, DCM/H₂O was layered. DCM layer was collected, dried, filtered and separated by preparative TLC (PE/EA = 4/1). Then target compound **75b** (175 mg) was obtained, which was white solids, with a yield of 63%.

MS (ESI) m/z: 530 (M+H⁺)

### Third step: (cis)-4-((4-ethylpyrrolidin-3-yl)-amino-1-(benzenesulfonyl)-1H -pyrrole[2,3-b]pyridin-5-carbonitrile 75c

Compound **75b** (175 mg, 0.33 mmol) was dissolved in 10 mL dichloromethane and 5 mL HBr (in acetic acid) was added. After the addition was completed, the mixture was stirred for 0.5h at room temperature to react. After the reaction was completed, the mixture was dried by a spin drier to remove solvent, freeze-dried with acetonitrile-water, thereby obtaining compound **75c,** which was used directly for next step.

MS (ESI) m/z: 396 (M+H⁺)

### Fourth step: (cis)-4-((4-ethylpyrrolidin-3-yl)-amino-1-(benzenesulfonyl)-1H -pyrrole[2,3-b]pyridin-5-carbonitrile 75d

Compound **75c** (80 mg) was dissolved in 5 mL of 1,4-dioxane, and then isopropyl isocyanate (20 mg) and 0.5 mL DIPEA were added and reacted for 1h at 100 °C. After the reaction was completed, the system was dried by a spin drier to remove solvent and separated by preparative TLC (DCM/MeOH = 20/1) to obtain target compound **75d** (60 mg), which was a colorless jelly, with a yield of 62%.

LCMS (ESI-MS) m/z: 481.3 (M+H⁺).

### Fifth step: (cis)-3-((5-cyano-1H-pyrrolo[2,3-b]pyridin-4-yl) amino)-4-ethyl-N-isobutylpyrrolidin-1-carboxamide H75

Compound **75d** (60 mg, 0.125 mmol) was dissolved in 5 mL methanol, and 2 mL sodium hydroxide solution was added. The mixture was stirred for 1h at room temperature to react. After the reaction was completed, dichloromethane and water were layered. Dichloromethane layer was dried by a spin drier to remove solvent, followed by preparative separation to obtain target compound **H75** (38 mg), which was white solids, with a yield of 62%.

MS (ESI) *m*/*z*: 341 (M+H⁺).

¹H NMR (400 MHz, MeOD) *δ* = 11.82 (s, 1H), 8.08 (s, 1H), 7.24 (s, 1H), 8.82 (s, 1H), 6.49 - 6.51 (m, 1H), 5.80 - 5.82 (d, *J* = 7.6 Hz, 1H), 4.77 - 4.79 (m, 1H), 3.67 - 3.76 (m, 1H), 3.59 - 3.52 (m, 3H), 3.22 - 3.27 (t, *J* = 10 Hz, 1H), 2.32 - 2.37 (m, 1H), 1.44 - 1.50 (m, 1H), 1.30 - 1.38 (m, 1H), 1.02 (s, 3H), 1.01 (s, 3H), 0.80 - 0.83 (t, *J* = 7.6 Hz, 3H).

### Example 76

### 4-(((cis)-4-ethyl-1-((3,3,3-trifluoropropyl)sulfonyl)pyrrolidin-3-yl)amino)-1H-pyrrolo[2,3-b]pyridin-5-carbonitrile H76

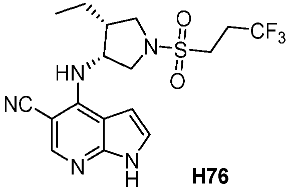

3,3,3-trifluoropropanesulfonyl chloride was used as a raw material to obtain compound **H76** by using the same preparation method as described in Example **75.**

MS m/z (ESI): 416 [M+H]+.

¹H NMR (400 MHz, DMSO-*d*₆) δ 11.90 (s, 1H), 8.12 (s, 1H), 7.28 - 7.19 (m, 1H), 6.65 - 6.59 (m, 1H), 6.56 - 6.44 (m, 1H), 4.73 - 4.64 (m, 1H), 3.67 -3.23 (m, 6H), 2.72 -2.60 (m, 2H), 2.51-2.37 (m, 1H), 1.60 - 1.22 (m, 2H), 0.88 - 0.78 (t, 3H).

### Example 77

### 4-(((cis)-4-ethyl-1-(4,4,4-trifluorobutyryl)pyrrolidin-3-yl)amino)-1H-pyrrolo[2,3-b]pyridin-5-carbonitrile H77

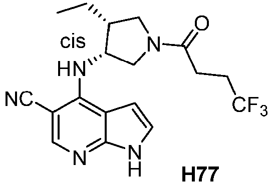

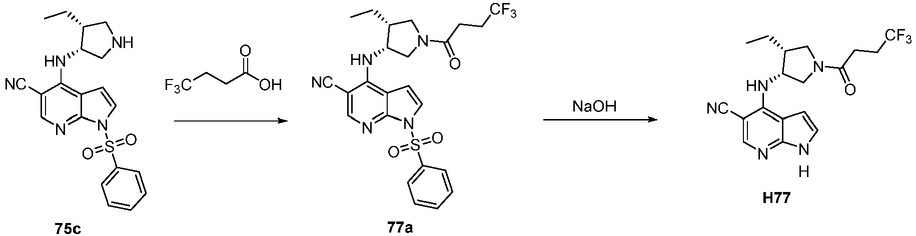

### First step: 4-(((cis)-4-ethyl-1-(4,4,4-trifluorobutyryl)pyrrolidin-3-yl)amino) -1-(benzenesulfonyl)-1H-pyrrolo[2,3-b]pyridin-5-acetonitrile 77a

Compound **75c** (100 mg, 0.25 mmol), compound 4,4,4-trifluorobutyric acid (36 mg, 0.25 mmol), EDCI (48mg, 0.25mmol), HOBT (34mg, 0.25mmol), DIPEA (65mg, 0.50mmol) and DCM (20 mL) were added into a flask. The system was reacted for 2h at room temperature. After the reaction was completed, the system was concentrated directly and purified by column chromatography (v/v, DCM/MeOH=100:1-50:1) to obtain compound **77a** (100 mg, 0.19 mmol) with a yield of 76.0%.

MS m/z (ESI): 520 [M+H]+.

### Second step: 4-(((cis)-4-ethyl-1-(4,4,4-trifluorobutyryl)pyrrolidin-3-yl)amino)-1H-pyrrolo [2,3-b]pyridin-5-acetonitrile H77

Compound **75a** (100 mg, 0.19 mmol), 2M sodium hydroxide solution (2mL) and methanol (5 mL) were added into a reaction flask. The system was stirred for 1h at room temperature. After the reaction was completed under TLC monitoring, the system was adjusted to pH=8-10 with diluted hydrochloric acid, concentrated and purified by column chromatography (v/v, DCM/MeOH=100:1-50:1) to obtain compound **H77** (50 mg, 0.13 mmol) with a yield of 68.4%.

MS m/z (ESI): 380 [M+H]+.

¹H NMR (600 MHz, DMSO-*d*₆) δ 11.87 (d, *J* = 7.0 Hz, 1H), 8.12 (s, 1H), 7.31 - 7.24 (m, 1H), 6.83 (d, *J* = 26.2 Hz, 1H), 6.57 (dd, *J* = 74.5, 8.9 Hz, 1H), 4.99 - 4.76 (m, 1H), 3.92 -3.38 (m, 4H), 2.64 - 2.30 (m, 5H), 1.58 - 1.25 (m, 2H), 0.83 (t, *J* = 7.4 Hz, 3H).

### Example 78

### 4-((4-ethyl-1-(2-cyanoacetyl)pyrrolidin-3-yl)amino)-1H-pyrrolo[2,3-b]pyridin-5-carbonitril e H78

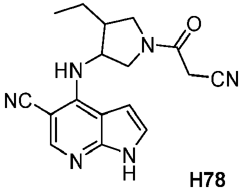

2-Cyanoacetic acid was used as a raw material to obtain compound **H78** by using the same preparation method as described in Example **77.**

MS m/z (ESI): 323 [M+H]+.

¹H NMR (400 MHz, DMSO-*d*₆) δ 11.91 - 11.79 (m, 1H), 8.08 (d, *J* = 6.8 Hz, 1H), 7.28 - 7.22 (m, 1H), 6.84 - 6.75 (m, 1H), 6.66 - 6.50 (m, 1H), 4.94 - 4.77 (m, 1H), 4.01 - 3.83 (m, 2H), 3.68 - 3.56 (m, 2H), 3.49 - 3.30 (m, 2H), 2.49 - 2.31 (m, 1H), 1.64 - 1.23 (m, 2H), 0.90 - 0.76 (m, 3H).

### Example 79

### (cis)-3-((5-cyano-1H-pyrrolo[2,3-b]pyridin-4-yl)amino)-4-ethyl-N-isobutylpyrrolidin-1-car boxamide H79

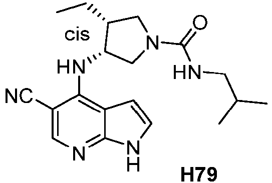

Isobutyl isocyanate was used as a raw material to obtain compound **H79** by using the same preparation method as described in Example **75.**

LCMS (ESI-MS) m/z: 502.1 (M+H⁺).

¹H NMR (400 MHz, DMSO-*d*₆) δ 11.82 (s, 1H), 8.07 (d, *J* = 6.7 Hz, 1H), 7.23 (td, *J* = 3.5, 2.5 Hz, 1H), 6.92 - 6.75 (m, 2H), 6.14 (t, *J* = 5.8 Hz, 1H), 4.44 (p, *J* = 7.6 Hz, 1H), 3.76 (dd, *J* = 10.3, 7.1 Hz, 1H), 3.64 - 3.54 (m, 1H), 3.54 - 3.42 (m, 1H), 3.31 - 3.12 (m, 1H), 2.95 (dd, *J* = 10.3, 8.3 Hz, 1H), 2.79 (t, *J* = 6.4 Hz, 2H), 2.30 (s, 1H), 1.72 - 1.40 (m, 2H), 1.40 - 1.18 (m, 1H), 0.91 -0.75 (m, 9H).

### Example 80

### 4-(((cis)-4-ethyl-1-(3-methoxy-1,2,4-thiadiazole-5-aminocarbonyl)pyrrolidin-3-yl)amino)-1 H-pyrrolo[2,3-b]pyridin-5-carbonitrile H80

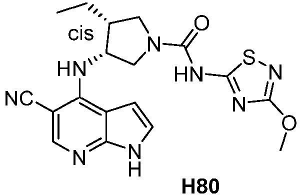

Compound **7h** was used as a raw material to obtain compound **H80** by using the same preparation method as described in Example **75.**

MS m/z (ESI): 413 [M+H]+.

¹H NMR (400 MHz, DMSO-*d*₆) δ 11.84 (s, 1H), 11.63 (s, 1H), 8.08 (s, 1H), 7.29 - 7.21 (m, 1H), 6.85 - 6.78 (m, 1H), 6.77 - 6.67 (m, 1H), 4.99 - 4.82 (m, 1H), 3.86 (s, 3H), 3.77 - 3.61 (m, 2H), 3.58 - 3.30 (m, 2H), 2.41 - 2.27 (m, 1H), 1.62 - 1.26 (m, 2H), 0.82 (t, *J* = 7.4 Hz, 3H).

### Example 81

### 4-(((cis)-1-(3-cyanopropionyl)-4-ethylpyrrolidin-3-yl)amino)-1H-pyrrolo[2,3-b]pyridin-5-c arbonitrile H81

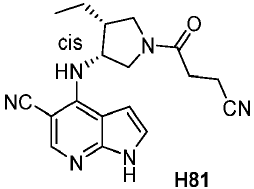

3-Cyanopropionic acid was used as a raw material to obtain compound **H81** by using the same preparation method as described in Example **77.**

MS m/z (ESI): 337 [M+1]⁺
¹H NMR (400 MHz, DMSO-*d*₆) δ 11.84 (s, 1H), 8.09 (s, 1H), 7.25 (qd, *J* = 3.6, 2.4 Hz, 1H), 6.80 (ddt, *J* = 15.9, 4.6, 2.3 Hz, 1H), 6.59 (dd, *J* = 48.2, 9.0 Hz, 1H), 4.85 - 4.75 (m, 1H), 3.75 - 3.51 (m, 3H), 2.75 - 2.25 (m, 4H), 1.59 - 1.27 (m, 2H), 0.84 (ddt, *J* = 17.6, 10.2, 7.4 Hz, 3H).

### Example 82

### 3-((5-cyano-1H-pyrrolo[2,3-b]pyridin-4-yl)amino)-4-ethyl-N-(cyclopropylmethyl)pyrrolidi n-1-carboxamide H82

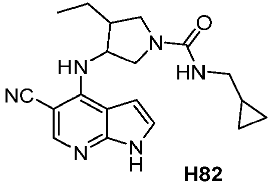

Cyclopropylmethyl isocyanate was used as a raw material to obtain compound **H82** by using the same preparation method as described in Example **75.**

MS (ESI) *m*/*z*: 353 (M+H) ⁺.

¹H NMR (400 MHz, DMSO-d6) δ 11.83 (s, 1H), 8.07 (d, J = 6.3 Hz, 1H), 7.23 (q, J = 2.9 Hz, 1H), 6.47 - 6.92 (m, 2H), 6.22 (t, J = 5.7 Hz, 1H), 4.83 - 4.37 (m, 1H), 3.85 - 3.39 (m, 3H), 3.27 - 3.13 (m, 1H), 2.99 - 2.82 (m, 2H), 2.41 - 2.27 (m, 1H), 1.61 - 1.42 (m, 1H), 1.40 - 1.26 (m, 1H), 1.20 (d, J = 3.6 Hz, 1H), 0.88 (q, J = 7.5 Hz, 2H), 0.82 (t, J = 7.4 Hz, 2H), 0.41 - 0.24 (m, 2H), 0.21 - 0.04 (m, 2H).

### Example 83

### 3-((5-cyano-1-H-pyrrolo[2,3-b]pyridin-4-yl)amino)-4-ethyl-N-(2,2,3,3,3-pentafluoropropyl) -pyrrolidin-1-carboxamide H83

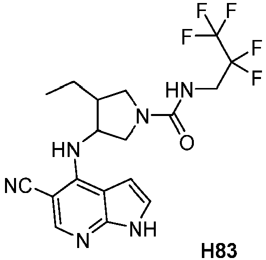

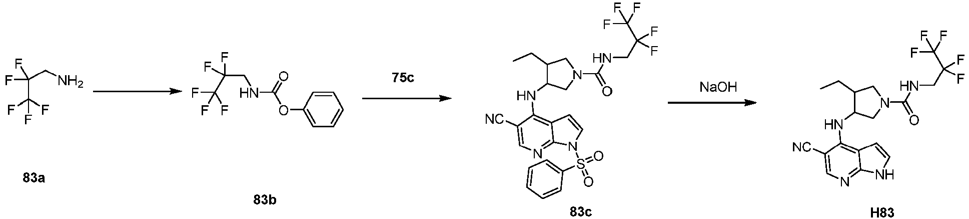

### First step: 2,2,3,3,3-pentafluoropropylphenyl carbamate 83b

Phenyl chloroformate (1.5g, 9.6mmol) was gradually added in 2,2,3,3,3-pentafluoropropylamino (1g, 6.7mmol) and triethylamine (2g, 20mmol) in DCM (20 mL) dropwise at an ice bath, stirred for 1h at room temperature, concentrated and separated by column chromatography (DCM:MeOH = 20:1) to obtain compound **83b** (1.3 g), with a yield of 72.0%.

¹H NMR (400 MHz, DMSO-*d*₆) δ 7.50 - 7.41 (m, 1H), 7.38 - 7.28 (m, 2H), 7.26 - 7.16 (m, 2H), 7.10 - 7.04 (m, 1H), 3.42 - 3.32 (m, 1H), 3.27 (d, J = 7.2 Hz, 1H).

### Second step: 3-((5-cyano-1-(benzenesulfonyl)-1H-pyrrolo[2,3-b]pyridin-4-yl)amino)-4 -ethyl-N-(2,2,3,3,3-pentafluoropropyl)-pyrrolidin-1-carboxamide 83c

Compound **75c** (200 mg, 0.51 mmol) was dissolved in 10 mL THF, and then compound **83b** (1.4 g, 1.49 mmol) and DIEA (300 mg, 2.33 mmol) were added, heated to 60 °C and reacted for 4h. After the reaction was completed, the system was concentrated and separated by preparative TLC to obtain compound **83c** (120 mg), with a yield of 41.6%.

MS (ESI) *m*/*z*: 571 (M+H) ⁺.

### Third step: 3-((5-cyano-1-H-pyrrolo[2,3-b]pyridin-4-yl)amino)-4-ethyl -N-(2,2,3,3,3-pentafluoropropyl)-pyrrolidin-1-carboxamide H83

Compound **83c** (120 mg, 0.21 mmol) was dissolved in 5 mL methanol, and NaOH (2 mL, 6 M in water) was added and reacted for 1h at room temperature. After the reaction was completed, the system was diluted with water (30 mL), extraxted with DCM (10 mL ×3). The organic phases were combined, dried with anhydrous Na₂SO₄, filtered, concentrated and separated by comparative TLC to obtain **H83** (35 mg) with a yield of 38.7%.

MS (ESI) *m*/*z*: 431 (M+H)⁺.

¹H NMR (400 MHz, DMSO-d6) δ11.83 (s, 1H), 8.08 (d, J = 6.2 Hz, 1H), 7.24 (dd, J = 3.5, 2.4 Hz, 1H), 6.55 - 6.77 (m, 3H), 4.42 - 4.85 (m, 1H), 3.90 - 3.75 (m, 2H), 3.64 (dd, J = 10.9, 5.6 Hz, 1H), 3.58 - 3.46 (m, 1H), 2.92 - 3.42 (m, 2H), 2.27 - 2.43 (m, 1H), 1.61 - 1.49 (m, 1H), 1.21 - 1.46 (m, 1H), 0.84 (dt, J = 20.1, 7.4 Hz, 3H).

### Example 84

### (cis)-3-((5-cyano-1H-pyrrolo[2,3-b]pyridin-4-yl)amino)-N-cyclopropyl-4-ethylpyrrolidin-1-carboxamide H84

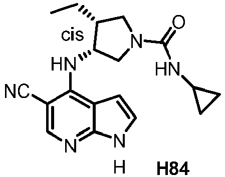

Cyclopropylamine was used as a raw material to obtain compound **H84** by using the same preparation method as described in Example **83.**

LCMS (ESI-MS) m/z: 339.2 (M+H⁺).

¹H NMR (400 MHz, DMSO-*d*₆) δ 11.84 (d, *J* = 5.4 Hz, 1H), 8.08 (d, *J =* 6.3 Hz, 1H), 7.23 (q, *J =* 3.1 Hz, 1H), 6.81 (ddd, *J =* 13.1, 3.6, 2.0 Hz, 1H), 6.53 (d, *J* = 8.8 Hz, 1H), 6.25 (d, *J =* 2.6 Hz, 1H), 4.78 (dtd, *J =* 8.8, 5.7, 2.8 Hz, 1H), 4.42 (p, *J =* 7.5 Hz, 1H), 3.62 - 3.51 (m, 1H), 3.51 - 3.40 (m, 1H), 3.27 - 3.10 (m, 1H), 2.93 (dd, *J =* 10.3, 8.2 Hz, 1H), 2.40 - 2.19 (m, 1H), 1.61 - 1.41 (m, 1H), 1.38 - 1.24 (m, 1H), 0.83 (dt, *J* = 20.5, 7.4 Hz, 3H), 0.48 (dt, *J* = 6.7, 3.3 Hz, 2H), 0.38 - 0.30 (m, 2H).

### Example 85

### (cis)-3-((5-cyano-1H-pyrrolo[2,3-b]pyridin-4-yl)amino)-4-ethyl-N-(3-methylbutane-2-yl) pyrrolidin-1-carboxamide H85

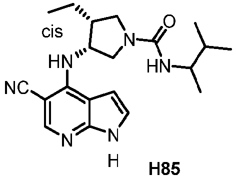

3-Methylbutane-2-ylamine was used as a raw material to obtain compound **H**85 by using the same preparation method as described inExample **83.**

LCMS (ESI-MS) m/z: 369 (M+H⁺).

1H NMR (400 MHz, DMSO-d6) δ 11.82 (d, J = 7.0 Hz, 1H), 8.07 (d, J = 7.1 Hz, 1H), 7.23 (td, J = 3.5, 2.4 Hz, 1H), 6.96 - 6.77 (m, 1H), 6.49 (d, J = 13.5 Hz, 1H), 5.70 (d, J = 8.5 Hz, 1H), 4.79 (s, 1H), 3.58 (d, J = 16.5 Hz, 1H), 3.52 - 3.38 (m, 2H), 3.21 (s, 1H), 2.95 (ddd, J = 21.9, 10.3, 8.6 Hz, 1H), 2.40 - 2.21 (m, 1H), 1.68 - 1.53 (m, 1H), 1.52 - 1.42 (m, 1H), 1.41 - 1.26 (m, 1H), 0.99 - 0.92 (m, 3H), 0.90 - 0.73 (m, 9H).

### Example 86

### (cis)-3-((5-cyano-1H-pyrrolo[2,3-b]pyridin-4-yl)amino)-4-ethyl-N-neopentyl-pyrrolidin-1-c arboxamide H86

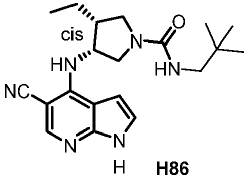

Neoamylamine was used as a raw material to obtain compound **H86** by using the same preparation method as described in Example **83.**

LCMS (ESI-MS) m/z: 369.3 (M+H⁺).

¹H NMR (400 MHz, DMSO-*d*₆) δ 11.83 (s, 1H), 8.08 (s, 1H), 7.24 (dd, J= 3.6, 2.4 Hz, 1H), 6.82 (dd, *J* = 3.6, 2.0 Hz, 1H), 6.46 (d, *J* = 8.7 Hz, 1H), 5.96 (t, *J* = 6.3 Hz, 1H), 4.80 (dt, *J* = 6.0, 3.2 Hz, 1H), 3.61 (dd, *J* = 10.9, 5.7 Hz, 1H), 3.50 (ddd, *J* = 10.9, 9.6, 5.4 Hz, 1H), 3.30 - 3.22 (m, 2H), 2.93 - 2.76 (m, 2H), 2.43 - 2.26 (m, 1H), 1.54 - 1.41 (m, 1H), 1.40 - 1.26 (m, 1H), 0.82 (t, *J* = 7.4 Hz, 3H), 0.77 (s, 9H).

### Example 87

### 3-((5-cyano-1H-pyrrolo[2,3-b]pyridin-4-yl)amino)-N-(2,2-difluoroethyl)-4-ethylpyrrolidin-1-carboxamide H87

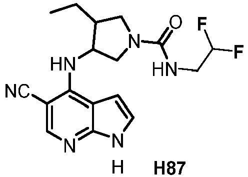

2,2-difluoroethylamine was used as a raw material to obtain compound **H87** by using the same preparation method as described in Example **83.**

LCMS (ESI-MS) m/z: 363.2 (M+H⁺).

¹H NMR (400 MHz, DMSO-*d*₆) δ 11.83 (s, 1H), 8.08 (d, *J* = 5.8 Hz, 1H), 7.24 (q, *J* = 2.6 Hz, 1H), 6.96 - 6.71 (m, 1H), 6.70 - 6.45 (m, 2H), 6.25 - 5.53 (m, 1H), 4.81 (d, *J* = 7.5 Hz, 1H), 4.52 - 3.73 (m, 1H), 3.62 (ddd, *J* = 9.8, 6.6, 3.4 Hz, 1H), 3.55 - 3.44 (m, 1H), 3.37 (ddd, *J* = 14.3, 11.4, 6.6 Hz, 2H), 3.10 (ddd, *J* = 85.1, 10.3, 7.5 Hz, 1H), 2.35 (ddt, *J* = 22.3, 14.6, 7.5 Hz, 1H), 1.63 - 1.42 (m, 1H), 1.40 - 1.20 (m, 1H), 0.84 (dt, *J* = 20.8, 7.4 Hz, 3H).

### Example 88

### 4-(((cis)-1-(2-cyanoethyl)-4-ethylpyrrolidin-3-yl)amino)-1H-pyrrolo[2,3-b]pyridin-5-carbon itrile H88

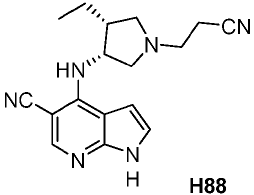

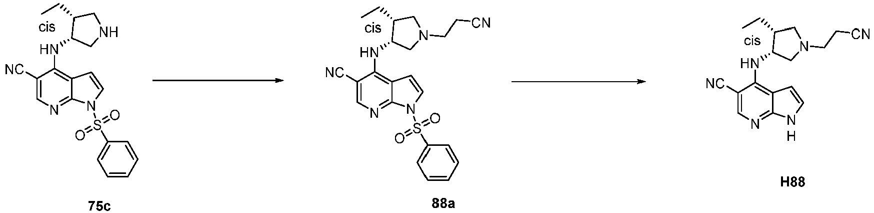

### First step: 4-(((cis)-1-(2-cyanoethyl)-4-ethylpyrrolidin-3-yl)amino)-1-(benzenesulfonyl)-1H-pyrrolo[2,3-b] pyridin-5-carbonitrile 88a

Compound **75c** (110mg, 0.28mmol, 1.0eq), acrylonitrile (45mg, 0.84mmol, 3.0eq) and DIPEA (0.11g, 0.84mmol, 3.0eq) were added in anhydrous MeOH (10ml) and reacted for 2h at room temperature. The system was concentrated and purified by flash column to obtain a colorless oily substance **88a** (99mg).

MS m/z (ESI): 449 [M+1]⁺

### Second step: 4-(((cis)1-(2-cyanoethyl)-4-ethylpyrrolidin-3-yl)amino)-1H-pyrrolo[2,3-b] pyridin-5-carbonitrile H88

Compound **88a** (99mg, 0.22mmol, 1.0eq) was dissolved in a mixed solvent of methanol (4ml) and THF (4ml), and then 2N of NaOH (0.55ml, 1.1mmol, 5.0eq) was added and reacted for 2h at room temperature to obtain a reaction liquid. The reaction liquid was mixed directly and purified by flash column (DCM~DCM/MeOH = 15: 1) to obtain white solid **H88** (36mg).

MS m/z (ESI): 309 [M+1]⁺
¹H NMR (400 MHz, DMSO-*d*₆) δ 11.78 (s, 1H), 8.04 (s, 1H), 7.20 (dd, *J =* 3.5, 2.4 Hz, 1H), 6.79 - 6.73 (m, 1H), 6.58 (s, 1H), 4.77 (d, *J* = 7.9 Hz, 1H), 3.20 (t, *J* = 8.1 Hz, 1H), 3.04 (t, *J* = 8.0 Hz, 1H), 2.67 (m, 4H), 2.56 - 2.49 (m, 1H), 2.28 (p, *J* = 7.9, 7.5 Hz, 1H), 2.18 (t, *J* = 8.8 Hz, 1H), 1.36 - 1.22 (m, 2H), 0.76 (t, *J* = 7.4 Hz, 3H).

### Example 89

### (cis)-3-((5-cyano-1H-pyrrolo[2,3-b]pyridin-4-yl)amino)-N-(2,2-difluoropropyl)-4-ethylpyrr olidin-1-carboxamide H89

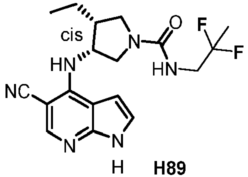

2,2-difluoropropylamine was used as a raw material to obtain compound **H89** by using the same preparation method as described in Example **83.**

LCMS (ESI-MS) m/z: 377.2 (M+H⁺).

¹H NMR (400 MHz, DMSO-*d*₆) δ 11.86 (s, 1H), 8.11 (s, 1H), 7.27 (dd, *J* = 3.6, 2.4 Hz, 1H), 6.85 (dd, *J* = 3.6, 2.0 Hz, 1H), 6.58 (q, *J* = 6.1 Hz, 2H), 4.84 (dq, *J* = 9.0, 2.7 Hz, 1H), 3.66-3.32 (m, 6H), 2.40 (q, *J* = 6.6 Hz, 1H), 1.53 (m, 4H), 1.38 (ddd, *J* = 13.8, 8.7, 7.1 Hz, 1H), 0.85 (t, *J* = 7.4 Hz, 3H).

### Example 90

### 7-((5-cyano-1H-pyrrolo[2,3-b]pyridin-4-yl)amino)-5,6,7,8-tetrahydroimidazo[1,2-a]pyridin-2-carbonitrile H90

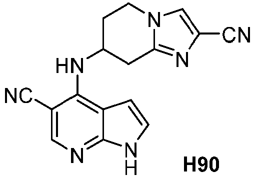

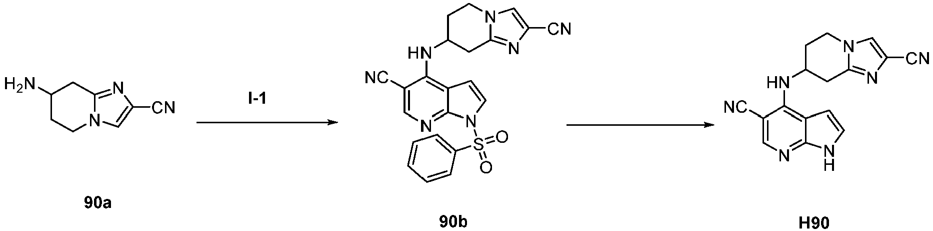

### First step: 7-((5-cyano-1-(benzenesulfonyl)-1H-pyrrolo[2,3-b]pyridin-4-yl)amino)-5,6,7,8-tetrahydroimidazo[1,2-a]pyridin-2-carbonitrile 90b

Compound **90a** (290mg, 1.8mmol, 1.0eq) and compound **I-1** (0.57g, 1.8mmol, 1.0eq) were dissolved in NMP (5ml), and DIPEA (1.2g, 9.0mmol, 5.0eq) was added and reacted for 4h at 110°C. The system was diluted with ethyl acetate (30ml), washed with brine (20ml×4), concentrated and purified with flash column (DCM - DCM/MeOH=10: 1) to obtain yellow solids **90b** (20mg).

MS m/z (ESI): 444 [M+H]⁺

### Second step: 7-((5-cyano-1H-pyrrolo[2,3-b]pyridin-4-yl)amino)-5,6,7,8-tetrahydroimidazo[1,2-a]pyridin-2-carbonitrile H90

Compound **90b** (20mg, 0.05mmol, 1.0eq) was dissolved in a mixed solvent of methanol (5ml) and THF (5ml), and then 2N of NaOH (0.13ml, 0.25mmol, 5.0eq) was added and reacted for 1h at room temperature to obtain a reaction liquid. The reaction liquid was dried under reduced pressure and purified by preparative plate (DCM - DCM/MeOH=15: 1) to obtain white solids **H90** (5mg).

MS m/z (ESI): 304 [M+H]⁺
¹H NMR (400 MHz, DMSO-*d*₆) δ 11.84 (s, 1H), 8.05 (d, *J* = 22.6 Hz, 2H), 7.24 (dd, *J* = 3.6, 2.4 Hz, 1H), 6.99 (d, *J* = 8.4 Hz, 1H), 6.74 (dd, *J* = 3.6, 2.0 Hz, 1H), 4.76 - 4.62 (m, 1H), 4.23 - 4.02 (m, 2H), 3.23-2.98 (m, 2H), 2.34 - 2.01 (m, 2H).

### Example 91

### (cis)-3-cyclopropyl-N-(2,2,2-trifluoroethyl)-4-((5-(trifluoromethyl)-1H-pyrrolo[2,3-b]pyridi n-4-yl)amino)pyrrolidin-1-carboxamide H91

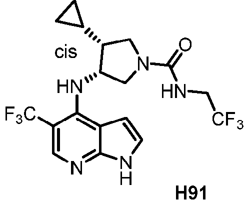

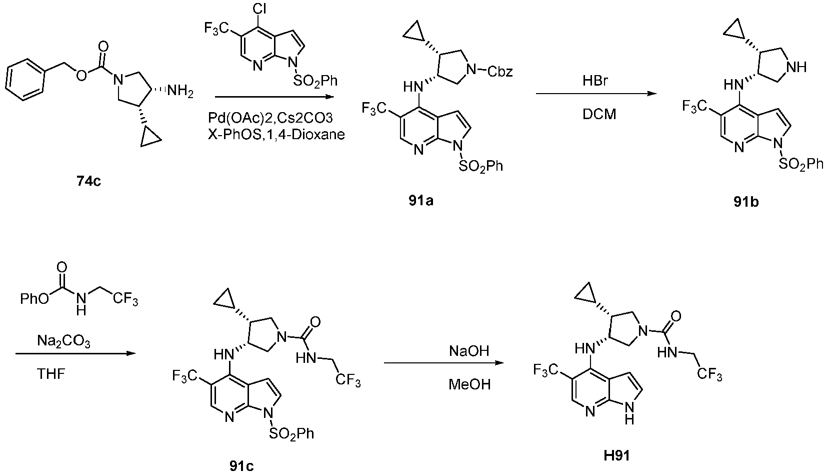

### Fifth step: (cis)-3-isopropyl-4-((1-(benzenesulfonyl)-5-(trifluoromethyl)-1H -pyrrole[2,3-b]pyridin-4-yl)amino)pyrrolidin-1-benzyl formate 91a

Compound 74c (546.6 mg, 2.1 mmol) was dissolved 3 mL of 1,4-dioxane, and 1-benzenesulfonyl-4-chloro-5-trifluoromethyl-1H-pyrrole[2,3-b]pyridine (500 mg, 1.4 mmol), Cs₂CO₃ (1.3 g, 4.2 mmol) and X-PhOS (162 mg, 0.28 mmol) were added. The system was substituted by nitrogen for three times, and then palladium acetate (62.9 mg, 0.28 mmol) was added, heated and subjected to a reflux reaction for 3h. After the reaction was completed, water was added. The system was extracted with EA and the organic phases were collected. Then the system was separated by column chromatography (PE: EA = 5:1) to obtain **91a** (460 mg), with a yield of 56%.

LCMS (ESI-MS) m/z: 585.3 (M+H+).

### Second step: N-((cis)-4-cyclopropylpyrrolidin-3-yl)-1-(phenylsulfanyl)-5-(trifluoromethyl) -1H-pyrrolo[2,3-b]pyridin-4-amine 91b

Compound **91a** (460 mg, 0.8 mmol) was dissolved in 2 mL dichloromethane, and then HBr (1 mL) was added and reacted for 1h at room temperature. After the reaction was completed, the system was concentrated and lyophilized directly to obtain crude products **91b** (300 mg) with a yield of 83%.

LCMS (ESI-MS) m/z: 451.2 (M+H+).

### Third step: (cis)-3-cyclopropyl-4-((1-(phenylsulfonyl)-5-(trifluoromethyl)-1H-pyrrolo [2,3-b]pyridin-4-yl)amino))-N-(2,2,2-trifluoroethyl)pyrrolidin-1-carboxamide 91c

Compound **91b** (300 mg, 0.6 mmol) was dissolved in 5 mL THF, and then compound Int-2 (263 mg, 1.2 mmol) and Na₂CO₃ (191 mg, 1.8 mmol) were added, heated to 70 °C and reacted for 3h. After the reaction was completed, the system was concentrated and separated by preparative TLC to obtain compound **91c** (150 mg), with a yield of 43%.

LCMS (ESI-MS) m/z: 576.3 (M+H+).

### Fourth step: (cis)-3-cyclopropyl-N-(2,2,2-trifluoroethyl)-4-((5-(trifluoromethyl)-1H-pyrrolo [2,3-b]pyridin-4-yl)amino)pyrrolidin-1-carboxamide H91

Compound **91c** (150 mg, 0.26 mmol) was dissolved in 2 mL methanol, and NaOH (1 mL, 1.5 mmol, 4 M in water) was added and reacted for 1h at room temperature. After the reaction was completed, water was added. The system was extracted with DCM, concentrated and separated by preparative TLC to obtain compound **H91** (23 mg) with a yield of 20%.

LCMS (ESI-MS) m/z: 436.3 (M+H+).

1H NMR (400 MHz, DMSO-d6) δ 11.77 (s, 1H), 8.10 (s, 1H), 7.32 (dd, J = 3.6, 2.4 Hz, 1H), 6.84 (t, J = 6.3 Hz, 1H), 6.71 (dd, J = 3.8, 1.8 Hz, 1H), 5.51 (d, J = 7.9 Hz, 1H), 4.82 (t, J = 6.8 Hz, 1H), 3.85 - 3.63 (m, 3H), 3.54 (dd, J = 10.3, 7.0 Hz, 1H), 3.37 - 3.31 (m, 2H), 1.85 (s, 1H), 0.79 (dt, J = 13.9, 5.6 Hz, 1H), 0.48 (dd, J = 8.1, 1.7 Hz, 2H), 0.28 - 0.01 (m, 2H).

### Example 92

### (cis)-3-ethyl-N-(2,2,2-trifluoroethyl)-4-((5-(trifluoromethyl)-1H-pyrrolo[2,3-b]pyridin-4-yl) amino)pyrrolidin-1-carboxamide H92

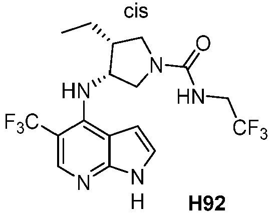

Ethyl magnesium bromide was used as a raw material to obtain compound **H92** by using the same preparation method as described in Example **91.**

LCMS (ESI-MS) m/z: 481.3 (M+H⁺).

¹H NMR (400 MHz, DMSO-*d*₆) δ 11.79 (s, 1H), 8.09 (s, 1H), 7.32 (dd, *J* = 3.7, 2.4 Hz, 1H), 6.84 (t, *J =* 6.3 Hz, 1H), 6.65 (dd, *J =* 3.7, 1.8 Hz, 1H), 5.06 - 4.98 (m, 1H), 4.76 (s, 1H), 3.79 - 3.56 (m, 4H), 3.48 (m, 1H), 3.07 (t, *J* = 10.2 Hz, 1H), 2.42 (m, 1H), 1.47 - 1.34 (m, 2H), 0.86 (t, *J* = 7.3 Hz, 3H).

### Example 93

### (cis)-3-ethyl-N-(cyanmethyl)-4-((5-(trifluoromethyl)-1H-pyrrolo[2,3-b]pyridin-4-yl)amino) pyrrolidin-1-carboxamide H93

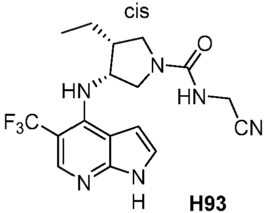

N-(cyanomethyl)phenyl carbamate was used as a raw material to obtain compound **H93** by using the same preparation method as described in Example **92.**

LCMS (ESI-MS) m/z: 481.3 (M+H⁺).

¹H NMR (400 MHz, DMSO-*d*₆) δ 11.80 (s, 1H), 8.09 (s, 1H), 7.32 (dd, J = 3.7, 2.5 Hz, 1H), 6.93 (t, *J* = 5.7 Hz, 1H), 6.65 (dd, *J* = 3.7, 1.9 Hz, 1H), 5.05 (d, *J* = 7.7 Hz, 1H), 4.77 (s, 1H), 3.95 (d, *J* = 5.5 Hz, 2H), 3.66 - 3.56 (m, 2H), 3.44 (d, *J* = 11.1 Hz, 1H), 3.06 (t, *J* = 10.1 Hz, 1H), 2.43 - 2.40 (m, 1H), 1.46 - 1.35 (m, 2H), 0.85 (t, *J* = 7.3 Hz, 3H).

### Biological test examples:

### Example 1 Inhibition test of the compounds of the invention on the JAK kinase

### 1. Test purpose

Inhibition test on activity of JAK kinase in vitro was carried out by the following methods.

### 1.1 Kits and test liquids

JAK1 LANCE^{®} Ultra assay, JAK2 LANCE^{®} Ultra assay and JAK3 LANCE^{®} Ultra assay were used to carry out the test separately, and kits of the above assays were supplied by PerkinElmer.

A reaction system of JAK1 LANCE^{®}Ultra kinase assay comprises 2 nM JAK1 (Intech, PV4775), 50 nM ULight TM-JAK-1 peptide (substrate, PerkinElmer, TRF0121-M) and 38 uM ATP (Sigma, A7699). A reaction system of JAK2 LANCE^{®}Ultra kinase assay comprises 0.03 nM JAK2 (Intech, PV4288), 50 nM ULight TM-JAK-1 peptide (substrate, PerkinElmer, TRF0121-M) and 12 uM ATP (Sigma, A7699). A reaction system of JAK3 LANCE^{®}Ultra kinase assay comprises 0.08 nM JAK3 (Intech, PV4080), 50 nM ULight TM-JAK-1 peptide (substrate, PerkinElmer, TRF0121-M) and 4 uM ATP (Sigma, A7699).

Buffers of an enzymatic reaction is 50 mM 4-hydroxyethylpiperazine ethane sulfonic acid (pH7.5) Intech, 15630130), 10 mM magnesium chloride (Sigma, 63020), 1 mM edetic acid (Intech, 1842C505), 2mM dithiothreitol (Intech, 43815) and 0.01% BRIJ-35 (Intech, B4184). JAK1, JAK2 and JAK3 kinase, ATP and substrates were dissolved and diluted with buffers.

A test liquid was 2 nM Eu-W1024 anti-phosphotyrosine (PerkinElmer, AD0069). A stop buffer was 10 mM edetic acid (Intech, 1842C505).

### 1.2 Formulation of the compound

Test compounds H01-H93 were dissolved in dimethyl sulfoxide, respectively, from 0.017nM to 1µM in triplicate dilutions for a total of 11 gradient concentrations.

### 1.3 Test procedure

2.5 µl of an 8-fold solution of the compound and 5 µl of a 4-fold solution of JAK1 or JAK2 or JAK3 kinase were added into a 384-well plate, respectively, and reacted for 10 minutes at room temperature. 2.5 µl of an 8-fold solution of ULight TM-JAK-1 peptide/ATP was added and reacted for 90 minutes at room temperature. Finally, 5µl of 4-fold edetic acid stop buffer and 5µl of 4-fold test liquid of Eu-W1024 anti-phosphotyrosine were added and incubated for 60 minutes. Luminescence values were read in an ENVISION Multi-Mode Plate Reader (PE, 2105-0010).

### 1.4 Test result

According to the formula: percentage of inhibition rate = (maximum value-transform value)/(maximum value-minimum value)×100, inhibition rate of each compound at various concentrations was calculated. The maximum value was the reference value of dimethyl sulfoxide added into the well plate, the minimum value was the blank value, and the transform value is the value obtained by the well of each compound. The IC₅₀ values of the corresponding compounds were then obtained by fitting by using the XLFit fitting tool in excel.

### 2. Test result and conclusion

IC₅₀ of the compounds of the invention on inhibiting the activities of JAK1-3 kniases was shown in the following table. The data showed that the compounds of the invention have different inhibiting effects on JAK1-3 kinases. The compounds of the invention showed better inhibiting activity on JAK1 and better selectivity on JAK family compared with JAK2 and JAK3, which indicated that the compounds of the invention have good specificity for target, strong pharmaceutical effect and less adverse reaction induced by other targets in the same family.

**Table 1 IC₅₀ of the compounds of the invention on inhibiting the activities of JAK1-3 kniases**

| Test compound number | IC₅₀ (nM) | | | |
|---|---|---|---|---|
| | JAK1 | JAK2 | JAK3 | JAK2/JAK1 |
| H01 | 0.79 | 6.70 | 50.00 | 8.48 |
| H02 | 0.98 | 7.50 | 66.00 | 7.65 |
| H03 | 2.86 | 22.00 | 133.00 | 7.69 |
| H04 | 5.96 | 18.00 | 186.00 | 3.02 |
| H05 | 7.86 | 12.33 | 256.00 | 1.56 |
| H06 | 6.68 | 29.86 | 96.00 | 4.47 |
| H07 | 0.53 | 4.96 | 47.00 | 9.35 |
| H08 | 0.39 | 3.00 | 63.00 | 7.69 |
| H09 | 3.86 | 26.50 | 156.00 | 6.86 |
| H10 | 4.98 | 36.00 | 143.00 | 7.22 |
| H11 | 1.28 | 9.86 | 77.00 | 7.70 |
| H12 | 0.56 | 7.63 | 66.00 | 13.63 |
| H13 | 2.96 | 21.00 | 58.90 | 7.09 |
| H14 | 1.78 | 9.95 | 34.20 | 5.59 |
| H15 | 2.36 | 16.60 | 244.00 | 7.03 |
| H16 | 9.16 | 132.00 | 633.00 | 14.41 |
| H17 | 5.00 | 13.00 | 27.00 | 2.60 |
| H18 | 0.65 | 5.70 | 40.00 | 8.80 |
| H19 | 2.00 | 15.00 | 99.00 | 7.50 |
| H20 | 5.80 | 36.00 | 161.00 | 6.20 |
| H21 | 9.40 | 146.00 | 223.00 | 15.40 |
| H22 | 0.35 | 5.80 | 58.00 | 16.40 |
| H23 | 7.16 | 135.00 | 533.00 | 18.85 |
| H24 | 5.10 | 12.60 | 28.00 | 2.40 |
| H25 | 0.56 | 6.70 | 58.00 | 11.96 |
| H26 | 2.60 | 18.00 | 101.00 | 6.92 |
| H27 | 5.60 | 33.00 | 171.00 | 5.89 |
| H28 | 9.50 | 135.00 | 220.00 | 14.21 |
| H29 | 0.36 | 6.80 | 60.00 | 18.89 |
| H30 | 0.57 | 6.40 | 76.00 | 11.23 |
| H31 | 13.00 | 32.00 | 77.00 | 2.46 |
| H32 | 5.00 | 13.00 | 27.00 | 2.60 |
| H33 | 0.65 | 5.70 | 40.00 | 8.77 |
| H34 | 2.00 | 15.00 | 99.00 | 7.50 |
| H35 | 8.40 | 120.00 | 172.00 | 14.86 |
| H36 | 7.50 | 65.00 | 160.00 | 8.67 |
| H37 | 5.60 | 48.00 | 356.00 | 8.57 |
| H38 | 6.40 | 30.00 | 440.00 | 4.69 |
| H39 | 0.35 | 21.00 | 150.00 | 60.00 |
| H40 | 2.60 | 15.60 | 144.00 | 6.00 |
| H41 | 0.73 | 6.70 | 96.00 | 9.17 |
| H42 | 3.60 | 21.30 | 263.00 | 5.92 |
| H43 | 2.40 | 20.00 | 125.00 | 8.33 |
| H44 | 0.67 | 15.60 | 80.00 | 23.28 |
| H45 | 5.90 | 50.00 | 77.00 | 8.47 |
| H46 | 1.13 | 9.80 | 68.00 | 8.67 |
| H47 | 2.67 | 20.00 | 130.00 | 7.49 |
| H48 | 0.86 | 7.40 | 56.00 | 8.60 |
| H49 | 5.43 | 15.00 | 178.00 | 2.76 |
| H50 | 4.65 | 33.00 | 137.00 | 7.10 |
| H51 | 6.89 | 30.94 | 101.00 | 4.49 |
| H52 | 0.79 | 7.95 | 69.00 | 10.06 |
| H53 | 0.64 | 4.00 | 68.00 | 6.25 |
| H54 | 3.64 | 24.80 | 148.00 | 6.81 |
| H55 | 2.59 | 17.70 | 253.00 | 6.83 |
| H56 | 1.43 | 10.03 | 82.00 | 7.01 |
| H57 | 0.89 | 6.40 | 48.00 | 7.19 |
| H58 | 2.74 | 19.00 | 55.70 | 6.93 |
| H59 | 2.06 | 11.60 | 39.60 | 5.63 |
| H60 | 5.60 | 35.00 | 153.00 | 6.25 |
| H61 | 8.23 | 126.00 | 589.00 | 15.31 |
| H62 | 5.80 | 16.00 | 40.00 | 2.76 |
| H63 | 1.42 | 7.30 | 64.00 | 5.14 |
| H64 | 2.50 | 18.00 | 113.00 | 7.20 |
| H65 | 3.20 | 21.00 | 124.00 | 6.56 |
| H66 | 8.90 | 126.00 | 197.00 | 14.16 |
| H67 | 0.67 | 9.50 | 76.00 | 14.18 |
| H68 | 6.87 | 122.00 | 386.00 | 17.76 |
| H69 | 0.81 | 7.30 | 90.00 | 9.01 |
| H70 | 6.70 | 13.90 | 47.00 | 2.07 |
| H71 | 1.13 | 9.80 | 70.00 | 8.67 |
| H72 | 10.30 | 29.00 | 75.00 | 2.82 |
| H73 | 4.90 | 30.00 | 157.00 | 6.12 |
| H74 | 9.10 | 128.00 | 180.00 | 14.07 |
| H75 | 2.60 | 17.30 | 89.00 | 6.65 |
| H76 | 0.95 | 7.80 | 91.00 | 8.21 |
| H77 | 4.20 | 49.00 | 273.00 | 11.67 |
| H78 | 6.00 | 16.00 | 42.00 | 2.67 |
| H79 | 0.96 | 6.50 | 59.00 | 6.77 |
| H80 | 2.90 | 18.60 | 152.00 | 6.41 |
| H81 | 9.30 | 117.00 | 162.00 | 12.58 |
| H82 | 8.90 | 69.00 | 172.00 | 7.75 |
| H83 | 3.70 | 34.00 | 146.00 | 9.19 |
| H84 | 6.90 | 38.00 | 387.00 | 5.51 |
| H85 | 5.47 | 15.66 | 48.00 | 2.86 |
| H86 | 1.41 | 29.00 | 169.00 | 20.57 |
| H87 | 5.10 | 45.00 | 73.00 | 8.82 |
| H88 | 0.86 | 7.90 | 112.00 | 9.19 |
| H89 | 2.90 | 19.70 | 241.00 | 6.79 |
| H90 | 0.76 | 5.71 | 68.00 | 7.51 |
| H91 | 0.92 | 18.70 | 96.00 | 20.33 |
| H92 | 8.21 | 14.90 | 258.00 | 1.81 |
| H93 | 0.64 | 7.10 | 79.00 | 11.09 |
| Tofacitinib | 6.4 | 2.1 | 0.39 | 0.32 |

### Example 2 Inhibiting test of the compounds of the invention on cell proliferation

### 1. Test purpose and method

Inhibiting test of the compounds of the invention on proliferation of Blast T cell and TF-1 cell (American type culture collection) was carried out by the following method.

### 1.1 Formulation of the compound

The tested compounds H01, H02, H03, H07, H08, H09, H11, H12, H14, H15, H18, H19, H22, H25, H26, H29, H30, H33, H34, H39, H40, H41, H43, H44, H50, H58, H62, H70, H85, and H92 were dissolved in dimethyl sulfoxide from 0.0006 to 10 µM in quadruplicate dilutions.

### 1.2 Cell proliferation test of IL-2 stimulated Blast T cell

A culture medium included RPMI 1640 culture medium (Grand Turk Island Biotechnology Company TM, 11875093), 10% fetal calf serum (Aosibina, 0986180), 55µM 2-mercaptoethanol and 1% penicillin-streptomycin (Grand Turk Island Biotechnology Company, 1902422). NuncTM Edge 2.0 96-well flat bottom (Cat. No. 167425).

Healthy human PBMC cells (drawn blood from healthy human) were isolated, and CD4⁺T cells were isolated using the EasySep^{™} human CD4 T cell isolation kit (Stemcell, 19052). 10µg/ml polyhydroxyalkanoate was added to stimulate and obtain cells with a density of 2x10⁶/mL, which were incubated for 3 days. The cells were washed with phosphate buffer for three times and IL-2 (100 u/ml) was added to stimulate for 1 week. Cells were suspended using the culture medium and seeded at 5x10⁴ cell/well density into 96-well plates, and incubated for 1h with the prepared test compound or DMSO control at various concentrations from 0.0006 to 10 µ M diluted by 4-fold volume. After incubation with 20u/ml of IL-2 (50µl/ well without reference well) for 72 hours, Celltiter Glo^{®} reagent (Promega, G7571) was added and mixed for 5 minutes, then incubated for 30 minutes at room temperature. The luminescence value was read on SpectraMax L (Molecular Device). Equation: inhibition rate = 1-(the value-minimum value)/(maximum value-minimum value) was used to calculate inhibition rate of each concentration, wherein the value is the luminescence value of each well, maximum value is reference value of dimethyl sulfoxide well and minimum value is the value without IL-2 stimulation. The IC₅₀ values of the corresponding compounds were then obtained by fitting by using the XLFit fitting tool in excel.

### 1.3 TF-1 cell proliferation test

A culture medium included RPMI 1640 culture medium (Grand Turk Island Biotechnology Company TM-11875093), 10% fetal calf serum (Aosibina, 0986180) and 1% penicillin-streptomycin (Grand Turk Island Biotechnology Company, 1902422).

TF-1 cells were incubated with culture medium containing 2ng/mL of human granulocyte macrophage colony stimulating factor to reach a density of 4.0x10⁵ cells/mL, then incubated with culture medium without human granulocyte macrophage colony stimulating factor for 24 h, centrifuged in 1000 rpm for 5 minutes, and suspended using the culture medium containing 2ng/mL of human granulocyte macrophage colony stimulating factor and seeded at 5000 cell/well density into 96-well plates. The above test compounds at various concentrations formulated in 1.1 or dimethyl sulfoxide control were added and incubated for 72h, and then Celltiter Glo^{®} reagent (Promega, G7571) was added. The luminescence value was read on Microplate Reader (Molecular Device). Equation: inhibition rate = 1-(the value-minimum value)/(maximum value-minimum value) was used to calculate inhibition rate of each concentration, wherein the value is the luminescence value of each well, maximum value is reference value of dimethyl sulfoxide well and minimum value is the value without IL-2 stimulation. The IC₅₀ values of the corresponding compounds were then obtained by fitting by using the XLFit fitting tool in excel.

### 2. Test result and conclusion

IC₅₀ of the compounds of the invention on inhibiting proliferation of Blast T cell and TF-1 cell was shown in following table. The data showed that IC₅₀ value of the compounds of the invention on proliferation of T cell was 13-50 nM, and IC₅₀ value on proliferation of TF-1 cell was 70-2500 nM. It was clear that the compounds of the invention had good inhibiting effect on proliferation of T cell and poor inhibiting effect on proliferation of TF-1 cell, which indicated that the compounds of the invention had strong activity on JAK1 and weak activity on JAK2 and had good selectivity. It had better pharmaceutical effect on animals and less adverse reaction.

**Table 2 IC₅₀ of the compounds of the invention on inhibiting proliferation of Blast T cell and TF-1 cell**

| Compound number | IC₅₀ (µM) | |
|---|---|---|
| | T cell | TF-1 cell |
| H01 | 0.013 | 0.67 |
| H02 | 0.48 | 1.34 |
| H03 | 0.027 | 0.76 |
| H07 | 0.024 | 0.073 |
| H08 | 0.026 | 1.30 |
| H09 | 0.036 | 1.56 |
| H11 | 0.013 | 0.68 |
| H12 | 0.011 | 1.67 |
| H14 | 0.017 | 0.63 |
| H15 | 0.014 | 0.073 |
| H18 | 0.040 | 1.56 |
| H19 | 0.024 | 2.30 |
| H22 | 0.019 | 1.76 |
| H25 | 0.020 | 0.73 |
| H26 | 0.027 | 1.98 |
| H29 | 0.016 | 0.87 |
| H30 | 0.018 | 1.23 |
| H33 | 0.023 | 1.10 |
| H34 | 0.044 | 1.54 |
| H39 | 0.015 | 0.69 |
| H40 | 0.036 | 1.24 |
| H41 | 0.055 | 1.64 |
| H43 | 0.043 | 1.44 |
| H44 | 0.014 | 0.73 |
| H50 | 0.034 | 0.85 |
| H58 | 0.041 | 0.98 |
| H62 | 0.029 | 1.32 |
| H70 | 0.048 | 1.53 |
| H85 | 0.032 | 1.47 |
| H92 | 0.027 | 0.91 |
| Tofacitinib | 0.065 | 0.047 |

### Example 3 Pharmacodynamics test of the compounds of the invention on paw swelling in mice

### 1. Abstract

The inhibiting effects of compounds H01, H07, H11, H12, H14, H15, H22, H25, H29, H30, H33, H39 and H44 on paw swelling in mice were determined after intragastric administration by using male ICR mice (ShanghaiSippr-BK laboratory animal Co. Ltd.), aged 8 weeks and weighing 22-24g as test animals. The anti-inflammatory pharmacodynamics characteristic of the compounds of the invention was discussed.

### 2. Test scheme

### 2.1 Compounds for tests

Compounds H01, H07, H11, H12, H14, H15, H22, H25, H29, H30, H33, H39, and H44.

### 2.2 Formulation of the compound

A certain amount of compounds were taken and dissolved in 4% Tween 80/0.5% hydroxy propyl methyl cellulose solution. A volume of intragastric administration in mice was 10 ml/kg.

### 2.3 Operation

Mice were given 10 mg/kg compounds H01, H07, H11, H12, H14, H15, H22, H25, H29, H30, H33, H39, and H44 and the positive drug indomethacin (Diamond Reagent Co., Ltd., China) by intragastric administration at the dosages shown in the table below and 20µL 1% (W/V) carrageenan was injected subcutaneously into the plantar 0.5h later. A paw volume was measured with a paw swelling meter before and at different time points after modeling (0.5 h, 1 h and 2 h). According to the formula: paw swelling rate = (paw volume after administration - basic paw volume)/basic paw volume ×100, the inhibiting effect of the compounds on paw volume was calculated. The basic paw volume was a paw volume before administration.

### 3. Test result and conclusion

Data in the following table showed that 10mg/kg compounds of the invention could relieve paw swelling rate of mice caused by carrageenan, especially over 1-2h after administration, which indicated that the compounds of the invention had a certain anti-acute inflammatory effect and had better effect on inhibiting paw swelling rate than indometacin.

### Example 3 Pharmacodynamics effect of the compounds of the invention on paw swelling in mice

| **Compound number** | **Dosage** (mg/kg) | **Paw swelling _0.5h (%)** | **Paw swelling rate_1h (%)** | **Paw swelling rate_2h (%)** |
|---|---|---|---|---|
| **Control group** | 0 | 36.43±2.56 | 49.24±2.7 | 61.57±3.41 |
| **Indomethacin** | 10 | 32.91±3.22 | 42.21±4.64 | 55.10±2.21 |
| H01 | 10 | 33.99±1.69 | 40.93±1.64 | 41.56±5.23 |
| H07 | 10 | 37.02±1.35 | 42.33±2.20 | 41.24±3.36 |
| H11 | 10 | 30.22±2.62 | 39.76±1.20 | 40.25±5.47 |
| H12 | 10 | 38.45±4.37 | 45.64±3.58 | 41.66±5.38 |
| H14 | 10 | 32.03±4.53 | 40.65±2.51 | 39.00±4.25 |
| H15 | 10 | 36.02±1.37 | 43.33±2.30 | 43.24±4.36 |
| H22 | 10 | 31.22±2.62 | 41.76±1.20 | 42.25±5.67 |
| H25 | 10 | 37.45±4.37 | 44.64±3.68 | 44.66±5.58 |
| H29 | 10 | 33.03±4.23 | 41.65±2.21 | 40.10±4.32 |
| H30 | 10 | 33.12±1.38 | 43.43±2.12 | 44.52±4.37 |
| H33 | 10 | 30.78±2.62 | 31.76±1.86 | 41.75±5.67 |
| H39 | 10 | 30.45±1.74 | 26.44±3.68 | 38.22±5.58 |
| H44 | 10 | 31.03±2.43 | 42.65±2.21 | 41.56±3.32 |

### Example 4 Effects test of the compounds of the invention on paw swelling in mice

### 1. Abstract

The inhibiting effects of compounds H13, H14, H09, H11 and compounds of Example 12 on paw swelling in mice were determined after intragastric administration by using Sprague-Dawley (SD) mice (ShanghaiSippr-BK laboratory animal Co. Ltd.), aged 6-7 weeks and weighing 160-180g as test animals. The anti-inflammatory pharmacodynamics characteristic of the compounds of the invention was discussed.

### 2. Test scheme

### 2.1 Compounds for tests

Compounds H01, H07, H11, H12, H14, H15, H22, H25, H29, H30, H33, H39, and H44.

### 2.2 Formulation of the compound

A certain amount of compounds were taken and dissolved in 4%Tween 80/0.5% hydroxy propyl methyl cellulose solution.

### 2.3 Operation

Mice were given compounds H01, H07, H11, H12, H14, H15, H22, H25, H29, H30, H33, H39, and H44 and the positive drug indomethacin (Diamond Reagent Co., Ltd., China) by intragastric administration at the dosages shown in the table below and 0.1ml 0.03%(W/V) concanavalin was injected subcutaneously into the plantar 0.5h later. A paw volume was measured with a paw swelling meter before modeling and 1h, 2h, 4h and 6h after modeling. According to the formula: paw swelling rate = (paw volume after administration - basic paw volume)/basic paw volume ×100, the inhibiting effect of the compounds on paw volume was calculated. The basic paw volume was a paw volume before administration.

### 3. Test result and conclusion

Data in the following table showed that 10mg/kg compounds of the invention could relieve the paw swelling in mice induced by concanavalin, which indicated that the compounds of the invention had a certain anti-acute inflammation effect and some compounds had a better anti-acute inflammation effect after 4h than indometacin.

### Example 4 Pharmacodynamics effect of the compounds of the invention on paw swelling in mice

| **Compound number** | **Dosage (mg/kg)** | **Paw swelling rate_1h (%)** | **Paw swelling rate_2h (%)** | **Paw swelling rate_4h (%)** | **Paw swelling rate_6h (%)** |
|---|---|---|---|---|---|
| **Control group** | 0 | 39.90±3.00 | 51.90±6.00 | 51.90±4.000 | 41.80±4.00 |
| **Indomethacin** | 10 | 34.00±2.30 | 44.80±3.00 | 42.70±2.00 | 36.20±2.00 |
| H01 | 10 | 25.70±1.50 | 25.20±2.40 | 31.20±3.10 | 30.20±3.10 |
| H07 | 10 | 25.20±2.10 | 32.10±1.70 | 22.30±4.20 | 21.30±4.20 |
| H11 | 10 | 23.50±2.40 | 25.80±3.30 | 31.00±2.10 | 32.00±2.10 |
| H12 | 10 | 28.90±2.00 | 23.10±3.00 | 24.00±2.90 | 24.00±3.00 |
| H14 | 10 | 32.10±5.20 | 25.90±5.00 | 25.50±4.00 | 25.10±4.20 |
| H15 | 10 | 24.50±2.50 | 25.90±3.40 | 32.00±2.30 | 31.00±2.30 |
| H22 | 10 | 29.80±2.00 | 29.10±3.00 | 31.00±2.90 | 31.00±2.90 |
| H25 | 10 | 32.10±5.20 | 26.90±5.00 | 24.50±4.00 | 24.50±4.00 |
| H29 | 10 | 23.70±1.60 | 23.20±2.60 | 32.20±3.50 | 29.20±3.50 |
| H30 | 10 | 24.40±2.40 | 25.20±3.40 | 26.00±2.30 | 24.50±2.30 |
| H33 | 10 | 27.80±2.00 | 29.20±3.10 | 30.10±2.90 | 30.10±2.90 |
| H39 | 10 | 31.10±2.30 | 25.60±4.00 | 26.30±3.30 | 25.30±3.30 |
| H44 | 10 | 23.80±1.60 | 24.80±2.50 | 24.20±2.90 | 23.50±2.90 |

### Example 5 Effects test of the compounds of the invention on rheumatoid arthritis in mice

### 1. Abstract

The effects of compounds H07, H12, H14, H25, H30, H39 and H44 on rheumatoid arthritis in mice were determined after long term administration by using Lewis male mice (Beijing Vital River Laboratory Animal Technology Co., Ltd.), aged 5-6 weeks and weighing 160g as test animals. The anti-inflammatory pharmacodynamics characteristic of the compounds of the invention was discussed.

### 2. Test scheme

### 2.1 Compounds for tests

Compounds H07, H12, H14, H25, H30, H39, and H44.

### 2.2 Formulation of the compound

A certain amount of compounds were taken and dissolved in 4% Tween 80/0.5% hydroxy propyl methyl cellulose solution. A volume of intragastric administration of mice was 10ml/kg.

### 2.3 Operation

Collagen type II was dissolved in 0.1M acetic acid and kept in a 4 °C refrigerator overnight at a collagen concentration of 4 mg/mL. Same volume of incomplete Freund adjuvant (Sigma, USA) was added in collagen before injection in test, which was emulsified high speed homogenizer (30000 rpm) to prepare a collagen emulsion. This procedure was carried out on ice.

Mice were immunologically injected with the collagen emulsion by two separate times for modeling on days 1 and 7 (the day before modeling was day 0). 0.1ml of collagen emulsion prepared as above was injected intradermally into a root of tail at a single point, and 0.2ml was injected intradermally into back at two points. The animals were subjected to clinical score and paw volume test from day 10 to day 14 after modeling. Animals successfully modeled (average clinical score ≥ 3 points/animal) were assigned to each administration group, with each group included 10 animals. They were administrated intragastrically with above compounds or resolvents at a dosage of 10 mg/kg twice/day, and administrated intragastrically with methotrexate (2mg/kg, Sigma, USA) every three days, until day 27. During the test, the mice were scored twice a week, and the paw volume and body weight were measured twice.

According to the different degrees of lesions (redness and joint deformation), the scores were ranged from 0 to 4 points, with the highest score of 4 points for each limb and 16 points for each animal. Scoring standards are as follows: 0, no redness; 1, slight redness on mid legs (tarsals) or ankles; 2, slight redness from ankles to mid legs (tarsals); 3, medium redness from ankles to tarsometatarsal joints; 4, severe redness from toes or fingers to ankles or wrist joints.

### 3. Test result and conclusion

Data in the following table showed that the compounds of the invention with the dosage of 10 mg/kg could decrease the clinical score of arthritis in mice and paw swelling volume after one week of intragastric administration. Animals could move normally after two weeks of administration, and the progress of arthritis was remarkably relieved. Therefore the compound was superior to methotrexate which was a front-line clinical medicine.

**Table 5 Scores of the compounds of the invention on rheumatoid arthritis models**

| **Group** | **Score** | | | | | | |
|---|---|---|---|---|---|---|---|
| | Day 0 | Day 13 | Day 14 | Day 18 | Day 21 | Day 25 | Day 27 |
| **Model control group** | 0.00±0.00 | 3.50±0.52 | 4.40±0.52 | 6.50±0.45 | 9.80±0.84 | 11.00±1.15 | 12.20±0.95 |
| **Methotrexate** | 0.00±0.00 | 3.10±0.43 | 3.60±0.37 | 4.10±0.47 | 6.45±0.23 | 8.87±0.21 | 9.65±0.13 |
| H07 | 0.00±0.00 | 3.10±0.56 | 4.10±0.41 | 3.10±0.30 | 3.00±0.11 | 2.00±0.25 | 1.00±0.17 |
| H12 | 0.00±0.00 | 3.40±0.39 | 3.90±0.61 | 2.90±0.67 | 1.23±0.29 | 0.60±0.17 | 0.50±0.14 |
| H14 | 0.00±0.00 | 3.30±0.32 | 4.10±0.50 | 3.20±0.73 | 3.20±0.61 | 2.20±0.45 | 1.10±0.27 |
| H25 | 0.00±0.00 | 2.90±0.48 | 3.40±0.27 | 2.13±0.21 | 1.10±0.10 | 0.93±0.18 | 0.74±0.34 |
| H30 | 0.00±0.00 | 3.20±0.37 | 4.00±0.45 | 3.50±0.50 | 2.83±0.29 | 2.00±0.27 | 1.20±0.27 |
| H39 | 0.00±0.00 | 3.10±0.44 | 4.10±0.50 | 3.0±0.37 | 2.33±0.31 | 2.20±0.52 | 1.10±0.27 |
| H44 | 0.00±0.00 | 2.80±0.45 | 3.40±0.27 | 2.13±0.21 | 1.70±0.10 | 0.83±0.48 | 0.64±0.34 |

### Example 6 Pharmacokinetic test of the compounds of the invention

### 1. Abstract

Drug concentrations of compounds H01, H07, H11, H12, H14, H15, H22, H25, H29, H30, H33, H39 and H44 intragastrically administrated in plasma by using LC/MS/MS method at various time points were determined by using Sprague-Dawley (SD) mice, aged 7-8 weeks and weighing 180-220g as test animals. Pharmacokinetic behavior of the compounds of the invention in mice was studied and its pharmacokinetic characteristics were evaluated.

### 2. Test scheme

### 2.1 Compounds for tests

Compounds H01, H07, H11, H12, H14, H15, H22, H25, H29, H30, H33, H39, H44.

### 2.2 Formulation of the compound

A certain amount of compounds was taken and dissolved in 50%PEG400/water to prepare a uniform solution.

### 2.3 Operation

Mice were administered intragastrically with the above compounds with a dosage of 5mg/kg. Blood (0.2ml) was taken from orbits before administration and 0.083h, 0.25h, 0.5h, 1.0h, 2.0h, 4.0h, 6.0h, 8.0h, 12.0h and 24.0h after administration. The blood was placed in anticoagulant tubes containing dipotassium ethylene glycol diethyl ether diamine tetraacetic acid, centrifugated at 6000 rpm at 4 °C for 10 minutes to separate plasma and saved at -80 °C.

50 µL plasma at various time was taken and 150µL acetonitrile solution containing internal standard tolbutamide (Sigma, USA) was added, mixed and stirred for 5 minutes. After centrifugation for 5 minutes at 12,000 rpm, 100µL supernatant was taken, mixed with 200µL water, following by sample injection and analysis.

### 2.4 Liquid chromatogram condition and analysis software

Liquid chromatogram system was LC-20AD UFLC High Performance Liquid Chromatography system. Mass spectrum system was AB Sciex API4000 triple quadrupole mass spectrometer equipped with Electron Spray Ionization (ESI) (Applied Biosystems, Canada). A software for controlling liquid chromatography-mass spectrometry and quantitative analysis was Anna Rust 1.6 (Applied Biosystems, Canada). Pharmacokinetic parameters were analyzed by WinNonlin (version 5.2, Pharsight, Mountain View, Canada) non-compartment model.

### 2.5 Standards and preparation of quality control solution

The test compound was dissolved in dimethyl sulfoxide to prepare a stock solution with a concentration of 1 mg/mL. The solution was diluted with 50% acetonitrile to obtain a series of standard working solutions with concentrations of 10 µg/mL, 3 µg/mL, 1 µg/mL, 0.3 µg/mL, 0.1 µg/mL, 0.03 µg/mL and 0.01 µg/mL, and a series of standard quality control solutions with concentrations of 8 µg/mL, 0.5 µg/mL, 0.03 µg/mL. 5 µL standard solution was mixed with 45 µL blank plasma substrate uniformly to obtain standard solutions of each concentration of a standard curve (1000 ng/mL, 500 ng/mL, 200 ng/mL, 100 ng/mL, 10 ng/mL, 5 ng/mL, 2 and 1 ng/mL) and quality control solutions (plasma samples: 800 ng/mL, 50 and 3 ng/mL).

Internal standard tolbutamide solid powder was dissolved in dimethyl sulfoxide to prepare a 1 mg/mL stock solution. The stock solution was diluted with 100 % acetonitrile to obtain 200 ng/mL solution as a protein precipitation solution.

### 3. Test result

**Table 6 Results of pharmacokinetic parameters of the compounds of the invention**

| **Compound** | **Dosag** 5mg/kg | | |
|---|---|---|---|
| | **Plasma concentration** (Cₘₐₓ, ng/ml) | **Area under the curve** (AUC₀₋₂₄, ng/ml.h) | **half life** |
| | | | (T_{1/2}, h) |
| H01 | 578.00±72.00 | 1720.00±220.00 | 2.13±0.27 |
| H07 | 578.00±43.00 | 1918.00±20.00 | 4.62±0.87 |
| H11 | 428.00±32.00 | 2620.00±10.00 | 8.53±0.67 |
| H12 | 644.00±82.00 | 1020.00±266.00 | 4.23±0.47 |
| H14 | 438.00±24.00 | 915.00±240.00 | 2.11±0.16 |
| H15 | 518.00±24.00 | 1515.00±241.00 | 5.11±0.16 |
| H22 | 213.00±22.00 | 757.00±169.00 | 3.11±0.64 |
| H25 | 501.00±17.00 | 1273.00±231.00 | 4.67±0.23 |
| H29 | 522.00±23.00 | 1529.00±176.00 | 4.43±0.32 |
| H30 | 433.00±16.00 | 923.00±263.00 | 2.47±0.46 |
| H33 | 440.00±89.00 | 1821.00±173.00 | 8.64±0.23 |
| H39 | 574.00±64.00 | 1556.00±230.00 | 4.65±0.15 |
| H44 | 619.00±39.00 | 1680.00±120.00 | 4.17±0.45 |

Data from the above table showed a pharmacokinetic feature that the compounds of the invention had a better pharmacokinetic absorption.

### Example 7 Inhibiting effect test of the compounds on hERG potassium channel current

### 1. Abstract

Effects of compounds H07, H12, H14, H25, H30, H39 and H44 on hERG potassium channel were detected by applying electrophysiological manual patch clamp to study an initial cardiac safety of the compounds of the invention.

### 2. Test scheme

### 2.1 Formulation of the compound

Test compounds were dissolved in dimethyl sulfoxide to prepare mother solutions (10mM, 3.3mM, 1.1mM and 0.37mM). Next an extracellular fluid was used for secondary dilution so that the test solution had final concentrations of 30 µM, 10 µM, 3.3 µM, 1.1 µM and 0.37 µM.

### 2.2 Preparation of solvents

Extracellular fluids: 130mM sodium chloride, 4 mM potassium chloride, 1.8 mM calcium chloride, 1 mM magnesium chloride, 10 mM glucose, 10 mM 4-hydroxyethylpiperazine ethane sulfonic acid (pH 7.4)

Intracellular fluid: 130 mM potassium chloride, 1 mM magnesium chloride, 5 mM ethylene glycol diethyl ether diamine tetraacetic acid, 5 mM triphosadenine and 10 mM 4-hydroxyethylpiperazine ethane sulfonic acid (pH7.2)

Compositions of a cell culture medium: Dulbecco's modified eagle medium (Grand Turk Island Biotechnology Company, 11330032), 15% fetal calf serum (Sigma, A15-101, USA), 1% penicillin-streptomycin (Biowest, L0022-100).

### 2.3 Test scheme of electrophysiological manual patch clamp system

HEK293 cells overexpressing hERG potassium channel (from Dr. Mohamed Boutjdir's laboratory, School of Medicine, New York University-PharmaCore Labs, Inc.) were incubated in a medium consisting of Dulbecco's modified eagle medium/15% fetal calf serum/1% penicillin-streptomycin in a 5% CO2 incubator at 37 °C. In the test, the cells were transferred to a cell bath embedded into a inverted microscope platform, and extracellular fluid was perfused. After stabilization for 5 minutes, the cells were precipitated and then the test could start. Membrane currents were recorded by using HEKA EPC-10 patch clamp amplifier and PATCHMASTER acquisition system (HEKA Instrument Co., Ltd., D-67466 Lambrcht, Palatinate, Germany). All tests were completed at room temperature (22 -24°C). P-97 microelectrode puller (Sutter Instrument Company, Novato, Canada 94949) was used to straighten the electrodes (BF150-110-10). The inner diameter of the electrode was 1-1.5 mm, and the water resistance after internal liquid filling was 2-4 MΩ.

Tests were carried out in whole-cell recording mode, with current values recorded according to the electrophysiological stimulation protocol below. First, the membrane voltage was clamped at -80 mV, and the cells were stimulated with +20mV for two seconds to activate hERG potassium channel, which was then repolarized to -50 mV for five seconds, producing an outward tail current with a stimulation frequency of every 15 seconds. The current value was a peak value of the tail current. In the test, the channel current was recorded in the whole-cell recording mode. First, extracellular fluid (about 2 ml per minute) was perfused and recorded continuously, and then the current was allowed to stabilize (current decay (run-down) was less than 5% within 5 minutes). At this time, the peak value of the tail current was the control current value. Subsequently, the extracellular fluid containing the drug to be tested was perfused and recorded continuously until the inhibiting effect of drug on hERG current reached a stable state, at which time the peak value of the tail current was the current value after adding drugs. After a stable situation was achieved, if hERG current recovered after perfusing and washing with extracellular fluid, perfusion could be continued to test other concentrations or drugs. The solution containing the drugs to be tested was perfused in descending order of concentration and the current values were recorded. PATCHMASTER V2X60 (HEKA Instrument Co., Ltd., D-67466 Lambrcht, Palatinate, Germany) was used to collect data. ORANGE 8.5 (ORANGE Laboratory Company, Northampton, UK) software was used for the analysis and statistics.

### 3. Result

IC₅₀ of the compounds of the invention for inhibiting hERG current was shown in Table 7 below. Data from the table showed the compounds of the invention had a weak effect on inhibiting hERG current and better safety.

**Table 7 Inhibiting effect of the compounds of the invention on hERG current**

| **Compound** | IC₅₀ (µM) |
|---|---|
| H07 | >30 |
| H12 | >30 |
| H14 | >30 |
| H25 | >30 |
| H30 | >30 |
| H39 | >30 |
| H44 | >30 |

### Example 8 Acute toxicity test of the compounds of the invention

### 1. ABSTRACT

ICR mice (Beijing Vital River Laboratory Animal Technology Co., Ltd.), 20-22 g and aged 8 weeks, half male and half female, were orally administered with the compounds of Example H07, Example H12, Example H14, Example H25, Example H30, Example H39 and Example H34 once for continuous observation for 14 days, including state observation on the edge of a cage, daily body weight weighing, and endpoint histopathological observation.

### 2. Test scheme

### 2.1 Compounds for tests

Compounds of Example H07, Example H12, Example H14, Example H25, Example H30, Example H39 and Example H34.

### 2.2 Formulation of the compound

A certain amount of compounds were taken and dissolved in 0.5% hydroxy propyl methyl cellulose to prepare a uniform solution.

### 2.3 Operation

Up-down method was used to observe acute toxicity of mice after oral administration once. There were 10 mice per dosage group. The dosage of administration was 100 mg/kg, 300 mg/kg and 1000mg/kg, and the volume of administration was 10 ml/kg.

### 2.4 Test statistics

LD₅₀ was calculated using Bliss software based on a death rate of animal of each dosage.

### 2.5 Test result

After continuous observation for 14 days, all the animals in the administration group survived, and their weights increased normally without any abnormal performance. After the 14-day observation period, all the animals were euthanized and underwent gross anatomical examination. No obvious abnormality was found on body surfaces and no visible lesion was found in the thoracic cavity, enterocoelia, pelvic cavity and cranial cavity.

Under the test conditions, the LD₅₀ of the compounds of the invention after oral administration to mice was more than 1000 mg/kg, which indicated the safety was good.

## Claims

1. A compound of formula (II) or tautomers, mesomers, racemes, enantiomers, diastereoisomers, or mixtures thereof, pharmaceutically acceptable salts, solvates or isotope derivatives thereof, 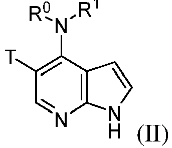
wherein, T is -CN or -CF₃;
R⁰ is -H or -C₁₋₃ alkyl;
R¹ is 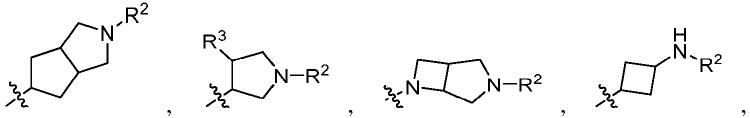 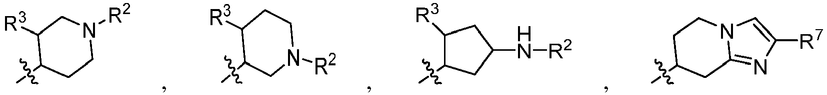 or 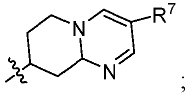
R² is -C(O)R⁴, -S(O)₂R⁴ or -C(O)NR⁵R⁶;
R³ is H, -C₁₋₄ alkyl or -C₃₋₆ cycloalkyl;
R⁴ is -C₁₋₅ alkyl, 4- to 6-membered heterocyclyl, 5- to 10-membered heteroaryl, phenyl, -heterocyclyl-O-R^{e}, -heterocyclyl-R^{4a}-O-R^{e} or -heterocyclyl-R^{4a}-NH-R^{e}, wherein the -C₁₋₅ alkyl can be substituted by the following groups: -OH, -C₁₋₃ alkyl, -O-C₁₋₄ alkyl, -NH₂, halogen, CF₃, phenyl or cyano;
wherein the heterocyclyl, heteroaryl or phenyl of R⁴ can be substituted by R^{e};
R^{4a} is -C₁₋₃ alkylene;
R⁵ is H or -C₁₋₃ alkyl;
R⁶ is -C₁₋₄ alkyl, -C₃₋₆ cycloalkyl, 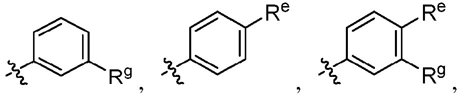 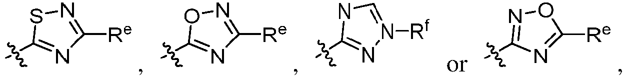 wherein the -C₁₋₄ alkyl can be substituted by the following groups: -OH, -C₁₋₃ alkyl, -C₃₋₆ cycloalkyl, -O-C₁₋₄ alkyl, -NH₂, halogen, CF₃, phenyl or cyano group;
R⁷ is hydrogen, -(CH₂)ₙ-OH, -(CH₂)ₙ-CF₃ or -(CH₂)ₙ-CN;
R^{e} and R^{g} are each independently halogen, -C₁₋₄ alkyl, alkoxy, 3- to 6-membered cycloalkyl, -NR^{c}R^{d}, -(CH₂)ₙ NR^{c}R^{d}, -(CH₂)ₙ-OH, -(CH₂)ₙ-CF₃, -(CH₂)ₙ-CN or C(O)OCH₃, wherein the -C₁₋₄ alkyl, alkoxy or cycloalkyl can be monosubstituted or disubstituted by the following groups: halogen, -OH, -NH₂, -C₁₋₃ alkyl, -O-C₁₋₄ alkyl, CF₃, phenyl or cyano;
R^{f} is -C₁₋₄ alkyl;
R^{c} and R^{d} are each independently hydrogen, -C₁₋₃ alkyl; and
n is 0, 1, 2 or 3.

2. The compound or tautomers, mesomers, racemes, enantiomers, diastereoisomers, or mixtures thereof, pharmaceutically acceptable salts, solvates or isotope derivatives thereof according to claim 1, wherein
R³ is H, -CH₃, -CH₂CH₃ or 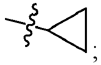
R⁴ is methyl, ethyl, propyl, -(CH₂)ₙ-CH₂CN, -(CH₂)ₙ-CH₂CF₃, -CH(OH)-(CH₂)ₙ CH₃, 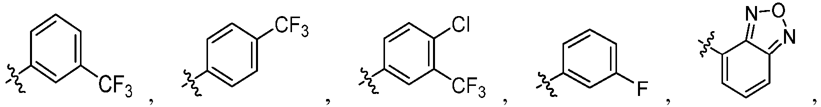 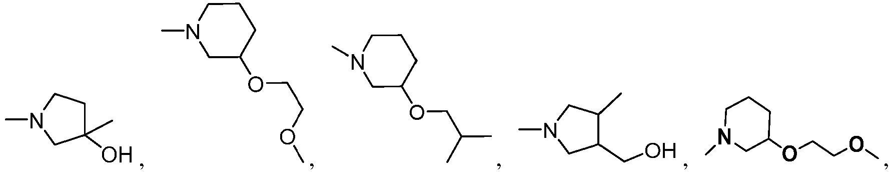 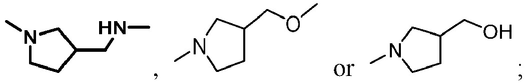
R⁵ is H;
R⁶ is -(CH₂)ₙ-CH₂CF₃, -(CH₂)ₙ-CF₂CF₃, -(CH₂)ₙ-CH₂CN, 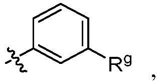 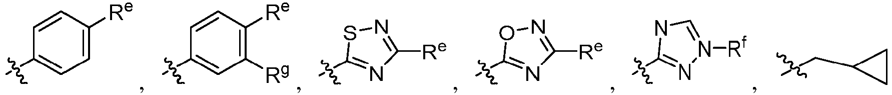 or 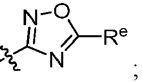
R^{e} and R^{g} are each independently -F, -Cl, methyl, ethyl, propyl, -CH(CH₃)-(CH₂)ₙCH₃, -CH₂CH(CH₃)₂, -CH(CH₃)CH(CH₃)₂, -CH₂C(CH₃)₃, -C(CH₃)₂-(CH₂)ₙCH₃, -O-(CH₂)ₙCH₃, -O-(CH₂)ₙCH₂-O-CH₃, -(CH₂)ₙOCH₃, -O-(CH₂)ₙ-CH₂-C(CH₃)₂-OH, -O-(CH₂)ₙ-CH₂-C(CH₃)₂-NH₂, C₃₋₆ cycloalkyl, -(CH₂)ₙNR^{c}R^{d}, -(CH₂)ₙ-OH, -(CH₂)ₙ-CF₃, -(CH₂)ₙ-CHF₂, -(CH₂)ₙ-CN or -C(O)-O-CH₃;
R^{f} is methyl, ethyl or propyl;
R^{c} and R^{d} are each independently -H, methyl, ethyl or propyl; and
n is 0 or 1.

3. The compound or tautomers, mesomers, racemes, enantiomers, diastereoisomers, or mixtures thereof, pharmaceutically acceptable salts, solvates or isotope derivatives thereof according to any one of claims 1-2, wherein the compound is:

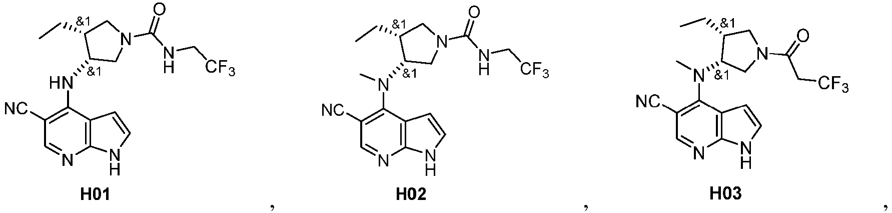

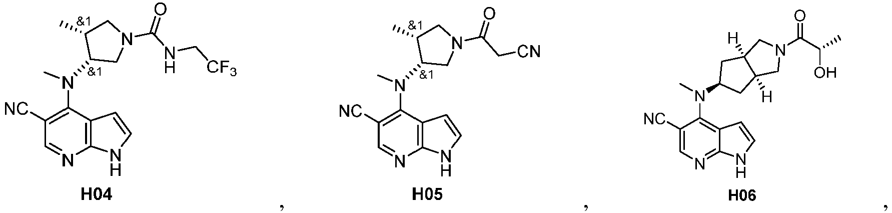

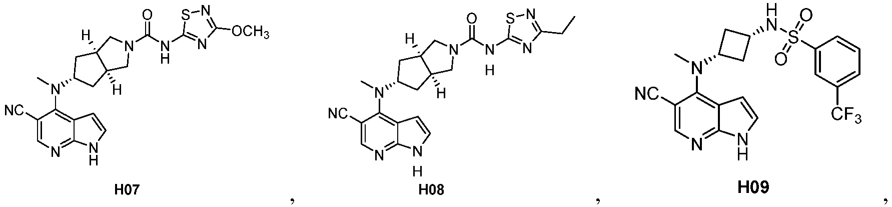

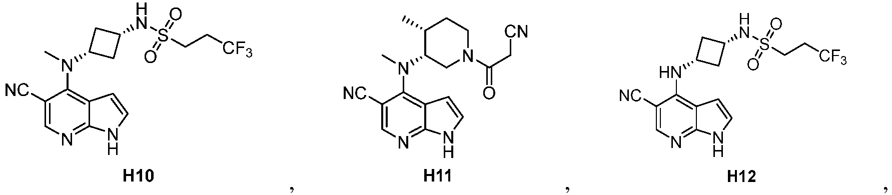

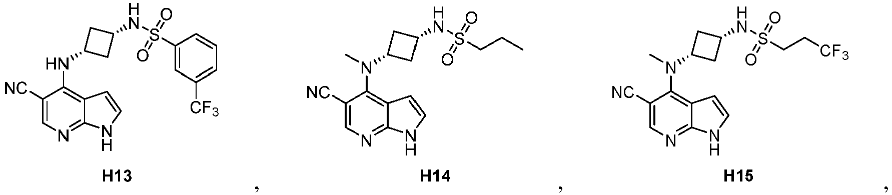

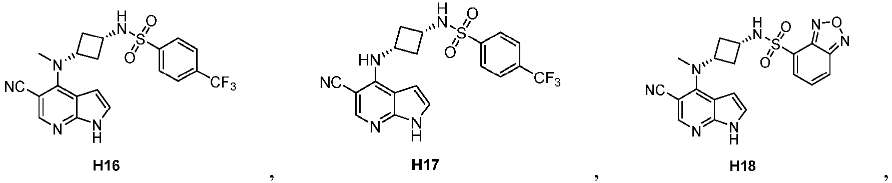

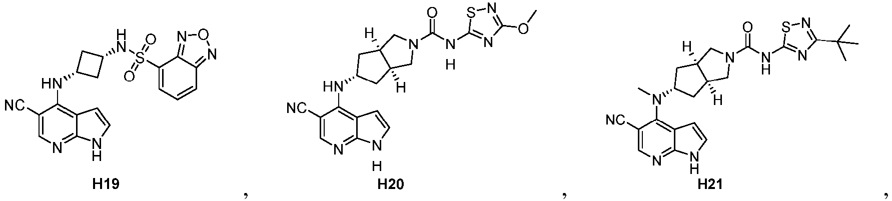

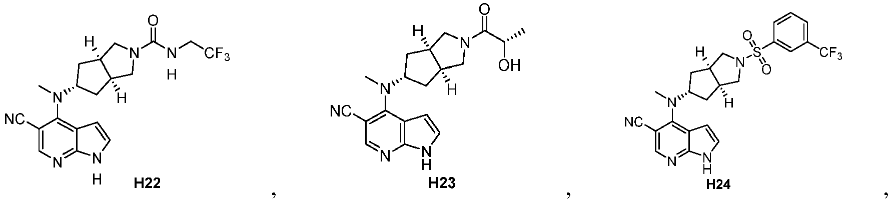

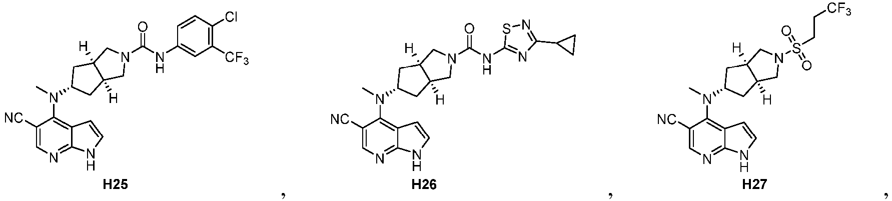

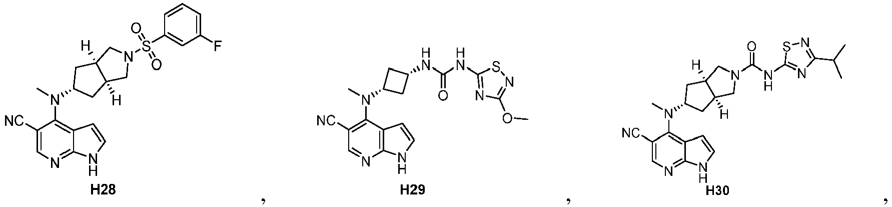

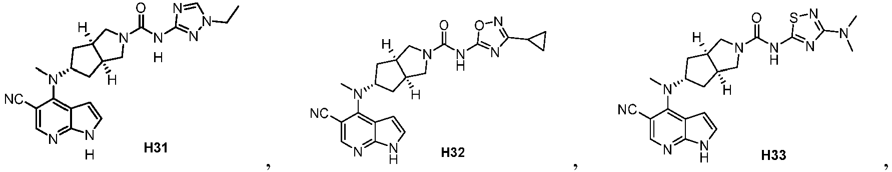

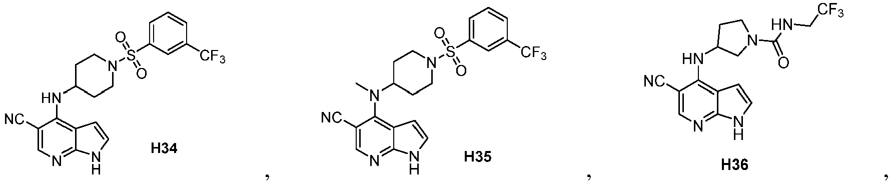

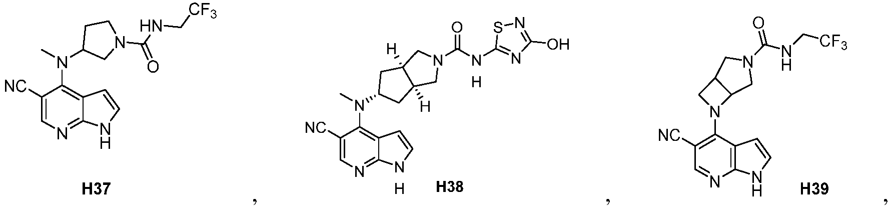

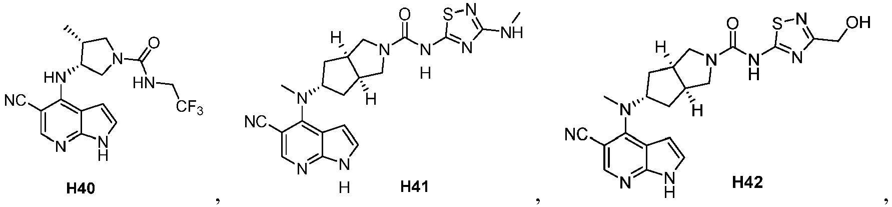

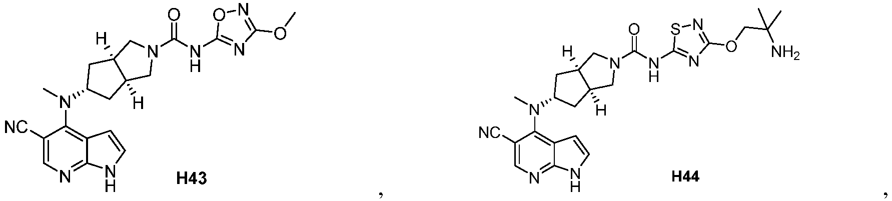

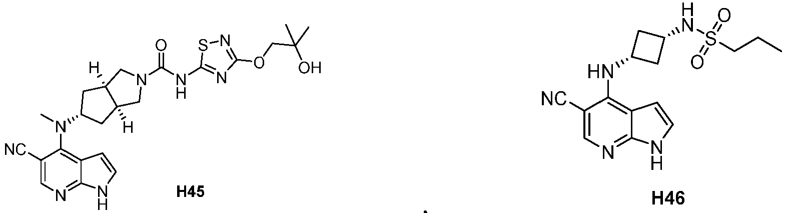

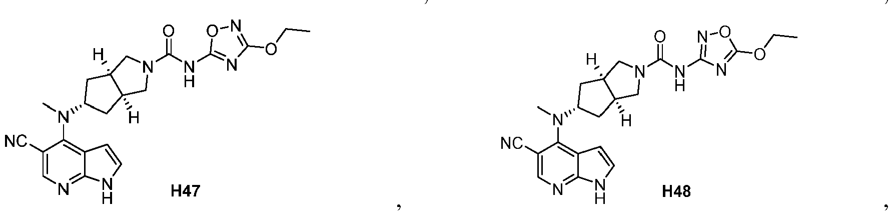

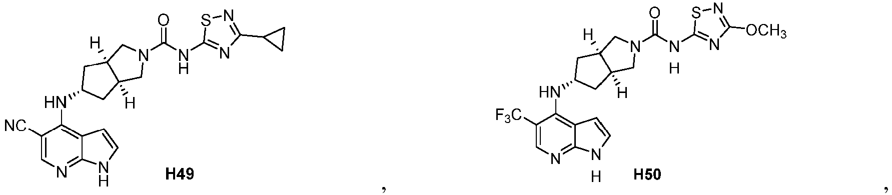

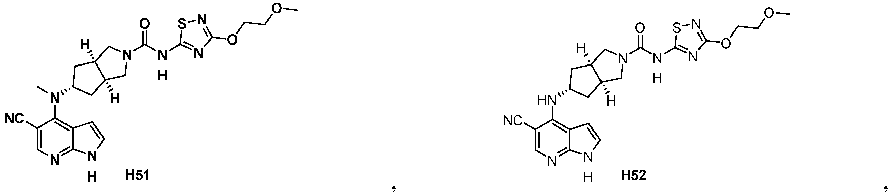

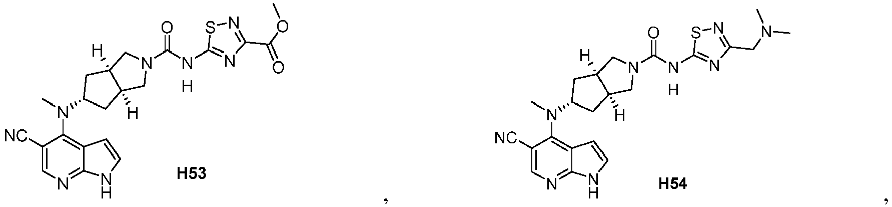

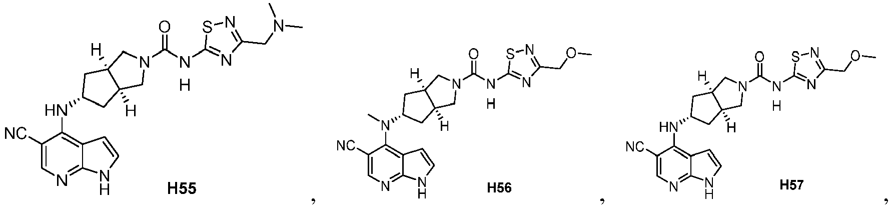

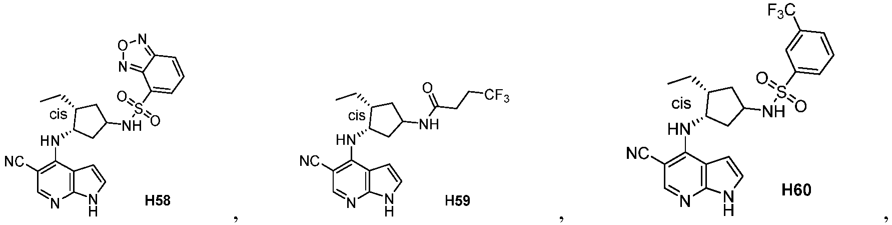

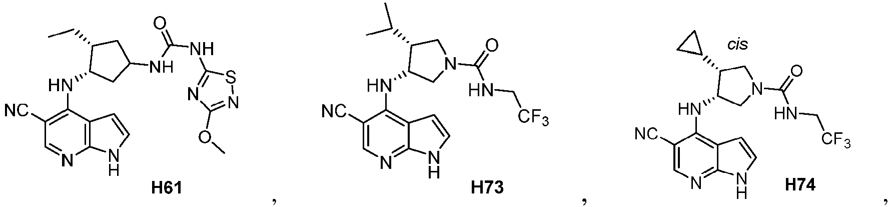

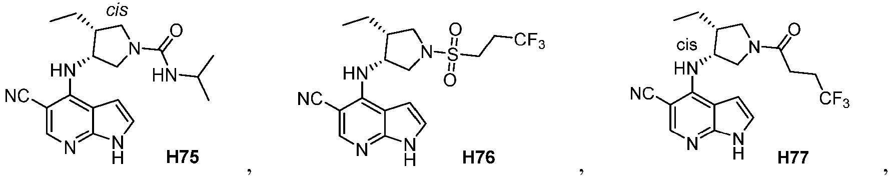

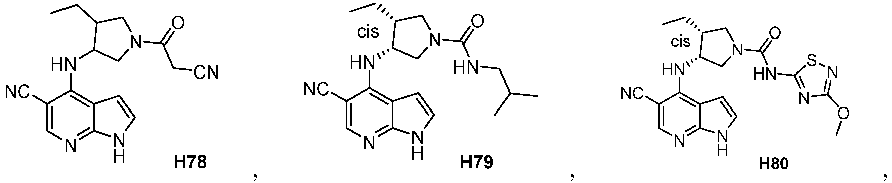

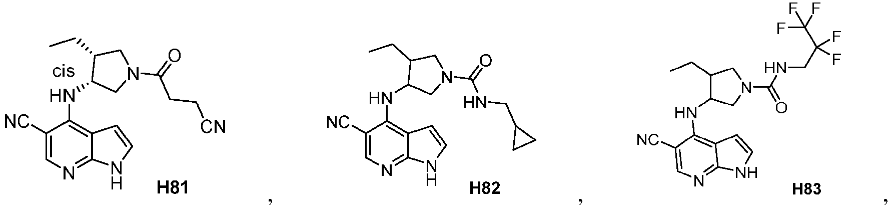

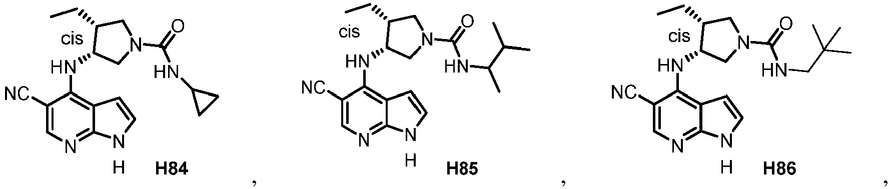

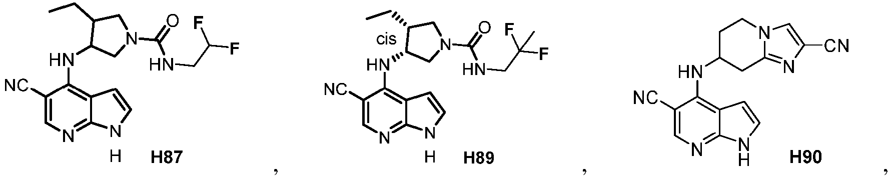

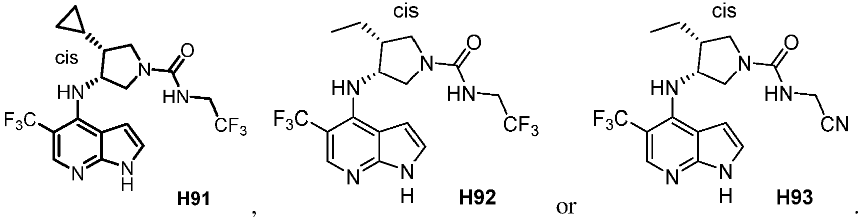

4. The compound or tautomers, mesomers, racemes, enantiomers, diastereoisomers, or mixtures thereof, pharmaceutically acceptable salts, solvates or isotope derivatives thereof according to any one of claims 1-3, wherein the pharmaceutically acceptable salts are acid addition salts, alkali addition salts, inner salts or betaines; the acid addition salts are, for example, hydrochlorides, sulfates, hydrosulfates, nitrates, phosphates, hydrophosphate, dihydrogenphosphates, hydrobromides, hydriodates, acetates, lactates, mesylates, citrates, malates, maleates, fumarate, tartrate, salicylates or stearates; the alkali addition salts are, for example, alkali metal salts, alkaline-earth metal salts or ammonium salts, such as sodium salts, potassium salts, calcium salts, magnesium salts or salts with ammonia or organic amines, such as ethylamines, ethanolamines, triethanolamines or amino acids.

5. A method for preparing the compound of formula (II) or tautomers, mesomers, racemes, enantiomers, diastereoisomers, or mixtures thereof, pharmaceutically acceptable salts, solvates or isotope derivatives thereof according to any one of claims 1-4, wherein the compound of formula (II) is prepared by: 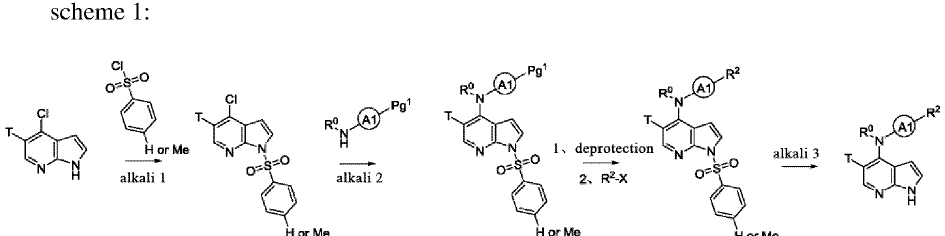 ; or 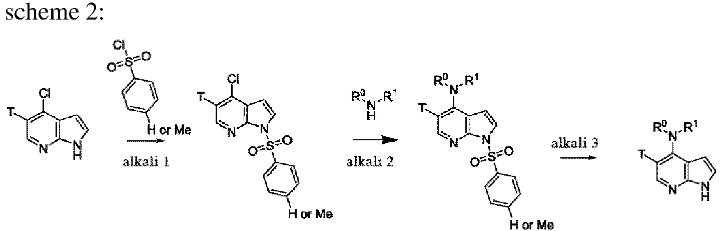 wherein, Pg¹ is an aliphatic amine protecting group, in particular tert-butoxycarbonyl or benzyloxycarbonyl, and A1 is 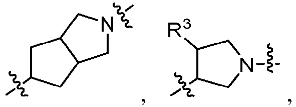 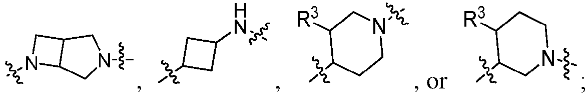 R²-X is an active form of R², and X is preferably selected from the group consisting of bromine, chloride, phenoxyl and p-nitrophenoxy, or R²-X is isocyanate form of R²; alkali 1, alkali 2 and alkali 3 are each independently selected from the group consisting of triethylamine, diisopropyl ethylamine, potassium carbonate, cesium carbonate, sodium carbonate, sodium hydroxide and potassium hydroxide.

6. A pharmaceutical composition, comprising a therapeutically effective amount of the compound or tautomers, mesomers, racemes, enantiomers, diastereoisomers, or mixtures thereof, pharmaceutically acceptable salts, solvates or isotope derivatives thereof according to any one of claims 1-4, and optionally one or more pharmaceutically acceptable carriers and excipients, wherein the carriers are, for example, lactoses, white sugar, sodium chlorides, glucoses, urea, starches, calcium carbonates, kaolins, crystalline celluloses and silicic acids; the excipients are, for example, adhesives such as water, ethanol, propanol, regular syrup, dextrose solutions, starch solutions, gelatin solutions, carboxymethyl celluloses, shellacs, methyl celluloses and potassium phosphates, polyvinylpyrrolidone; disintegrants, such as dry starches, sodium alginates, agar powder, kelp powder, sodium bicarbonates, calcium carbonates, polyoxyethylene sorbitan fatty acid esters, sodium dodecyl sulfates, monoglyceride stearates, starches and lactoses; disintegration inhibiting agents, such as white sugar, glycerol tristearates, coconut oil and hydrogenated oil; absorptive accelerators, such as quaternary ammonium hydroxides and sodium dodecyl sulfates; humectants, such as glycerol and starches; adsorbents, such as starches, lactoses, kaolins, bentonites and colloid silicic acids; and lubricants, such as pure talcs, stearates, boric acid powder and polyethylene glycol, wherein the pharmaceutical composition also comprises additional one or more reagents, anticarcinogens or anti-inflammatory agents that regulate immune systems of mammals.

7. The compound or tautomers, mesomers, racemes, enantiomers, diastereoisomers, or mixtures thereof, pharmaceutically acceptable salts, solvates or isotope derivatives thereof according to any one of claims 1-4 or the pharmaceutical composition according to claim 6 for use as a medicament.

8. The compound or tautomers, mesomers, racemes, enantiomers, diastereoisomers, or mixtures thereof, pharmaceutically acceptable salts, solvates or isotope derivatives thereof according to any one of claims 1-4 or the pharmaceutical composition according to claim 6 for use in preventing and/or treating JAK-mediated diseases, wherein the JAK-mediated diseases include: immune system diseases; autoimmune diseases; dermatosis; allergic diseases; viral diseases; type 1 diabetes and diabetic complication; alzheimer disease; xerophthalmia; myelofibrosis; thrombocytosis; polycythemia or leukemia; cancers; inflammatory bowel disease; myelodysplastic syndrome; myeloma.

9. The compound for use according to claim 8, wherein the JAK-mediated diseases include: organ-graft rejection such as allograft rejection and graft-versus-host disease; lupus, multiple sclerosis, rheumatoid arthritis, juvenile arthritis, psoriasis, ulcerative colitis, Crohn's disease and autoimmune thyroid diseases; psora, skin rash, atopic dermatitis; allergies, asthma and rhinitis; hepatitis B, hepatitis C and varicella-zoster virus; solid tumor such as prostate cancer, renal carcinoma, liver cancer, pancreatic cancer, gastric cancer, breast cancer, lung cancer, head-neck carcinoma, thyroid cancer, glioblastoma, melanoma, hematologic malignancy, such as lymphoma, leukemia, skin cancer (cutaneous T-cell lymphoma, cutaneous B-cell lymphoma).

## Patentansprüche

1. Verbindung der Formel (II) oder Tautomere, Mesomere, Raceme, Enantiomere, Diastereomere oder Gemische davon, pharmazeutisch verträgliche Salze, Solvate oder Isotopenderivate davon, 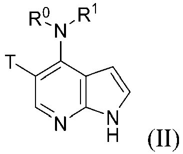
wobei T -CN oder -CF₃ ist;
R⁰ -H oder -C₁₋₃-Alkyl ist;
R¹ 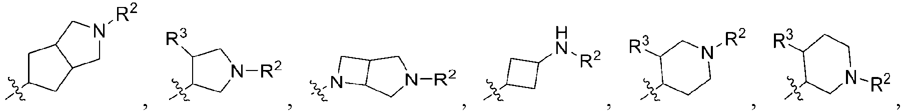 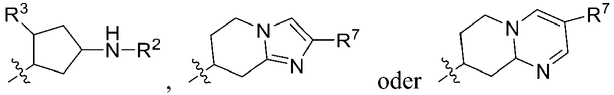 ist;
R² -C(O)R⁴, -S(O)₂R⁴ oder -C(O)NR⁵R⁶ ist;
R³ H, -C₁₋₄-Alkyl oder -C₃₋₆-Cycloalkyl ist;
R⁴ -C₁₋₅-Alkyl, 4- bis 6-gliedriges Heterocyclyl, 5- bis 10-gliedriges Heteroaryl, Phenyl, - Heterocyclyl-O-R^{e}, -Heterocyclyl-R^{4a}-O-R^{e} oder -Heterocyclyl-R^{4a}-NH-R^{e} ist, wobei das -C₁₋₅-Alkyl durch die folgenden Gruppen substituierbar ist: -OH, -C₁₋₃-Alkyl, -O-C₁₋₄-Alkyl, -NH₂, Halogen, CF₃, Phenyl oder Cyano;
wobei das Heterocyclyl, Heteroaryl oder Phenyl von R⁴ durch R^{e} substituierbar ist;
R^{4a} -C₁₋₃-Alkylen ist;
R⁵ H oder -C₁₋₃-Alkyl ist;
R⁶ -C₁₋₄-Alkyl, -C₃₋₆-Cycloalkyl, 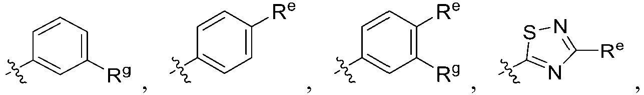 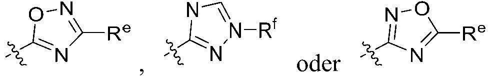 ist, wobei das -C₁₋₄-Alkyl durch die folgenden Gruppen substituierbar ist: -OH, -C₁₋₃-Alkyl, -C₃₋₆-Cycloalkyl, -O-C₁₋₄-Alkyl,-NH₂, Halogen, CF₃, Phenyl- oder Cyanogruppe;
R⁷ Wasserstoff, -(CH₂)ₙ-OH, -(CH₂)ₙ-CF₃ oder -(CH₂)ₙ-CN ist;
R^{e} und R^{g} jeweils unabhängig Halogen, -C₁₋₄-Alkyl, Alkoxy, 3- bis 6-gliedriges Cycloalkyl, -NR^{c}R^{d}, -(CH₂)ₙNR^{c}R^{d}, -(CH₂)ₙ-OH, -(CH₂)ₙ-CF₃, -(CH₂)ₙ-CN oder C(O)OCH₃ sind, wobei das -C₁₋₄-Alkyl-, Alkoxy oder Cycloalkyl durch die folgenden Gruppen monosubstituierbar oder disubstituierbar sind: Halogen, -OH, -NH₂, -C₁₋₃-Alkyl, -O-C₁₋₄-Alkyl, CF₃, Phenyl oder Cyano;
R^{f} -C₁₋₄-Alkyl ist;
R^{c} und R^{d} jeweils unabhängig Wasserstoff, -C₁₋₃-Alkyl sind; und
n 0, 1, 2 oder 3 ist.

2. Verbindung oder Tautomere, Mesomere, Raceme, Enantiomere, Diastereomere oder Gemische davon, pharmazeutisch verträgliche Salze, Solvate oder Isotopenderivate davon nach Anspruch 1, wobei
R³ H, -CH₃, -CH₂CH₃ oder 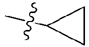 ist;
R⁴ Methyl, Ethyl, Propyl, -(CH₂)ₙ-CH₂CN, -(CH₂)ₙ-CH₂CF₃, -CH(OH)-(CH₂)ₙCH₃, 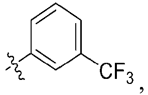 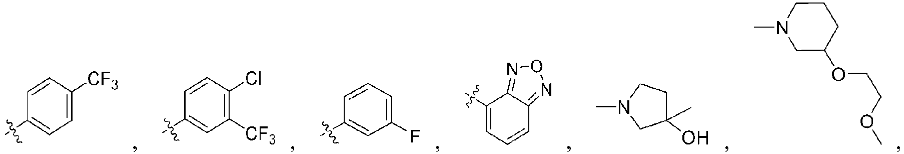 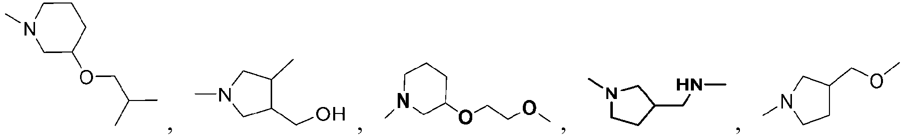 oder 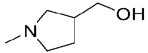 ist;
R⁵ H ist;
R⁶ -(CH₂)ₙ-CH₂CF₃, -(CH₂)ₙ-CF₂CF₃, -(CH₂)ₙ-CH₂CN, 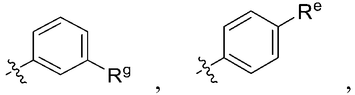 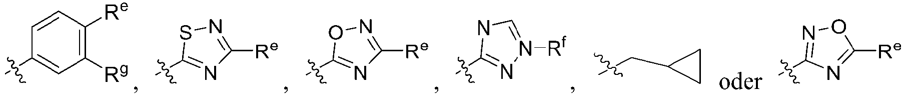 ist;
R^{e} und R^{g} jeweils unabhängig -F, -Cl, Methyl, Ethyl, Propyl, -CH(CH₃)-(CH₂)ₙCH₃, -CH₂CH(CH₃)₂, -CH(CH₃)CH(CH₃)₂, -CH₂C(CH₃)₃, -C(CH₃)₂-(CH₂)ₙCH₃, -O-(CH₂)ₙCH₃, -O-(CH₂)ₙCH₂-O-CH₃, - (CH₂)ₙOCH₃, -O-(CH₂)ₙ-CH₂-C(CH₃)₂-OH, -O-(CH₂)ₙ-CH₂-C(CH₃)₂-NH₂, C₃₋₆-Cycloalkyl, - (CH₂)ₙNR^{c}R^{d}, -(CH₂)ₙ-OH, -(CH₂)ₙ-CF₃, -(CH₂)ₙ-CHF₂, -(CH₂)ₙ-CN oder -C(O)-O-CH₃ sind;
R^{f} Methyl, Ethyl oder Propyl ist;
R^{c} und R^{d} jeweils unabhängig -H, Methyl, Ethyl oder Propyl sind; und
n 0 oder 1 ist.

3. Verbindung oder Tautomere, Mesomere, Raceme, Enantiomere, Diastereomere oder Gemische davon, pharmazeutisch verträgliche Salze, Solvate oder Isotopenderivate davon nach einem der Ansprüche 1-2, wobei die Verbindung Folgendes ist:

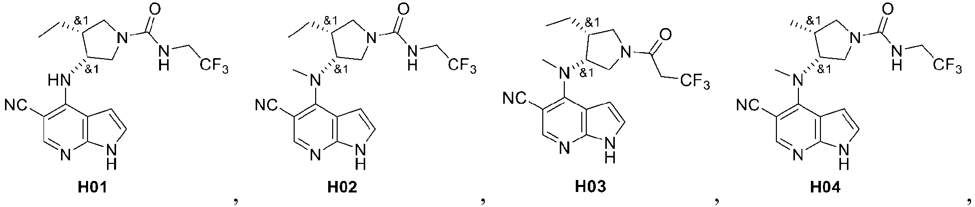

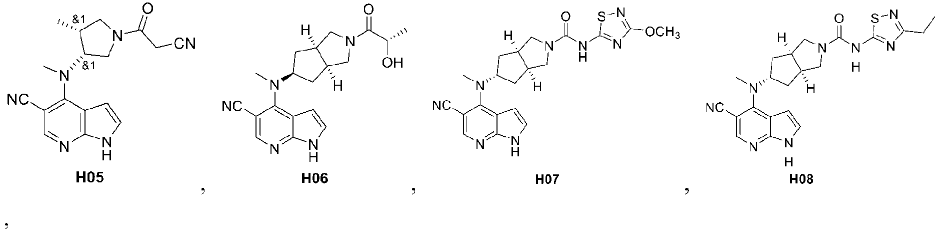

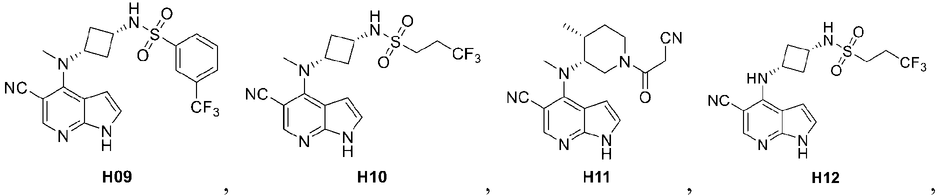

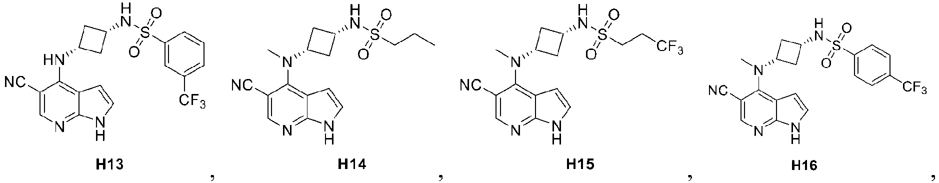

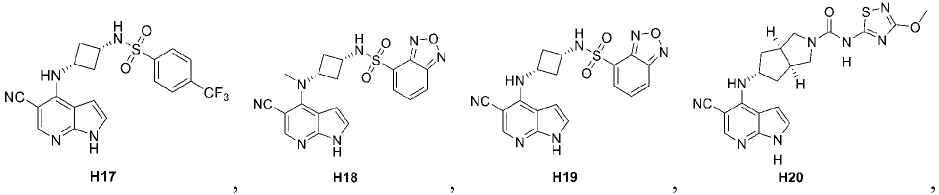

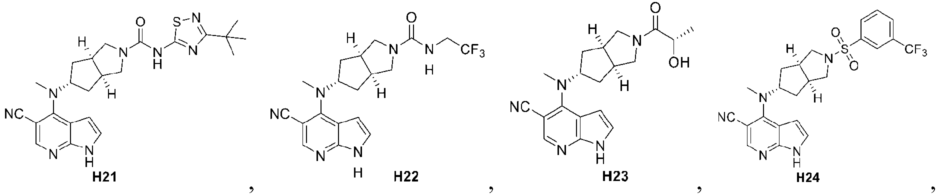

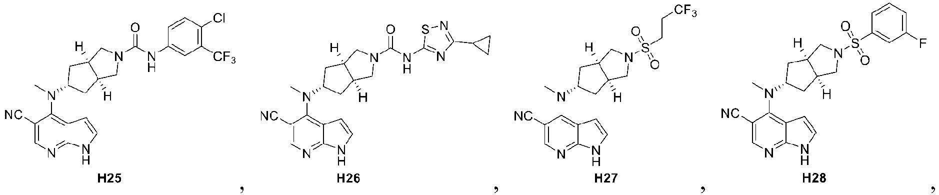

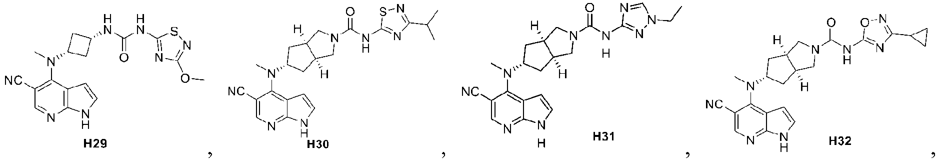

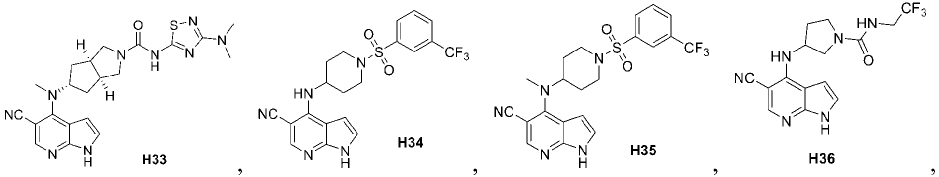

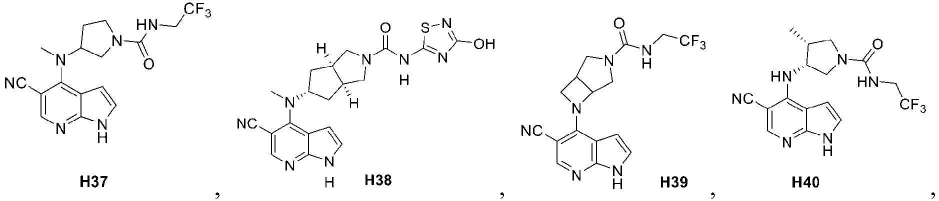

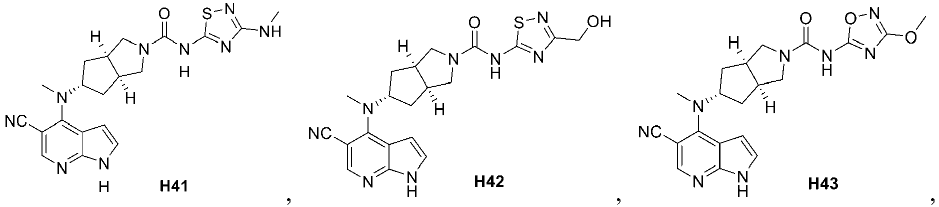

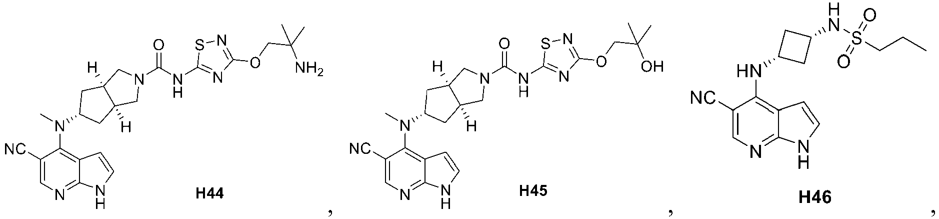

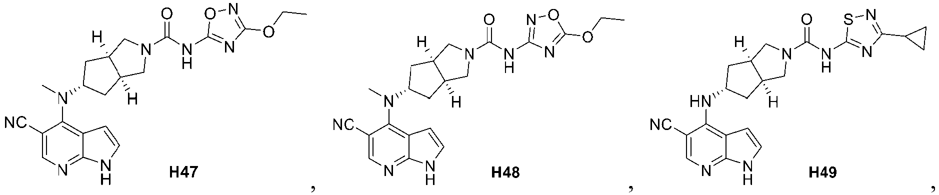

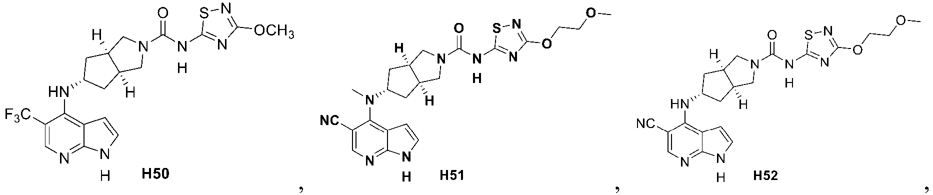

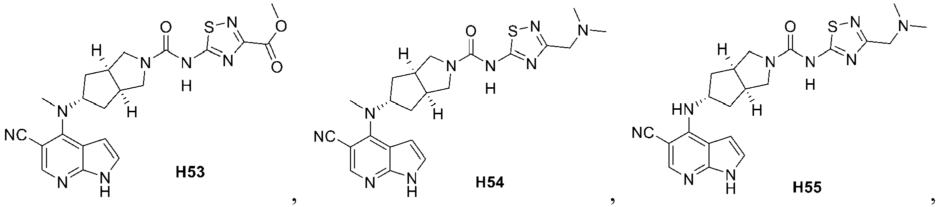

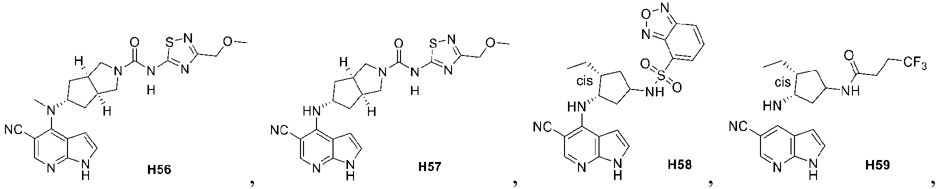

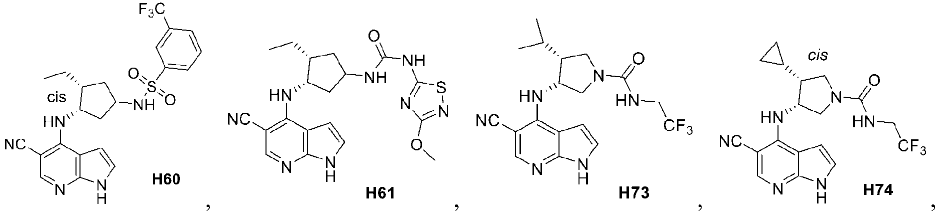

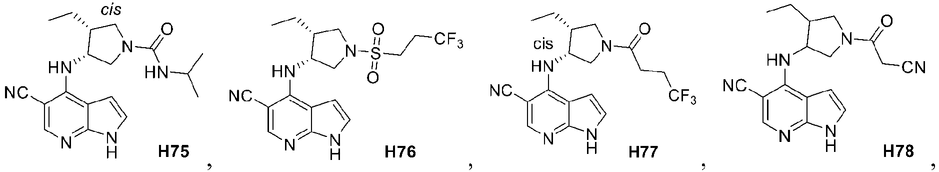

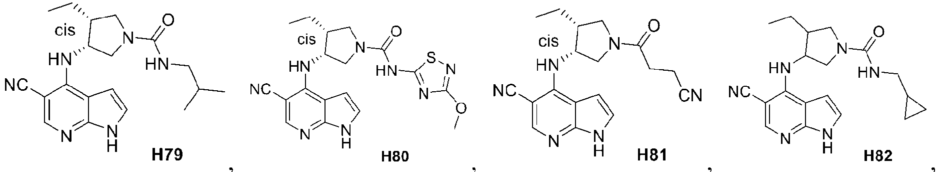

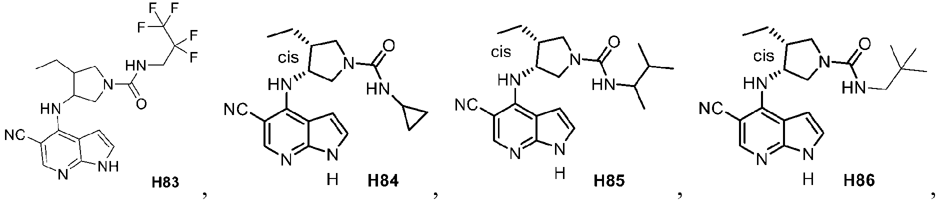

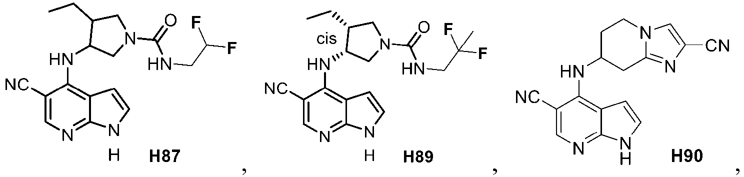

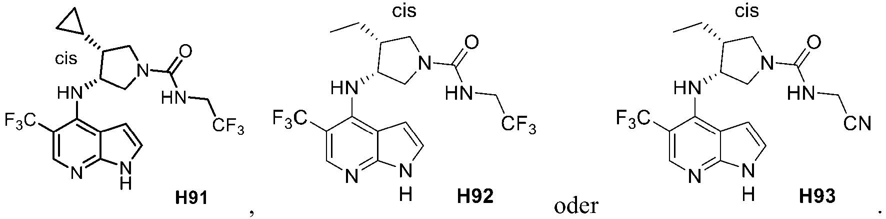

4. Verbindung oder Tautomere, Mesomere, Raceme, Enantiomere, Diastereomere oder Gemische davon, pharmazeutisch verträgliche Salze, Solvate oder Isotopenderivate davon nach einem der Ansprüche 1-3, wobei die pharmazeutisch verträglichen Salze Säureadditionssalze, Alkaliadditionssalze, innere Salze oder Betaine sind; wobei die Säureadditionssalze zum Beispiel Hydrochloride, Sulfate, Hydrosulfate, Nitrate, Phosphate, Hydrophosphate, Dihydrogenphosphate, Hydrobromide, Hydroiodate, Acetate, Lactate, Mesylate, Citrate, Malate, Maleate, Fumarat, Tartrat, Salicylate oder Stearate sind; wobei die Alkaliadditionssalze zum Beispiel Alkalimetallsalze, Erdalkalimetallsalze oder Ammoniumsalze, wie Natriumsalze, Kaliumsalze, Calciumsalze, Magnesiumsalze oder Salze mit Ammoniak oder organischen Aminen, wie Ethylaminen, Ethanolaminen, Triethanolaminen oder Aminosäuren sind.

5. Verfahren zum Herstellen der Verbindung der Formel (II) oder von Tautomeren, Mesomeren, Racemen, Enantiomeren, Diastereomeren oder Gemischen davon, pharmazeutisch verträglichen Salzen, Solvaten oder Isotopenderivaten davon nach einem der Ansprüche 1-4, wobei die Verbindung der Formel (II) hergestellt wird durch: 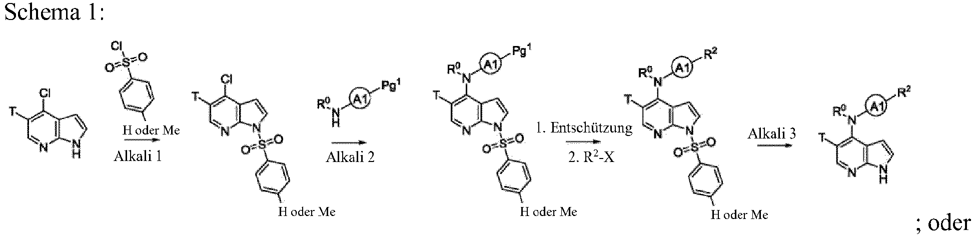 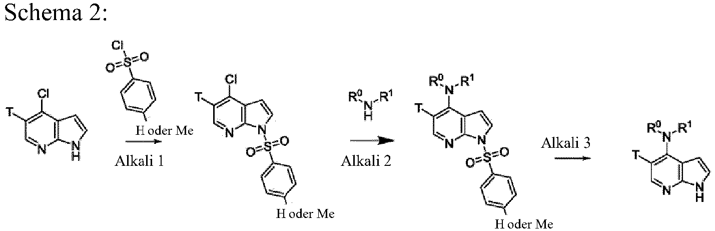 wobei Pg¹ eine aliphatische Amin-Schutzgruppe, insbesondere tert-Butoxycarbonyl oder Benzyloxycarbonyl ist, und A1 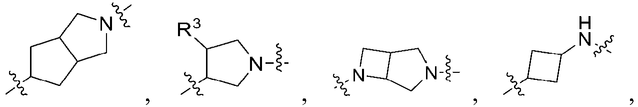 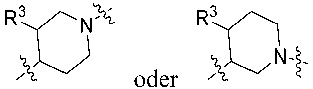 ist; wobei R²-X eine aktive Form von R² ist und X vorzugsweise ausgewählt ist aus der Gruppe bestehend aus Brom, Chlorid, Phenoxyl und p-Nitrophenoxy oder R²-X die Isocyanatform von R² ist; Alkali 1, Alkali 2 und Alkali 3 jeweils unabhängig ausgewählt sind aus der Gruppe bestehend aus Triethylamin, Diisopropylethylamin, Kaliumcarbonat, Cäsiumcarbonat, Natriumcarbonat, Natriumhydroxid und Kaliumhydroxid.

6. Pharmazeutische Zusammensetzung, umfassend eine therapeutisch wirksame Menge der Verbindung oder Tautomeren, Mesomeren, Racemen, Enantiomeren, Diastereomeren oder Gemischen davon, pharmazeutisch verträglichen Salzen, Solvaten oder Isotopenderivaten davon gemäß einem der Ansprüche 1-4 und optional einen oder mehrere pharmazeutisch verträgliche Träger und Hilfsstoffe, wobei die Träger zum Beispiel Laktosen, Weißzucker, Natriumchloride, Glucosen, Harnstoff, Stärken, Calciumcarbonate, Kaoline, kristalline Cellulosen und Kieselsäuren sind; wobei die Hilfsstoffe zum Beispiel Adhesive wie Wasser, Ethanol, Propanol, normaler Sirup, Dextroselösungen, Stärkelösungen, Gelatinelösungen, Carboxymethylcellulosen, Schellacke, Methylcellulosen und Kaliumphosphate, Polyvinylpyrrolidone; Sprengmittel wie trockene Stärken, Natriumalginate, Agar-Pulver, Kelp-Pulver, Natriumbicarbonate, Calciumcarbonate, Polyoxyethylensorbitan-Fettsäureester, Natriumdodecylsulfate, Monoglyceridstearate, Stärken und Laktosen; Zersetzungshemmungsmittel wie Weißzucker, Glycerintristearate, Kokosöl und gehärtetes Öl; Absorptionsbeschleuniger wie quartäre Ammoniumhydroxide und Natriumdodecylsulfate; Feuchthaltemittel wie Glycerin und Stärken; Adsorbentien wie Stärken, Laktosen, Kaoline, Bentonite und kolloidale Kieselsäuren; und Gleitmittel wie reine Talke, Stearate, Borsäurepulver und Polyethylenglykol sind, wobei die pharmazeutische Zusammensetzung außerdem ein oder mehrere zusätzliche Reagenzien, Antikarzinogene oder entzündungshemmende Mittel umfasst, die das Immunsystem von Säugetieren regulieren.

7. Verbindung oder Tautomere, Mesomere, Raceme, Enantiomere, Diastereomere oder Gemische davon, pharmazeutisch verträgliche Salze, Solvate oder Isotopenderivate davon nach einem der Ansprüche 1-4 oder pharmazeutische Zusammensetzung nach Anspruch 6 zur Verwendung als Arzneimittel.

8. Verbindung oder Tautomere, Mesomere, Raceme, Enantiomere, Diastereomere oder Gemische davon, pharmazeutisch verträgliche Salze, Solvate oder Isotopenderivate davon nach einem der Ansprüche 1-4 oder pharmazeutische Zusammensetzung nach Anspruch 6 zur Verwendung beim Vorbeugen und/oder Behandeln von JAK-vermittelten Erkrankungen, wobei die JAK-vermittelten Erkrankungen Folgendes umfassen: Erkrankungen des Immunsystems; Autoimmunerkrankungen; Dermatose; allergische Erkrankungen; Viruserkrankungen; Typ-1-Diabetes und diabetische Komplikationen; Alzheimer-Krankheit; Xerophthalmie; Myelofibrose; Thrombozytose; Polyzythämie oder Leukämie; Krebserkrankungen; entzündliche Darmerkrankung; myelodysplastisches Syndrom; Myelom.

9. Verbindung zur Verwendung nach Anspruch 8, wobei die JAK-vermittelten Erkrankungen Folgendes umfassen: Organtransplantatabstoßung wie Allotransplantatabstoßung und Graft-versus-Host-Erkrankung; Lupus, Multiple Sklerose, rheumatoide Arthritis, juvenile Arthritis, Psoriasis, Colitis ulcerosa, Morbus Crohn und Autoimmunerkrankungen der Schilddrüse; Psora, Hautausschlag, atopische Dermatitis; Allergien, Asthma und Rhinitis; Hepatitis B, Hepatitis C und Varicella-Zoster-Virus; solide Tumore wie Prostatakrebs, Nierenkarzinom, Leberkrebs, Pankreaskrebs, Magenkrebs, Brustkrebs, Lungenkrebs, Kopf-Hals-Karzinom, Schilddrüsenkrebs, Glioblastom, Melanom, hämatologisches Malignom wie Lymphom, Leukämie, Hautkrebs (kutanes T-Zell-Lymphom, kutanes B-Zell-Lymphom).

## Revendications

1. Composé de formule (II) ou tautomères, mésomères, racèmes, énantiomères, diastéréoisomères de celui-ci, ou mélanges de ceux-ci, sels, solvates ou dérivés isotopiques pharmaceutiquement acceptables de celui-ci, 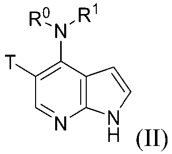
où, T représente -CN ou -CF₃ ;
R⁰ est -H ou -alkyle en C₁₋₃ ;
R¹ est 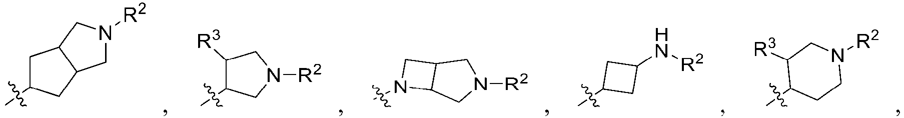 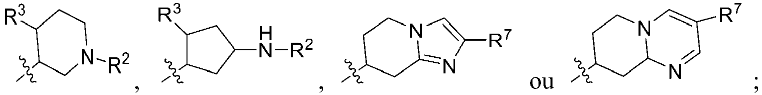
R² est -C(O)R⁴, -S(O)₂R⁴ ou -C(O)NR⁵R⁶ ;
R³ est H, -alkyle en C₁₋₄ ou -cycloalkyle en C₃₋₆ ;
R⁴ est -alkyle en C₁₋₅, hétérocyclyle à 4 à 6 chaînons, hétéroaryle à 5 à 10 chaînons, phényle, - hétérocyclyl-O-R^{e}, -hétérocyclyl-R^{4a}-O-R^{e} ou -hétérocyclyl-R^{4a}-NH-R^{e}, dans lequel l'alkyle en C₁₋₅ peut être substitué par les groupes suivants : -OH, -alkyle en C₁₋₃, -O-alkyle en C₁₋₄, -NH₂, halogène, CF₃, phényle ou cyano ;
dans lequel l'hétérocyclyle, l'hétéroaryle ou le phényle de R⁴ peut être remplacé par R^{e} ;
R^{4a} est -alkylène en C₁₋₃ ;
R⁵ est H ou -alkyle en C₁₋₃ ;
R⁶ est -alkyle en C₁₋₄, -cycloalkyle en C₃₋₆, 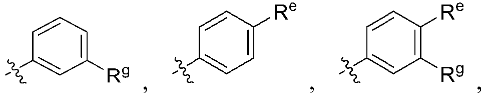 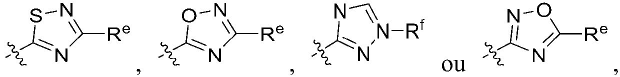 dans lequel l'alkyle en C₁₋₄ peut être substitué par les groupes suivants : -OH, -alkyle en C₁₋₃, -cycloalkyle en C₃₋₆, -O-alkyle en C₁₋₄, - NH₂, halogène, CF₃, groupe phényle ou cyano ;
R⁷ est hydrogène, -(CH₂)ₙ-OH, -(CH₂)ₙ-CF₃ ou -(CH₂)ₙ-CN ;
R^{e} et R^{g} sont chacun indépendamment halogène, -alkyle en C₁₋₄, alcoxy, cycloalkyle à 3 à 6 chaînons, -NR^{c}R^{d}, -(CH₂)ₙ NR^{c}R^{d}, -(CH₂)ₙ-OH, -(CH₂)ₙ-CF₃, -(CH₂)ₙ-CN ou C(O)OCH₃, dans lequel l'alkyle en C₁₋₄, l'alcoxy ou le cycloalkyle peuvent être monosubstitués ou disubstitués par les groupes suivants : halogène, -OH, -NH₂, -alkyle en C₁₋₃, -O-alkyle en C₁₋₄, CF₃, phényle ou cyano ;
R^{f} est -alkyle en C₁₋₄ ;
R^{c} et R^{d} sont chacun indépendamment hydrogène, -alkyle en C₁₋₃ ; et
n est 0, 1, 2 ou 3.

2. Composé ou tautomères, mésomères, racèmes, énantiomères, diastéréoisomères de celui-ci, ou mélanges de ceux-ci, sels, solvates ou dérivés isotopiques pharmaceutiquement acceptables de celui-ci selon la revendication 1, dans lequel
R³ est H, -CH₃, -CH₂CH₃ ou 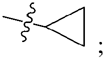
R⁴ est méthyle, éthyle, propyle, -(CH₂)ₙ-CH₂CN, -(CH₂)ₙ-CH₂CF₃, -CH(OH)-(CH₂)ₙ CH₃, 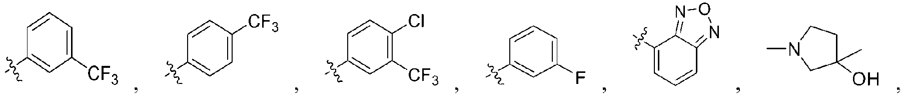 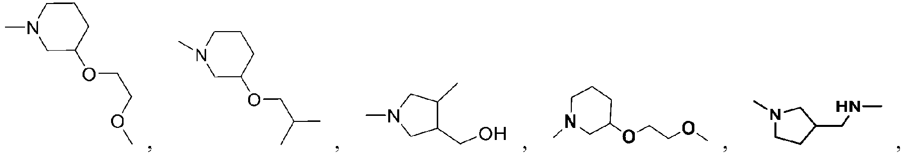 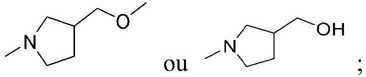
R⁵ est H ;
R⁶ est -(CH₂)ₙ-CH₂CF₃, -(CH₂)ₙ-CF₂CF₃, -(CH₂)ₙ-CH₂CN, 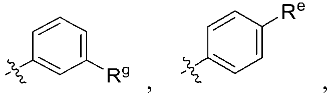 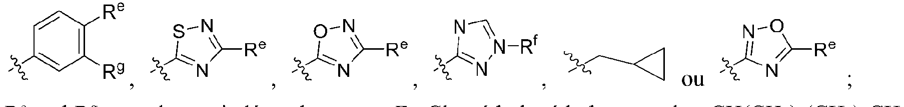
R^{e} and R^{g} sont chacun indépendamment -F, -Cl, méthyle, éthyle, propyle, -CH(CH₃)-(CH₂)ₙCH₃, - CH₂CH(CH₃)₂, -CH(CH₃)CH(CH₃)₂, -CH₂C(CH₃)₃, -C(CH₃)₂-(CH₂)ₙCH₃, -O-(CH₂)ₙCH₃, -O-(CH₂)ₙCH₂-O-CH₃, -(CH₂)ₙOCH₃, -O-(CH₂)ₙ-CH₂-C(CH₃)₂-OH, -O-(CH₂)ₙ-CH₂-C(CH₃)₂-NH₂, cycloalkyle en C₃₋₆, -(CH₂)ₙNR^{c}R^{d}, -(CH₂)ₙ-OH, -(CH₂)ₙ-CF₃, -(CH₂)ₙ-CHF₂, -(CH₂)ₙ-CN ou -C(O)-O-CH₃ ;
R^{f} est méthyle, éthyle ou propyle ;
R^{c} et R^{d} sont chacun indépendamment -H, méthyle, éthyle ou propyle ; et
n est 0 ou 1.

3. Composé ou tautomères, mésomères, racèmes, énantiomères, diastéréoisomères de celui-ci, ou mélanges de ceux-ci, sels, solvates ou dérivés isotopiques pharmaceutiquement acceptables de celui-ci selon l'une quelconque des revendications 1-2, dans lequel le composé est :

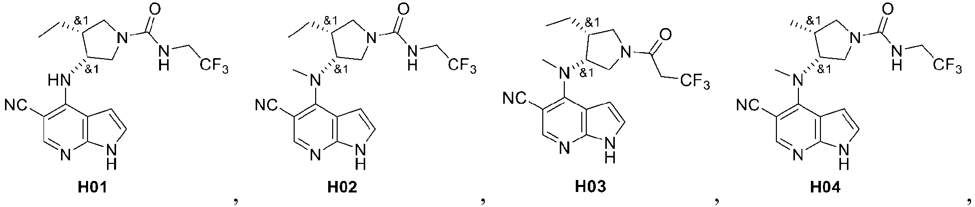

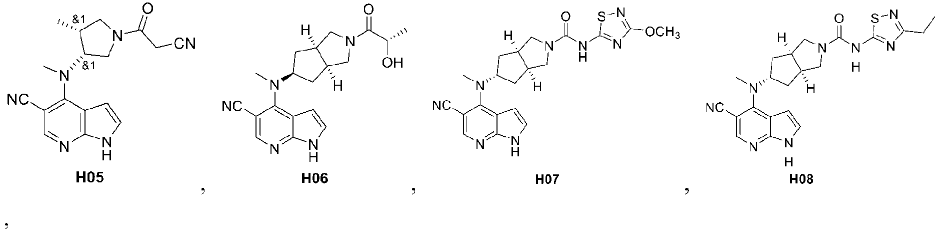

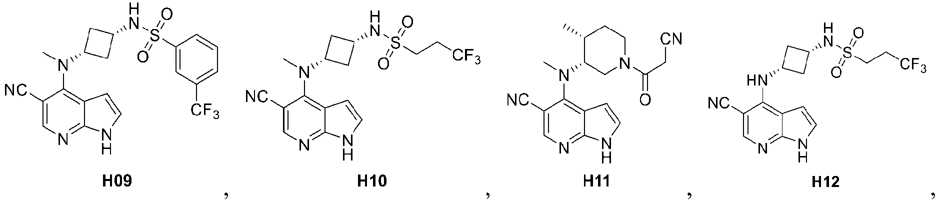

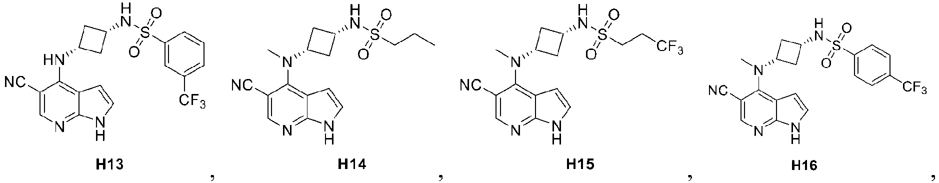

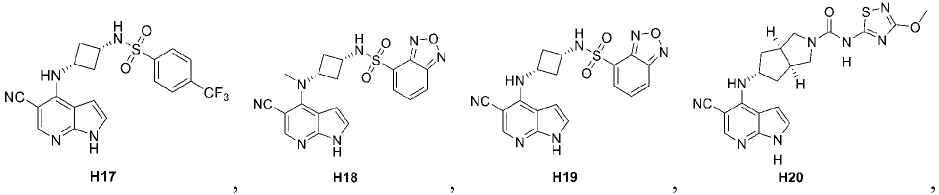

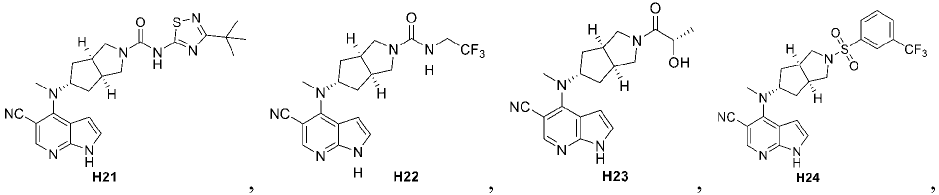

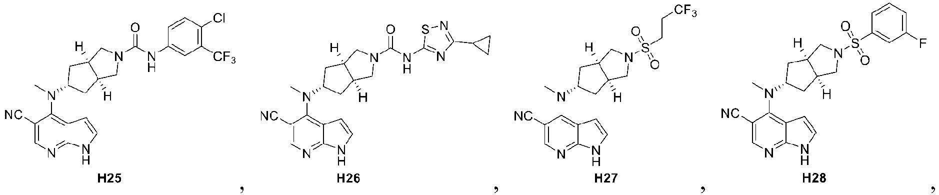

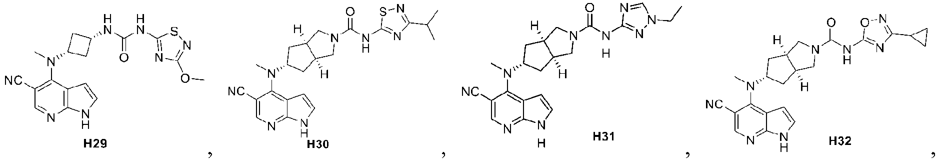

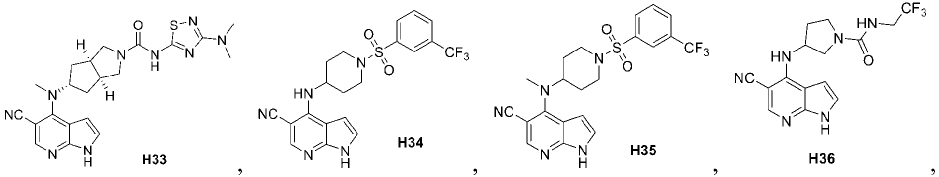

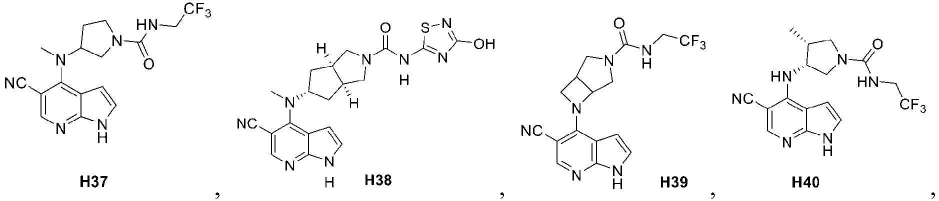

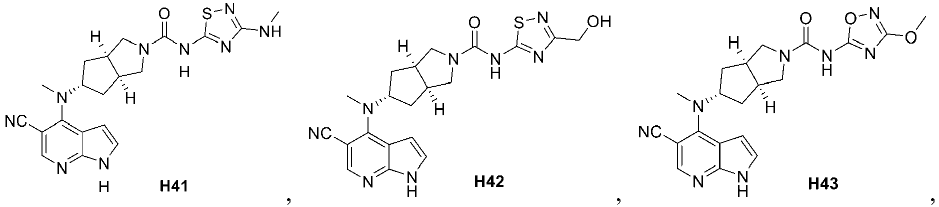

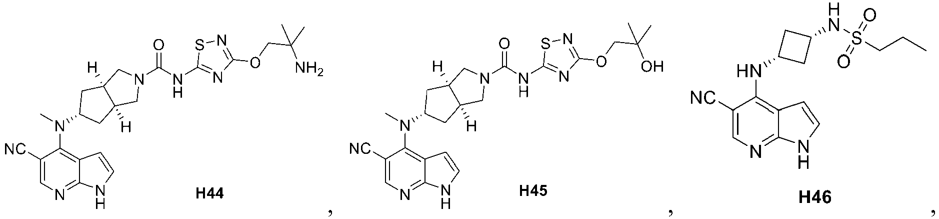

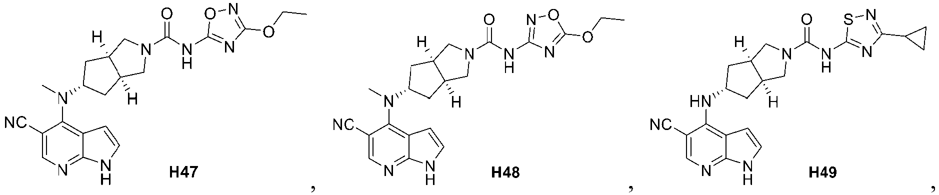

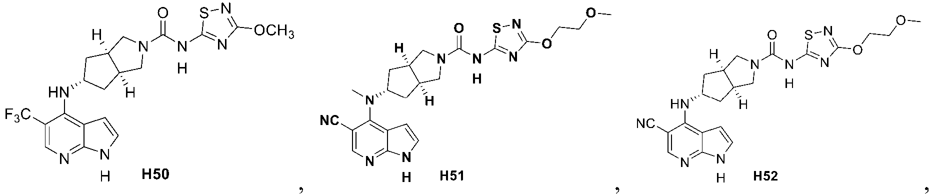

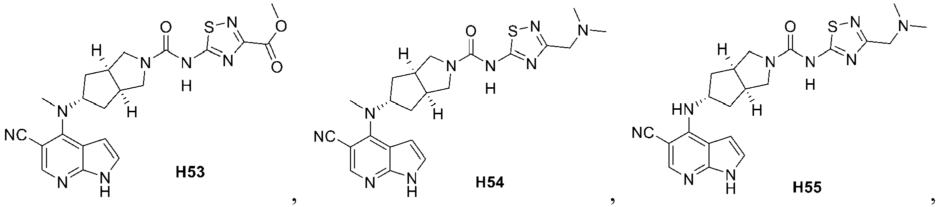

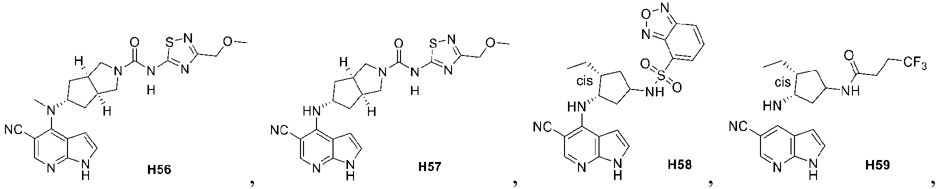

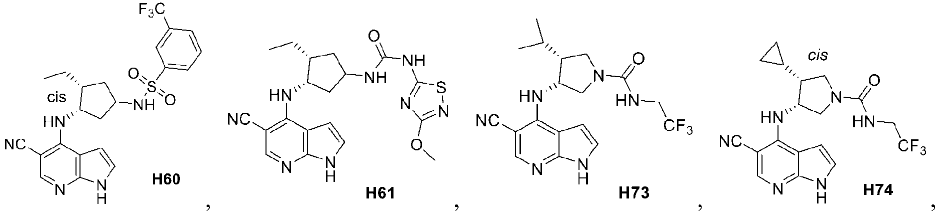

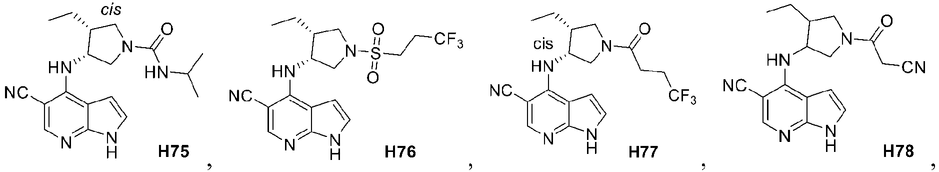

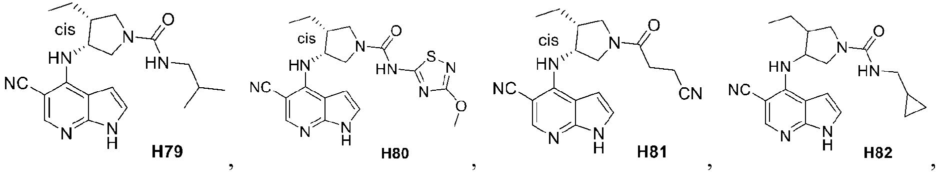

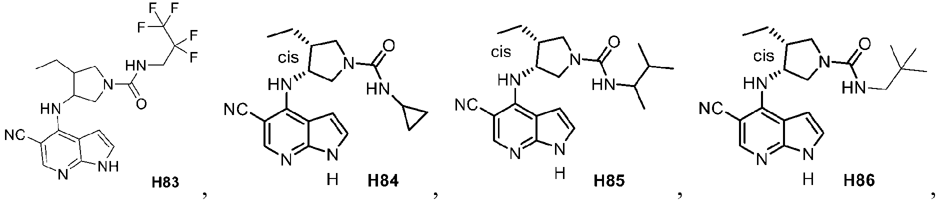

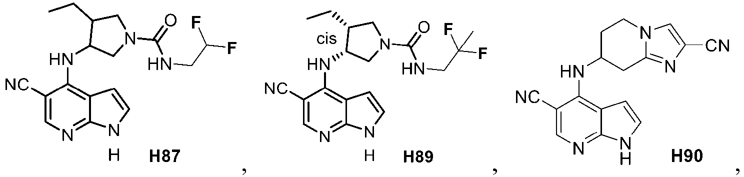

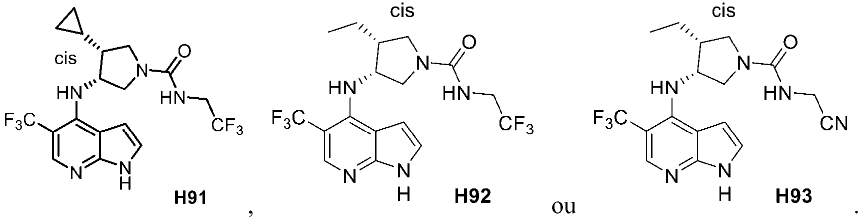

4. Composé ou tautomères, mésomères, racèmes, énantiomères, diastéréoisomères de celui-ci, ou mélanges de ceux-ci, sels, solvates ou dérivés isotopiques pharmaceutiquement acceptables de celui-ci selon l'une quelconque des revendications 1-3, dans lequel les sels pharmaceutiquement acceptables sont des sels d'addition d'acide, des sels d'addition d'alcali, des sels internes ou des bétaïnes ; les sels d'addition d'acide sont, par exemple, des chlorhydrates, des sulfates, des hydrosulfates, des nitrates, des phosphates, des hydrophosphates, des dihydrogénophosphates, des bromhydrates, des hydroiodates, des acétates, des lactates, des mésylates, des citrates, des malates, des maléates, des fumarates, des tartrates, des salicylates ou des stéarates ; les sels d'addition d'alcali sont, par exemple, des sels de métaux alcalins, des sels de métaux alcalino-terreux ou des sels d'ammonium, tels que des sels de sodium, des sels de potassium, des sels de calcium, des sels de magnésium ou des sels avec de l'ammoniac ou des amines organiques, telles que des éthylamines, des éthanolamines, des triéthanolamines ou des acides aminés.

5. Procédé de préparation du composé de formule (II) ou de tautomères, mésomères, racèmes, énantiomères, diastéréoisomères de celui-ci, ou de mélanges de ceux-ci, de sels, solvates ou dérivés isotopiques pharmaceutiquement acceptables de celui-ci selon l'une quelconque des revendications 1-4, dans lequel le composé de formule (II) est préparé par : 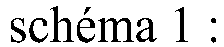 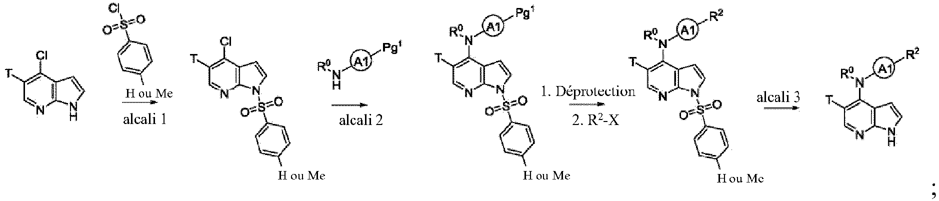 ou 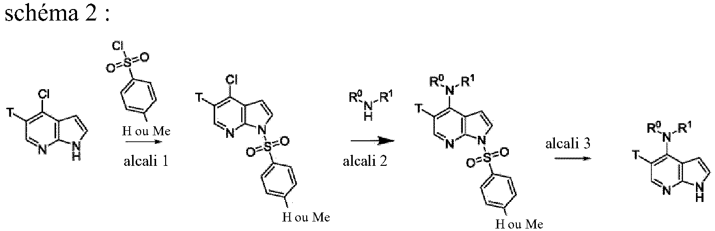 dans lequel, Pg¹ est un groupe protecteur d'amine aliphatique, en particulier tert-butoxycarbonyle ou benzyloxycarbonyle, et A1 est 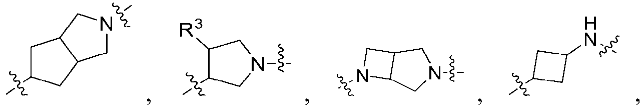 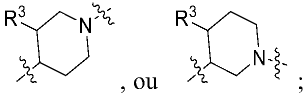 R²-X est une forme active de R² et X est de préférence choisi parmi le groupe constitué par brome, chlorure, phénoxyle et p-nitrophénoxy, ou R²-X est la forme isocyanate de R² ; alcali 1, alcali 2 et alcali 3 sont chacun choisis indépendamment dans le groupe constitué par triéthylamine, diisopropyléthylamine, carbonate de potassium, carbonate de césium, carbonate de sodium, hydroxyde de sodium et hydroxyde de potassium.

6. Composition pharmaceutique comprenant une quantité thérapeutiquement efficace du composé ou de tautomères, mésomères, racèmes, énantiomères, diastéréoisomères de celui-ci, ou de mélanges de ceux-ci, de sels, solvates ou dérivés isotopiques pharmaceutiquement acceptables de celui-ci selon l'une quelconque des revendications 1-4, et facultativement un ou plusieurs véhicules et excipients pharmaceutiquement acceptables, dans laquelle les véhicules sont, par exemple, lactoses, sucre blanc, chlorures de sodium, glucoses, urée, amidons, carbonates de calcium, kaolins, celluloses cristallines et acides siliciques ; les excipients sont, par exemple, des adhésifs tels que eau, éthanol, propanol, sirop régulier, solutions de dextrose, solutions d'amidon, solutions de gélatine, carboxyméthylcelluloses, gommes laques, méthylcelluloses et phosphates de potassium, polyvinylpyrrolidone ; des désintégrants, tels qu'amidons secs, alginates de sodium, poudre d'agar-agar, poudre de varech, bicarbonates de sodium, carbonates de calcium, esters d'acides gras de sorbitan polyoxyéthyléné, dodécylsulfates de sodium, stéarates de monoglycérides, amidons et lactoses ; des inhibiteurs de désintégration, tels que sucre blanc, tristéarates de glycérol, huile de coco et huile hydrogénée ; des accélérateurs d'absorption, tels que hydroxydes d'ammonium quaternaire et dodécylsulfates de sodium ; des humectants, tels que glycérol et amidons ; des adsorbants, tels qu'amidons, lactoses, kaolins, bentonites et acides siliciques colloïdaux ; et des lubrifiants, tels que talcs purs, stéarates, poudre d'acide borique et polyéthylène glycol, dans laquelle la composition pharmaceutique comprend également en supplément un ou plusieurs réactifs, agents anticancérigènes ou anti-inflammatoires qui régulent le système immunitaire des mammifères.

7. Composé ou tautomères, mésomères, racèmes, énantiomères, diastéréoisomères de celui-ci, ou mélanges de ceux-ci, sels, solvates ou dérivés isotopiques pharmaceutiquement acceptables de celui-ci selon l'une quelconque des revendications 1-4 ou composition pharmaceutique selon la revendication 6 destinés à être utilisés comme médicament.

8. Composé ou tautomères, mésomères, racèmes, énantiomères, diastéréoisomères de celui-ci, ou mélanges de ceux-ci, sels, solvates ou dérivés isotopiques pharmaceutiquement acceptables de celui-ci selon l'une quelconque des revendications 1-4 ou composition pharmaceutique selon la revendication 6, destinés à être utilisés dans la prévention et/ou le traitement des maladies médiées par JAK, dans lesquels les maladies médiées par JAK comprennent : maladies du système immunitaire ; maladies auto-immunes ; dermatoses ; maladies allergiques ; maladies virales ; diabète de type 1 et les complications du diabète ; maladie d'Alzheimer ; xérophtalmie ; myélofibrose ; thrombocytose ; polycythémie ou leucémie ; cancers ; maladies inflammatoires chroniques de l'intestin ; syndrome myélodysplasique ; myélome.

9. Composé destiné à être utilisé selon la revendication 8, dans lequel les maladies médiées par JAK comprennent : le rejet de greffe d'organe tel que le rejet d'allogreffe et la maladie du greffon contre l'hôte ; le lupus, la sclérose en plaques, la polyarthrite rhumatoïde, l'arthrite juvénile, le psoriasis, la recto-colite hémorragique, la maladie de Crohn et les maladies auto-immunes de la thyroïde ; le psora, les éruptions cutanées, la dermatite atopique ; les allergies, l'asthme et la rhinite ; l'hépatite B, l'hépatite C et le virus varicelle-zona ; les tumeurs solides telles que le cancer de la prostate, le carcinome rénal, le cancer du foie, le cancer du pancréas, le cancer de l'estomac, le cancer du sein, le cancer du poumon, le carcinome de la tête et du cou, le cancer de la thyroïde, le glioblastome, le mélanome, les hémopathies malignes, telles que le lymphome, la leucémie, le cancer de la peau (lymphome cutané à cellules T, lymphome cutané à cellules B).
